# EUROPEAN PATENT APPLICATION

(11) **EP 4 261 830 A1**
(43) Date of publication of application: **18.10.2023**
(21) Application number: 21903440.2
(22) Date of filing: 08.12.2021
(51) Int. Cl.: G16C 20/50, C07D 471/08, C07K 1/00

(54) **NOVEL PEPTIDOMIMETIC COMPOUND AND DESIGN**

(30) Priority: 09.12.2020 JP 2020204334
(71) Applicant: Oita University Institute of Advanced Medicine, Inc., Oita-shi, Oita 870-0037 (JP)
(72) Inventor: KOUJI, Hiroyuki, Fujisawa-shi, Kanagawa 251-8555 (JP); YOSHIMORI, Atsushi, Fujisawa-shi, Kanagawa 251-8555 (JP)
(74) Representative: Engelhard, Markus
(86) International application number: PCT/JP2021/045163
(87) International publication number: WO 2022/124341

(57) **Abstract**

The present disclosure provides a design and synthesis of a useful carbon skeleton, a design and synthesis of a peptidomimetic molecule and applications and uses based thereon. More particularly, provided is a method for designing a compound skeleton with a desired structure, said method comprising, with the use of a computer: (1) a step for generating a molecular formula of a carbon-containing compound; (2) a step for randomly synthesizing molecules using the adjacency matrix and extracting compounds that correspond to the molecular formula among the randomly synthesized molecules; and (3) a step for selecting a compound that satisfies preset conditions.

## Description

### [Technical Field]

The present disclosure relates to a compound with a novel peptidomimetic backbone and a design thereof.

### [Background Art]

Peptidomimetics have drawn attention as a target of pharmaceutical products. Various theoretical approaches have been taken, but there are very few cases of successful development.

### [Summary of Invention]

### [Solution to Problem]

The present disclosure provides a compound with a refined peptidomimetic backbone, a backbone that is a foundation of the compound, and a design thereof.

The present disclosure provides, for example, the following.

### [Item 1]

A method of designing a compound backbone with a desired structure, comprising the steps of causing a computer to:
(1) generate a molecular formula of a compound comprising carbon;
(2) randomly generate molecules using an adjacency matrix to extract a compound falling under the molecular formula among the randomly generated molecules; and
(3) select a compound satisfying a predefined condition.

### [Item 1A]

The method of item 1, wherein the molecular formula of the compound further comprises at least one of hydrogen, oxygen, nitrogen, and sulfur.

### [Item 2]

The method of any one of the preceding items, wherein the desired structure is a cage-like carbon backbone.

### [Item 3]

The method of any one of the preceding items, wherein the predefined condition is to satisfy at least one of:
condition 1: all carbon atoms and bonds are within a 5- or 6- membered ring;
condition 2: there is no spiro ring;
condition 3: there are two or more atoms that are present in common in at least three ring structures within a molecule (ring structure under condition 1 is a 5- or 6-membered ring); and
condition 4: energy is 150 kcal/mol or less in an MMFF (Merck Molecular Force Field) when all are calculated as a single bond upon generation of a 3D structure.

### [Item 3A]

The method of any one of the preceding items, wherein energy is 100 kcal/mol or less in the MMFF.

### [Item 3B]

The method of any one of the preceding items, wherein energy is 50 to 150 kcal/mol in the MMFF.

### [Item 4]

The method of any one of the preceding items, wherein all of condition 1 to condition 4 are satisfied.

### [Item 4A]

The method of any one of the preceding items, wherein the atom comprises a carbon, nitrogen, oxygen, or sulfur atom.

### [Item 4B]

The method of any one of the preceding items, wherein the atom comprises carbon.

### [Item 4C]

The method of any one of the preceding items, comprising at least condition 1.

### [Item 5]

The method of any one of the preceding items, wherein the molecular formula is one selected from the group consisting of C₁₀H₁₆, C₁₁H₁₆, C₁₁H₁₈, C₁₂H₁₈, C₁₂H₂₀, C₁₃H₂₀, and C₁₃H₂₂ .

### [Item 6]

A method of generating a compound having a compound backbone with a desired structure, comprising the steps of:
(1) causing a computer to generate a molecular formula of a compound comprising carbon;
(2) causing a computer to randomly generate molecules using an adjacency matrix to extract a compound falling under the molecular formula among the randomly generated molecules;
(3) causing a computer to select a compound satisfying a predefined condition; and
(4) producing a compound having a backbone structure selected in (3).

### [Item 7]

The method of any one of the preceding items, wherein the compound comprises at least one type of bond selected from a single bond, a double bond, and a triple bond.

### [Item 7A]

The method of any one of the preceding items, wherein the compound comprises a single bond.

### [Item 8]

A method of designing a peptidomimetic molecule framework, comprising the steps of causing a computer to:
(A) provide structural data for a carbon moiety of a predefined carbon backbone;
(B) generate an attachment point (AP), which is a position where a side chain structure is given from the structural data for the carbon backbone;
(C) randomly select APs at M locations;
(D) optionally, generate a carbon backbone with N APs by repeating steps (A) to (C) N times, wherein N is a positive integer;
(E) optionally, comprehensively add the same or different amino acid-like structure to each AP at M locations to generate an amino acid-like structure-containing carbon backbone; and
(F) fit the amino acid-like structure-containing carbon structure to a pharmacophore obtained from a three-dimensional structure of a predefined peptide, and select a carbon structure that has fit as a peptidomimetic molecule framework.

### [Item 9]

The method of any one of the preceding items, wherein the carbon backbone is a cage-like carbon backbone.

### [Item 10]

The method of item 8 or 9, wherein N is 1000 or greater in step (D).

### [Item 10A]

The method of any one of the preceding items, wherein N is preferably 3000 or greater in step (D).

### [Item 11]

The method of any one of the preceding items, wherein M is an integer from 2 to 6.

### [Item 11A]

The method of any one of the preceding items, wherein M is an integer from 2 to 5 or from 2 to 4.

### [Item 12]

The method of any one of the preceding items, wherein the amino acid comprises at least one selected from the group consisting of leucine, phenylalanine, isoleucine, and tryptophan in step (E).

### [Item 13]

The method of any one of the preceding items, wherein step (F) comprises causing the computer to select top K carbon backbones with a best fitting score of the pharmacophore.

### [Item 14]

The method of any one of the preceding items, wherein the carbon backbone is a backbone generated by the method of any one of the preceding items.

### [Item 15]

A method of generating a compound having a peptidomimetic molecule framework, comprising the steps of:
(A) providing to a computer structural data for a carbon moiety of a predefined carbon backbone;
(B) causing a computer to generate an attachment point (AP), which is a position where a side chain structure is given from the structural data for the carbon backbone;
(C) causing a computer to randomly select APs at M locations;
(D) optionally, causing a computer to generate a carbon backbone with N APs by repeating step (C) N times, wherein N is a positive integer;
(E) optionally, causing a computer to comprehensively add the same or different amino acid-like structure to each AP at M locations to generate a carbon backbone with an amino acid side chain structure;
(F) causing a computer to fit the carbon structure with the amino acid side chain structure to a pharmacophore obtained from a three-dimensional structure of a predefined peptide, and select a carbon structure that has fit as a peptidomimetic molecule framework; and
(G) producing a compound with the peptidomimetic molecule framework selected in (F).

### [Item 16]

The method of any one of the preceding items, wherein M is 3 or greater.

### [Item 17]

The method of any one of the preceding items, wherein the method comprises step (D), and N is 1000 or greater.

### [Item 18]

The method of any one of the preceding items, wherein the compound comprises at least one type selected from a single bond, a double bond, and a triple bond.

### [Item 19]

A cyclic compound having a structure satisfying, when calculated in terms of hydrocarbon:
condition 1: all carbon atoms and bonds are within a 5- or 6- membered ring;
condition 2: there is no spiro ring;
condition 3: there are two or more atoms that are present in common in at least three ring structures within a molecule (ring structure under condition 1 is a 5- or 6-membered ring); and
condition 4: energy is 150 kcal/mol or less in an MMFF upon generation of a 3D structure;
wherein the cyclic compound optionally has a heteroatom in place of a carbon atom within a backbone and optionally has one or more substituents.

### [Item 20]

A peptidomimetic compound having a backbone satisfying when calculated in terms of hydrocarbon:
condition 1: all carbon atoms and bonds are within a 5- or 6- membered ring;
condition 2: there is no spiro ring;
condition 3: there are two or more atoms that are present in common in at least three ring structures within a molecule (ring structure under condition 1 is a 5- or 6-membered ring); and
condition 4: energy is 150 kcal/mol or less in an MMFF upon generation of a 3D structure;
and M substituents (wherein M is any integer from 1 to (number of atoms in a backbone moiety that can have a substituent)) corresponding to M amino acid-like structures (amino acid side chain or an equivalent thereof) from the backbone, wherein the peptidomimetic compound optionally has a heteroatom in place of a carbon atom within a backbone.

### [Item 21]

A cyclic compound having any one of the following backbones: 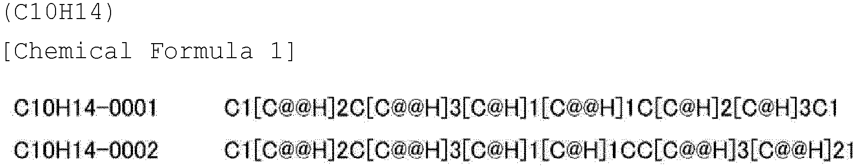 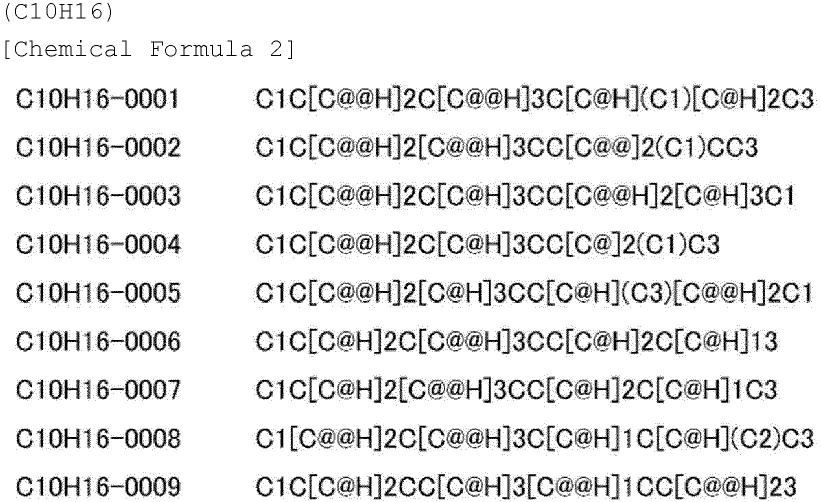 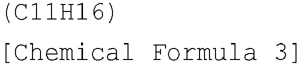 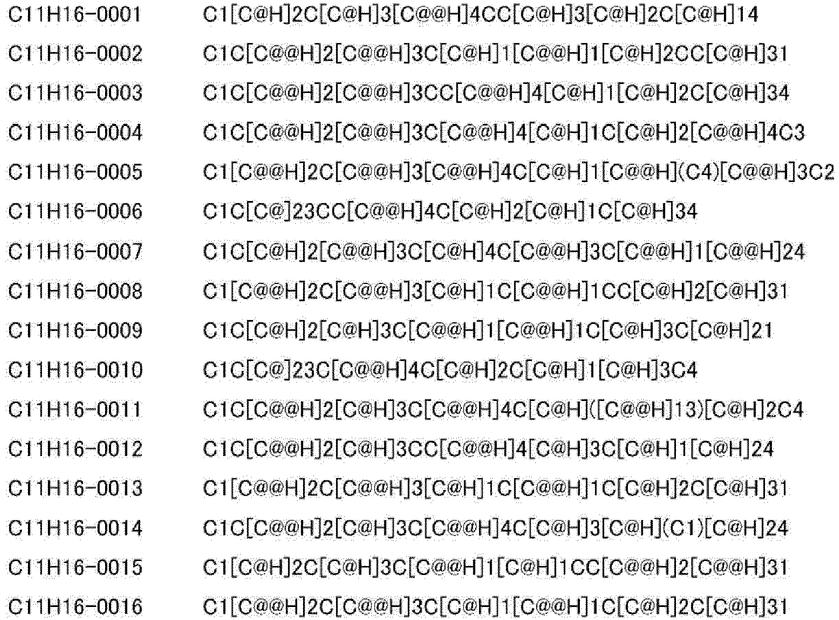 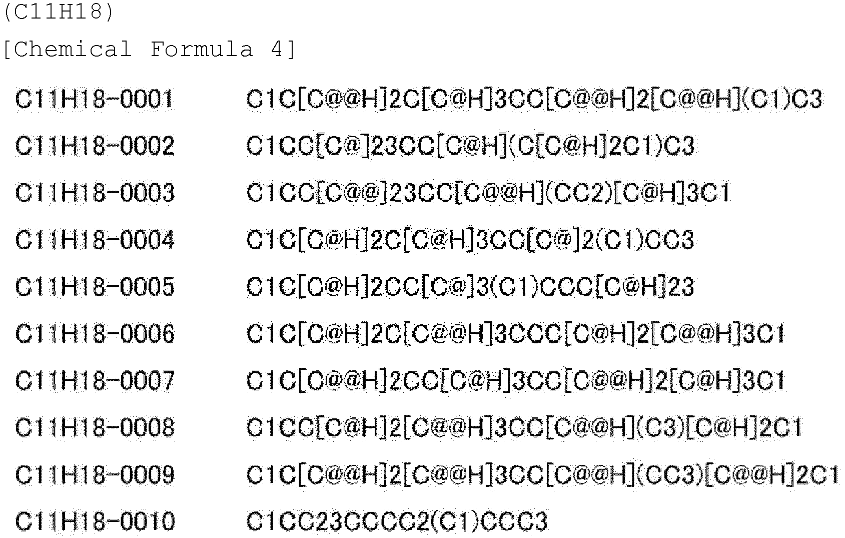 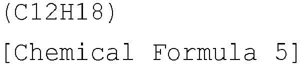 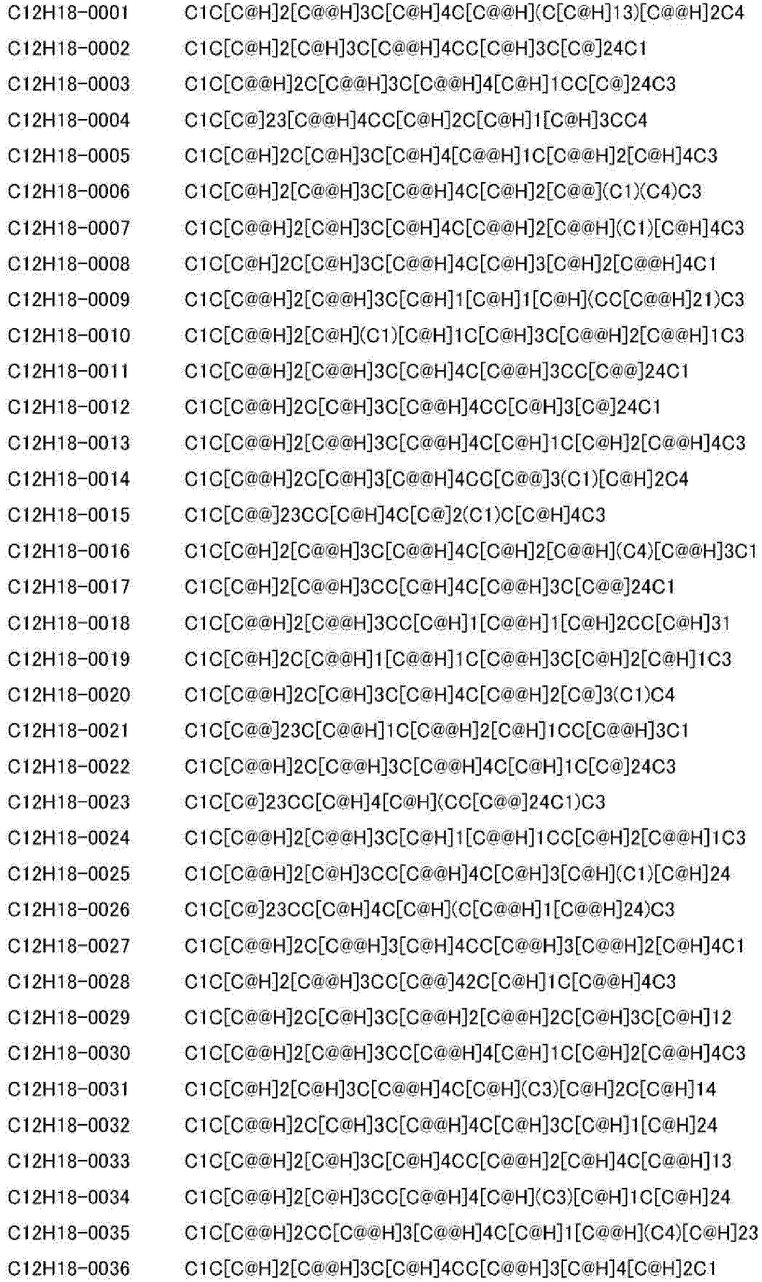 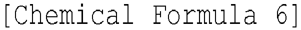 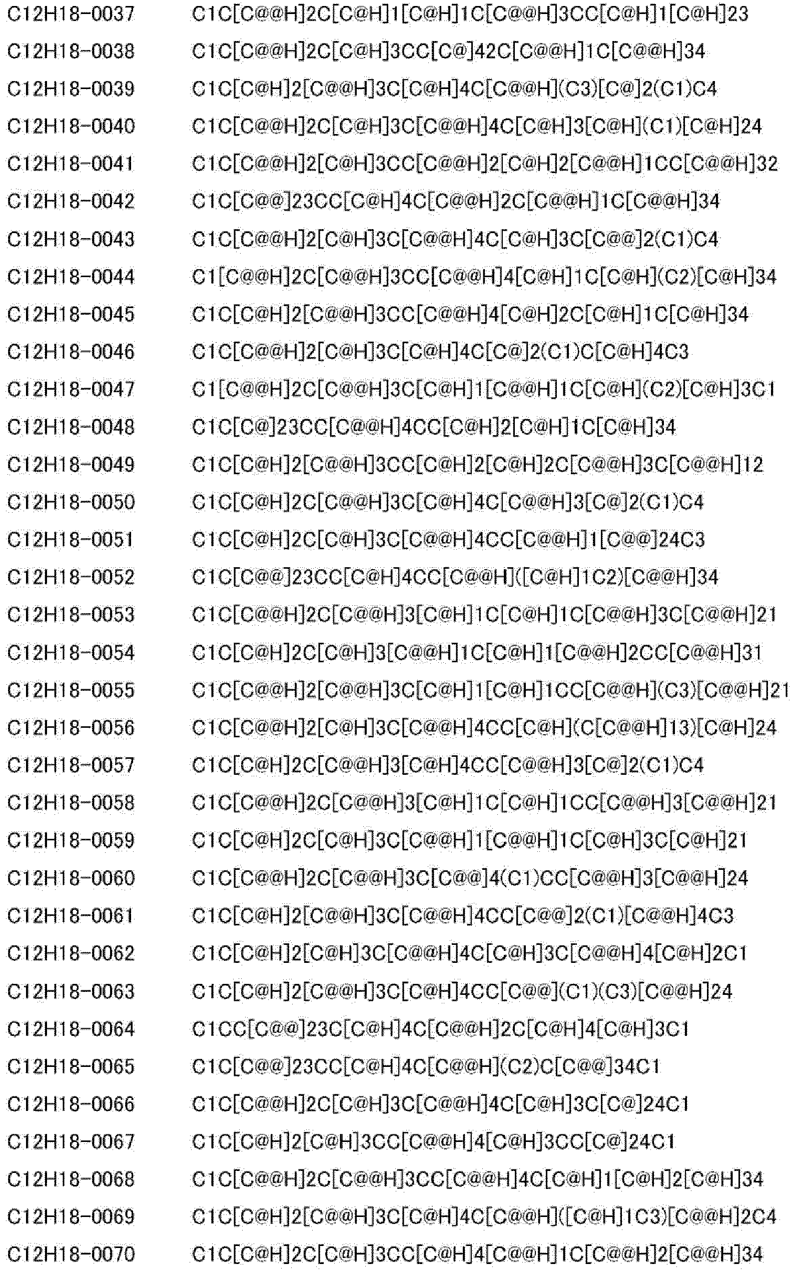 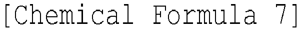 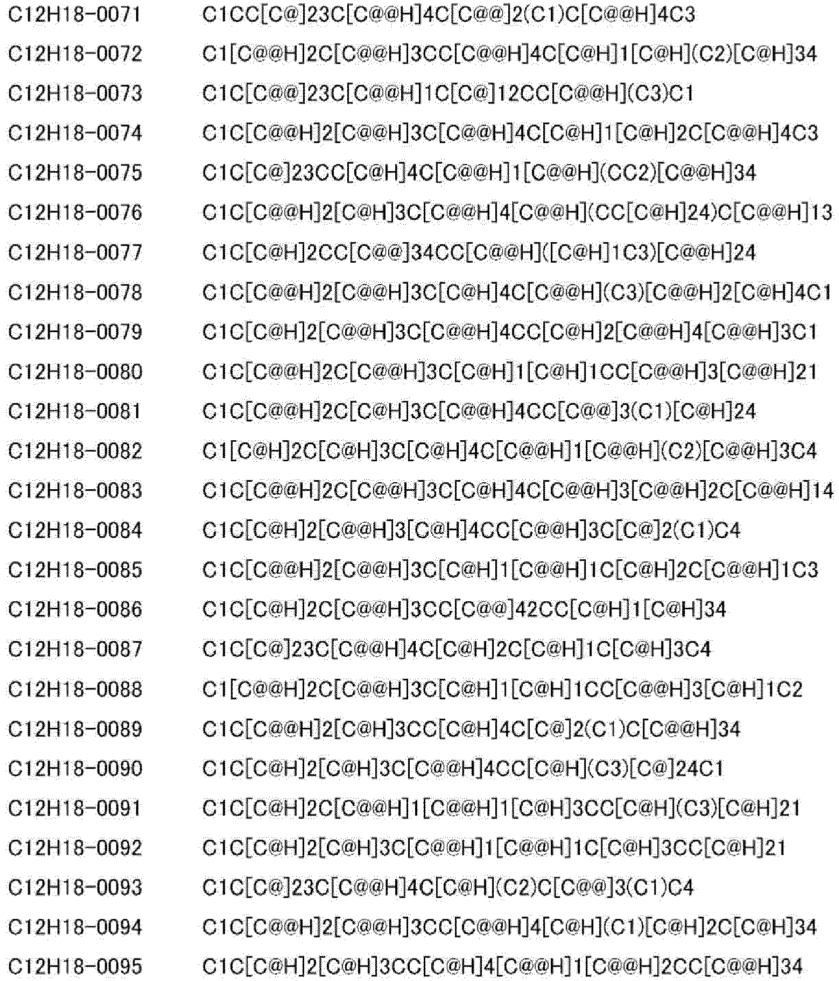 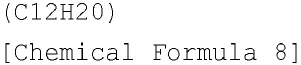 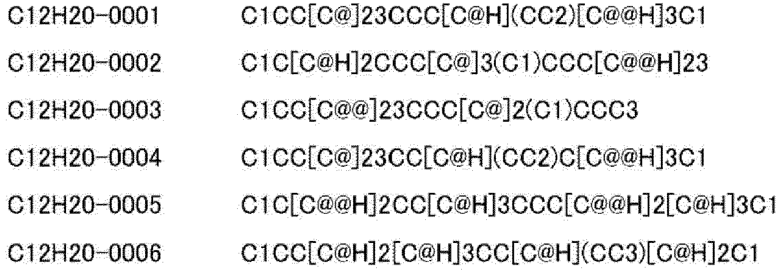 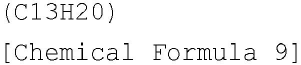 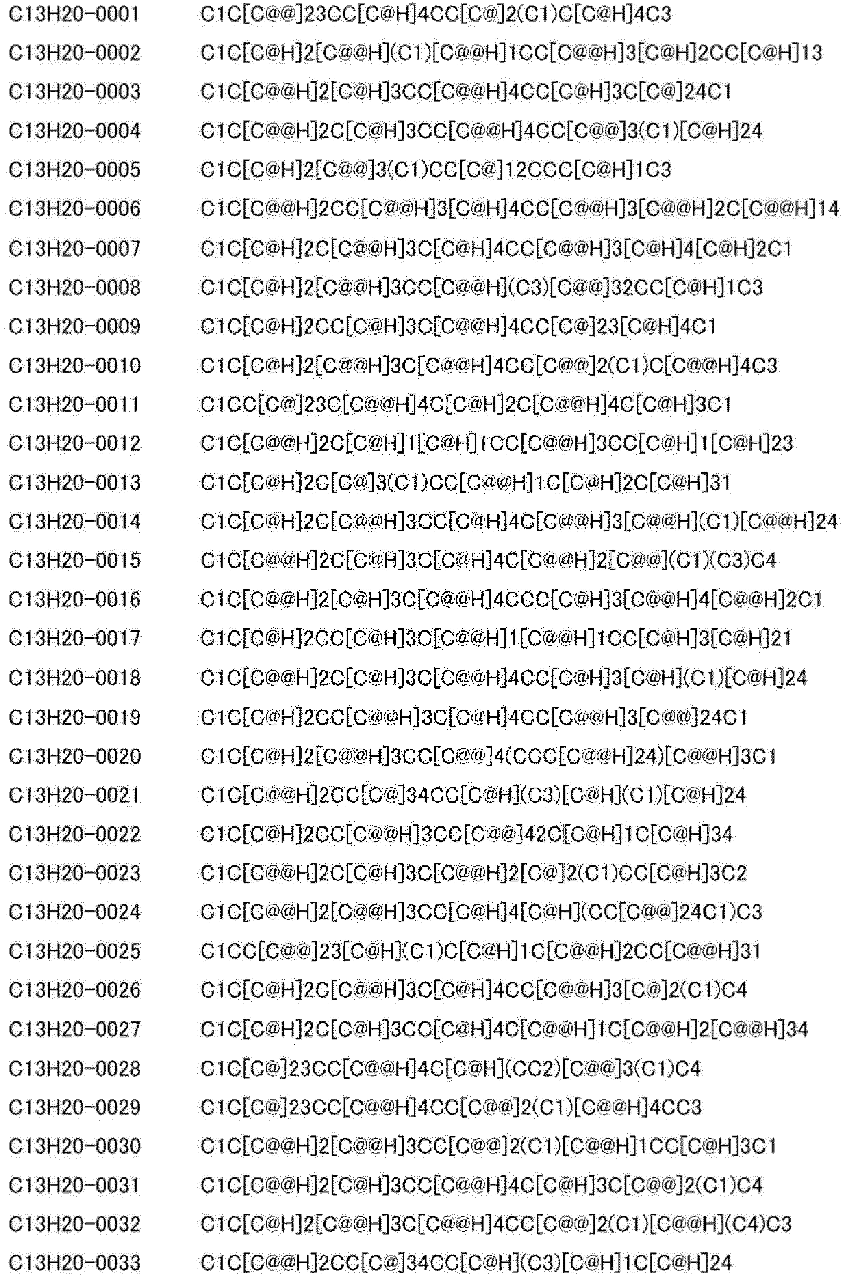 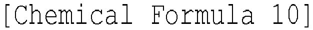 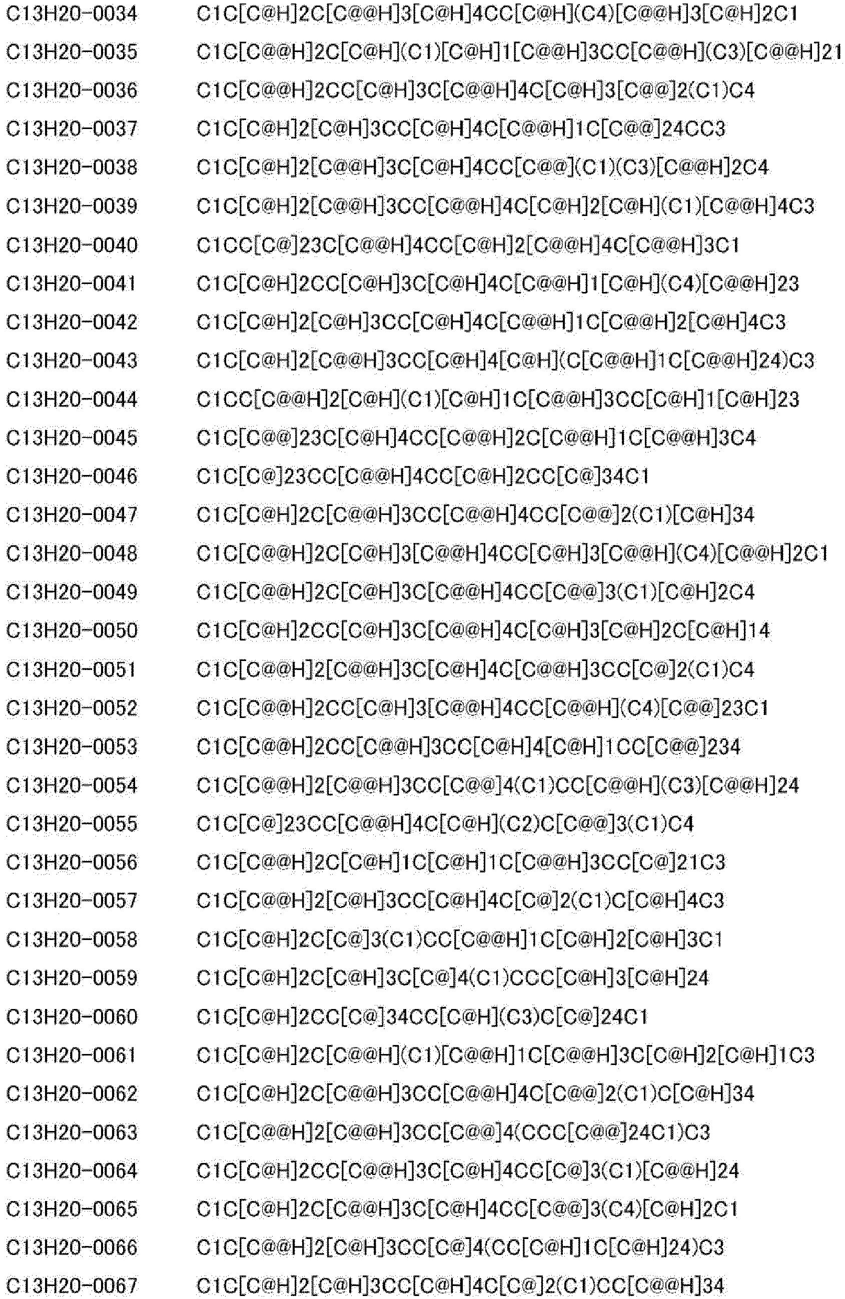 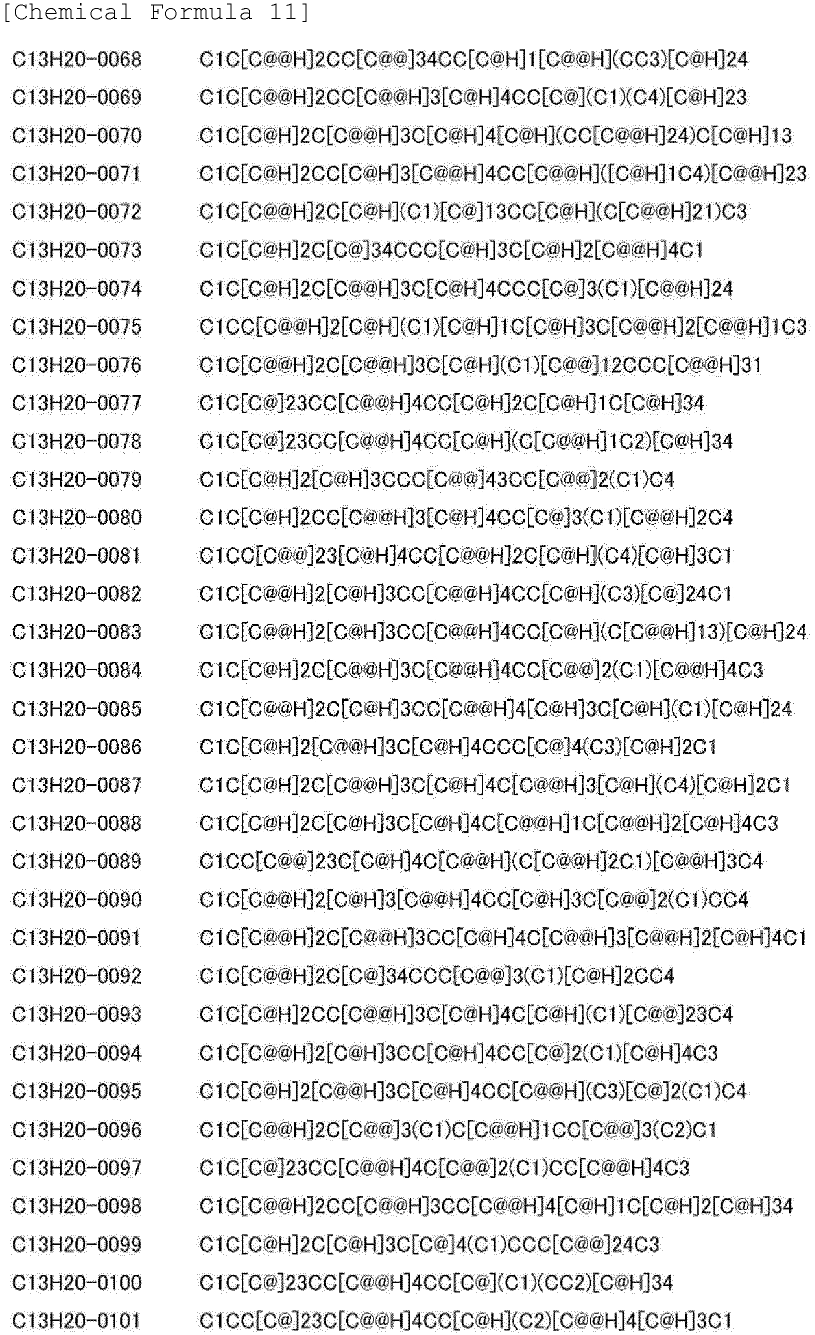 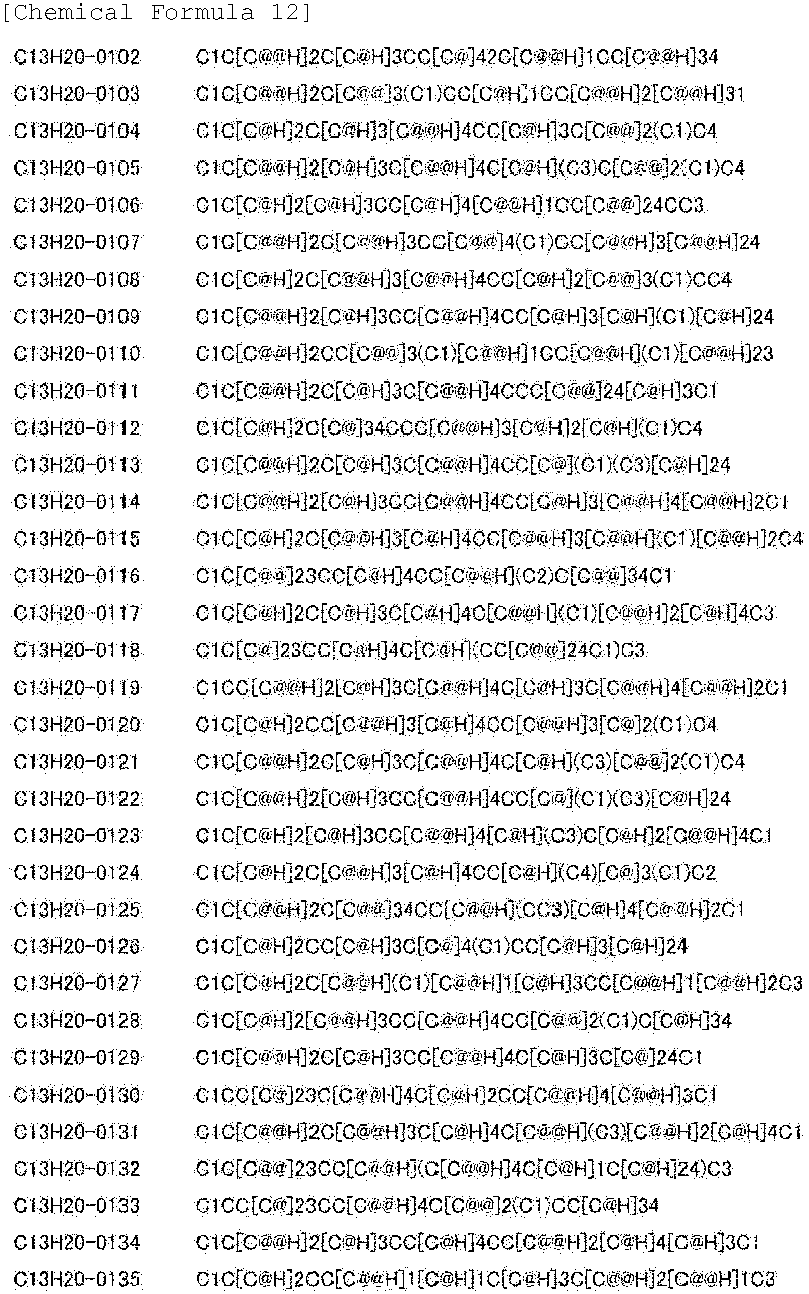 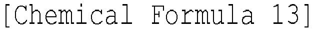 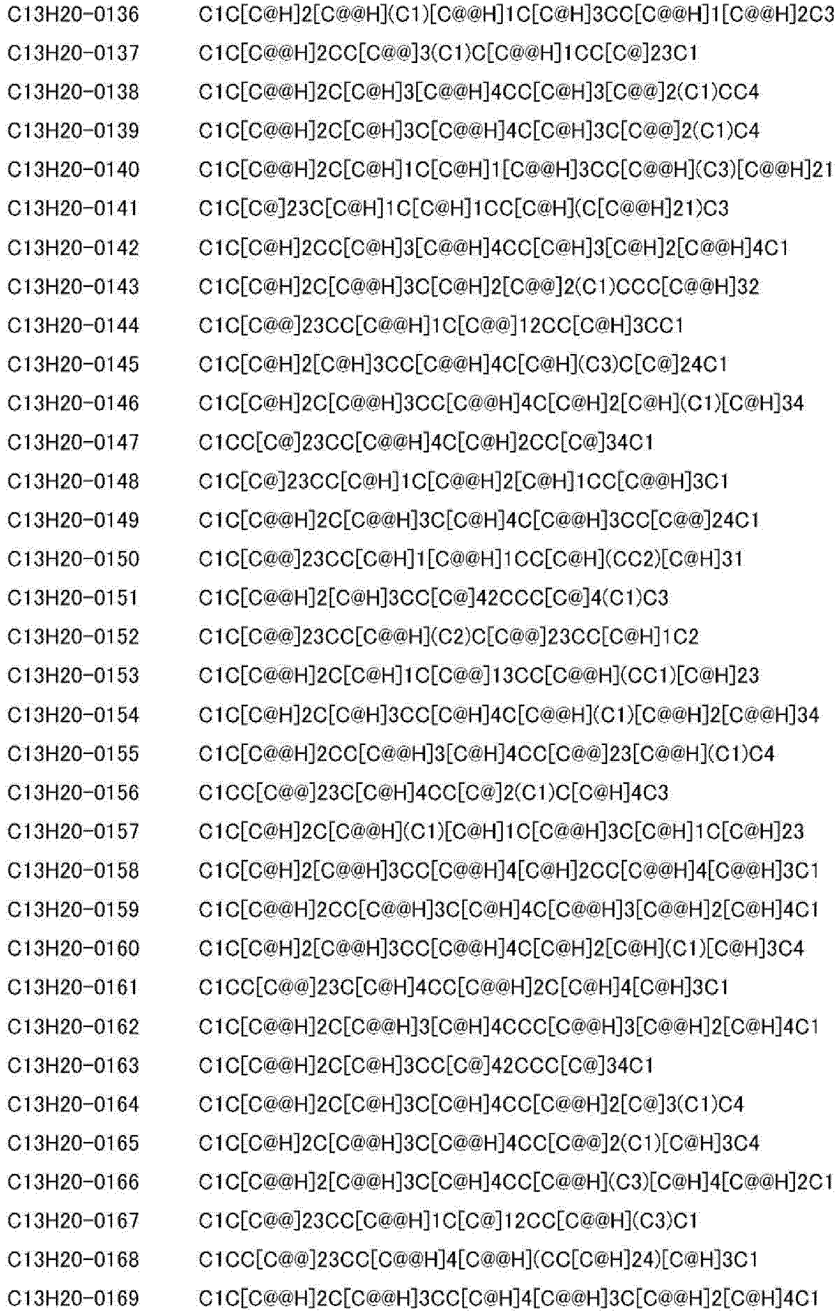 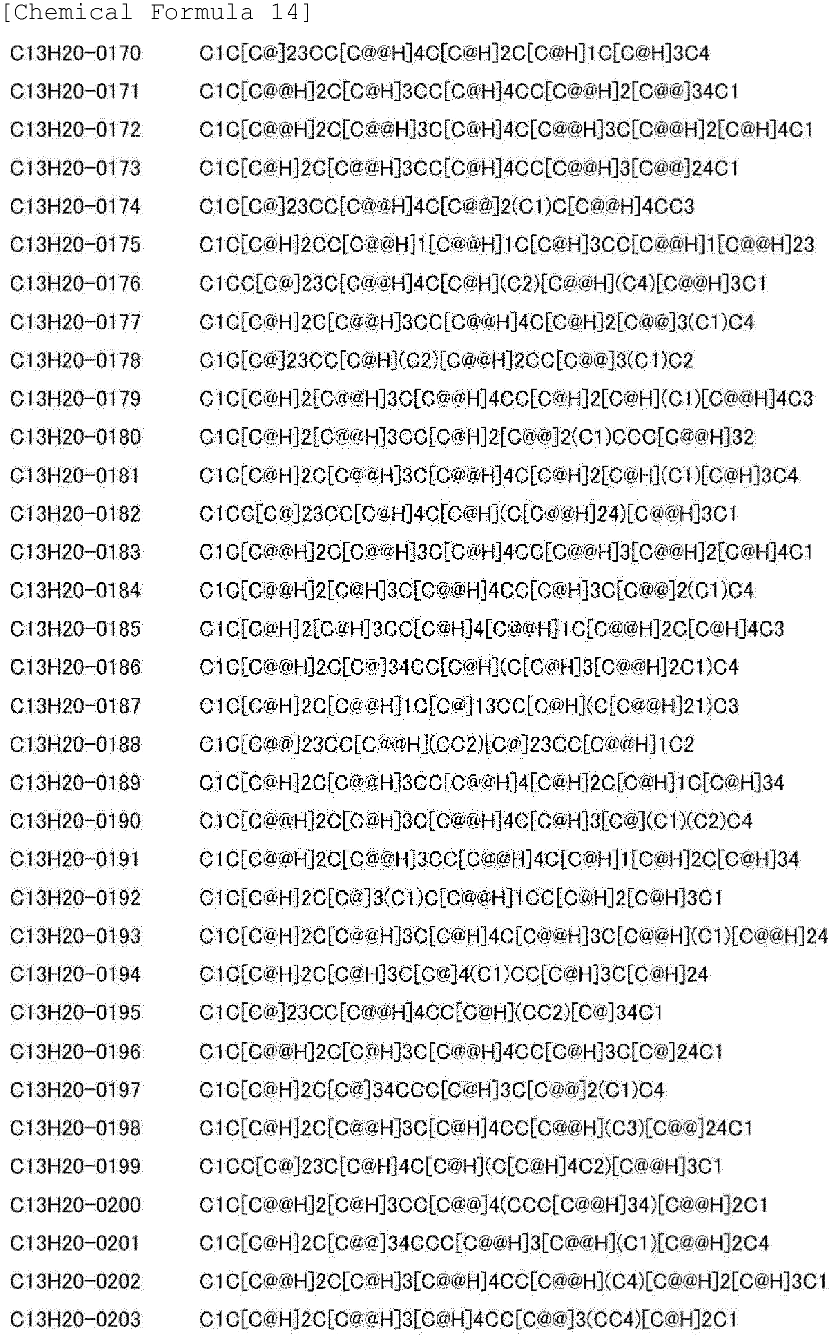 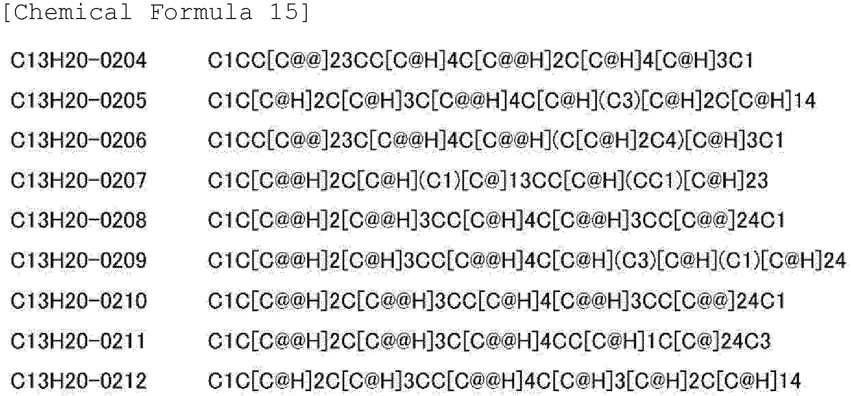 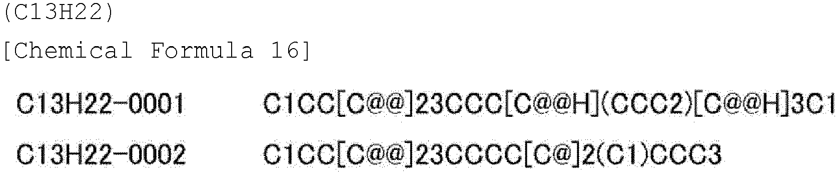 or an enantiomer thereof, optionally having a heteroatom in place of a carbon atom within a backbone and optionally having one or more substituents.

### [Item 22]

A peptidomimetic compound having any one of the following backbones 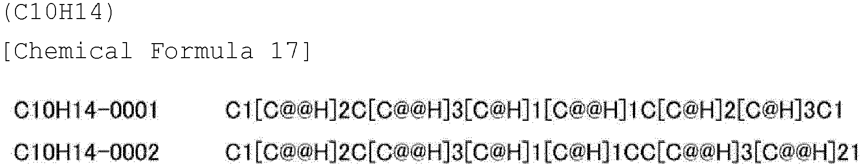 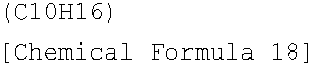 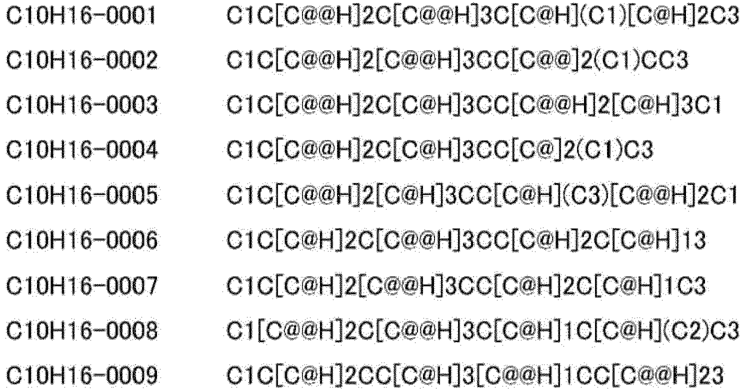 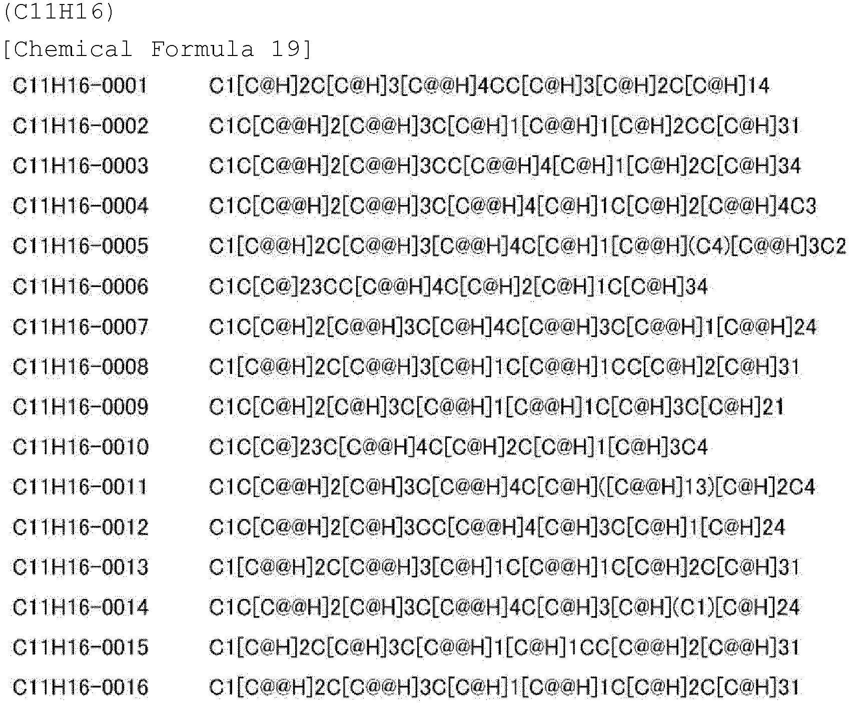 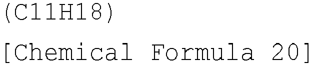 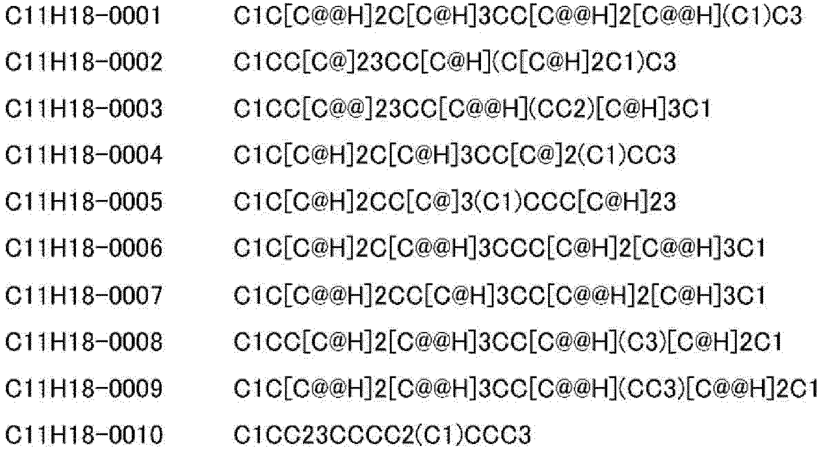 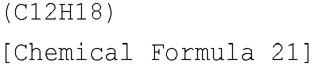 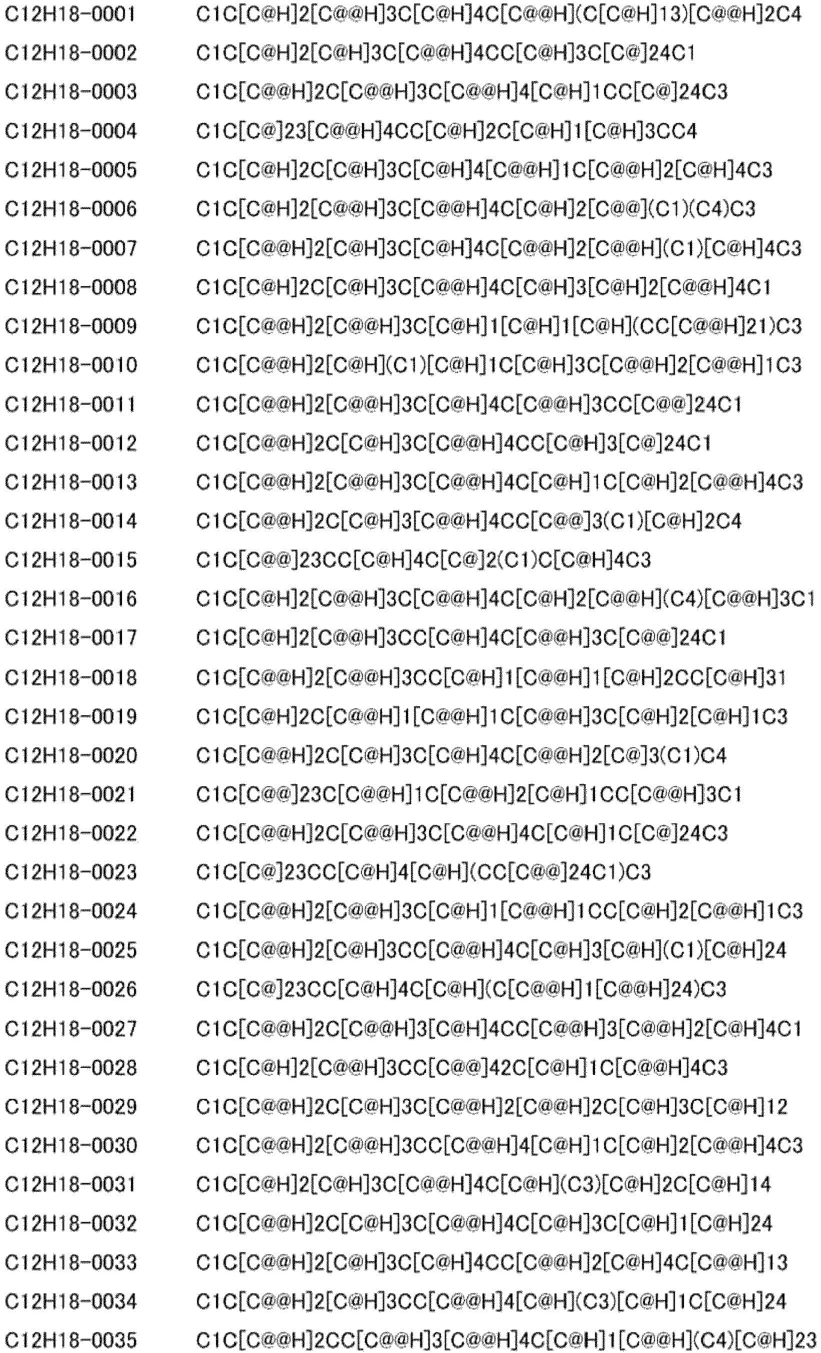 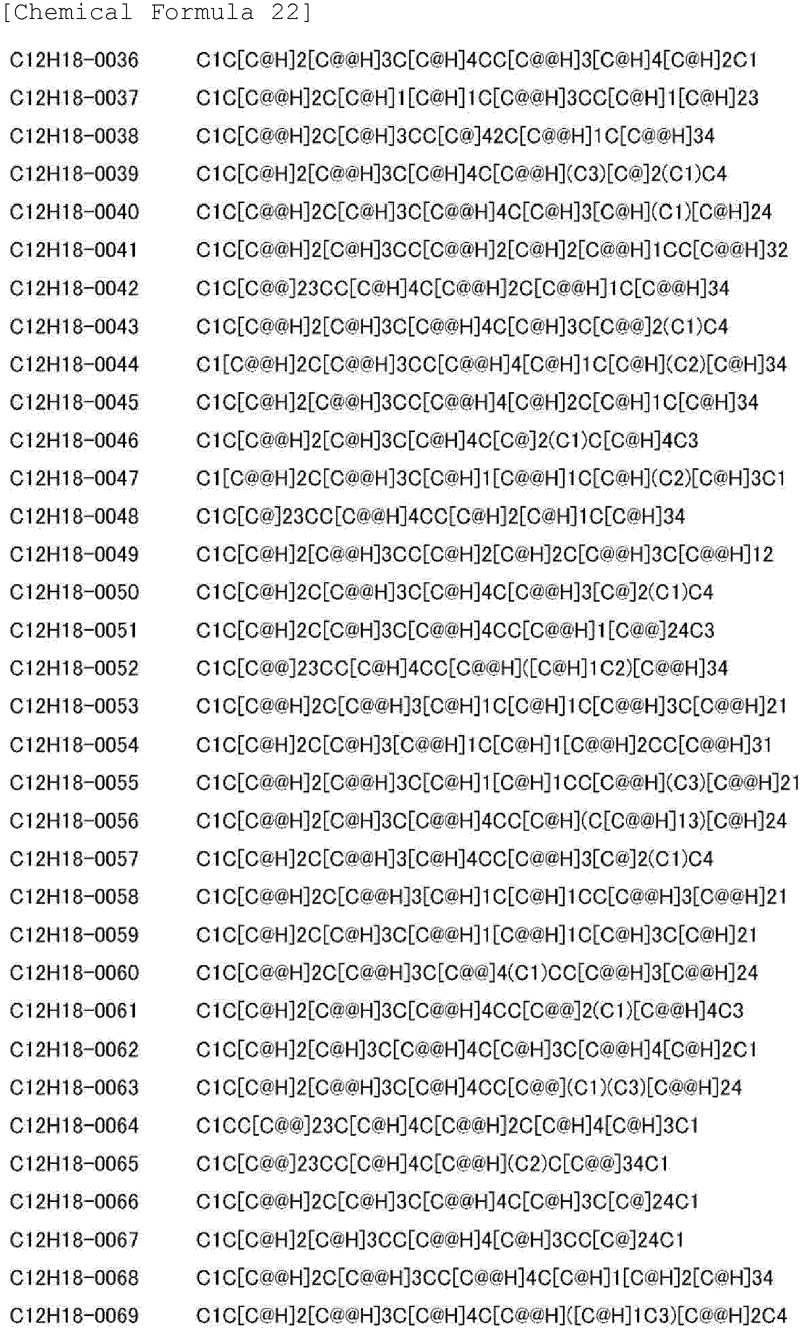 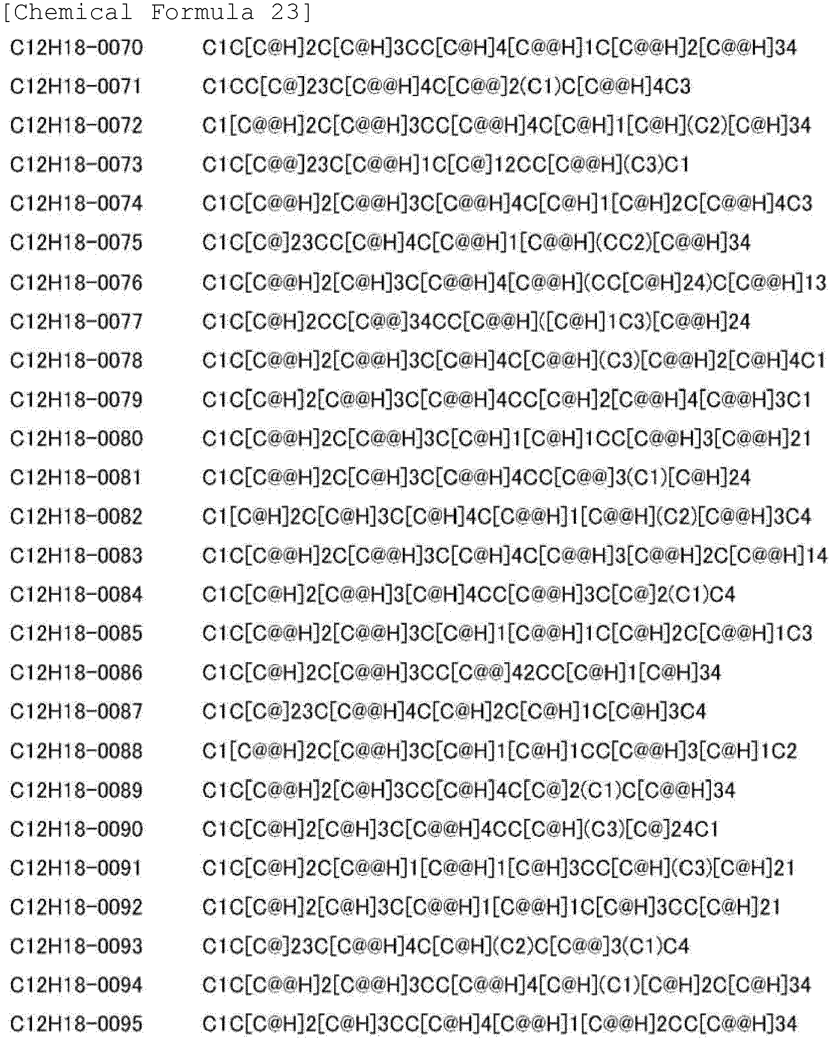 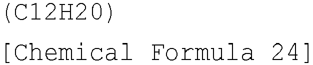 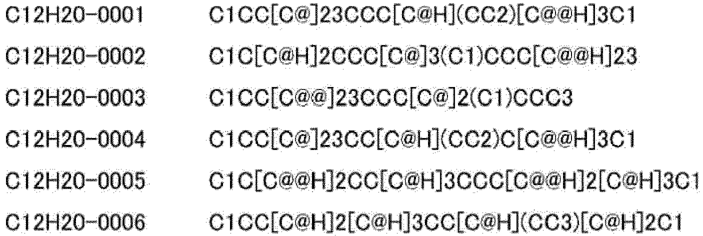 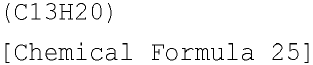 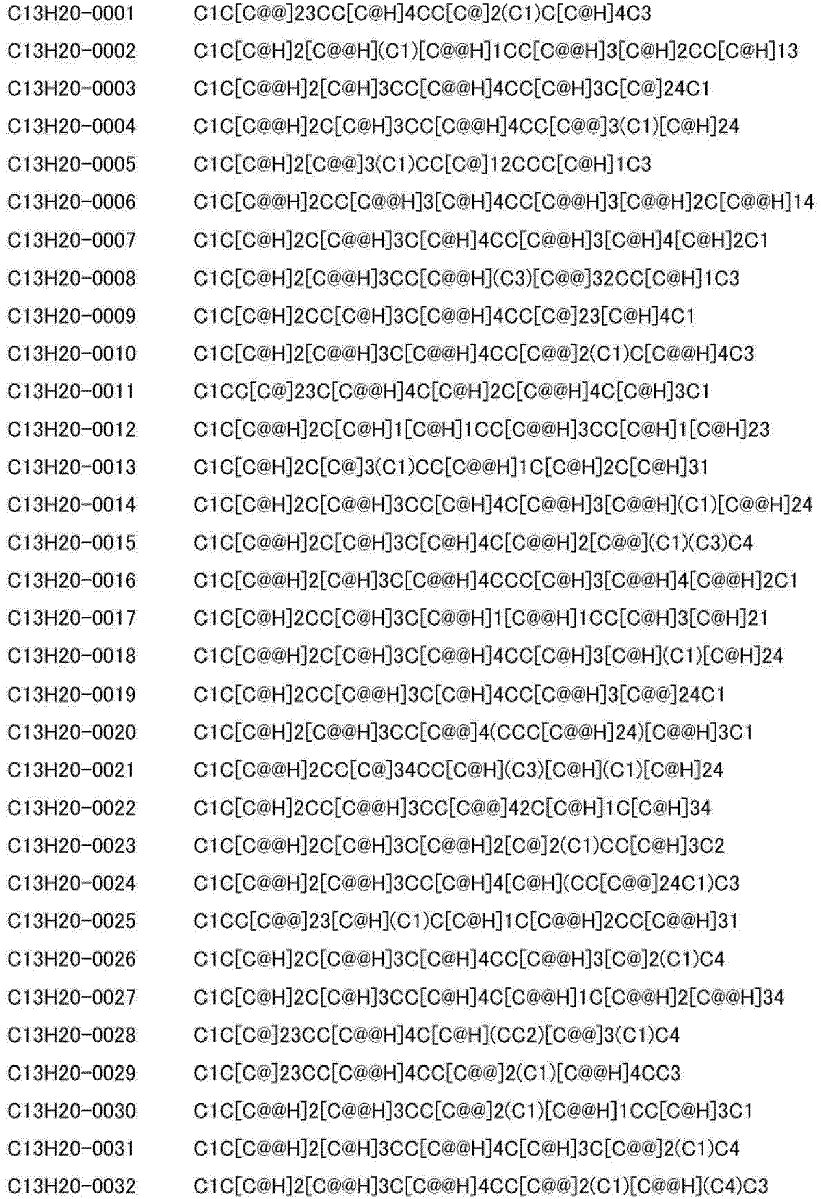 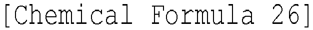 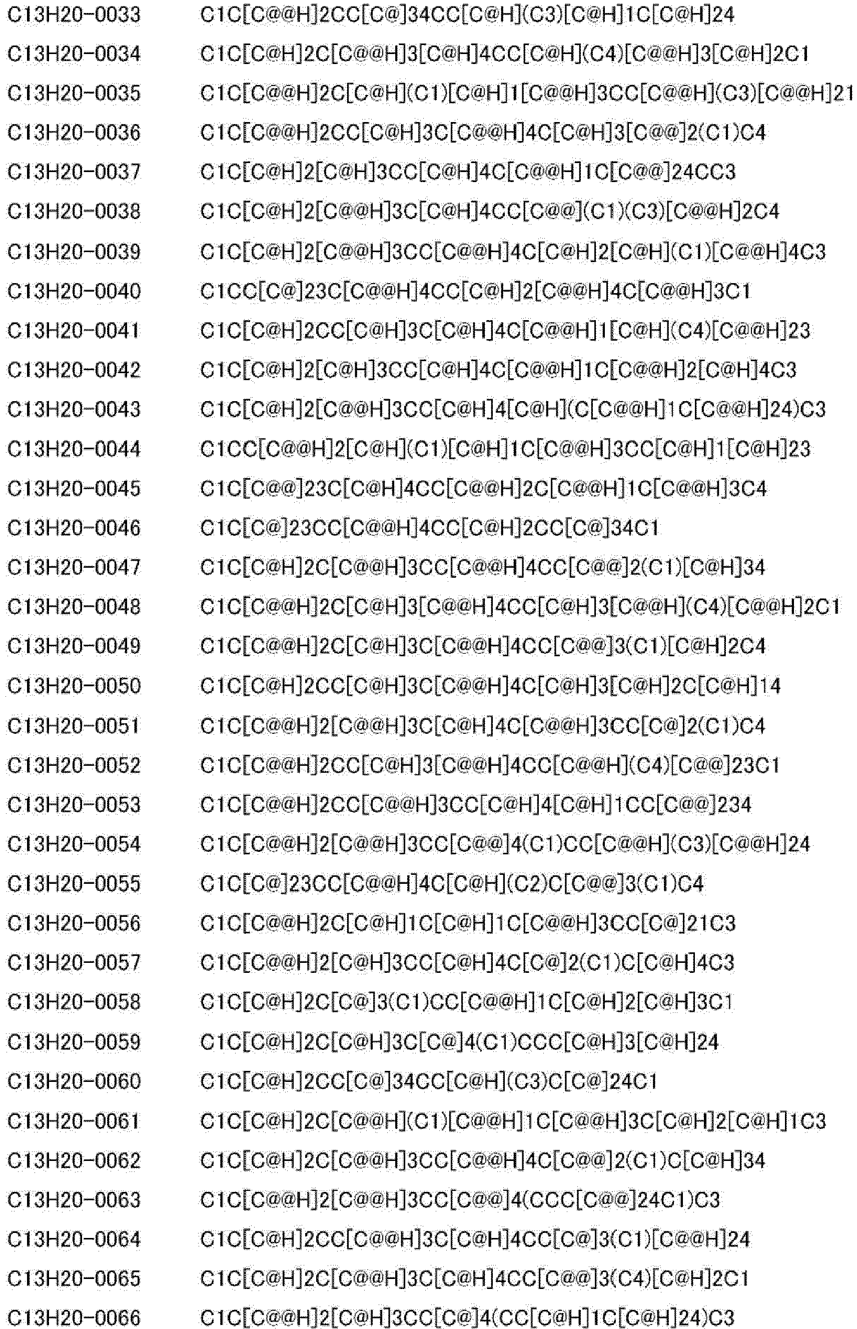 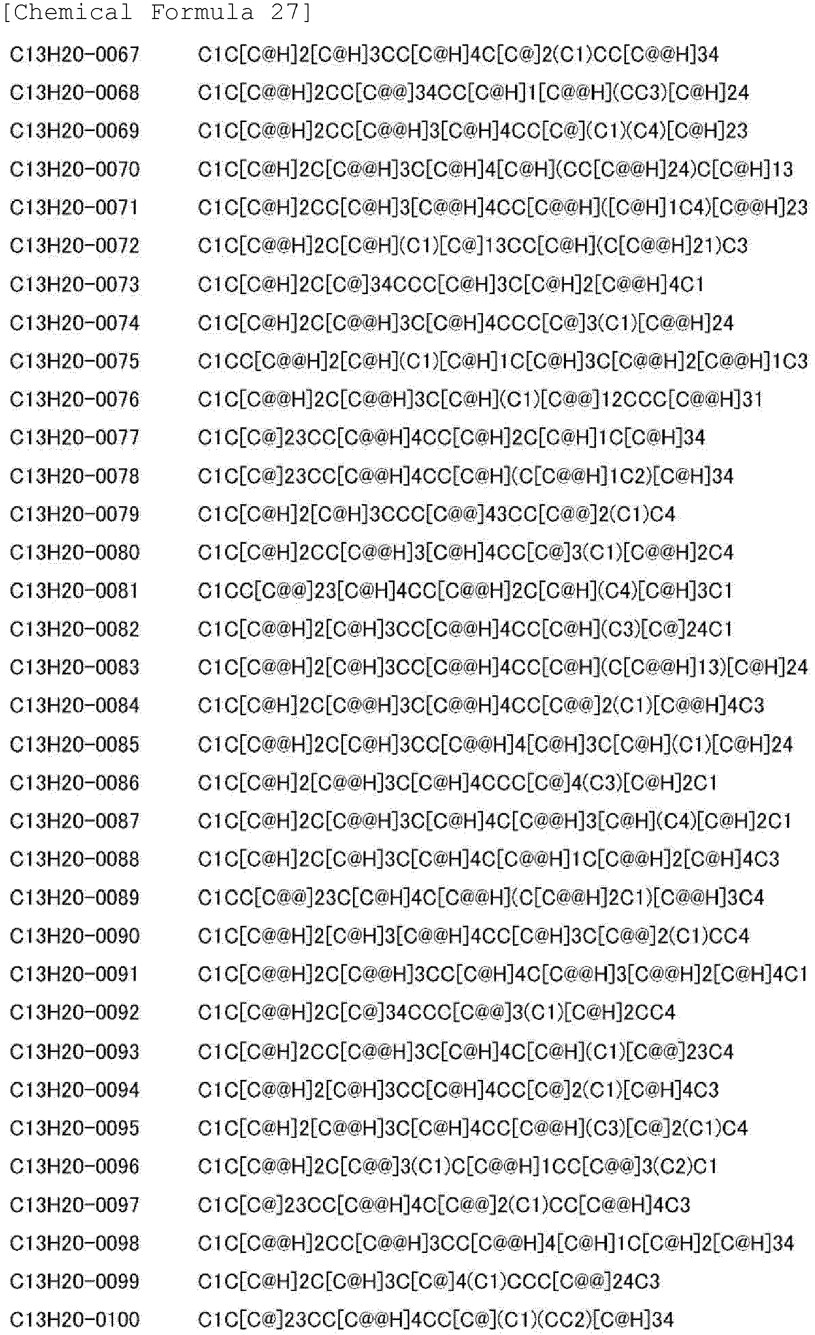 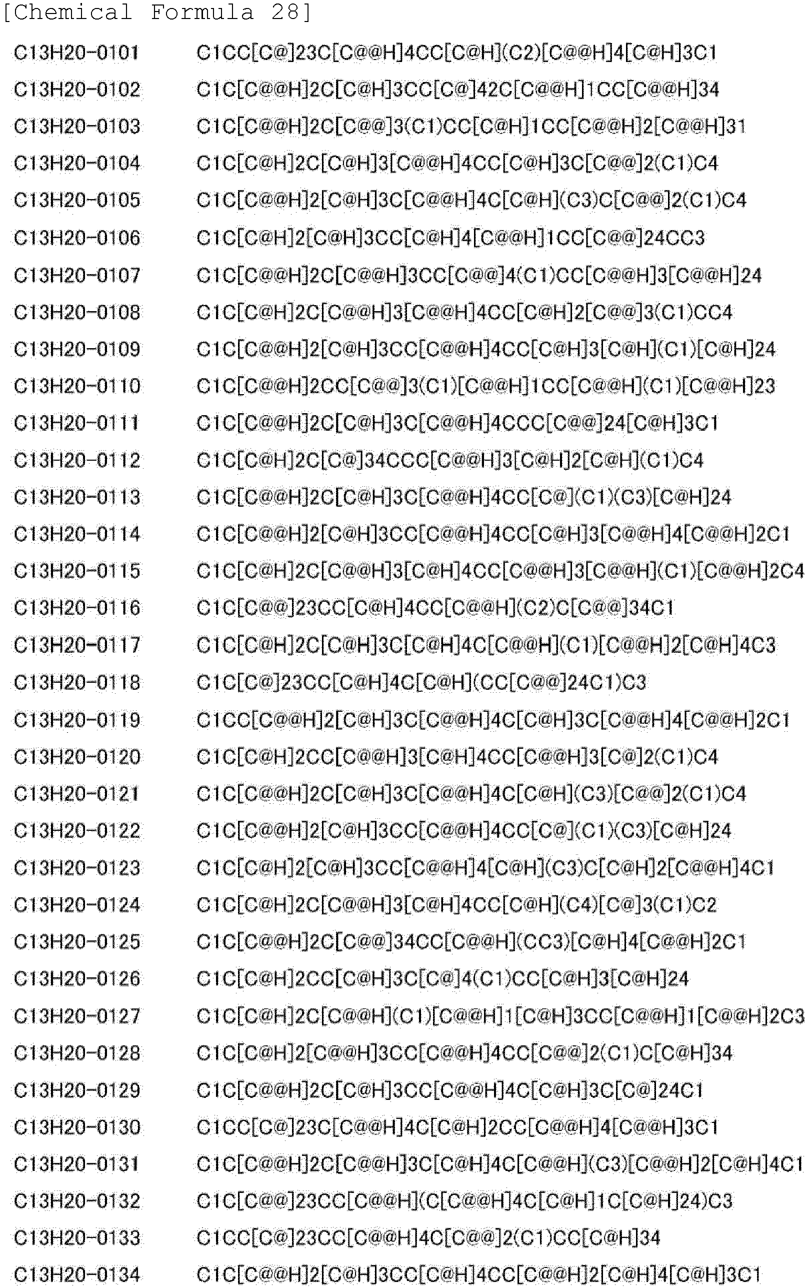 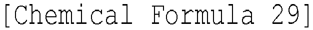 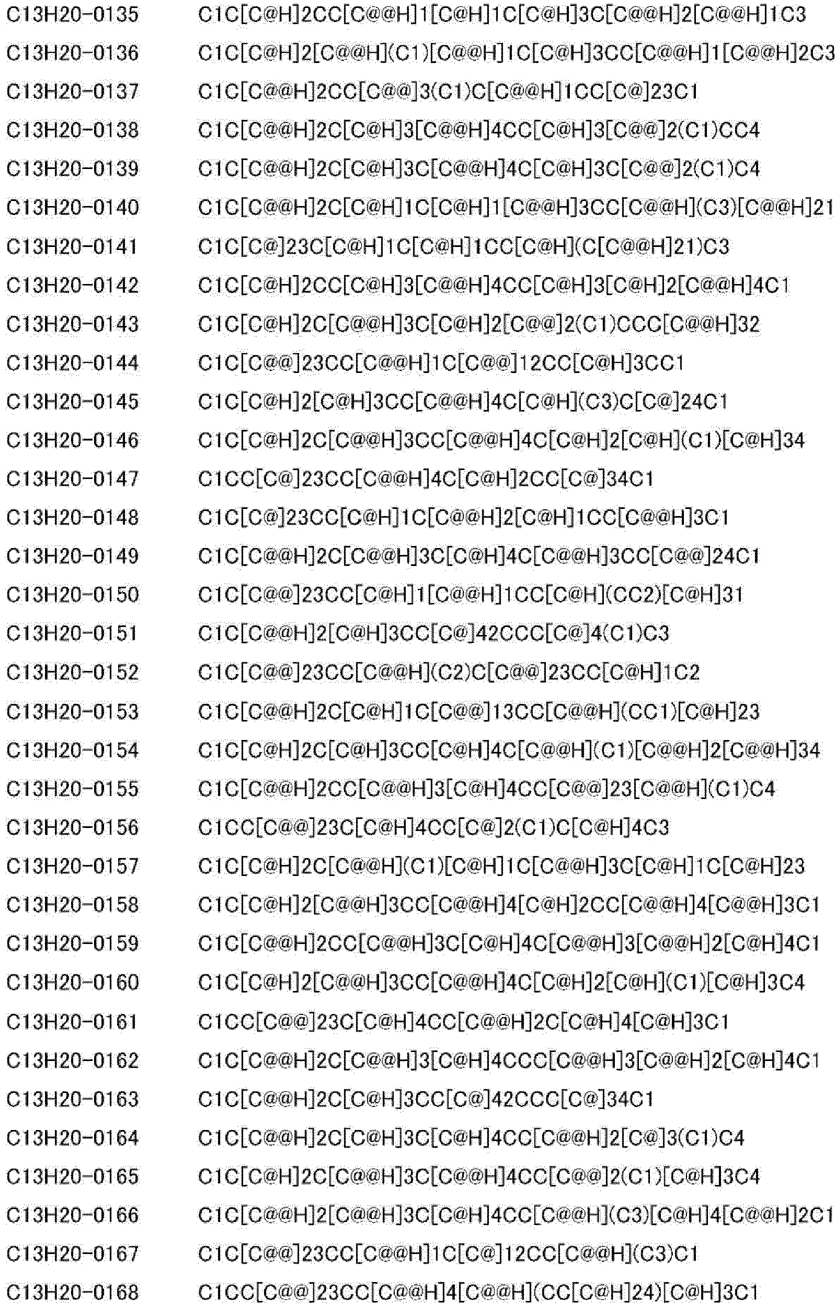 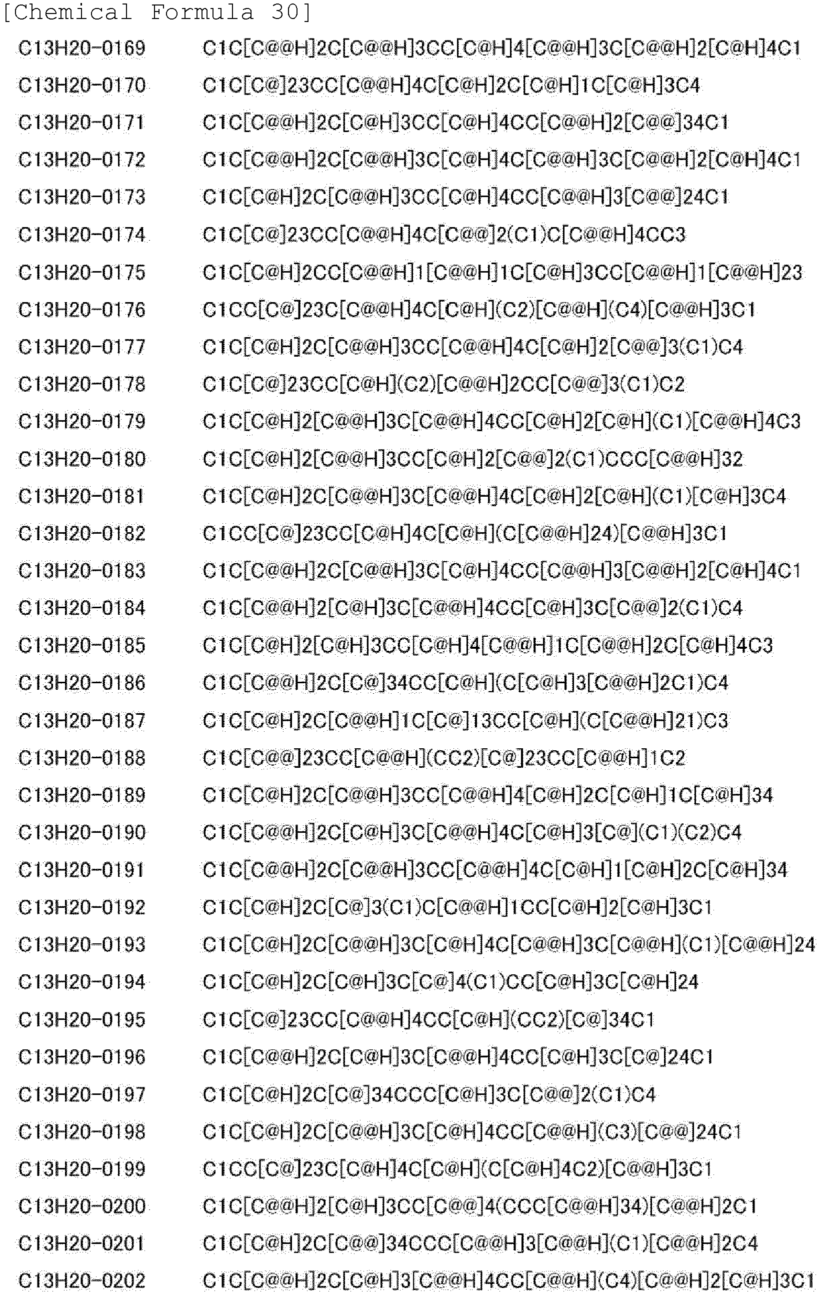 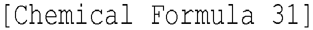 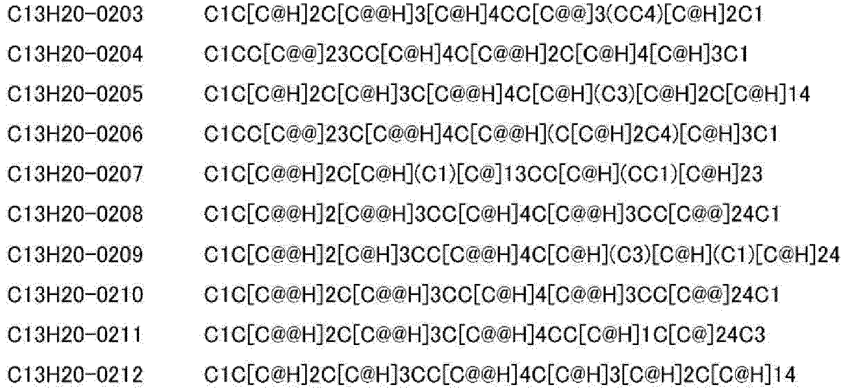 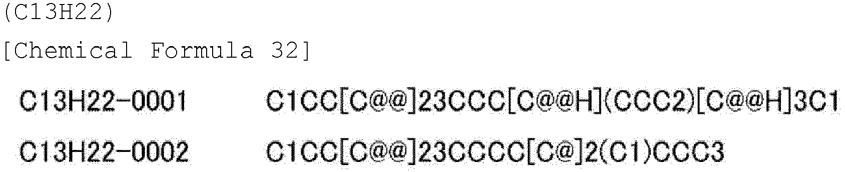 or a backbone having an enantiomer thereof, and M substituents (wherein M is any integer from 1 to (number of atoms in a backbone moiety that can have a substituent)) corresponding to M amino acid-like structures (amino acid side chain or an equivalent thereof) from the backbone, optionally having a heteroatom in place of a carbon atom within a backbone.

### [Item 23]

A composition for interacting a target molecule and a peptide base, comprising a compound, the compound comprising the peptidomimetic compound of C1 or E1,
wherein a substituent of the peptidomimetic compound comprises an amino acid-like structure corresponding to an amino acid contained in the peptide.

### [Item 24]

A medicament comprising the compound or composition of any one of the preceding items.

### [Item A1]

A program encoded with a procedure for executing a method of designing a compound backbone with a desired structure, comprising the steps of causing a computer to:
(1) generate a molecular formula of a compound comprising carbon;
(2) randomly generate molecules using an adjacency matrix to extract a compound falling under the molecular formula among the randomly generated molecules; and
(3) select a compound satisfying a predefined condition.

### [Item A1A]

The program of item A1, wherein the molecular formula of the compound further comprises at least one of hydrogen, oxygen, nitrogen, and sulfur.

### [Item A2]

The program of any one of the preceding items, wherein the desired structure is a cage-like carbon backbone.

### [Item A3]

The program of any one of the preceding items, wherein the predefined condition is to satisfy at least one of:
condition 1: all carbon atoms and bonds are within a 5- or 6- membered ring;
condition 2: there is no spiro ring;
condition 3: there are two or more atoms that are present in common in at least three ring structures within a molecule (ring structure under condition 1 is a 5- or 6-membered ring); and
condition 4: energy is 150 kcal/mol or less in an MMFF (Merck Molecular Force Field) when all are calculated as a single bond upon generation of a 3D structure.

### [Item A3A]

The program of any one of the preceding items, wherein energy is 100 kcal/mol or less in the MMFF.

### [Item A3B]

The program of any one of the preceding items, wherein energy is 50 to 150 kcal/mol in the MMFF.

### [Item A4]

The program of any one of the preceding items, wherein all of condition 1 to condition 4 are satisfied.

### [Item A4A]

The program of any one of the preceding items, wherein the atom comprises a carbon, nitrogen, oxygen, or sulfur atom.

### [Item A4B]

The program of any one of the preceding items, wherein the atom comprises carbon.

### [Item A4C]

The program of any one of the preceding items, comprising at least condition 1.

### [Item A5]

The program of any one of the preceding items, wherein the molecular formula is one selected from the group consisting of C₁₀H₁₆, C₁₁H₁₆, C₁₁H₁₈, C₁₂H₁₈, C₁₂H₂₀, C₁₃H₂₀, and C₁₃H₂₂ .

### [Item A6]

A combination comprising a program encoded with a procedure for causing a computer to execute a method of generating a compound having a compound backbone with a desired structure, comprising the steps of:
(1) causing a computer to generate a molecular formula of a compound comprising carbon;
(2) causing a computer to randomly generate molecules using an adjacency matrix to extract a compound falling under the molecular formula among the randomly generated molecules; and
(3) causing a computer to select a compound satisfying a predefined condition; and
means for (4) producing a compound having a backbone structure selected in (3).

### [Item A7]

The program or combination of any one of the preceding items, wherein the compound comprises at least one type of bond selected from a single bond, a double bond, and a triple bond.

### [Item A7A]

The program or combination of any one of the preceding items, wherein the compound comprises a single bond.

### [Item A8]

A program encoded with a procedure for causing a computer to execute a method of designing a peptidomimetic molecule framework, comprising the steps of causing a computer to:
(A) provide structural data for a carbon moiety of a predefined carbon backbone;
(B) generate an attachment point (AP), which is a position where a side chain structure is given from the structural data for the carbon backbone;
(C) randomly select APs at M locations;
(D) optionally, generate a carbon backbone with N APs by repeating steps (A) to (C) N times, wherein N is a positive integer;
(E) optionally, comprehensively add the same or different amino acid-like structure to each AP at M locations to generate an amino acid-like structure-containing carbon backbone;
(F) fit the amino acid-like structure-containing carbon structure to a pharmacophore obtained from a three-dimensional structure of a predefined peptide, and select a carbon structure that has fit as a peptidomimetic molecule framework.

### [Item A9]

The program of any one of the preceding items, wherein the carbon backbone is a cage-like carbon backbone.

### [Item A10]

The program of item A8 or A9, wherein N is 1000 or greater in step (D).

### [Item A10A]

The program of any one of the preceding items, wherein N is preferably 3000 or greater in step (D).

### [Item A11]

The program of any one of the preceding items, wherein M is an integer from 2 to 6.

### [Item A11A]

The program of any one of the preceding items, wherein M is an integer from 2 to 5 or from 2 to 4.

### [Item A12]

The program of any one of the preceding items, wherein the amino acid is leucine, phenylalanine, isoleucine, and tryptophan in step (E).

### [Item A13]

The program of any one of the preceding items, wherein step (F) comprises causing a computer to select top K carbon backbones with a best fitting score of the pharmacophore.

### [Item A14]

The program of any one of the preceding items, wherein the carbon backbone is a backbone generated by the method of any one of the preceding items.

### [Item A15]

A combination for generating a compound having a peptidomimetic molecule framework, comprising:
a program encoded with a procedure for causing a computer to execute a method comprising the steps of:
(A) providing to a computer structural data for a carbon moiety of a predefined carbon backbone;
(B) causing a computer to generate an attachment point (AP), which is a position where a side chain structure is given from the structural data for the carbon backbone;
(C) causing a computer to randomly select APs at M locations;
(D) optionally, causing a computer to generate a carbon backbone with N APs by repeating step (C) N times, wherein N is a positive integer;
(E) optionally, causing a computer to comprehensively add the same or different amino acid-like structure to each AP at M locations to generate a carbon backbone with an amino acid side chain structure; and
(F) causing a computer to fit the carbon structure with the amino acid side chain structure to a pharmacophore obtained from a three-dimensional structure of a predefined peptide, and select a carbon structure that has fit as a peptidomimetic molecule framework; and
   means for
(G) producing a compound with the peptidomimetic molecule framework selected in (F).

### [Item A16]

The program or combination of any one of the preceding items, wherein M is 3 or greater.

### [Item A17]

The program or combination of any one of the preceding items, wherein the program or combination comprises step (D), and N is 1000 or greater.

### [Item A18]

The program or combination of any one of the preceding items, wherein the compound comprises at least one type selected from a single bond, a double bond, and a triple bond.

### [Item B1]

A recording medium for storing a program encoded with a procedure for causing a computer to execute a method of designing a compound backbone with a desired structure, comprising the steps of causing a computer to:
(1) generate a molecular formula of a compound comprising carbon;
(2) randomly generate molecules using an adjacency matrix to extract a compound falling under the molecular formula among the randomly generated molecules; and
(3) select a compound satisfying a predefined condition.

### [Item B1A]

The recording medium of item B1, wherein the molecular formula of the compound further comprises at least one of hydrogen, oxygen, nitrogen, and sulfur.

### [Item B2]

The recording medium of any one of the preceding items, wherein the desired structure is a cage-like carbon backbone.

### [Item B3]

The recording medium of any one of the preceding items, wherein the predefined condition is to satisfy at least one of:
condition 1: all carbon atoms and bonds are within a 5- or 6- membered ring;
condition 2: there is no spiro ring;
condition 3: there are two or more atoms that are present in common in at least three ring structures within a molecule (ring structure under condition 1 is a 5- or 6-membered ring); and
condition 4: energy is 150 kcal/mol or less in an MMFF (Merck Molecular Force Field) when all are calculated as a single bond upon generation of a 3D structure.

### [Item B3A]

The recording medium of any one of the preceding items, wherein energy is 100 kcal/mol or less in the MMFF.

### [Item B3B]

The recording medium of any one of the preceding items, wherein energy is 50 to 150 kcal/mol in the MMFF.

### [Item B4]

The recording medium of any one of the preceding items, wherein all of condition 1 to condition 4 are satisfied.

### [Item B4A]

The recording medium of any one of the preceding items, wherein the atom comprises a carbon, nitrogen, oxygen, or sulfur atom.

### [Item B4B]

The recording medium of any one of the preceding items, wherein the atom comprises carbon.

### [Item B4C]

The recording medium of any one of the preceding items, comprising at least condition 1.

### [Item B5]

The recording medium of any one of the preceding items, wherein the molecular formula is one selected from the group consisting of C₁₀H₁₆, C₁₁H₁₆, C₁₁H₁₈, C₁₂H₁₈, C₁₂H₂₀, C₁₃H₂₀, and C₁₃H₂₂ .

### [Item B6]

A combination for generating a compound having a compound backbone with a desired structure, comprising:
a program encoded with a procedure for causing a computer to execute a method comprising the steps of:
(1) causing a computer to generate a molecular formula of a compound comprising carbon;
(2) causing a computer to randomly generate molecules using an adjacency matrix to extract a compound falling under the molecular formula among the randomly generated molecules; and
(3) selecting a compound satisfying a predefined condition; and
   means for
(4) producing a compound having a backbone structure selected in (3).

### [Item B7]

The recording medium or combination of any one of the preceding items, wherein the compound comprises at least one type of bond selected from a single bond, a double bond, and a triple bond.

### [Item B7A]

The recording medium or combination of any one of the preceding items, wherein the compound comprises a single bond.

### [Item B8]

A recording medium for storing a program encoded with a procedure for causing a computer to execute a method of designing a peptidomimetic molecule framework, comprising the steps of causing a computer to:
(A) provide structural data for a carbon moiety of a predefined carbon backbone;
(B) generate an attachment point (AP), which is a position where a side chain structure is given from the structural data for the carbon backbone;
(C) randomly select APs at M locations;
(D) optionally, generate a carbon backbone with N APs by repeating steps (A) to (C) N times, wherein N is a positive integer;
(E) optionally, comprehensively add the same or different amino acid-like structure to each AP at M locations to generate an amino acid-like structure-containing carbon structure; and
(F) fit the amino acid-like structure-containing carbon structure to a pharmacophore obtained from a three-dimensional structure of a predefined peptide, and select a carbon structure that has fit as a peptidomimetic molecule framework.

### [Item B9]

The recording medium of any one of the preceding items, wherein the carbon backbone is a cage-like carbon backbone.

### [Item B10]

The recording medium of item B8 or B9, wherein N is 1000 or greater in step (D).

### [Item B10A]

The recording medium of any one of the preceding items, wherein N is preferably 3000 or greater in step (D).

### [Item B11]

The recording medium of any one of the preceding items, wherein M is an integer from 2 to 6.

### [Item B11A]

The recording medium of any one of the preceding items, wherein M is an integer from 2 to 5 or from 2 to 4.

### [Item B12]

The recording medium of any one of the preceding items, wherein the amino acid comprises at least one selected from the group consisting of leucine, phenylalanine, isoleucine, and tryptophan in step (E).

### [Item B13]

The recording medium of any one of the preceding items, wherein step (F) comprises causing a computer to select top K carbon backbones with a best fitting score of the pharmacophore.

### [Item B14]

The method of any one of the preceding items, wherein the carbon backbone is a backbone generated by the method of any one of the preceding items.

### [Item B15]

A combination for generating a compound having a peptidomimetic molecule framework, comprising:
a program encoded with a procedure for causing a computer to execute a method comprising the steps of:
(A) providing to a computer structural data for a carbon moiety of a predefined carbon backbone;
(B) causing a computer to generate an attachment point (AP), which is a position where a side chain structure is given from the structural data for the carbon backbone;
(C) causing a computer to randomly select APs at M locations;
(D) optionally, causing a computer to generate a carbon backbone with N APs by repeating step (C) N times, wherein N is a positive integer;
(E) optionally, causing a computer to comprehensively add the same or different amino acid-like structure to each AP at M locations to generate a carbon backbone with an amino acid side chain structure; and
(F) causing a computer to fit the carbon structure with the amino acid side chain structure to a pharmacophore obtained from a three-dimensional structure of a predefined peptide, and select a carbon structure that has fit as a peptidomimetic molecule framework; and
   means for
(G) producing a compound with the peptidomimetic molecule framework selected in (F).

### [Item B16]

The recording medium or combination of any one of the preceding items, wherein M is 3 or greater.

### [Item B17]

The recording medium or combination of any one of the preceding items, wherein the method comprises step (D), and N is 1000 or greater.

### [Item B18]

The recording medium or combination of any one of the preceding items, wherein the compound comprises at least one type selected from a single bond, a double bond, and a triple bond.

### [Item C1]

A system for designing a compound backbone with a desired structure, comprising:
(1) means or a molecular formula generation unit for generating a molecular formula of a compound comprising carbon;
(2) means or a compound extraction unit for randomly generating molecules using an adjacency matrix to extract a compound falling under the molecular formula among the randomly generated molecules; and
(3) means or a compound selection unit for selecting a compound satisfying a predefined condition.

### [Item C1A]

The system of item C1, wherein the molecular formula of the compound further comprises at least one of hydrogen, oxygen, nitrogen, and sulfur.

### [Item C2]

The system of any one of the preceding items, wherein the desired structure is a cage-like carbon backbone.

### [Item C3]

The system of any one of the preceding items, wherein the predefined condition is to satisfy at least one of:
condition 1: all carbon atoms and bonds are within a 5- or 6- membered ring;
condition 2: there is no spiro ring;
condition 3: there are two or more atoms that are present in common in at least three ring structures within a molecule (ring structure under condition 1 is a 5- or 6-membered ring); and
condition 4: energy is 150 kcal/mol or less in an MMFF (Merck Molecular Force Field) when all are calculated as a single bond upon generation of a 3D structure.

### [Item C3A]

The system of any one of the preceding items, wherein energy is 100 kcal/mol or less in the MMFF.

### [Item C3B]

The system of any one of the preceding items, wherein energy is 50 to 150 kcal/mol in the MMFF.

### [Item C4]

The system of any one of the preceding items, wherein all of condition 1 to condition 4 are satisfied.

### [Item C4A]

The system of any one of the preceding items, wherein the atom comprises a carbon, nitrogen, oxygen, or sulfur atom.

### [Item C4B]

The system of any one of the preceding items, wherein the atom comprises carbon.

### [Item C4C]

The system of any one of the preceding items, comprising at least condition 1.

### [Item C5]

The system of any one of the preceding items, wherein the molecular formula is one selected from the group consisting of C₁₀H₁₆, C₁₁H₁₆, C₁₁H₁₈, C₁₂H₁₈, C₁₂H₂₀, C₁₃H₂₀, and C₁₃H₂₂ .

### [Item C6]

A system for generating a compound having a compound backbone with a desired structure, comprising:
(1) means or a molecular formula generation unit for generating a molecular formula of a compound comprising carbon;
(2) means or a compound extraction unit for randomly generating molecules using an adjacency matrix to extract a compound falling under the molecular formula among the randomly generated molecules;
(3) means or a compound selection unit for selecting a compound satisfying a predefined condition; and
(4) means or a compound production unit for producing a compound having a backbone structure selected in (3).

### [Item C7]

The system of any one of the preceding items, wherein the compound comprises at least one type of bond selected from a single bond, a double bond, and a triple bond.

### [Item C7A]

The system of any one of the preceding items, wherein the compound comprises a single bond.

### [Item C8]

A system for designing a peptidomimetic molecule framework, comprising:
(A) means or structural data provision unit for providing structural data for a carbon moiety of a predefined carbon backbone;
(B) means or an AP generation unit for generating an attachment point (AP), which is a position where a side chain structure is given from the structural data for the carbon backbone;
(C) means or an AP selection unit for randomly selecting APs at M locations;
   wherein a carbon backbone with N APs is optionally generated by repeating steps (A) to (C) N times, wherein N is a positive integer;
(E) optionally, means or a backbone generation unit for comprehensively adding the same or different amino acid-like structure to each AP at M locations to generate an amino acid-like structure-containing carbon backbone; and
(F) means or a framework selection unit for fitting the amino acid-like structure-containing carbon structure to a pharmacophore obtained from a three-dimensional structure of a predefined peptide, and selecting a carbon structure that has fit as a peptidomimetic molecule framework.

### [Item C9]

The system of any one of the preceding items, wherein the carbon backbone is a cage-like carbon backbone.

### [Item C10]

The system of item C8 or C9, wherein N is 1000 or greater in step (D).

### [Item C10A]

The system of any one of the preceding items, wherein N is preferably 3000 or greater in step (D).

### [Item C11]

The system of any one of the preceding items, wherein M is an integer from 2 to 6.

### [Item C11A]

The system of any one of the preceding items, wherein M is an integer from 2 to 5 or from 2 to 4.

### [Item C12]

The system of any one of the preceding items, wherein the amino acid comprises at least one selected from the group consisting of leucine, phenylalanine, isoleucine, and tryptophan in step (E).

### [Item C13]

The system of any one of the preceding items, wherein step (F) comprises selecting top K carbon backbones with a best fitting score of the pharmacophore.

### [Item C14]

The system of any one of the preceding items, wherein the carbon backbone is a backbone generated by the system of any one of the preceding items.

### [Item C15]

A system for generating a compound having a peptidomimetic molecule framework, comprising:
(A) means or structural data provision unit for providing structural data for a carbon moiety of a predefined carbon backbone;
(B) means or an AP generation unit for generating an attachment point (AP), which is a position where a side chain structure is given from the structural data for the carbon backbone;
(C) means or an AP selection unit for randomly selecting APs at M locations;
   wherein a carbon backbone with N APs is optionally generated by repeating steps (A) to (C) N times, wherein N is a positive integer;
(E) optionally, means or a backbone generation unit for comprehensively adding the same or different amino acid-like structure to each AP at M locations to generate an amino acid-like structure-containing carbon backbone;
(F) means or a framework selection unit for fitting the amino acid-like structure-containing carbon structure to a pharmacophore obtained from a three-dimensional structure of a predefined peptide, and selecting a carbon structure that has fit as a peptidomimetic molecule framework; and
(G) means or a compound production unit for producing a compound with the peptidomimetic molecule framework selected in (F).

### [Item C16]

The system of any one of the preceding items, wherein M is 3 or greater.

### [Item C17]

The system of any one of the preceding items, wherein the system comprises step (D), and N is 1000 or greater.

### [Item C18]

The system of any one of the preceding items, wherein the compound comprises at least one type selected from a single bond, a double bond, and a triple bond.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

The present disclosure provides a peptidomimetic compound which is compatible with a target at a level not seen in conventional art. An effect at the quality and level that was not provided in conventional art, such as drug efficacy that is more suited to a target and reduction in side effects, is expected in biotechnology related products such as pharmaceutical products. In addition, any action related to proteins (e.g., interaction between proteins, interaction between a protein and a nucleic acid, etc.) can be controlled using the present disclosure to enable broad applications such as utilization in the field of pesticides where new development is expected and treatment for improvement of the environment.

### [Brief Description of Drawings]

[Figure 1] Figure **1** is a schematic diagram upon extracting a pharmacophore of the FxxFL region by using LigandScout.
[Figure 2] Figure **2** is a schematic diagram of fitting C10H16-0007-11737 to a pharmacophore.
[Figure 3] Figure **3** is a schematic diagram of superposing a peptide of the FxxFL region with C10H16-0007-11737.
[Figure 4] Figure **4** is a schematic diagram of fitting a compound of IF-801 to a pharmacophore.
[Figure 5] Figure **5** is a schematic diagram of superposing a peptide of the FxxFL region with a compound of IF-801.

### [Description of Embodiments]

The present disclosure is described hereinafter in further detail. Throughout the entire specification, a singular expression should be understood as encompassing the concept thereof in the plural form, unless specifically noted otherwise. Thus, singular articles (e.g., "a", "an", "the", etc. in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

### (1 Definition)

As used herein, "molecular formula" is used in the conventional meaning, referring to a methodology of expressing a compound. A molecular formula may also be used for designing and expressing a compound backbone herein and can be expressed to include carbon. A molecular formula may also comprise hydrogen in addition to carbon herein, depending on the setting of calculation. For example, another heteroatom (nitrogen, oxygen, sulfur, etc.) may be comprised when using the technology in the present disclosure.

A molecular formula may be represented in accordance with IUPAC, preferably in the form of Simplified Molecular Input Line Entry System (SMILES) for calculation. In a preferred embodiment, SMILES format that denotes a chemical structure with character strings can be employed.

The SMILES format can be converted into a chemical structural formula that is generally used unambiguously and vice versa. For example, backbones having a molecular formula such as C₁₀H₁₄, C₁₀H₁₆, C₁₁H₁₆, C₁₁H₁₈, C₁₂H₁₈, C₁₂H₂₀, C₁₃H₂₀, or C₁₃H₂₂ can be expressed in the SMILES format in the present disclosure.

As used herein, "molecular formula of a compound comprising carbon" is a formula representing a molecule of any compound comprising carbon, which is represented by a chemical formula comprising Cx (where x is a positive integer) as is conventional or by other suitable formats.

As used herein, "adjacency matrix" is used in the conventional meaning and is used as a method of expressing a molecular structure. In this regard, this is the same for SMILES. For adjacency matrices, an adjacency matrix can be converted to SMILES in a computer. SMILES is preferable because it is highly versatile and can be expressed as character strings.

As used herein, "random generation" refers to generating some type of calculation results (e.g., structural data for a compound backbone) at random and can be expressed as follows in an adjacency matrix. Specifically, an adjacency matrix would be a matrix comprised of 0, 1, and

-1. The molecular structure is expressed by 0 indicating no bond and 1 indicating having a bond (single bond). Diagonal elements (bond to itself) are set to -1. Various molecular structures can be generated by randomly generating 0 and 1 (see below the details of an adjacency matrix).

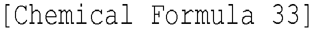

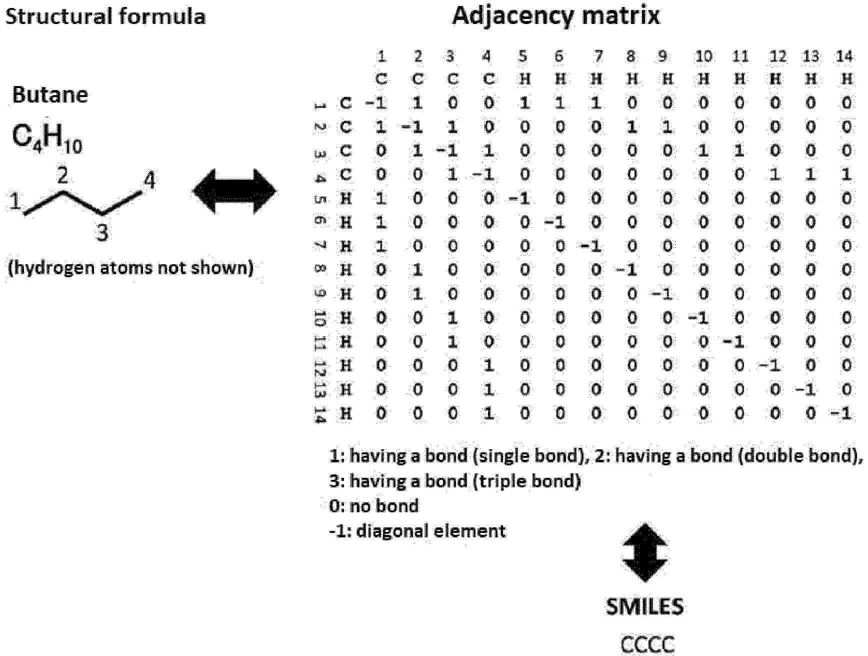

An adjacency matrix of a molecule consisting of n atoms can be expressed as an n × n square matrix A having the bond order of a bond between all atoms as elements thereof. Aᵢⱼ indicates the bond orders of atom i and atom j. The left and right are symmetric with respect to the diagonal line. For example, Kemoinformatikkusu Yosoku to Sekkei no tameno Kagaku Jouhougaku [Chemoinformatics: Chemoinformatics for Prediction and Design], Maruzen, 2005, ISBN4-621-07552-7 can be referenced.

As used herein, "cage-like carbon backbone" refers to a backbone having cyclic structures which are configured not to be substantially on a plane. This refers to, for example, a backbone forming a cage-like shape by fusion of three or more cyclic structures.

As used herein, "compound backbone" or "backbone" refers to the primary structural moiety forming a crosslinked moiety, excluding the substituents. In relation to peptidomimetics, this is a peptidomimetic backbone, which is a foundational structure having an amino acid side chain.

As used herein, "falls under a molecular formula" refers to matching with a molecular formula subjected to analysis (match in elements constituting a molecule such as the type of atom or number of a specific atom (e.g., number of carbons)).

As used herein, "predefined condition" of a "compound" satisfying the "predefined condition" is a condition that can be determined arbitrarily for calculation, and is a condition that can be determined arbitrarily by those skilled in the art as needed for calculation. For example, RDKit (see RDKit: https://www.rdkit.org/) can be used in the following manner. For example, the number of 5-membered rings within a molecule and atoms constituting the 5-membered rings can be obtained by the function GetSubstructMatches of RDKit. Likewise, the number of 6-membered rings within a molecule and atoms constituting the 6-membered rings can be obtained by the function GetSubstructMatches of RDKit. If the returned value is 0 by using the function CalcNumSpiroAtoms, it can be determined that "there is no spiro ring".

As used herein, "all carbon atoms and bonds are within a 5- or 6- membered ring" means that when a certain compound backbone is formed, all carbon atoms are within the backbone and there are carbon-carbon bonds, which are not on a side chain. Specifically, this can be checked by "checking whether all carbon atoms and bonds are within a ring" during processing using SMILES. In addition to all carbon atoms, other atoms (e.g., heteroatoms, etc.) and bonds can also be within a 5- or 6-membered ring. In such a case, it can be confirmed whether it is suitable by checking whether target atoms in addition to carbon atoms are a match.

As used herein, "there is no spiro ring" refers to any state without a spiro ring in a cyclic compound. The number of spiro atoms can be calculated using, for example, the function CalcNumSpiroAtoms of RDKit as described above in calculation using SMILES. If this value is zero in such a case, it can be determined that "there is no spiro ring".

As used herein, "there are two or more atoms that are present in common in at least three ring structures within a molecule (ring structure under condition 1 is a 5- or 6-membered ring)" refers to searching for all 5- or 6-membered rings within a molecule, retrieving constituent atoms from the obtained 5- or 6-membered rings, sorting out atoms that are present in common in at least three ring structures, and checking that there are two or more obtained atoms. This can be materialized by writing a program as described below. The presence or absence thereof can be checked with a program. For example, this can be materialized by, for example, programming the following:
1 search for all 5- or 6-membered rings within a molecule,
2 retrieve constituent atoms from the 5- or 6-membered rings obtained in 1,
3 sort out atoms that are present in common in at least three ring structures from 2, and
4 check that there are two or more atoms obtained in 3.

For example, software known as RDKit can search for 5- or 6-membered rings and retrieve constituent atoms thereof.

As used herein, "energy is, for example, 100 kcal/mol or less in an MMFF (when all bonds are single bonds) upon constructing a 3D structure" means that energy of MMFF calculated upon generation of a predefined compound backbone and generating a three-dimensional structure is 100 kcal/mol or less. The value of energy in an MMFF may be set to 50 kcal/mol or less, 150 kcal/mol or less, 50 to 150 kcal/mol, or any value therebetween in accordance with the objective of the present disclosure.

Unless specifically noted otherwise, MMFF is from calculation of the field while assuming that "backbone comprises only a single bond". MMFF is an abbreviation for (Merck Molecular Force Field). For example, the article: Thomas A. Halgren (1996). "Merck molecular force field. I. Basis, form, scope, parameterization, and performance of MMFF94". J. Comp. Chem.: 490-519. can be referenced.

As used herein, "Attachment Point (AP)" refers to a position where a side chain structure is added. Specifically, attachment point refers to a position of a hydrogen atom of a representative "cage-like carbon backbone" of the present disclosure.

As used herein, generating an "attachment point (AP), which is a position where a side chain structure is given from the structural data for the carbon backbone" refers to setting a position where a side chain structure can be given, typically a position where a hydrogen atom attaches.

As used herein, "carbon backbone with AP" refers to any carbon backbone that can generate a side chain structure.

As used herein, "amino acid-like structure" refers to a structure that can have a structure similar to amino acid in a part of a certain compound. In particular, an amino acid-like structure can exert a function that is similar to (including inferior) or improved as compared to an amino acid in a biological reaction. Said structure as used herein may be typically a side chain moiety of an amino acid or an analogue thereof. For an analogue, it can be preferable to use an analogue with better fit from the viewpoint of fitting a peptidomimetic molecule to a target for an amino acid side chain. Those skilled in the art can identify a suitable amino acid-like structure by, for example, analysis of a three-dimensional structure.

Examples of such fitting optimized amino acid-like structures include groups of a side chain of a naturally-occurring amino acid such as hydrogen (glycine-like), methyl (alanine-like), isopropyl (valine-like), isobutyl (leucine-like), sec-butyl (isoleucine-like), hydroxymethyl (serine-like), 1-hydroxyethyl (threonine-like), carboxymethyl (aspartic acid-like), carboxyethyl (glutamic acid-like), aminocarbonylmethyl (asparagine-like), aminocarbonylethyl (glutamine-like), aminobutyl (lysine-like), guanidylpropyl (arginine-like), 4-imidazoylmethyl (histidine-like), phenylmethyl (phenylalanine-like), 4-hydroxyphenylmethyl (tyrosine-like), and 3-indolylmethyl (tryptophan-like), groups of a side chain of a non-naturally-occurring amino acid such as ethyl, propyl, butyl, pentyl, hexyl, heptyl, octyl, nonyl, decyl, isopentyl, isohexyl, isoheptyl, isooctyl, isononyl, isodecyl, tert-butyl, cyclobutyl, cyclopentyl, cyclohexyl, cyclobutylmethyl, cyclopentylmethyl, cyclohexylmethyl, phenyl, phenylethyl, phenylpropyl, 1-naphthyl, 2-naphthyl, 1-naphthylmethyl, 2-naphthylmethyl, 1-naphthylethyl, 2-naphthylethyl, 2-pyridyl, 3-pyridyl, 4-pyridyl, 2-pyridylmethyl, 3-pyridylmethyl, 4-pyridylmethyl, 2-pyridylethyl, 3-pyridylethyl, 4-pyridylethyl, 2-quinolyl, 3-quinolyl, 4-quinolyl, 5-quinolyl, 6-quinolyl, 7-quinolyl, 8-quinolyl, 2-benzothiazolyl, 2-benzothiazolylmethyl, 2-benzothiazolylethyl, 2-quinolylmethyl, 3-quinolylmethyl, 4-quinolylmethyl, 5-quinolylmethyl, 6-quinolylmethyl, 7-quinolylmethyl, 8-quinolylmethyl, 2-quinolylethyl, 3-quinolylethyl, 4-quinolylethyl, 5-quinolylethyl, 6-quinolylethyl, 7-quinolylethyl, 8-quinolylethyl, 2-benzothiazolyl, 2-benzothiazolylmethyl, and 2-benzothiazolylethyl, optionally substituted phenyl, optionally substituted phenylmethyl, optionally substituted phenylethyl, optionally substituted naphthyl, optionally substituted naphthylmethyl, optionally substituted naphthylethyl, optionally substituted quinolyl, optionally substituted quinolylmethyl, optionally substituted quinolylethyl, optionally substituted 2-benzothiazolyl, optionally substituted 2-benzothiazolylmethyl, optionally substituted 2-benzothiazolylethyl, etc.

Examples of amino acid-like structure that is typically used include isobutyl (leucine-like), sec-butyl (isoleucine-like), phenylmethyl (phenylalanine-like), 3-indolylmethyl (tryptophan-like), etc.

Adjustment can be made for fitting, such as changing the number of carbon atoms, in a side chain of an amino acid provided, such as a naturally-occurring amino acid.

Amino acid-like structures of leucine or isoleucine include isobutyl, ethyl, propyl, butyl, pentyl, hexyl, heptyl, octyl, nonyl, decyl, isopentyl, isohexyl, isoheptyl, isooctyl, isononyl, isodecyl, tert-butyl, cyclobutyl, cyclopentyl, cyclohexyl, cyclobutylmethyl, cyclopentylmethyl, and cyclohexylmethyl.

Amino acid-like structures of phenylalanine include phenylmethyl, phenyl, phenylethyl, phenylpropyl, 2-pyridyl, 3-pyridyl, 4-pyridyl, 2-pyridylmethyl, 3-pyridylmethyl, 4-pyridylmethyl, 2-pyridylethyl, 3-pyridylethyl, 4-pyridylethyl, optionally substituted phenyl, optionally substituted phenylmethyl, and optionally substituted phenylethyl.

Amino acid-like structures of tryptophan include 3-indolylmethyl, 1-naphthyl, 2-naphthyl, 1-naphthylmethyl, 2-naphthylmethyl, 1-naphthylethyl, 2-naphthylethyl, 2-quinolyl, 3-quinolyl, 4-quinolyl, 5-quinolyl, 6-quinolyl, 7-quinolyl, 8-quinolyl, 2-benzothiazolyl, 2-benzothiazolylmethyl, 2-benzothiazolylethyl, 2-quinolylmethyl, 3-quinolylmethyl, 4-quinolylmethyl, 5-quinolylmethyl, 6-quinolylmethyl, 7-quinolylmethyl, 8-quinolylmethyl, 2-quinolylethyl, 3-quinolylethyl, 4-quinolylethyl, 5-quinolylethyl, 6-quinolylethyl, 7-quinolylethyl, 8-quinolylethyl, optionally substituted naphthyl, optionally substituted naphthylmethyl, optionally substituted naphthylethyl, optionally substituted quinolyl, optionally substituted quinolylmethyl, optionally substituted quinolylethyl, optionally substituted 2-benzothiazolyl, optionally substituted 2-benzothiazolylmethyl, and optionally substituted 2-benzothiazolylethyl.

For example, if an amino acid is phenylalanine, a phenethyl group can be provided, with a benzyl group as the basis. If this is isoleucine which is an asymmetric carbon, cyclohexyl can be provided. The length of a side chain is not limited, and can be appropriately extended or contracted in accordance with the biological activity of interest.

A "corresponding" amino acid-like structure corresponding to an amino acid herein refers to any structure (substituent) which has, or is predicted to have, the same structure/action as a given amino acid in a peptide, polypeptide, or protein that is the baseline of comparison, or a portion thereof, in a peptidomimetic molecule. Representative examples thereof include a side chain of an amino acid subjected to comparison and can include derivatives thereof (e.g., those with the number of carbons changed, etc.).

As used herein, "amino acid-like structure-containing carbon backbone" refers to a carbon backbone comprising an amino acid-like structure directly or as a part thereof, structural data depicting such a backbone, etc. As used herein, "amino acid-like structure-containing carbon backbone" and "carbon backbone with an amino acid side chain structure" can be interchangeably used.

As used herein, "peptidomimetic" refers to anything similar to a peptide, structural data which is a depiction thereof, etc.

As used herein, "pharmacophore" refers to a group of functional groups with a characteristic required for the interaction with a target protein and relative three-dimensional arrangement thereof. Pharmacophore can be depicted as a specific structural formula or a collection thereof and can also be expressed by SMILES. For example, various software such as LigandScout can be used therefor. Pharmacophore can be expressed (stored) in a PML format, which is the storage format for LigandScout. See, for example, an article on LigandScout: J. Chem. Inf. Model. 2005, 45, 160-169. Exemplary pharmacophores include those in the PML format, which is as follows when pharmacophore0 is shown as an example.

### [Numeral 1]

```
 <?xml version="1.0" encoding="UTF-8"?>
 <pharmacophore name="[A] MOL438" id="pharmacophore0"
 pharmacophoreType="LIGAND_SCOUT"><point name="H" featureld="11500169456600017567"
 optional="false" disabled="false" weight="1.0" coreCompound="" envCompound=""
 id=" featureo"><position x3="223.65666" y3="231.44966" z3="106.4125" tolerance="1.5"
 /></point><point name="H" featureld="11500169982530019316" optional="false" disabled="false"
 weight="1.0" coreCompound="" envCompound="" id="feature1"><position x3="221.004"
 y3="225.786" z3="110.441666" tolerance=" 1.5" /></point><point name="H"
 featureId="11500169201430017142" optional="false" disabled="false" weight="1.0"
 coreCompound="" envCompound="" id="feature2"><position x3="227.24283" y3="225.45366"
 z3="109.228836" tolerance="1.5" /></point><plane name="AR" featured="11579230736430026684"
 optional="true" disabled="false" weight=" 1.0" coreCompound="" envCompound=""
 id="feature3"><position x3="223.65666" y3="231.44966" z3="106.4125" tolerance="0.9" /><normal
 x3="0.765492" y3="-0.550102" z3="-0.33378094" tolerance="0.43633232" /></plane><plane
 name="AR" featureId="11582293253510026695" optional="true" disabled="false" weight="1.0"
 coreCompound="" envCompound="" id="feature4"><position x3="227.24283" y3="225.45367"
 z3="109.228836" tolerance="0.9" /><normal x3="-0.11025418" y3="0.9930941" z3="0,040101208"
 tolerance="0.43633232" /></plane><volume type="exclusion" featureld="11500169244590017149"
 optional="false" disabled="false" weight="1.0" id="feature5"><position x3="224.38867"
 y3="229.80183" z3="110.85267" tolerance="1,5" /></volume><volume type="exclusion"
 feetureId="11531180345260022054" optional="false" disabled="false" weight="1.0"
 id="feature6"><position x3="221.361" y3="232.867" z3="110.313" tolerance="1.5"
 /></volume><volume type="exclusion" featureld="11531180126770021713" optional="false"
 disabled="false" weight="1.0" id="feature7"><position x3="218.86" y3="229.561" z3="107.902"
 tolerance="1.5" /></volume>< volume type="exclusion" featureId="11531180322440022022"
 optional="false" disabled="false" weight="1.0"
id="feature8"><position x3="221.305" y3="234.168"
 z3="107.219" tolerance="1.5" /></volume><volume type= exclusion"
 featureld="11500169248370017159" optional="faise" disabled="false" weight="1.0"
 id="feature9"><position x3="227.07913" y3="229.46796" z3="105.38747" tolerance="1.5"
 /></volume><volume type="exclusion" featureld="11531180173350021824" optional="false "
 disabled="false" weight="1.0" id="feature10"><position x3="219.044" y3="231.107" z3="112.031"
 tolerance="1.5" /></volume><volume type="exclusion" featureid="11500170526880020072"
 optional="false" disabled="false" weight="1.0"
id="feature11"><position x3="226.072" y3="234.813"
 z3="109.262" tolerance="1.0" /></volume><volume type="exclusion"
 featured="11500170565890020073" optional="false" disabled="false" weight="1.0"
```

### [Numeral 2]

```
 id="feature12"><position x3="217.162" y3="229.654" z3="104.757" tolerance="1.0"
 /></volume><volume type="exclusion" featureld=" 11531180640980022224" optional="false"
 disabled="false" weight="1.0" id="feature13"><position x3="217.887" y3="233.328" z3="104.774"
 tolerance="1.5" 1></volume><volume type="exclusion" featureId="11531180335900022041"
 optional="false" disabled="false" weight=" 1.0"
id="feature 14"><position x3="225.815" y3="231.615"
 z3="102.05" tolerance="1.5" /></volume><volume type="exclusion"
 featureld="11531180135030021787" optional="false" disabled="false" weight="1.0"
 id="feature15"><position x3="215.877" y3="229.46" z3="110.722" tolerance="1.5"
 /></volume><volume type="exclusion" featureId="11531180737180022310" optional="false"
 disabled="false" weight="1.0" id="feature16"><position x3="224.254" y3="235.57" z3="112.509"
 tolerance="1.5" /></volume><volume type="exclusion" featureld="11500169525140017746"
 optional="false" disabled="false" weight="1.0" id="feature17"><position x3="221.4842"
 y3="233.2158" z3="101.786804" tolerance="1.5" /></volume><volume type="exclusion"
 featureld="11531180026800021646" optional="false" disabled="false" weight="1.0"
 id="feature18"><position x3-"226.111" y3-"227.482" z3="114.396 " tolerance-" 1.5"
 /></volume><volume type="exclusion" featureld=" 11500169150220018850" optional="false"
 disabled="false" weight=" 1.0" id="feature 19"><position x3="219.70941" y3="226.43436"
 z3="114.26077" tolerance="1.5" /></volume><volume type="exclusion"
 featureId="11531180400530022089" optional="false" disabled="false" weight="1.0"
 id="feature20"><position x3="229.384" y3="233.209" z3=108.926" tolerance="1.5"
 /></volume><volume type=" exclusion" featureId="11531180398560022088" optional="false"
 disabled="false" weight="1.0" id="feature21 "><position x3="228.784" y3="232,438" z3="104.601 "
 tolerance="1.5" /></volume><volume type="exclusion" featureld="11531180412480022098"
 optional="false" disabled="false" weight="1.0"
id="feature22"><position x3="225.093" y3="235.87"
 z3=" 104.206" tolerance="1.5" /></volume><volume type=" exclusion"
 featureld="111531180296560021990" optional="false" disabled="false" weight="1.0"
 id=" feature23 "><position x3="218.835" y3="229.445" z3="101.41" tolerance="1.5"
 /></volume><volume type="exclusion" featureld="11531180219410021888" optional="false"
 disabled="false" weight="1.0" id="feature24"><position x3="223.254" y3="229.053" z3="116.169"
 tolerance="1.5" /></volume><volume type="exclusion" featureld="11531180578100022175"
 optional="false" disabled="false" weight="1.0"
id="feature25" ><position x3="221.442" y3="237.163"
 z3="105.13" tolerance="1.5" /></volume><volume type="exclusion"
 featureld="11500169131090017141" optional="false" disabled="false" weight=" 1.0"
 id="feature26"><position x3="230.48778" y3="229.53227" z3="110.08813" tolerance="1.5"
 /></volume><volume type="exclusion" featureld="1153'1180093060021750" optional="false"
 disabled="false" weight="1.0" id="feature27"><position x3="217.99" y3="222.336" z3="108.025"
```

### [Numeral 3]

```
 tolerance="1.5" /></volume><volume type="exclusion" featuretd="11531480697800022278"
 optional="false" disabled="false" weight="1.0"
id="feature28"><position x3="225.627" y3:="238.344"
 z3="108.012" tolerance="1.5" /></volume><volume type="exclusion"
 featureld="11531180922000022473" optional="false" disabled="false" weight="1.0"
 id="feature29"><position x3="214.48" y3="227.101" z3="105.243" tolerance="1.5"
 /></volume><volume type="exclusion" featureld="115311801 14710021758" optional="false"
 disabled="false" weight="1.0" id="feature30"><position x3="217.355" y3="222.965" z3="111.328"
 tolerance="1.5" /></volume><volume type::" exclusion" featureId="11500170612450020074"
 optianal="false" disabled="false" weight="1.0"
id="feature31"><position x3="229.641" y3="227.311"
 z3="113.745" tolerance=" 1.0" /></volume><volume type="exclusion"
 featureId="11531180161910021816" optional="false" disabled="false" weight="1.0"
 id="feature32"><positíon x3="214.056" y3="226.017" z3="108.97" tolerance="1.5'
 /></volume><volume type="exclusion" featureId="11531180291560021983" optional="false"
 disabled="false" weight="1.0" id="feature33"><position x3="215.508" y3="224.866" z3="102.158"
 tolerance="1.5" /></volume><volume type="exclusion" featureId="11531179987690021611"
 optional="false" disabled="false" weight="1.0"
id="feature34"><position x3="231,192" y3="223.738"
 z3="11 1.506" tolerance="1.5" /></volume></pharmacophore>
```

As used herein, "fitting" refers to superposing the pharmacophore of a target protein with a group of functional groups of a compound. Fitting can be performed with a program by using SMILES. Fitting can be performed using various software such as LigandScout. See, for example, an article on LigandScout: J. Chem. Inf. Model. 2005, 45, 160-169.

As used herein, "fitting score" refers to an indicator indicating the degree of overlap of pharmacophore of a target protein with a group of functional groups of a compound. The score can be expressed as a relative ratio thereof with a maximum value of 100, or as an absolute value. The fitting score may also be referred to as "pharmacophore score" or "FS" herein, which can be used interchangeably.

As used herein, "peptidomimetic molecule framework" refers to a backbone moiety of a compound that can be used as a peptide mimetic molecule by appropriately arranging substituents or structural data thereof. A peptidomimetic molecule can be freely manufactured by using such a peptidomimetic molecule framework.

Amino acids can be referred to herein by either their generally known three-letter symbols or the one-letter symbols recommended by the IUPAC-IUB Biochemical Nomenclature Commission. Nucleotides can also be referred to by the generally recognized one-letter codes. Herein, comparison of similarity, identity, and homology of amino acid sequences and base sequences is calculated with default parameters using a tool for sequence analysis, BLAST. The identity search can be performed using, for example, NCBI's BLAST 2.2.28 (published on April 2, 2013) (Proc. Natl. Acad. Sci. USA 90: 5873-5877 ,1993). The value of identity herein usually refers to the value obtained by alignment under the default conditions by using the aforementioned BLAST. However, if a higher value is obtained by varying the parameters, the highest value obtained is deemed as the value for the identity. When identity is evaluated in multiple regions, the highest value among them is deemed as the value for the identity. Similarity is a numerical value that takes into account similar amino acids in addition to identity. Blastp can be used with default settings for the algorithm in the comparison between amino acid sequences in BLAST. The measurement results are quantified as Positives or Identities. The homology of amino acid sequence and base sequence can be determined by the algorithm BLAST by Karlin and Altschul. Based on this algorithm, programs known as BLASTN and BLASTX have been developed (Altschul et al. J. Mol. Biol. 215: 403-410, 1990). When a base sequence is analyzed by BLASTN based on BLAST, parameters are set as, for example, score = 100 and wordlength = 12. When an amino acid sequence is analyzed by BLASTX based on BLAST, parameters are set as, for example, score = 50 and wordlength = 3. When BLAST and Gapped BLAST programs are used, the default parameters of each program are used. Specific techniques of these analysis methods are known (http://www.ncbi.nlm.nih.gov.) .

As used herein, "protein", "polypeptide", "oligopeptide", and "peptide" are used with the same meaning herein and refer to a polymer of amino acids of any length. The polymer may be linear, branched, or cyclic. Amino acids may be naturally-occurring, non-naturally-occurring, or altered amino acids. The term may can encompass those assembled into a complex of multiple polypeptide chains. The term also encompasses naturally or artificially altered amino acid polymers. Examples of such alterations include disulfide bond formation, glycosylation, lipidation, acetylation, phosphorylation, methylation, and any other manipulation or alteration (e.g., conjugation with a labeling component). This definition also encompasses, for example, polypeptides including one or more analogs of amino acids (including, for example, non-naturally-occurring amino acids), peptide-like compounds (e.g., peptoids), and other alterations known in the art. As used herein, "amino acid" is a general term for organic compounds having an amino group and a carboxyl group. When the antibody according to the embodiments of the present disclosure includes a "specific amino acid sequence", any amino acid in the amino acid sequence may be chemically modified. In addition, any amino acid in the amino acid sequence may form a salt or a solvate. Further, any amino acid in the amino acid sequence may have an L-form or D-form. Even in such cases, the protein according to the embodiments of the present disclosure can be deemed as comprising the "specific amino acid sequence" described above. Known chemical modifications that an amino acid included in a protein undergoes in vivo include N-terminal modification (e.g., acetylation, myristoylation, etc.), C-terminal modification (e.g., amidation, glycosylphosphatidylinositol addition, etc.), side chain modification (e.g., phosphorylation, glycosylation, etc.) or the like. Proteins may be naturally-occurring or non-naturally-occurring, as long as objectives of the present disclosure are satisfied.

As used herein, an "agent" is used in a broad sense, and may be any substance or other elements (e.g., energy such as light, radiation, heat, and electricity) as long as the intended object can be achieved. Examples of such a substance include, but are not limited to, proteins, polypeptides, oligopeptides, peptides, polynucleotides, oligonucleotides, nucleotides, nucleic acids (e.g., including DNA such as cDNA and genomic DNA, and RNA such as mRNA), polysaccharides, oligosaccharides, lipids, organic small molecules (e.g., hormones, ligands, information transmitting substances, organic small molecules, molecules synthesized by combinatorial chemistry, small molecules which can be utilized as a pharmaceutical product (e.g., a low molecular weight ligand), etc.), and composite molecules thereof.

For oral administration, an agent may be formulated into various forms such as tablets, granules, fine granules, powder, and capsules for use. An additive commonly used in a formulation such as a binding agent, covering agent, excipient, lubricant, disintegrant, or humectant may also be included. In addition thereto, formulations for oral administration may be formulated as a liquid formulation such as an aqueous solution for internal use, suspension, emulsion, or syrup. The formulation may also be formulated as a dry formulation that is redissolved in a solvent upon use.

For parenteral administration, an agent may be formulated to be contained in a unit dose ampule or multidose container or tube. An additive such as a stabilizer, buffer, preservative, or isotonizing agent may also be included. A formulation for parenteral administration may also be formulated into a powder form that can be redissolved in a suitable carrier (sterilized water, etc.) upon use.

As used herein, "treat" refers to the prevention of exacerbation, preferably maintaining of the current state, more preferably alleviation, and still more preferably elimination of a disease or disorder, in case of such a disease or disorder, including being capable of exerting an effect of improving or preventing a disease of a patient or one or more symptoms accompanying the disease. Preliminary diagnosis with suitable therapy is referred to as "companion therapy" and a diagnostic agent therefor may be referred to as "companion diagnostic agent". Since the present disclosure targets genetic diseases, genes may be inspected in advance to treat a patient.

As used herein, "therapeutic drug (agent)" broadly refers to any agent that can treat a condition of interest (e.g., cancer, infection, etc.). In one embodiment of the present disclosure, "therapeutic drug" may be a pharmaceutical composition comprising an active ingredient and one or more pharamacologically acceptable carriers. A pharmaceutical composition can be manufactured by any method that is known in the technical field of pharmaceutical science, such as mixing an active ingredient with the carrier described above. The mode of use of a therapeutic drug is not limited, as long as the drug can be used for treatment. A therapeutic drug may be an active ingredient alone, or a mixture of an active ingredient with any component. The form of the carrier described above is not limited. Examples thereof include solids and liquids (e.g., buffer).

As used herein, "prevention (or prophylaxis)" refers to the action of taking a measure against a disease or disorder (e.g., cancer or infection) from being in such a condition, prior to being in such a condition. It is possible to use the agent of the present disclosure to perform diagnosis, and if necessary use the agent of the present disclosure to prevent or take measures to prevent a retinal degenerative disease, etc. As used herein, "prophylactic drug (agent)" broadly refers to any agent capable of preventing a condition of interest (e.g., vesicular trafficking disorder, apoptosis, etc.).

As used herein, "kit" refers to a unit providing parts to be provided (e.g., compound, test drug, diagnostic drug, therapeutic drug, antibody, label, user manual, etc.) which are generally separated into two or more segments. When it is preferable to administer a specific drug (compound, etc.) only to a suitable patient after first identifying a patient to be administered with a reagent for determining the characteristic of a patient such as a companion diagnostic drug, etc., a diagnostic drug and a therapeutic drug can be prepared as a kit when provided in combination. Alternatively, such a kit form is preferred when providing a composition, which should not be provided in a mixed state for stability, etc., such as a specific unstable drug, and is preferably used by mixing immediately prior to use. Such a kit advantageously comprises an instruction or user manual describing how the provided parts (e.g., compound, test drug, diagnostic drug, and therapeutic drug) are used or how the reagent should be processed. When the kit is used as a reagent kit herein, the kit generally comprises instructions describing how to use a test drug, diagnostic drug, therapeutic drug, antibody, etc.

As used herein, "active ingredient" refers to an ingredient contained at an amount needed for a composition of the present disclosure, etc. to attain an effect such as treatment, prevention, or suppression of progression of interest. Other ingredients may also be contained, as long as the effect is not reduced below a desired level. The drug, composition, etc. of the present disclosure may also be formulated. Further, the route of administration of the drug, composition, etc. of the present disclosure may be either oral or parenteral. The route of administration can be appropriately determined depending on the form of formulation, etc.

As used herein, "instruction" (including package insert, label used by the USFDA, etc.) is a document with an explanation for the method of use of the present disclosure for a physician or other users. Such an instruction has an instructive description for the method of detection of the present disclosure, how to use a diagnostic drug, or instruction for administration of a drug, etc. The instruction may have a description instructing oral or topical administration as a site of administration (e.g., by injection, etc.). The instruction is prepared in accordance with a format specified by the regulatory agency of the country in which the present disclosure is practiced (e.g., the Ministry of Health, Labour and Welfare in Japan, Food and Drug Administration (FDA) in the U.S., etc.), with an explicit description showing approval by the regulatory agency. An instruction is a so-called package insert or label. An instruction is generally provided in paper media, but is not limited thereto. An instruction can also be provided in a form such as electronic media (e.g., web sites provided on the Internet or emails).

### (2 Schematic description of display/calculation method using structural data for a compound)

The compound structure data of the present disclosure can be denoted via any methodology of the art. As a practical example, SMILES (simplified molecular input line entry system), as well as methodologies such as Wiswesser Line Notation (WLN), ROSDAL, SLN (Tripos), SMARTS, and SMIRKS can also be used. For example, Kemoinformatikkusu Yosoku to Sekkei no tameno Kagaku Jouhougaku [Chemoinformatics: Chemoinformatics for Prediction and Design], Maruzen, 2005, ISBN4-621-07552-7 can be referenced. The following is an example thereof.

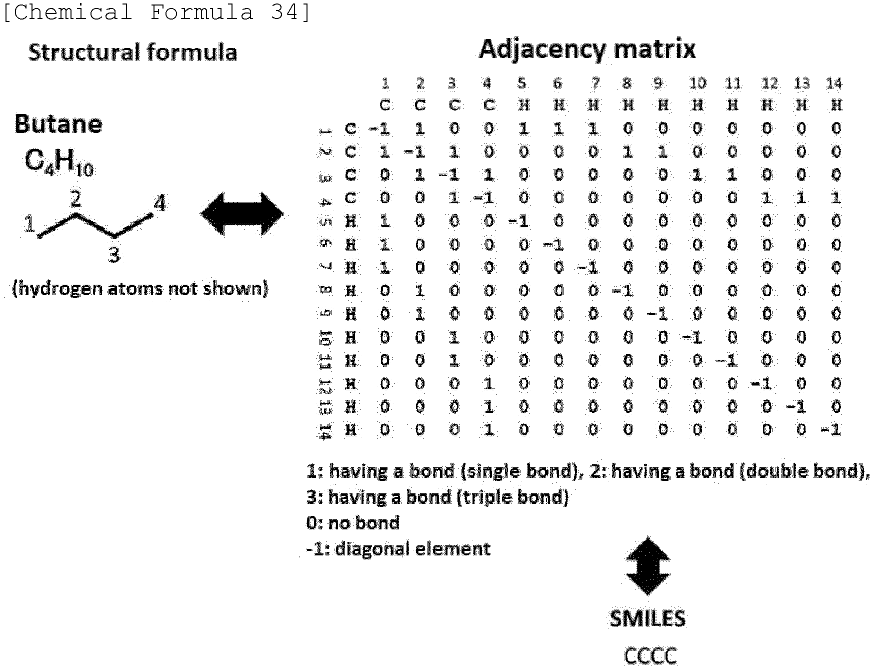

SMILES (simplified molecular input line entry system) is a method of denotation for converting a chemical structure of a molecule into ASCII code alphanumerical character strings without structural ambiguity. SMILES character strings can be imported into many types of molecule editors and represented as a two-dimensional diagram or three-dimensional model. In other words, SMILES is character strings expressing nodes (atoms) and edges (bonds) from a depth-first tree traversal of a chemical graph. A molecular graph is constructed by first removing hydrogen atoms of the system (except for the chiral center), breaking open formed cycles to turn it into a spanning tree, and including numerical suffix label for broken cycles to indicate connected nodes. Parentheses ("()") are used to indicate points of branching on the tree. Atoms are in brackets ("[]"), which may be omitted when the atoms are common atoms forming an organic compound (organic subset, i.e., B, C, N, O, P, S, F, Cl, Br, or I), have no formal charge, not require explicit designation of isotopes, and are chiral centers. In such a case, hydrogen is implied to be attached based on the valency. For instance, O and N are water and ammonium, respectively (water may be explicitly written as [H]O[H], but is hardly ever written in this manner). A formal charge is indicated by + or - followed by a number (e.g., [NH4+] for ammonium ion and [Fe+2] for iron (II)). Isotopes are explicitly specified with an integer mass preceding the atomic symbol (e.g., carbon-14 is [14C]). A bond is represented by -, =, #, and $, in order from a single bond (however, single bond - is generally omitted). The orientation of single bonds adjacent to double bonds = are represented by / or ¥ to distinguish cis-trans isomers. For example, C/C=C¥C and C/C=C/C are cis/trans-2-butane, respectively. Non-bond is indicated with . (e.g., hydrogen peroxide is OO, while 0.0 is two water molecules). In a cyclic structure, a number is labeled after an atom to be bound. For example, propane and cyclopropane are represented as CCC and C1CC1 in SMILES, respectively. A label of already formed ring with a pair may be reused. A label is deemed as a single digit number. For example, C12 is carbon attached to labels 1 and 2. A two-digit label is represented by preceding % (e.g., C%12 is label 12). Constituent atoms of an aromatic ring (not only carbon, but also nitrogen, oxygen, etc.) are in lower-case forms. For example, cyclohexane is C1CCCCC1, while benzene is c1ccccc1. Representation of bonds of an aromatic ring by single/double bonds (e.g., C1=CC=CC=C1) is known as Kekule form. The chiral centers are followed by @ or @@, representing that the subsequent atom group is arranged in a counter-clockwise or clockwise direction, respectively, when viewed from the direction of the base (because @ is counter-clockwise). For example, SMILES for S-alanine is N[C@@H](C)C(=O)O, with the amino group as the base (may be written as N[C@@]([H])(C)C(=O)O).

SMILES for a certain system is not necessarily uniquely determined. For example, S-alanine can be represented as not only the SMILES described above, but also as C[C@H](N)C(=O)O, C[C@@H](C(=O)O)N, OC(=O)[C@H](C)N, etc. For this reason, representations converted to be unique for a system based on an algorithm is termed canonical SMILES. However, algorithms may be different depending on the database or program. Chemical reactions are represented by reactant>>product or reactant>catalyst, etc.>product. For example, a reaction of adding water to propane to produce propan-2-ol is represented as CC=C.O>>CC(O)C.

As used herein, the term "group" refers to a monovalent group, unless especially noted otherwise. Examples of a group that is not a monovalent group include alkylene groups (divalent), etc. The term "group" may also be abbreviated in the following description of substituents, etc.

As used herein, the number of substituents when a group is defined as "optionally substituted" or "substituted" is not particularly limited as long as it is substitutable and is one or more, unless specifically limited. The description for each substituent is also applicable when the substituent is a part of or a substituent on another substituent, unless specifically noted otherwise.

As used herein, "maximum substitutable number" is the maximum number of substituents that a group can have. The number can vary for each group. For example, the number is 3 for a methyl group, 5 for an ethyl group, 7 for a benzyl group, and 11 for a naphthalenyl ethyl group.

For a group that is modified by "optionally substituted" or "substituted" herein, any portion of the group may be substituted. For example, "optionally substituted arylalkyl" and "substituted arylalkyl" may have the aryl moiety substituted, the alkyl moiety substituted, or both the aryl moiety and the alkyl moiety substituted.

As used herein, the substituent used when "optionally substituted" can be, for example, one or more of the same or different substituents in the substituent group. While the types of atoms within a substituent associated with attachment are not particularly limited by the type of substituent, if the atom to which a substituent attaches is an oxygen atom, a nitrogen atom, or a sulfur atom, the atom is limited to and selected from those with a poiont of attachment in the following substituents that is a carbon atom.

Substituent group consists of, for example, halogen, hydroxy, oxo, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureide, amidino, amidinoamino, unsubstituted or substituted alkyl, unsubstituted or substituted alkenyl, unsubstituted or substituted alkynyl, unsubstituted or substituted aryl, unsubstituted or substituted cycloalkyl, unsubstituted or substituted heteroaryl, unsubstituted or substituted heterocycloalkyl, unsubstituted or substituted alkyloxy, unsubstituted or substituted alkenyloxy, unsubstituted or substituted alkynyloxy, unsubstituted or substituted aryl-L_{X}-oxy, unsubstituted or substituted cycloalkyl-L_{X}-oxy, unsubstituted or substituted heteroaryl-L_{X}-oxy, unsubstituted or substituted heterocycloalkyl-L_{X}-oxy, unsubstituted or substituted alkyloxyalkyl, unsubstituted or substituted alkenyloxyalkyl, unsubstituted or substituted alkynyloxyalkyl, unsubstituted or substituted aryloxyalkyl, unsubstituted or substituted cycloalkyloxyalkyl, unsubstituted or substituted heteroaryloxyalkyl, unsubstituted or substituted heterocycloalkyloxyalkyl, unsubstituted or substituted alkyloxyalkyloxy, unsubstituted or substituted alkenyloxyalkyloxy, unsubstituted or substituted alkynyloxyalkyloxy, unsubstituted or substituted aryloxyalkyloxy, unsubstituted or substituted cycloalkyloxyalkyloxy, unsubstituted or substituted heteroaryloxyalkyloxy, unsubstituted or substituted heterocycloalkyloxyalkyloxy, unsubstituted or substituted alkylcarbonyl, unsubstituted or substituted alkenylcarbonyl, unsubstituted or substituted alkynylcarbonyl, unsubstituted or substituted aryl-L_{X}-carbonyl, unsubstituted or substituted cycloalkyl-L_{X}-carbonyl, unsubstituted or substituted heteroaryl-L_{X}-carbonyl, unsubstituted or substituted heterocycloalkyl-L_{X}-carbonyl, unsubstituted or substituted alkylcarbonyloxy, unsubstituted or substituted alkenylcarbonyloxy, unsubstituted or substituted alkynylcarbonyloxy, unsubstituted or substituted aryl-L_{X}-carbonyloxy, unsubstituted or substituted cycloalkyl-L_{X}-carbonyloxy, unsubstituted or substituted heteroaryl-L_{X}-carbonyloxy, unsubstituted or substituted heterocycloalkyl-L_{X}-carbonyloxy, unsubstituted or substituted alkylcarbonylamino, unsubstituted or substituted alkenylcarbonylamino, unsubstituted or substituted alkynylcarbonylamino, unsubstituted or substituted aryl-L_{X}-carbonylamino, unsubstituted or substituted cycloalkyl-L_{X}-carbonylamino, unsubstituted or substituted heteroaryl-L_{X}-carbonylamino, unsubstituted or substituted heterocycloalkyl-L_{X}-carbonylamino, unsubstituted or substituted alkylcarbonylthio, unsubstituted or substituted alkenylcarbonylthio, unsubstituted or substituted alkynylcarbonylthio, unsubstituted or substituted aryl-L_{X}-carbonylthio, unsubstituted or substituted cycloalkyl-L_{X}-carbonylthio, unsubstituted or substituted heteroaryl-L_{X}-carbonylthio, unsubstituted or substituted heterocycloalkyl-L_{X}-carbonylthio, unsubstituted or substituted alkylcarbonylimino, unsubstituted or substituted alkenylcarbonylimino, unsubstituted or substituted alkynylcarbonylimino, unsubstituted or substituted aryl-L_{X}-carbonylimino, unsubstituted or substituted cycloalkyl-L_{X}-carbonylimino, unsubstituted or substituted heteroaryl-L_{X}-carbonylimino, unsubstituted or substituted heterocycloalkyl-L_{X}-carbonylimino, unsubstituted or substituted alkylthio, unsubstituted or substituted alkenylthio, unsubstituted or substituted alkynylthio, unsubstituted or substituted aryl-L_{X}-thio, unsubstituted or substituted cycloalkyl-L_{X}-thio, unsubstituted or substituted heteroaryl-L_{X}-thio, unsubstituted or substituted heterocycloalkyl-L_{X}-thio, unsubstituted or substituted alkylamino, unsubstituted or substituted alkenylamino, unsubstituted or substituted alkynylamino, unsubstituted or substituted alkynylamino, unsubstituted or substituted aryl-L_{X}-amino, unsubstituted or substituted cycloalkyl-L_{X}-amino, unsubstituted or substituted heteroaryl-L_{X}-amino, unsubstituted or substituted heterocycloalkyl-L_{X}-amino, unsubstituted or substituted alkylsulfonyl, unsubstituted or substituted alkenylsulfonyl, unsubstituted or substituted alkynylsulfonyl, unsubstituted or substituted aryl-L_{X}-sulfonyl, unsubstituted or substituted cycloalkyl-L_{X}-sulfonyl, unsubstituted or substituted heteroaryl-L_{X}-sulfonyl, unsubstituted or substituted heterocycloalkyl-L_{X}-sulfonyl, unsubstituted or substituted alkylsulfonylamino, unsubstituted or substituted alkenylsulfonylamino, unsubstituted or substituted alkynylsulfonylamino, unsubstituted or substituted aryl-L_{X}-sulfonylamino, unsubstituted or substituted cycloalkyl-L_{X}-sulfonylamino, unsubstituted or substituted heteroaryl-L_{X}-sulfonylamino, unsubstituted or substituted heterocycloalkyl-L_{X}-sulfonylamino, unsubstituted or substituted alkylimino, unsubstituted or substituted alkenylimino, unsubstituted or substituted alkynylimino, unsubstituted or substituted aryl-L_{X}-imino, unsubstituted or substituted cycloalkyl-L_{X}-imino, unsubstituted or substituted heteroaryl-L_{X}-imino, unsubstituted or substituted heterocycloalkyl-L_{X}-imino, unsubstituted or substituted alkyloxyimino, unsubstituted or substituted alkenyloxyimino, unsubstituted or substituted alkynyloxyimino, unsubstituted or substituted aryl-L_{X}-oxyimino, unsubstituted or substituted cycloalkyl-L_{X}-oxyimino, unsubstituted or substituted heteroaryl-L_{X}-oxyimino, unsubstituted or substituted heterocycloalkyl-L_{X}-oxyimino, unsubstituted or substituted alkyloxycarbonyl, unsubstituted or substituted alkenyloxycarbonyl, unsubstituted or substituted alkynyloxycarbonyl, unsubstituted or substituted aryl-L_{X}-oxycarbonyl, unsubstituted or substituted cycloalkyl-L_{X}-oxycarbonyl, unsubstituted or substituted heteroaryl-L_{X}-oxycarbonyl, unsubstituted or substituted heterocycloalkyl-L_{X}-oxycarbonyl, unsubstituted or substituted alkyloxycarbonylamino, unsubstituted or substituted alkenyloxycarbonylamino, unsubstituted or substituted alkynyloxycarbonylamino, unsubstituted or substituted aryl-L_{X}-oxycarbonylamino, unsubstituted or substituted cycloalkyl-L_{X}-oxycarbonylamino, unsubstituted or substituted heteroaryl-L_{X}-oxycarbonylamino, unsubstituted or substituted heterocycloalkyl-L_{X}-oxycarbonylamino, unsubstituted or substituted alkylsulfanyl, unsubstituted or substituted alkenylsulfanyl, unsubstituted or substituted alkynylsulfanyl, unsubstituted or substituted aryl-L_{X}-sulfanyl, unsubstituted or substituted cycloalkyl-L_{X}-sulfanyl, unsubstituted or substituted heteroaryl-L_{X}-sulfanyl, unsubstituted or substituted heterocycloalkyl-L_{X}-sulfanyl, unsubstituted or substituted alkylsulfinyl, unsubstituted or substituted alkenylsulfinyl, unsubstituted or substituted alkynylsulfinyl, unsubstituted or substituted aryl-L_{X}-sulfinyl, unsubstituted or substituted cycloalkyl-L_{X}-sulfinyl, unsubstituted or substituted heteroaryl-L_{X}-sulfinyl, unsubstituted or substituted heterocycloalkyl-L_{X}-sulfinyl, unsubstituted or substituted alkylcarbamoyl, unsubstituted or substituted alkenylcarbamoyl, unsubstituted or substituted alkynylcarbamoyl, unsubstituted or substituted aryl-L_{X}-carbamoyl, unsubstituted or substituted cycloalkyl-L_{X}-carbamoyl, unsubstituted or substituted heteroaryl-L_{X}-carbamoyl, unsubstituted or substituted heterocycloalkyl-L_{X}-carbamoyl, unsubstituted or substituted alkylsulfamoyl, unsubstituted or substituted alkenylsulfamoyl, unsubstituted or substituted alkynylsulfamoyl, unsubstituted or substituted aryl-L_{X}-sulfamoyl, unsubstituted or substituted cycloalkyl-L_{X}-sulfamoyl, unsubstituted or substituted heteroaryl-L_{X}-sulfamoyl, and unsubstituted or substituted heterocycloalkyl-L_{X}-sulfamoyl, wherein L_{X} is a single bond or unsubstituted or substituted alkylene,
wherein the substituted alkyl, substituted alkenyl, substituted alkynyl, substituted aryl, substituted cycloalkyl, substituted heteroaryl, substituted heterocycloalkyl, and substituted alkylene moieties (fully or partially) in the substituent group each independently have one to the maximum substitutable number of the same or different substituents selected from the group consisting of halogen, hydroxy, oxo, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureide, amidino, amidinoamino, alkyl, alkenyl, alkynyl, aryl, cycloalkyl, heteroaryl, heterocycloalkyl, alkyloxy, alkenyloxy, alkynyloxy, aryl-L_{X}-oxy, cycloalkyl-L_{X}-oxy, heteroaryl-L_{X}-oxy, heterocycloalkyl-L_{X}-oxy, alkyloxyalkyl, alkenyloxyalkyl, alkynyloxyalkyl, aryloxyalkyl, cycloalkyloxyalkyl, heteroaryloxyalkyl, heterocycloalkyloxyalkyl, alkyloxyalkyloxy, alkenyloxyalkyloxy, alkynyloxyalkyloxy, aryloxyalkyloxy, cycloalkyloxyalkyloxy, heteroaryloxyalkyloxy, heterocycloalkyloxyalkyloxy, alkylcarbonyl, alkenylcarbonyl, alkynylcarbonyl, aryl-L_{X}-carbonyl, cycloalkyl-L_{X}-carbonyl, heteroaryl-L_{X}-carbonyl, heterocycloalkyl-L_{X}-carbonyl, alkylcarbonyloxy, alkenylcarbonyloxy, alkynylcarbonyloxy, aryl-L_{X}-carbonyloxy, cycloalkyl-L_{X}-carbonyloxy, heteroaryl-L_{X}-carbonyloxy, heterocycloalkyl-L_{X}-carbonyloxy, alkylcarbonylamino, alkenylcarbonylamino, alkynylcarbonylamino, aryl-L_{X}-carbonylamino, cycloalkyl-L_{X}-carbonylamino, heteroaryl-L_{X}-carbonylamino, heterocycloalkyl-L_{X}-carbonylamino, alkylcarbonylthio, alkenylcarbonylthio, alkynylcarbonylthio, aryl-L_{X}-carbonylthio, cycloalkyl-L_{X}-carbonylthio, heteroaryl-L_{X}-carbonylthio, heterocycloalkyl-L_{X}-carbonylthio, alkylcarbonylimino, alkenylcarbonylimino, alkynylcarbonylimino, aryl-L_{X}-carbonylimino, cycloalkyl-L_{X}-carbonylimino, heteroaryl-L_{X}-carbonylimino, heterocycloalkyl-L_{X}-carbonylimino, alkylthio, alkenylthio, alkynylthio, aryl-L_{X}-thio, cycloalkyl-L_{X}-thio, heteroaryl-L_{X}-thio, heterocycloalkyl-L_{X}-thio, alkylamino, alkenylamino, alkynylamino, alkynylamino, aryl-L_{X}-amino, cycloalkyl-L_{X}-amino, heteroaryl-L_{X}-amino, heterocycloalkyl-L_{X}-amino, alkylsulfonyl, alkenylsulfonyl, alkynylsulfonyl, aryl-L_{X}-sulfonyl, cycloalkyl-L_{X}-sulfonyl, heteroaryl-L_{X}-sulfonyl, heterocycloalkyl-L_{X}-sulfonyl, alkylsulfonylamino, alkenylsulfonylamino, alkynylsulfonylamino, aryl-L_{X}-sulfonylamino, cycloalkyl-L_{X}-sulfonylamino, heteroaryl-L_{X}-sulfonylamino, heterocycloalkyl-L_{X}-sulfonylamino, alkylimino, alkenylimino, alkynylimino, aryl-L_{X}-imino, cycloalkyl-L_{X}-imino, heteroaryl-L_{X}-imino, heterocycloalkyl-L_{X}-imino, alkyloxyimino, alkenyloxyimino, alkynyloxyimino, aryl-L_{X}-oxyimino, cycloalkyl-L_{X}-oxyimino, heteroaryl-L_{X}-oxyimino, heterocycloalkyl-L_{X}-oxyimino, alkyloxycarbonyl, alkenyloxycarbonyl, alkynyloxycarbonyl, aryl-L_{X}-oxycarbonyl, cycloalkyl-L_{X}-oxycarbonyl, heteroaryl-L_{X}-oxycarbonyl, heterocycloalkyl-L_{X}-oxycarbonyl, alkyloxycarbonylamino, alkenyloxycarbonylamino, alkynyloxycarbonylamino, aryl-L_{X}-oxycarbonylamino, cycloalkyl-L_{X}-oxycarbonylamino, heteroaryl-L_{X}-oxycarbonylamino, heterocycloalkyl-L_{X}-oxycarbonylamino, alkylsulfanyl, alkenylsulfanyl, alkynylsulfanyl, aryl-L_{X}-sulfanyl, cycloalkyl-L_{X}-sulfanyl, heteroaryl-L_{X}-sulfanyl, heterocycloalkyl-L_{X}-sulfanyl, alkylsulfinyl, alkenylsulfinyl, alkynylsulfinyl, aryl-L_{X}-sulfinyl, cycloalkyl-L_{X}-sulfinyl, heteroaryl-L_{X}-sulfinyl, heterocycloalkyl-L_{X}-sulfinyl, alkylcarbamoyl, alkenylcarbamoyl, alkynylcarbamoyl, aryl-L_{X}-carbamoyl, cycloalkyl-L_{X}-carbamoyl, heteroaryl-L_{X}-carbamoyl, heterocycloalkyl-L_{X}-carbamoyl, alkylsulfamoyl, alkenylsulfamoyl, alkynylsulfamoyl, aryl-L_{X}-sulfamoyl, cycloalkyl-L_{X}-sulfamoyl, heteroaryl-L_{X}-sulfamoyl, and heterocycloalkyl-L_{X}-sulfamoyl.

In an exemplary embodiment, a hydroxyl group in the aforementioned substituent group may also be substituted with a silyl protecting group. In an exemplary embodiment, an amino group in the substituent group described above may also be further protected with a nitrogen protecting group.

As used herein, "C₁₋₆" means that the number of carbon atoms is 1 to 6. The same applies to other numbers. For example, "C₁₋₄" means that the number of carbon atoms is 1 to 4, and "C₁₋₃" means that the number of carbon atoms is 1 to 3. A description with a limitation in the number of carbons herein is only a preferred numerical range. It is intended so that groups with a substituent with a number of carbons other than the number of carbons specified in the present disclosure are also within the scope of the present disclosure.

As used herein, "hydrocarbon group" is also referred to as a hydrocarbyl group, referring to a group generated by removing at least one hydrogen from "hydrocarbon" comprising at least one carbon and at least one hydrogen.

As used herein, "functional group" refers to any group conferring some type of functionality, encompassing a carboxyl group, nitrile group, carbonyl group, hydroxyl group, amino group, imino group, nitro group, halogen group, as well as alkyl group, and more broadly acid anhydrides and groups formed by a bond such as an ester bond, amide bond, or ether bond.

As used herein, "heteroatom" refers to atoms other than carbon atoms and hydrogen atoms such as oxygen atoms, nitrogen atoms, and sulfur atoms. A group comprising a heteroatom is also known as a hetero ... group (e.g., heteroaryl group (means that an aryl group comprises at least a heteroatom), heterocyclic group (means that a cyclic group (carbocyclic group) comprises at least one heteroatom)), etc.

As used herein, "halogen atom" is an atom belonging to the halogen group, referring to a fluorine atom, chlorine atom, bromine atom, iodine atom, etc., and is preferably a fluorine atom or chlorine atom. A "halogen atom" is also referred to as "halogen" or "halo".

As used herein, "hydroxyl group" is a monovalent group of -OH. This group is also referred to as a "hydroxy group" or "hydroxy".

As used herein, "carboxyl group" is a monovalent group of -COOH. This group is also referred to as a "carboxy group", "carboxy", or "carboxyl".

As used herein, "cyano group" is a monovalent group of -CN.

As used herein, "amino" is a monovalent group of -NH₂. This group is also referred to as an "amino group".

As used herein, "alkyl" refers to a linear or branched saturated aliphatic hydrocarbon group. "C₁₋₁₂ alkyl" is an alkyl group with 1 to 12 carbon atoms. Examples thereof include, but are not limited to, C₁₋₆ alkyl, heptyl, isoheptyl, octyl, isooctyl, nonyl, isononyl, decyl, isodecyl, undecyl, isoundecyl, dodecyl, isododecy, etc. "C₁₋₁₂ alkyl" is an alkyl group with 1 to 12 carbon atoms. "C₁₋₆ alkyl" is an alkyl group with 1 to 6 carbon atoms, which is preferably "C₁₋₄ alkyl", more preferably "C₁₋₃ alkyl", and still more preferably "C₁₋₂ alkyl". Specific examples of "C₁₋₄ alkyl" include methyl, ethyl, n-propyl, isopropyl, n-butyl, isobutyl, tert-butyl, sec-butyl, etc. Specific examples of "C₁₋₆ alkyl" include, but are not limited to, C₁₋₄ alkyl, n-pentyl, isopentyl, neopentyl, tert-pentyl, 1,2-dimethylpropyl, n-hexyl, etc. Specific examples of "C₁₋₁₂ alkyl" include, but are not limited to, C₁₋₆ alkyl, n-heptyl, isoheptyl, n-octyl, isooctyl, n-nonyl, n-decanyl, n-undecyl, n-dodecyl, etc.

As used herein, "alkenyl" refers to a linear or branched unsaturated aliphatic hydrocarbon group comprising at least one carbon-carbon double bond. "C₂₋₁₂ alkenyl" is an alkenyl group with 2 to 12 carbon atoms. Examples thereof include, but are not limited to, heptenyl, isoheptenyl, octenyl, isooctenyl, nonenyl, isononenyl, decenyl, isodecenyl, undecenyl, isoundecenyl, dodecenyl, isododecenyl, etc. "C₂₋₆ alkenyl" is an alkenyl group with 2 to 6 carbon atoms. Preferred examples thereof include "C₂₋₄ alkenyl". Specific examples of "C₂₋₆ alkenyl" include, but are not limited to, vinyl, 1-propenyl, 2-propenyl, 1-butenyl, 2-butenyl, 3-butenyl, 2-methyl-1-propenyl, 2-methyl-2-propenyl, etc.

As used herein, "alkynyl" refers to a linear or branched unsaturated aliphatic hydrocarbon group comprising at least one carbon-carbon triple bond. "C₂₋₁₂ alkynyl" is an alkynyl group with 2 to 12 carbon atoms. Examples thereof include, but are not limited to, heptynyl, isoheptynyl, octynyl, isooctynyl, nonynyl, isononynyl, decynyl, isodecynyl, undecynyl, isoundecynyl, dodecynyl, isododecynyl, etc. "C₂₋₆ alkynyl" is an alkynyl group with 2 to 6 carbon atoms. Preferred examples thereof include "C₂₋₄ alkynyl". Specific examples of "C₂₋₆ alkynyl" include, but are not limited to, ethynyl, 1-propynyl, 2-propynyl, 1-butynyl, 1-methyl-2-propynyl, 3-butynyl, 1-pentynyl, 1-hexynyl, etc.

As used herein, "aryl" refers to a monovalent group of a monocyclic or bicyclic aromatic hydrocarbon ring. "C₆₋₁₀ aryl" refers to an aryl group with 6 to 10 carbon atoms. Examples of "aryl" include, but are not limited to, C₆ aryl, C₁₀ aryl, etc. Specific examples of C₆ aryl include, but are not limited to, phenyl, etc. Specific examples of C₁₀ aryl include, but are not limited to, 1-naphthyl, 2-naphthyl, etc.

An aryl group as a substituent or a portion thereof may be fused to an alicyclic group. For example, a phenyl group may be fused to a cyclohexane ring to form a 1,2,3,4-tetrahydronaphthalenyl group. In such a case, one of the possible carbon atoms on a benzene ring attaches to the backbone, or to a group near the backbone, or to its atom. An aryl group encompasses 5,6,7,8-tetrahydronaphthalen-1-yl and 5,6,7,8-tetrahydronaphthalen-2-yl.

As used herein, "arylalkyl" refers to alkyl substituted with at least one aryl. "C₆₋₁₀ aryl C₁₋₆ alkyl" refers to C₁₋₆ alkyl substituted with at least one C₆₋₁₀ aryl. Specific examples of C₆₋₁₀ aryl C₁₋₆ alkyl include, but are not limited to, benzyl (i.e., phenyl-CH₂-), phenethyl (i.e., phenyl-CH₂CH₂-), 1-phenylethyl, naphthalen-1-ylmethyl, naphthalen-2-ylmethyl, 2-(naphthalen-1-yl)ethyl, 2-(naphthalen-2-yl)ethyl, 1-(naphthalen-1-yl)ethyl, 1-(naphthalen-2-yl)ethyl, etc.

As used herein, "(optionally substituted amino)-arylalkyl" refers to arylalkyl substituted with an optionally substituted amino group, wherein the alkyl group, the aryl group, or both is substituted with an amino group. An amino group of such an arylalkyl group may be unsubstituted, or substituted with 1, 2, or 3 substituents, such as optionally substituted alkyl (e.g., unsubstituted C₁₋₆ alkyl, C₃₋₆ cycloalkyl-C₁₋₆ alkyl, C₃₋₆ cycloalkylcarbonyl, etc.).

Examples of (optionally substituted amino)-C₆₋₁₀ aryl C₁₋₆ alkyl include, but are not limited to, (di(alkyl)amino)benzyl, ((cycloalkylalkyl)amino)benzyl, ((cycloalkylcarbonyl)amino)benzyl, ((carbamoylalkyl)carbonylamino)benzyl, ((carboxyalkyl)carbonyl)aminobenzyl, (di(alkyl)amino)naphthalenylmethyl, ((cycloalkylalkyl)amino)naphthalenylmethyl, ((cycloalkylcarbonyl)amino)naphthalenylmethyl, ((carbamoylalkyl)carbonylamino)naphthalenylmethyl, ((carboxyalkyl)carbonyl)aminonaphthalenylmethyl, etc.

As used herein, the aryl moiety of "arylthio" is defined the same as the aryl described above. Preferred examples of "C₆₋₁₀ arylthio" include "C₆ or C₁₀ arylthio". Specific examples of "C₆₋₁₀ aryloxy" include, but are not limited to, phenylthio, 1-naphthylthio, 2-naphthylthio, etc.

As used herein, "aryl sulfonyl" refers to sulfonyl substituted with the "aryl" described above. "C₆₋₁₀ aryl sulfonyl" is preferably "C₆ or C₁₀ aryl sulfonyl". Specific examples of "C₆₋₁₀ aryl sulfonyl" include, but are not limited to, phenylsulfonyl, 1-naphthylsulfonyl, 2-naphthylsulfonyl, etc.

As used herein, "heteroaryl" refers to a monovalent group of a monocyclic or bicyclic aromatic heterocycle comprising 1 to 4 same or different heteroatoms selected from the group consisting of an oxygen atom, nitrogen atom, and sulfur atom.

As used herein, "5- to 6-membered heteroaryl" refers to a monovalent group of a monocyclic aromatic heterocycle consisting of 5 to 6 atoms, comprising 1 to 4 same or different heteroatoms selected from the group consisting of an oxygen atom, nitrogen atom, and sulfur atom. Specific examples of "5- to 6-membered heteroaryl" include, but are not limited to, pyrrolyl, furyl, thienyl, pyrazolyl, imidazolyl, oxazolyl, isoxazolyl, oxadiazolyl, thiazolyl, isothiazolyl, thiadiazolyl, tetrazolyl, pyridyl, furyl, pyridazinyl, pyrimidinyl, pyrazinyl, etc.

As used herein, "5- to 10-membered heteroaryl" refers to a monovalent group of a monocyclic or bicyclic aromatic heterocycle consisting of 5 to 10 atoms, comprising 1 to 4 same or different heteroatoms selected from the group consisting of an oxygen atom, nitrogen atom, and sulfur atom. Specific examples of "5- to 10-membered heteroaryl" include, but are not limited to, 5- or 6-membered heteroaryl, quinolyl, isoquinolyl, naphthyridinyl, quinoxalinyl, cinnolinyl, quinazolinyl, phthalazinyl, imidazopyridyl, imidazothiazolyl, imidazooxazolyl, benzothiazolyl, benzoxazolyl, benzoimidazolyl, indolyl, isoindolyl, indazolyl, pyrrolopyridyl, thienopyridyl, furopyridyl, benzothiadiazolyl, benzoxadiazolyl, pyridopyrimidinyl, benzofuryl, benzothienyl, benzo[1,3]dioxole, thienofuryl, chromenyl, chromanyl, coumarinyl, quinolonyl, etc.

As used herein, "heteroarylalkyl" refers to alkyl substituted with at least one heteroaryl. "5- to 10-membered heteroaryl C₁₋₆ alkyl" refers to C₁₋₆ alkyl substituted with at least one 5- to 10-membered heteroaryl. Specific examples of 5- to 10-membered heteroaryl C₁₋₆ alkyl include, but are not limited to, pyridin-2-ylmethyl, pyridin-4-ylmethyl, 2-(quinolin-8-yl)ethyl, 2-(quinolin-5-yl)ethyl, 2-(quinoxalin-5-yl)ethyl, 1H-indol-3-ylmethyl, 2-(1H-indol-3-yl)ethyl, etc.

As used herein, "alicyclic group" refers to a monovalent group of a monocyclic, bicyclic, or tricyclic non-aromatic hydrocarbon ring, including those that have a partially unsaturated bond, those that have a partially crosslinked structure, those that are partially a spiro, and those having 1, 2, or more carbonyl structures. An "alicyclic group" encompasses cycloalkyl, cycloalkenyl, and cycloalkynyl. "C₃₋₂₀ alicyclic group" is preferably a "C₃₋₁₀ alicyclic group", more preferably a "C₃₋₆ alicyclic group". Specific examples of "C₃₋₂₀ alicyclic group" include, but are not limited to, cyclopropyl, cyclobutyl, cyclopentyl, cyclohexyl, cycloheptyl, cyclooctyl, cyclohexadinyl, cycloheptadinyl, cyclooctadinyl, adamantyl, norbornyl, etc.

An alicyclic group may be a fused ring between a non-aryl ring and an aryl and/or heteroaryl ring. For example, cycloalkyl fused with C₆₋₁₀ aryl or 5- to 6-membered heteroaryl is encompassed by an alicyclic group. Examples of fused alicyclic groups include a monovalent group with one hydrogen atom removed from 1,2,3,4-tetrahydronaphthalene, indane, 1,2,3,4-tetrahydroanthracene, and 5,6,7,8-tetrahydroquinoline, etc. Specific examples thereof include 1,2,3,4-tetrahydronaphthalen-1-yl, 1,2,3,4-tetrahydronaphthalen-2-yl, indan-1-yl, indan-2-yl, 5,6,7,8-tetrahydroquinolin-5-yl, 5,6,7,8-tetrahydroquinolin-6-yl, etc. A fused alicyclic group attaches from any one of the possible cyclic structure atoms on a non-aryl ring to the backbone.

As used herein, "C₃₋₁₀ alicyclic group" refers to a substituent in which "C₃₋₁₀ alicyclic group" is a monovalent group among the aforementioned "C₃₋₂₀ alicyclic group".

As used herein, "alicyclic oxy" refers to an (alicyclic group)-O- group, and the alicyclic moiety is defined the same as an alicyclic group. "C₃₋₆ alicyclic oxy" refers to a (C₃₋₆ alicyclic group) -O- group, and the C₃₋₆ alicyclic moiety is defined the same as a C₃₋₆ alicyclic group. "C₃₋₆ alicyclic oxy" is preferably "C₃₋₅ alicyclic oxy". Specific examples of "C₃₋₆ alicyclic oxy" include, but are not limited to, cyclopropyloxy, cyclobutyloxy, cyclopentyloxy, cyclohexyloxy, etc.

As used herein, "alicyclic carbonyl" refers to carbonyl substituted with the "alicyclic group" described above. "C₃₋₁₀ alicyclic carbonyl" is preferably "C₃₋₆ alicyclic carbonyl". Specific examples of "C₃₋₁₀ alicyclic carbonyl" include, but are not limited to, cyclopropylcarbonyl, cyclobutylcarbonyl, cyclopentylcarbonyl, cyclohexylcarbonyl, etc.

As used herein, "alicyclic thio" refers to an (alicyclic group)-S- group, and the alicyclic moiety is defined the same as above. "C₃₋₁₀ alicyclic thio" is preferably "C₃₋₆ alicyclic thio". Specific examples of "C₃₋₆ alicyclic thio" include, but are not limited to, cyclopropylthio, cyclobutylthio, cyclopentylthio, cyclohexylthio, etc.

As used herein, "alicyclic sulfonyl" refers to a sulfonyl group substituted with the "alicyclic group" described above. "C₃₋₁₀ alicyclic sulfonyl" is preferably "C₃₋₆ alicyclic sulfonyl". Specific examples of "C₃₋₁₀ alicyclic sulfonyl" include, but are not limited to, cyclopropylsulfonyl, cyclobutylsulfonyl, cyclopentylsulfonyl, cyclohexylsulfonyl, etc.

As used herein, "cycloalkyl" refers to a non-aromatic saturated hydrocarbon ring group, including those that have a partially crosslinked structure, those that are partially spiro, and those having 1, 2, or more carbonyl structures. "C₃₋₂₀ cycloalkyl" refers to monocyclic or bicyclic cycloalkyl with 3 to 20 carbon atoms. "C₃₋₆ cycloalkyl" refers to monocyclic cycloalkyl with 3 to 6 carbon atoms. Specific examples of C₃₋₆ cycloalkyl include, but are not limited to, cyclopropyl, cyclobutyl, cyclopentyl, and cyclohexyl. "C₃₋₁₀ cycloalkyl" refers to a monocyclic or bicyclic cycloalkyl with 3 to 10 carbon atoms. Specific examples of C₃₋₁₀ cycloalkyl include, but are not limited to, C₃₋₆ cycloalkyl, cycloheptyl, cyclooctyl, cyclononyl, cyclodecyl, bicyclo[4.1.0]heptyl, bicyclo[3.3.0]octyl, bicyclo[4.2.0]octyl, bicyclo[4.3.0]nonyl, decahydronaphthyl, etc.

A cycloalkyl group may be fused to aryl and/or heteroaryl ring as a substituent or a portion thereof. For example, a cyclohexyl group may be fused to a benzene ring to form a 1,2,3,4-tetrahydronaphthalenyl group. In such a case, one of the possible carbon atoms on the cyclohexane ring attaches to the backbone, to a group near the backbone, or to its atom. A cycloalkyl group encompasses 1,2,3,4-tetrahydronaphthalen-1-yl, 1,2,3,4-tetrahydronaphthalen-2-yl, indan-1-yl, indan-2-yl, 5,6,7,8-tetrahydroquinolin-5-yl, and 5,6,7,8-tetrahydroquinolin-6-yl.

As used herein, "cycloalkylalkyl" refers to alkyl substituted with at least one cycloalkyl. "C₃₋₁₀ cycloalkyl C₁₋₆ alkyl" refers to C₁₋₆ alkyl substituted with at least one C₃₋₁₀ cycloalkyl, and "C₃₋₆ cycloalkyl C₁₋₆ alkyl" refers to C₁₋₆ alkyl substituted with at least one C₃₋₆ cycloalkyl. Specific examples of C₃₋₆ cycloalkyl C₁₋₆ alkyl include, but are not limited to, cyclopropylmethyl, cyclobutylmethyl, cyclopentylmethyl, cyclohexylmethyl, 2-cyclopropylethyl, 2-cyclobutylethyl, 2-cyclopentylethyl, 2-cyclohexylethyl, 3-cyclopropylpropyl, 3-cyclobutylpropyl, 3-cyclopentylpropyl, 3-cyclohexylpropyl, etc. Specific examples of C₃₋₁₀ cycloalkyl C₁₋₆ alkyl include, but are not limited to, C₃₋₆ cycloalkylmethyl, C₃₋₆ cycloalkylethyl, cycloheptylmethyl, cycloheptylethyl, cyclooctylmethyl, cyclooctylethyl, cyclononylmethyl, cyclononylethyl, cyclodecylmethyl, cyclodecylethyl, etc.

As used herein, "heterocycloalkyl" refers to a non-aromatic saturated or partially unsaturated heterocycle comprised of 3 or more atoms, comprising 1, 2, or more same or different heteroatoms selected from the group consisting of an oxygen atom, nitrogen atom, and sulfur atom, including those that have a partially crosslinked structure and those that are partially spiro. "Heterocycloalkyl" encompasses "non-aryl heterocycle". Heterocycloalkyl can have a structure where a non-aromatic heterocycle is fused to an aryl ring and/or heteroaryl ring.

As used herein, "non-aryl heterocycle" refers to a monocyclic or bicyclic non-aromatic heterocycle comprised of 3 or more atoms, comprising 1, 2, or more same or different heteroatoms selected from the group consisting of an oxygen atom, nitrogen atom, and sulfur atom, including saturated non-aryl heterocycles, those that have a partially unsaturated attachment, those that have a partially crosslinked structure, and those that are partially spiro. A non-aryl heterocycle may form a fused ring with aryl or heteroaryl. For example, a non-aryl heterocycle fused to C₆₋₁₀ aryl or 5- to 6-membered heteroaryl is also encompassed by a heterocycle. 1, 2, or more carbonyl, thiocarbonyl, sulfinyl, or sulfonyl may be comprised to constitute the non-aryl heterocycle. For example, lactam, thiolactam, lactone, thiolactone, cyclic imide, cyclic carbamate, cyclic thiocarbamate, and other cyclic groups are also encompassed by the non-aryl heterocycle. In this regard, an oxygen atom of carbonyl, sulfinyl, and sulfonyl and a sulfur atom of thiocarbonyl are not included in the number of members of the ring (ring size) or the number of heteroatoms constituting the ring.

As used herein, "4- to 10-membered non-aryl heterocycle" refers to a substituent wherein a"4- to 10-membered non-aryl heterocycle" is a monovalent group among the "non-aryl heterocycle" described above.

As used herein, the non-aryl heterocycle moiety of "non-aryl heterocyclyloxy" is defined the same as the "4- to 10-membered non-aryl heterocycle" described above. "4- to 10-membered non-aryl heterocyclyloxy" is preferably "4- to 6-membered non-aryl heterocyclyloxy". Specific examples of "4- to 10-membered non-aryl heterocyclyloxy" include, but are not limited to, tetrahydrofuranyloxy, tetrahydropyranyloxy, azetidinyloxy, pyrrolidinyloxy, piperidinyloxy, etc.

As used herein, the non-aryl heterocycle moiety of "non-aryl heterocyclylthio" is defined the same as the "non-aryl heterocycle" described above. "4- to 10-membered non-aryl heterocyclylthio" is preferably "4- to 6-membered non-aryl heterocyclylthio". Specific examples of "4- to 10-membered non-aryl heterocyclylthio" include, but are not limited to, tetrahydropyranylthio, piperidinylthio, etc.

As used herein, "non-aryl heterocyclylcarbonyl" refers to a carbonyl group substituted with the "non-aryl heterocycle" described above. "4- to 10-membered non-aryl heterocyclylcarbonyl" is preferably "4- to 6-membered non-aryl heterocyclylcarbonyl". Specific examples of "4- to 10-membered non-aryl heterocyclylcarbonyl" include, but are not limited to, azetidinylcarbonyl, pyrrolidinylcarbonyl, piperidinylcarbonyl, morpholinylcarbonyl, etc.

As used herein, "non-aryl heterocyclylsulfonyl" refers to a sulfonyl group substituted with the "non-aryl heterocycle" described above. "4- to 10-membered non-aryl heterocyclylsulfonyl" is preferably "4- to 6-membered non-aryl heterocyclylsulfonyl". Specific examples of "4- to 10-membered non-aryl heterocyclylsulfonyl" include, but are not limited to, azetidinylsulfonyl, pyrrolidinylsulfonyl, piperidinylsulfonyl, morpholinylsulfonyl, etc.

As used herein, "5- or 6-membered heterocycloalkyl" and "5- to 6-membered heterocycloalkyl" refers to heterocycloalkyl comprised of 5 to 6 cyclic atoms, comprising 1, 2, or more same or different heteroatoms selected from the group consisting of an oxygen atom, nitrogen atom, and sulfur atom.

As used herein, "heterocycloalkylalkyl" refers to alkyl substituted with at least one heterocycloalkyl.

As used herein, "alkylcarbonyl" is a monovalent group of -C(=O)-alkyl. Preferred examples of alkylcarbonyl include C₁₋₆ alkylcarbonyl. Specific examples of C₁₋₆ alkylcarbonyl include, but are not limited to, acetyl (CH₃C(=O)-), n-propanoyl (CH₃CH₂C(=O)-), n-butanoyl (CH₃CH₂CH₂C(=O)-), n-pentanoyl (CH₃(CH₂)₃C(=O)-), n-hexanoyl (CH₃(CH₂)₄C(=O)-), n-heptanoyl (CH₃(CH₂)₅C(=O)-), etc.

As used herein, "alkoxy" is a monovalent group of -O-alkyl. Preferred examples of alkoxy include C₁₋₆ alkoxy (i.e., C₁₋₆ alkyl-O-), C₁₋₄ alkoxy (i.e., C₁₋₄ alkyl-O-), etc. Specific examples of C₁₋₄ alkoxy include methoxy (CH₃O-), ethoxy (CH₃CH₂O-), n-propoxy (CH₃(CH₂)₂O-), isopropoxy ((CH₃)₂CHO-), n-butoxy (CH₃(CH₂)₃O-), isobutoxy ((CH₃)₂CHCH₂O-), tert-butoxy ((CH₃)₃CO-), sec-butoxy (CH₃CH₂CH(CH₃)O-), etc. Specific examples of C₁₋₆ alkoxy include, but are not limited to, C₁₋₄ alkoxy, n-pentyloxy (CH₃(CH₂)₄O-), isopentyloxy ((CH₃)₂CHCH₂CH₂O-), neopentyloxy ((CH₃)₃CCH₂O-), tert-pentyloxy (CH₃CH₂C(CH₃)₂O-), 1,2-dimethylpropoxy (CH₃CH(CH₃)CH(CH₃)O-), etc.

As used herein, "alkoxycarbonyl" is a monovalent group of -C(=O)-O-alkyl. Examples of alkoxycarbonyl include, but are not limited to, C₁₋₆ alkoxycarbonyl, preferably C₁₋₄ alkoxycarbonyl. Specific examples of C₁₋₄ alkoxycarbonyl include methoxycarbonyl, ethoxycarbonyl, n-propoxycarbonyl, isopropoxycarbonyl, n-butoxycarbonyl, sec-butoxycarbonyl, tert-butoxycarbonyl, isobutoxycarbonyl, etc. Specific examples of C₁₋₆ alkoxycarbonyl include, but are not limited to, C₁₋₄ alkoxycarbonyl, n-pentyloxycarbonyl, isopentyloxycarbonyl, neopentyloxycarbonyl, tert-pentyloxycarbonyl, 1,2-dimethylpropyloxycarbonyl, n-hexyloxycarbonyl, etc.

As used herein, "alkoxycarbonylamino" is a monovalent group of -NH-C(=O)-O-alkyl. Examples of alkoxycarbonylamino include, but are not limited to, C₁₋₆ alkoxycarbonylamino, preferably C₁₋₄ alkoxycarbonylamino. Specific examples of C₁₋₄ alkoxycarbonylamino include methoxycarbonylamino, ethoxycarbonylamino, n-propoxycarbonylamino, isopropoxycarbonylamino, n-butoxycarbonylamino, sec-butoxycarbonylamino, tert-butoxycarbonylamino, isobutoxycarbonylamino, etc. Specific examples of C₁₋₆ alkoxycarbonylamino include, but are not limited to, C₁₋₄ alkoxycarbonylamino, n-pentyloxycarbonylamino, isopentyloxycarbonylamino, neopentyloxycarbonylamino, tert-pentyloxycarbonylamino, 1,2-dimethylpropyloxycarbonylamino, n-hexyloxycarbonylamino, etc.

As used herein, "haloalkyl" is a monovalent group of halogenated alkyl, having one or more hydrogen on an alkyl group substituted with halogen. The term "perhaloalkyl" refers to haloalkyl with all hydrogen on the alkyl group substituted with halogen. For example, perfluoroethyl is - CF₂CF₃, and perchloro-n-propyl is -CCl₂CCl₂CCl₃. Examples of haloalkyl include C₁₋₆ haloalkyl, C₁₋₄ haloalkyl, C₁₋₃ haloalkyl, etc. Specific examples of C₁₋₃ alkyl include, but are not limited to, fluoromethyl, chloromethyl, bromomethyl, difluoromethyl, dichloromethyl, dibromomethyl, trifluoromethyl, trichloromethyl, tribromomethyl, fluorochloromethyl, difluorochloromethyl, fluorodichloromethyl, fluoroethyl, chloroethyl, bromoethyl, trifluoroethyl, trichloroethyl, tribromoethyl, perfluoroethyl, perchloroethyl, perbromoethyl, perfluoropropyl, perchloropropyl, perbromopropyl, perfluoroisopropyl, perchloroisopropyl, perbromoisopropyl, etc. Specific examples of C₁₋₄ alkyl include, but are not limited to, C₁₋₃ haloalkyl, perfluorobutyl, perchlorobutyl, perbromobutyl, perfluoroisobutyl, perfluoro-t-butyl, etc. Specific examples of C₁₋₆ alkyl include, but are not limited to, C₁₋₄ haloalkyl, perfluoro-n-pentyl, perfluoroisopentyl, perfluoroneopentyl, perfluoro-tert-pentyl, perfluoro-1,2-dimethylpropyl, etc.

As used herein, "haloalkoxy" as well as "haloalkyloxy" is a monovalent group of -O-haloalkyl with one or more hydrogen on the alkyl group substituted with halogen. The term "perhaloalkoxy" refers to haloalkoxy with all hydrogen on the alkyl group substituted with halogen. For example, perfluoroethoxy is -OCF₂CF₃, and perchloro-n-propoxy is - OCCl₂CCl₂CCl₃. Preferred examples of haloalkoxy include C₁₋₆ haloalkoxy, C₁₋₄ haloalkoxy, C₁₋₃ haloalkoxy, etc. Specific examples of C₁₋₃ alkoxy include, but are not limited to, fluoromethoxy, chloromethoxy, bromomethoxy, difluoromethoxy, dichloromethoxy, dibromomethoxy, trifluoromethoxy, trichloromethoxy, tribromomethoxy, fluorochloromethoxy, difluorochloromethoxy, fluorodichloromethoxy, fluoroethoxy, chloroethoxy, bromoethoxy, trifluoroethoxy, trichloroethoxy, tribromoethoxy, perfluoroethoxy, perchloroethoxy, perbromoethoxy, perfluoropropoxy, perchloropropoxy, perbromopropoxy, perfluoroisopropoxy, perchloroisopropoxy, perbromoisopropoxy, etc. Specific examples of C₁₋₄ alkoxy include, but are not limited to, C₁₋₃ haloalkoxy, perfluorobutoxy, perchlorobutoxy, perbromobutoxy, perfluoroisobutoxy, perfluoro-t-butoxy, etc. Specific examples of C₁₋₆ alkoxy include, but are not limited to, C₁₋₄ haloalkoxy, perfluoro-n-pentyloxy, perfluoroisopentyloxy, perfluoroneopentyloxy, perfluoro-tert-pentyloxy, perfluoro-1,2-dimethylpropoxy, etc.

As used herein, "alkylsulfonyl" refers to a sulfonyl group substituted with the "alkyl" described above. "C₁₋₆ alkylsulfonyl" is preferably "C₁₋₄ alkylsulfonyl". Specific examples of "C₁₋₆ alkylsulfonyl" include, but are not limited to, methylsulfonyl, propionylsulfonyl, butyrylsulfonyl, etc.

As used herein, the alkyl moiety of "alkylthio" is defined the same as the alkyl described above. Examples of "C₁₋₆ alkylthio" include "C₁₋₄ alkylthio", and preferred examples thereof include "C₁₋₃ alkylthio". Specific examples of "C₁₋₆ alkylthio" include, but are not limited to, methylthio, ethylthio, n-propylthio, n-butylthio, isopropylthio, isobutylthio, tert-butylthio, sec-butylthio, isopentylthio, neopentylthio, tert-pentylthio, 1,2-dimethylpropylthio, etc.

As used herein, "arylcarbonyl" is a monovalent group of -C(=O)-aryl. Preferred examples of arylcarbonyl include C₆₋₁₀ arylcarbonyl. Specific examples of C₆₋₁₀ arylcarbonyl include, but are not limited to, benzoyl (i.e., phenyl-C(=O)-), 1-naphthylcarbonyl, 2-naphthylcarbonyl, etc.

As used herein, the aryl moiety of "aryloxy" is defined the same as the aryl described above. Preferred examples of "C₆₋₁₀ aryloxy" include "C₆ or C₁₀ aryloxy". Specific examples of "C₆₋₁₀ aryloxy group" include, but are not limited to, a phenoxy group, 1-naphthyloxy group, 2-naphthyloxy group, etc.

As used herein, "heteroarylcarbonyl" is a monovalent group of -C(=O)-heteroaryl.

As used herein, "heteroarylcarbonyl group" refers to a carbonyl group substituted with the "heteroaryl" described above. Specific examples of "5- or 6-membered heteroarylcarbonyl group" include, but are not limited to, pyrazoylcarbonyl group, triazoylcarbonyl group, thiazoylcarbonyl group, thiadiazoylcarbonyl group, pyridylcarbonyl group, pyridazoylcarbonyl group, etc.

As used herein, the heteroaryl moiety of the "heteroaryloxy group" is defined the same as the "heteroaryl" described above. The 5- or 6-membered heteroaryl moiety of the "5- or 6-membered heteroaryloxy group" is defined the same as "5-membered heteroaryl" or "6-membered heteroaryl", respectively. Specific examples of "5- or 6-membered heteroaryloxy group" include, but are not limited to, a pyrazoyloxy group, triazoyloxy group, thiazoyloxy group, thiadiazoyloxy group, pyridyloxy group, pyridazoyloxy group, etc.

As used herein, the heteroaryl moiety of a "heteroarylthio group" is defined the same as the "heteroaryl" described above. The 5- or 6-membered heteroaryl moiety of "5- or 6-membered heteroarylthio group" is defined the same as "5-membered heteroaryl" or "6-membered heteroaryl", respectively. Specific examples of "5- or 6-membered heteroarylthio group" include, but are not limited to, pyrazoylthio group, triazoylthio group, thiazoylthio group, thiadiazoylthio group, pyridylthio group, pyridazoylthio group, etc.

As used herein, the heteroaryl moiety of a "heteroarylsulfonyl group" is defined the same as the "heteroaryl" described above. A "5- or 6-membered heteroarylsulfonyl group" refers to a sulfonyl group substituted with the "5- or 6-membered heteroaryl" described above. Specific examples of "5- or 6-membered heteroarylsulfonyl group" include, but are not limited to, pyrazoylsulfonyl group, triazoylsulfonyl group, thiazoylsulfonyl group, thiadiazoylsulfonyl group, pyridylsulfonyl group, pyridazoylsulfonyl group, etc.

As used herein, "acyl" refers to a monovalent group of -C(=O)-R_{acyl}, wherein R_{acyl} is hydrogen, optionally substituted alkyl, optionally substituted alkenyl, optionally substituted alkynyl, optionally substituted aryl, optionally substituted heteroaryl, or optionally substituted heterocycloalkyl. Specific examples of acyl include, but are not limited to, formyl, groups exemplified as alkylcarbonyl, arylcarbonyl, and heteroarylcarbonyl, etc.

As used herein, an "optionally substituted carbonyl" group refers to a monovalent group of -C(=O)- (hydrogen or any group selected from the substituent group described herein). Examples of "optionally substituted carbonyl" group include, but are not limited to, formyl, optionally substituted carbamoyl, alkylcarbonyl, alkoxycarbonyl, alkenylcarbonyl, alkenyloxycarbonyl, alkynylcarbonyl, alkynyloxycarbonyl, arylcarbonyl, aryloxycarbonyl, cycloalkylcarbonyl, cycloalkyloxycarbonyl, heteroarylcarbonyl, heteroaryloxycarbonyl, heterocycloalkylcarbonyl, heterocycloalkyloxycarbonyl, etc. A carbonyl group substituted with hydrogen is a formyl group. A carbonyl group substituted with amino is a carbamoyl group.

As used herein, an "optionally substituted oxy" group refers to a monovalent group of -O- (hydrogen or any group selected from the substituent group described herein). Examples of "optionally substituted oxy" group include, but are not limited to, hydroxy, optionally substituted alkyloxy, alkenyloxy, alkynyloxy, aryloxy, heteroaryloxy, heterocycloalkyloxy, alkylcarbonyloxy, alkenylcarbonyloxy, alkynylcarbonyloxy, arylcarbonyloxy, heteroarylcarbonyloxy, heterocycloalkylcarbonyloxy, etc. An oxy group substituted with hydrogen is a hydroxy group.

As used herein, "carbamoyl" is a monovalent group of -C(=O)-NH₂.

As used herein, "amidinoamino" is a monovalent group of -NH-C(=NH)-NH₂. An "amidinoamino" group is also referred to as a "guanidine" group or "guanidyl" group. Such terms are interchangeably used.

As used herein, the phrase "(a substituent)-substituted group" means that the group is substituted with at least one substituent. For example, "hydroxy-substituted C₁₋₆ alkyl" refers to C₁₋₆ alkyl that has at least one hydroxy substitution.

As used herein, "carbamoyl-substituted C₁₋₆ alkyl" is C₁₋₆ alkyl substituted with at least one -C(=O)-NH₂ group. Examples of "carbamoyl-substituted C₁₋₆ alkyl" include, but are not limited to, carbamoyl-substituted C₁₋₄ alkyl, 6-amino-6-oxohexyl (i.e., H₂NC(=O)-(CH₂)₅- or carbamoylpentyl), 7-amino-7-oxoheptyl (i.e., H₂NC(=O)-(CH₂)₆- or carbamoylhexyl), etc. Specific examples of "carbamoyl-substituted C₁₋₄ alkyl" include, but are not limited to, 2-amino-2-oxoethyl (i.e., H₂NC(=O)-CH₂- or carbamoylmethyl), 3-amino-3-oxopropyl (i.e., H₂NC(=O)-CH₂CH₂- or carbamoylethyl), 4-amino-4-oxobutyl (i.e., H₂NC(=O)-(CH₂)₃- or carbamoylpropyl), 5-amino-5-oxopentyl (i.e., H₂NC(=O)-(CH₂)₄- or carbamoylbutyl), etc.

As used herein, "amidinoamino-substituted alkyl" or "guanidino-substituted alkyl" is alkyl substituted with at least one -NH-C(=NH)-NH₂ group, wherein the nitrogen atom of the amidinoamino group may be protected with a nitrogen protecting group (e.g., tert-butoxycarbonyl group). Examples of "amidinoamino-substituted C₁₋₆ alkyl" include, but are not limited to, "amidinoamino-substituted C₁₋₄ alkyl", etc. Specific examples of "amidinoamino-substituted C₁₋₄ alkyl" include, but are not limited to, (amidinoamino)methyl, 2-(amidinoamino)ethyl, 3-(amidinoamino)propyl, 4-(amidinoamino)butyl, etc. Specific examples of "amidinoamino-substituted C₁₋₆ alkyl" include, but are not limited to, amidinoamino-substituted C₁₋₄ alkyl, 5-(amidinoamino)pentyl, 6-(amidinoamino)hexyl, etc. Examples of amidinoamino groups protected with a nitrogen protecting group include 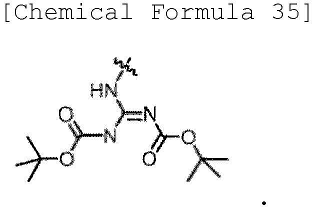 As used herein, "amidinoamino" is synonymous with "guanidino".

As used herein, "carboxy-substituted alkyl" is alkyl substituted with at least one -COOH group. Examples of "carboxy-substituted C₁₋₆ alkyl" include, but are not limited to, carboxy-substituted C₁₋₄ alkyl, 5-carboxypentyl, 6-carboxyhexyl, etc. Specific examples of "carboxy-substituted C₁₋₄ alkyl" include, but are not limited to, carboxymethyl, 2-carboxyethyl, 3-carboxypropyl, 4-carboxybutyl, etc.

As used herein, "arylalkyl substituted with alkyl-substituted amino" is arylalkyl substituted with at least one alkyl-substituted amino. Specific examples of C₁₋₆ alkyl-substituted amino include, but are not limited to, -NH(CH₃), -N(CH₃)₂, -NH(CH₂CH₃), -N(CH₂CH₃)₂, -NH((CH₂)₂CH₃), - N((CH₂)₂CH₃)₂, -NH(CH(CH₃)₂), -N(CH(CH₃)₂)₂, -NH((CH₂)₃CH₃), - N((CH₂)₃CH₃)₂, -NH(CH₂CH(CH₃)₂), -N(CH₂CH(CH₃)₂)₂, - NH((CH₂)₄CH₃), -N((CH₂)₄CH₃)₂, -NH((CH₂)₅CH₃), -N((CH₂)₅CH₃)₂, etc. As used herein, "alkyl-substituted amino" is synonymous with "alkylamino".

A "protecting group" refers to a group of atoms that blocks, reduces, or prevents reactivity of a functional group when attached to a reactive functional group in a molecule. The compound of the present disclosure may have a substitution with a protecting group when appropriate or needed at any of R₁ to R₄ or any position of a substituent thereof or other substituents, etc. Compounds comprising such a protecting group are also within the scope of the present disclosure. Typically, a protecting group can be selectively removed during a synthesis process if desired. Examples of protecting groups are found in Greene and Wuts, Protective Groups in Organic Chemistry, 5th Edition, 2014, John Wiley & Sons, NY and Harrison et al., Compendium of Synthetic Organic Methods, Vol. 1 to 8, John Wiley & Sons, NY, etc. As used herein, a "protecting group" can fall under some or all of the definitions for substituents. In such a case, "X) Protecting group" may be described as "X) protecting group other than 1) to X-1)" in the substituent group. Representative examples of nitrogen protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilylethanesulfonyl ("TES"), trityl and substituted trityl group, allyloxycarbonyl, 9-fluorenylmethyloxycarbonyl ("FMOC"), nitro-veratryloxycarbonyl ("NVOC"), groups represented by "Protect" herein, etc. Representative examples of hydroxyl protecting groups include, but are not limited to, groups that acylate (esterify) or alkylate a hydroxyl group, such as benzyl and trityl ether, as well as alkyl ether, tetrahydropyranyl ether, trialkylsilyl ether (e.g., TMS, triethylsilyl, t-butyldimethylsilyl (TBDMS), and triisopropylsilyl (TIPS)), alkyldiarylsilyl ether (e.g., t-butyldiphenylsilyl (TBDPS)), triarylsilyl ether (e.g., triphenylsilyl), glycol ether (e.g., ethylene glycol ether, propylene glycol ether, etc.), and allyl ether.

An amino group of the compound of the present disclosure (e.g., amino group of the backbone, amino group as a substituent, amino group in a substituent of said compound, etc.) may be protected with a nitrogen protecting group or a group represented by "Protect". An amino group in a substituent listed in the substituent group may be further protected with a nitrogen protecting group or a group represented by "Protect". A protected substituent may also be used as a substituent.

A hydroxy group of the compound of the present disclosure (e.g., hydroxy group as a substituent, a hydroxy group in a substituent of said compound, a hydroxy group in a substituent group described above, etc.) may also be protected with a protecting group of a hydroxy group. A hydroxy group in a substituent listed in a substituent group may be further protected with a hydroxyl protecting group (silyl ether, etc.) described herein. A protected substituent may also be used as a substituent.

### (2.2) Basic denotation method of the compounds of the present disclosure

The present disclosure can denote compounds in the following manner.
Examples of structural data for compounds:

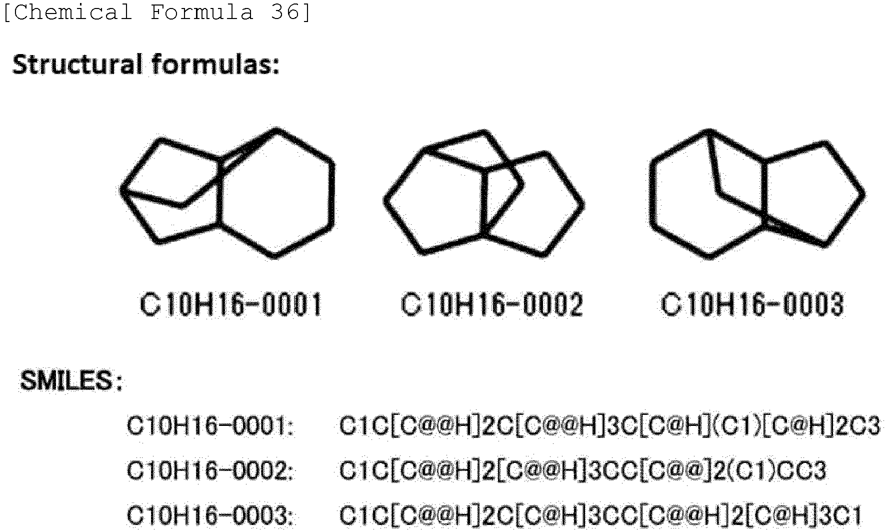

### (3 Schematic explanation of generation of the structure of a compound)

The structure of a compound is generated in order to ultimately generate a peptidomimetic molecule frame. First, an adjacency matrix is generated to generate a carbon backbone. Next, an Attachment Point (AP), which refers to the location where an amino acid-like structure is added, is added to the carbon backbone to generate a carbon backbone with APs. Finally, an amino acid-like side chain is added to the carbon backbone with APs to generate a peptidomimetic molecule frame.

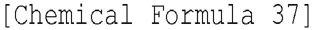

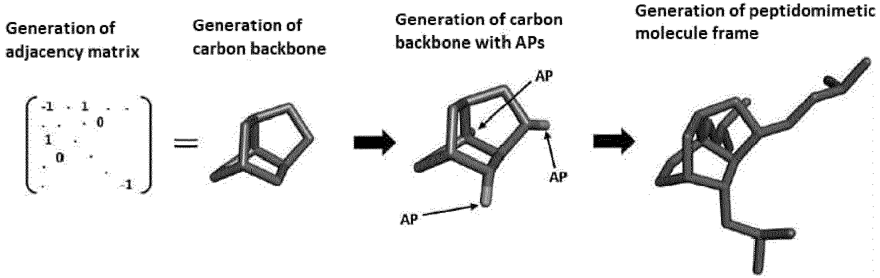

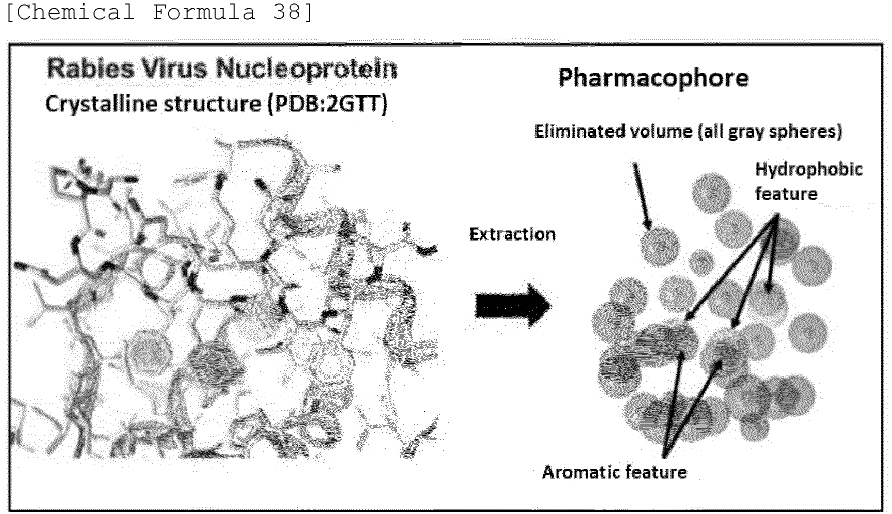

### (3.1) Backbone generation

The procedure for generating a backbone of the present disclosure is described hereinafter. A backbone can be randomly generated, generated through deliberate calculation, or generated to match a certain condition by using such a condition.

### (3.1.1) Generation step using an adjacency matrix

An adjacency matrix of a molecule consisting of n atoms can be expressed as an n × n square matrix with bond orders of bonds between all atoms as elements thereof. If a molecular formula is designated as C4H10, the adjacency matrix is expressed as a 14 × 14 square matrix. Elements of an adjacency matrix indicate the bond order, except for the diagonal elements. Specifically, 0- indicates no bond, 1 indicates a single bond, 2 indicates a double bond, and 3 indicates a triple bond.

When generating a structure of a compound comprised of only a single bond, 0 and 1 are randomly generated in the top triangular region of an adjacency matrix. Since an adjacency matrix is symmetric to the left and right with respect to a diagonal line, the bottom triangular region would have the same elements as the top triangular region.

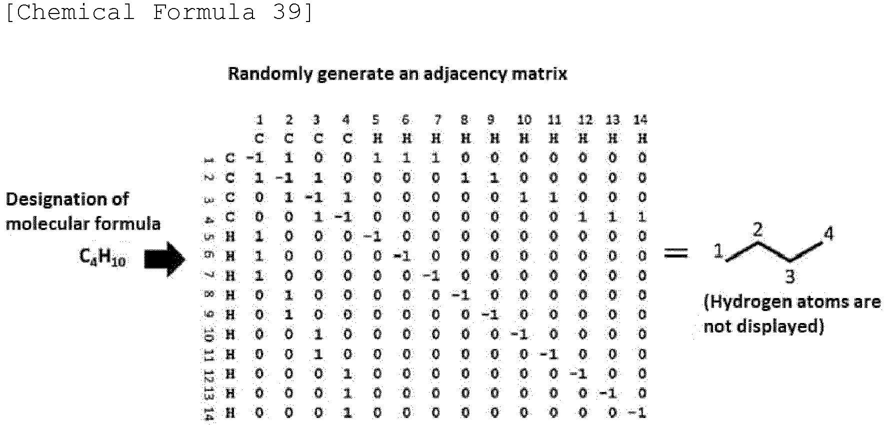

### (3.1.2) Judgment on extraction/relevance/selection of a compound

Extraction, relevance, and selection of a compound is now described.

Representative examples of criteria of judgment on molecular formulas are shown below.
*The valence of atoms constituting a molecule satisfies the condition of 4 for carbon and 1 for hydrogen.
*A molecule expressed by an adjacency matrix is one molecule.
*The molecular formula of a molecule expressed by an adjacency matrix is the same as the designated molecular formula.

Obviously, it is understood that those skilled in the art can use these conditions and criteria after making appropriate changes.

In a preferred embodiment, a carbon backbone satisfying each of condition 1 to condition 4 is selected.
condition 1: all carbon atoms and bonds are within a 5- or 6- membered ring;
condition 2: there is no spiro ring;
condition 3: there are two or more atoms that are present in common in at least three ring structures within a molecule (ring structure under condition 1 is a 5- or 6-membered ring); and
condition 4: energy is, for example, 100 kcal/mol or less in an MMFF upon generation of a 3D structure.

Obviously, it is understood that those skilled in the art can add or substitute other conditions after appropriate review.

The technology of the present disclosure may determine whether a predefined condition is satisfied to select a generated compound or structural data thereof based on whether a predefined condition is satisfied.

Examples of "predefined condition" as used herein includes anything related to a compound. Examples thereof include condition A: "all atoms or carbon atoms and bonds or carbon-carbon bonds are within a 5- or 6- membered ring"; condition B: "there is no spiro ring"; condition C: "there are two or more atoms that are present in common in at least three same ring structures (e.g., ring structure is a 5- or 6-membered ring) within a molecule" (condition is, for example, at least three rings are formed by a carbon atom used, and the rings are 5- or 6-membered rings); condition D: "energy is 150 kcal/mol or less in an MMFF (Merck Molecular Force Field) when a backbone comprising only a single bond is assumed upon generation of a 3D structure", etc.

The descriptions provided above are just representative examples, which can be appropriately modified. For example, atoms can include one or more atoms other than carbon atoms such as oxygen atoms, nitrogen atoms, or sulfur atoms. MMFF may be 150 kcal/mol or less, 140 kcal/mol or less, 130 kcal/mol or less, 120 kcal/mol or less, 110 kcal/mol or less, 100 kcal/mol or less, 90 kcal/mol or less, 80 kcal/mol or less, 70 kcal/mol or less, 60 kcal/mol or less, or 50 kcal/mol or less, or, providing a lower limit as 30 kcal/mol or greater, 40 kcal/mol or greater, 50 kcal/mol or greater, 60 kcal/mol or greater, 70 kcal/mol or greater, 80 kcal/mol or greater, 90 kcal/mol or greater, 100 kcal/mol or greater, etc. These upper limits and lower limits can be appropriately combined. For example, a range such as 50 to 150 kcal/mol can be designated.

### (3.2) Generation of a peptidomimetic molecule frame

Generation of a peptidomimetic molecule frame is described.

In one aspect, the present disclosure provides a method of generating a peptidomimetic molecule framework, comprising the steps of:
(A) providing structural data for a carbon moiety of a predefined carbon backbone;
(B) generating an attachment point (AP), which is a position where a side chain structure is given from the structural data for the carbon backbone;
(C) randomly selecting APs at M locations;
(D) optionally, generating a carbon backbone with N APs by repeating step (C) N times, wherein N is a positive integer;
(E) optionally, comprehensively adding the same or different amino acid-like structure to each AP at M locations to generate a carbon backbone with an amino acid side chain structure; and
(F) fitting the carbon backbone with the amino acid side chain structure to a pharmacophore obtained from a three-dimensional structure of a predefined peptide, and selecting a carbon structure that has fit as a peptidomimetic molecule framework.

In another aspect, the present disclosure provides a method of generating a peptidomimetic molecule framework, comprising the steps of:
(A) providing to a computer structural data for a carbon moiety of a predefined carbon backbone;
(B) causing a computer to generate an attachment point (AP), which is a position where a side chain structure is given from the structural data for the carbon backbone;
(C) causing a computer to randomly select APs at M locations;
(D) optionally, causing a computer to generate a carbon backbone with N APs by repeating step (C) N times, wherein N is a positive integer;
(E) optionally, causing a computer to comprehensively add the same or different amino acid-like structure to AP at M locations to generate a carbon backbone with an amino acid side chain structure; and
(F) causing a computer to fit the carbon backbone with the amino acid side chain structure to a pharmacophore obtained from a three-dimensional structure of a predefined peptide, and select a carbon structure that has fit as a peptidomimetic molecule framework.

In one embodiment, the carbon backbone is a backbone generated by the method described herein (e.g., backbone generation section). In one embodiment, the carbon backbone is a cage-like carbon backbone.

The number of times N of repeating step (C) in step (D) is a positive integer. N can be 1 or greater, 50 or greater, 100 or greater, 200 or greater, 300 or greater, 400 or greater, 500 or greater, 600 or greater, 700 or greater, 800 or greater, 900 or greater, 1000 or greater, 2000 or greater, 3000 or greater, 4000 or greater, 5000 or greater, 6000 or greater, 7000 or greater, 8000 or greater, 9000 or greater, 10000 or greater, 20000 or greater, 30000 or greater, 40000 or greater, 50000 or greater, 60000 or greater, 70000 or greater, 80000 or greater, 90000 or greater, 100000 or greater, 250000 or greater, 500000 or greater, 750000 or greater, 1000000 or greater, or greater. In one embodiment, N in step (D) is 1000 or greater. In one embodiment, N is preferably 3000 or greater.

In the step of randomly selecting APs at M locations, M can be an integer that is 1 or greater, 2 or greater, 3 or greater, 4 or greater, 5 or greater, 6 or greater, 7 or greater, 8 or greater, 9 or greater, 10 or greater, or greater. The lower limit value of M can be an integer that is 1, 2, 3, 4, 5, 6, 7, 8, 9, 10, or greater, and the upper limit value of M can be an integer that is 2, 3, 4, 5, 6, 7, 8, 9, 10, 11, 12, 13, 14, 15, 16, 17, 18, 19, 20, or greater. Any combination of an upper limit and lower limit can be selected. The range can be, for example, 2 to 3, 2 to 4, 2 to 5, 2 to 6, 2 to 7, 2 to 8, 2 to 9, 2 to 10, 2 to 11, etc. In one embodiment, M is an integer from 2 to 6. In one embodiment, M is an integer from 2 to 5 or 2 to 4. In one embodiment, M is 3 or greater.

In step (E) in one embodiment, the amino acid comprises at least one selected from the group consisting of leucine, phenylalanine, isoleucine, and tryptophan.

In one embodiment, step (F) comprises fitting each generated carbon backbone with the amino acid side chain structure to a target protein to determine whether the carbon backbone with the amino acid side chain structure satisfies a pharmacophore thereof. If it is determined that a pharmacophore is satisfied, a fitting score of the carbon backbone with the amino acid side chain structure is computed. If it is determined that a pharmacophore is not satisfied, the fitting score of such a carbon backbone with an amino acid side chain structure is 0.

In one embodiment, step (F) comprises causing a computer to select top K carbon backbones with a best fitting score of the pharmacophore.

In one embodiment, step (F) fits each generated carbon backbone with the amino acid side chain structure to a target protein to calculate FS. In one embodiment, step (F) comprises selecting top FS when arranged in order from the highest value from a plurality of calculated FS. Selected K FS is typically about top 20%, but can be about top 1%, 2%, 3%, 4%, 5%, 6%, 7%, 8%, 9%, 10%, 12%, 14%, 16%, 18%, 20%, 22%, 24%, 26%, 28%, 30%, 32%, 34%, 36%, 38%, 40%, 42%, 44%, 46%, 48%, 50%, 55%, 60%, 65%, 70%, 75%, 80%, 85%, 90%, 95%, or 99%.

In step (F), a "fitted compound" selected as a peptidomimetic molecule framework refers to a compound wherein superposing a group of functional groups of the compound with a pharmacophore of a target protein results in the group of functional groups satisfying the pharmacophore. "Satisfying a pharmacophore" means a pharmacophore score greater than 0, and "not satisfying a pharmacophore" means a pharmacophore score of 0. When "satisfying a pharmacophore", the score has a value that is greater than 0 and less than or equal to 1, depending on the degree of satisfaction of a pharmacophore. In other words, the group of functional groups is determined as "satisfying a pharmacophore" if the pharmacophore score is greater than 0 in step (F).

In one embodiment, the compound comprises at least one type of bond selected from a single bond, a double bond, and a triple bond.

### (3.2.1) Provision of structural data for a carbon moiety of a predefined carbon backbone

Structural data for a carbon moiety of a predefined carbon backbone is typically provided in the SMILES format, but the format is not limited thereto. Such data can be provided by other methodologies. Examples of other methodologies include, but are not limited to, a chemical structural formula, rational formula, MOL (MDL) file, PDB code, CML, SMARTS, SLN, WLN, etc.

Structural data for a group of backbones specifying the backbone with any substituent can be provided in, for example, the SMILES format.

Data can be represented, for example, as follows.

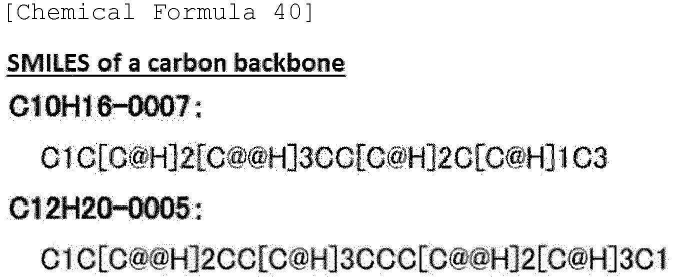

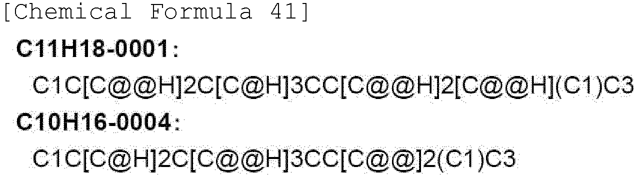

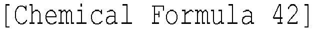

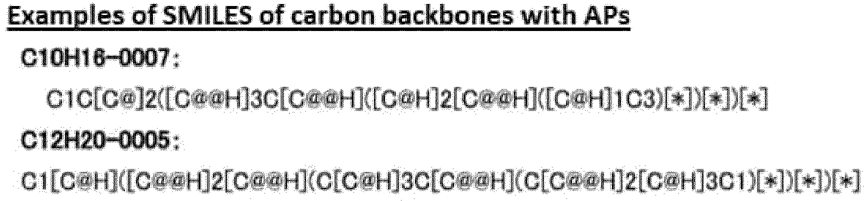

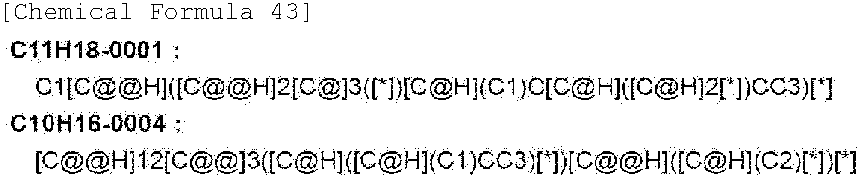

### (3.2.2) Generation of an attachment point (AP), which is a position where a side chain structure is given from the structural data for the carbon backbone

An important viewpoint of the technology of the present disclosure is the generation of an AP. An AP is a position where a side chain structure is given (position of a hydrogen atom in a "cage-like carbon backbone") and can be denoted in the SMILES format as described above and appropriately calculated by those skilled in the art in such a manner.

Any M locations (M is an integer that is 1 or greater, and the maximum value of M is the number of hydrogen atoms of the generated carbon backbone (e.g., cage-like carbon backbone) can be selected from the generated APs. Any random function generator can be used for randomly selecting APs at M locations. For example, this can be denoted in the SMILES format as described above and appropriately calculated by those skilled in the art in such a manner.

A carbon backbone with N APs can also be generated by repeating provision of a backbone, AP generation, and selection N times.

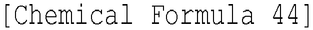

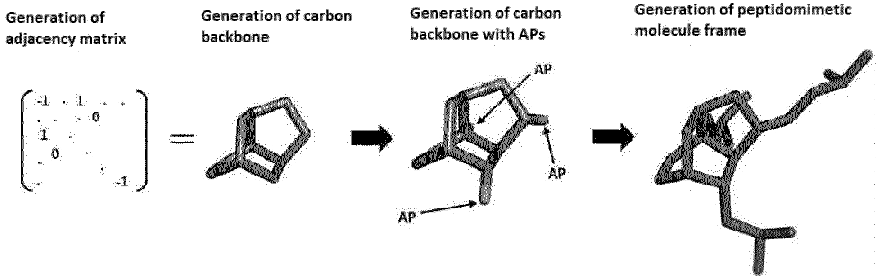

### (3.2.3) Step of comprehensively adding an amino acid-like structure to an AP to generate an amino acid-like structure-containing carbon structure

Next, the same or different amino acid-like structure can be comprehensively added to each AP at M locations to generate an amino acid-like structure-containing carbon backbone. If there are APs at three locations and five amino acid-like side chains, a 5 × 5 × 5 structure is generated. Structural information on a carbon backbone with APs is within a program. An amino acid-like structure can be prepared as a file in the SMILES format. After reading in the SMILES format of an amino acid-like structure, an AP of a carbon backbone with an AP can be attached to the base portion of an amino acid-like side chain without extraordinary calculation.

### (3.2.4) Pharmacophore

A pharmacophore is the concept of features (group of functional groups and relative three-dimensional arrangement thereof) of a molecule required for the molecule recognition of a ligand by a biopolymer. A model representing this concept is referred to as a pharmacophore model. Commercially available software can be used for all computation associated with pharmacophore. For example, LigandScout can be used. LigandScout can also be used for displaying pharmacophore. See the article on LigandScout: J. Chem. Inf. Model. 2005, 45, 160-169.

### (3.2.5) Fitting

The amino acid-like structure-containing carbon backbone or peptidomimetic molecule framework of the present disclosure can be fitted to a target.

For example, fitting can be performed with various software such as LigandScout. See, for example, an article on LigandScout: J. Chem. Inf. Model. 2005, 45, 160-169.

### (4 Schematic description of synthesis of a compound)

For a designed backbone, an appropriate synthesis method can be set based on the backbone.

### (4.1 Design/synthesis of actual compound from calculated backbone)

Once a backbone is generated, those skilled in the art can design and synthesize a compound by using the descriptions herein and known technologies. Steps of producing a compound with a peptidomimetic molecule framework of the present disclosure can comprise, for example, the steps of:
determining a route for producing a compound with a peptidomimetic molecule framework by using a synthetic route designing program; and
synthesizing the compound in accordance with the determined route.

The step for determining a route for production can comprise, for example, the steps of:
replacing one or more backbone atoms of the compound with the peptidomimetic molecule framework; and
investigating whether the compound generated through the replacement can be synthesized by using a synthetic route designing program. The step of determining a route for production can comprise:
   after the investigation step, fitting the compound generated by the replacement to confirm that a fitting score is 0.1 or greater.

The fitting score upon such fitting is typically a numerical value from 0 to 1. FS = 1 means that a group of functional groups of a compound subjected to fitting completely overlaps with a pharmacophore of a target protein. FS = 0 means that a group of functional groups of a compound subjected to fitting does not overlap with a pharmacophore of a target protein at all. A fitting score required in the step of confirming FS after the investigation step is typically about 0.1 or greater, but can be about 0.15 or greater, about 0.20 or greater, about 0.25 or greater, about 0.30 or greater, about 0.35 or greater, about 0.40 or greater, about 0.45 or greater, about 0.50 or greater, about 0.55 or greater, about 0.80 or greater, about 0.85 or greater, about 0.90 or greater, or about 0.95 or greater.

The basic design and synthesis method are disclosed below as an example.

### (4.2 Design/synthesis of actual compound from a calculated backbone)

Design/synthesis of actual compound from a calculated backbone is now described.

In one embodiment, an AP is generated after generating the backbone (SK-1) of C10H16-0007. Formula "SK-1 + AP" described below is an example of generating APs at three locations. Next, an AP of a carbon backbone with APs is attached to the base of an amino acid-like side chain. When amino acid-like side chains are attached to formula "SK-1 + AP", formula "SK-1 + AP + AL" described below is obtained, wherein R_{A1}, R_{A2}, and R_{A3} are groups having any amino acid-like side chain. Next, fitting is performed for formula "SK-1 + AP + AL", and a synthetic route is considered for those satisfying the criteria for a fitting score. For a compound of formula SK-1 + AP + AL, a compound of formula "SK-1 + AP + AL + HA" with two backbone carbon atoms replaced with nitrogen atoms and one backbone carbon atom replaced with oxygen can be given. A compound of formula IB was obtained in the Examples herein.

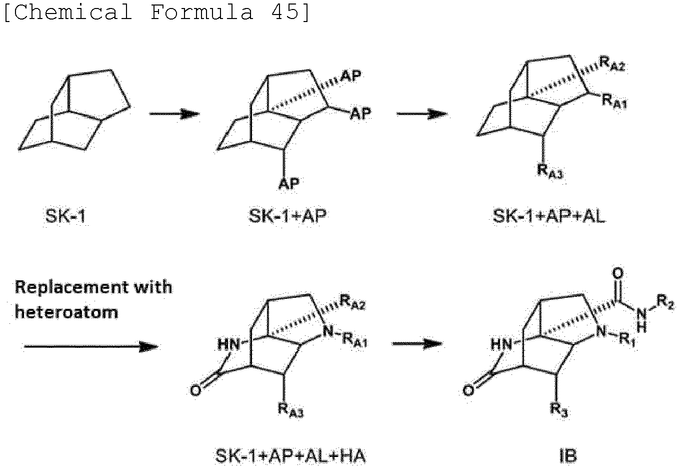

In one embodiment, an AP is generated after generating the backbone (SK-2) of C12H20-0005. Formula "SK-2 + AP" described below is an example of generating APs at three locations. Next, an AP of a carbon backbone with APs is attached to the base of an amino acid-like side chain. When amino acid-like side chains are attached to formula "SK-2 + AP", formula "SK-2 + AP + AL" described below is obtained, wherein R_{A4}, R_{A5}, and R_{A6} are groups having any amino acid-like side chain. Next, fitting is performed for formula "SK-2 + AP + AL", and a synthetic route is considered for those satisfying the criteria for a fitting score. For a compound of formula SK-2 + AP + AL, a compound of formula "SK-2 + AP + AL + HA" with two backbone carbon atoms replaced with nitrogen atoms can be given. A compound of formula LXII was obtained in the Examples herein.

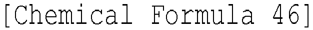

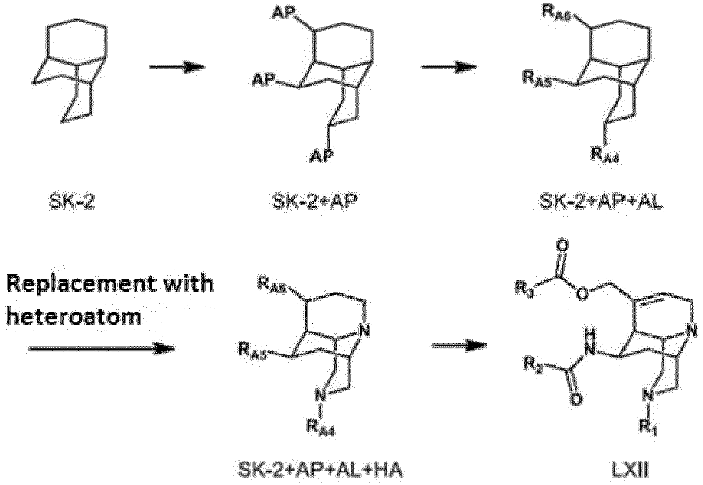

In one embodiment, an AP is generated after generating the backbone (SK-3) of C11H18-0001. Formula "SK-3 + AP" described below is an example of generating APs at four locations. Next, an AP of a carbon backbone with APs is attached to the base of an amino acid-like side chain. When amino acid-like side chains are attached to formula "SK-3 + AP", formula "SK-3 + AP + AL" described below is obtained, wherein R_{A7}, R_{A8}, R_{A9}, and R_{A10} are groups having any amino acid-like side chain. Next, fitting is performed for formula "SK-3 + AP + AL", and a synthetic route is considered for those satisfying the criteria for a fitting score. For a compound of formula SK-3 + AP + AL, a compound of formula "SK-3 + AP + AL + HA" with two backbone carbon atoms replaced with nitrogen atoms can be given. A compound of formula LLIIB was obtained in the Examples herein.

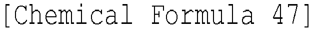

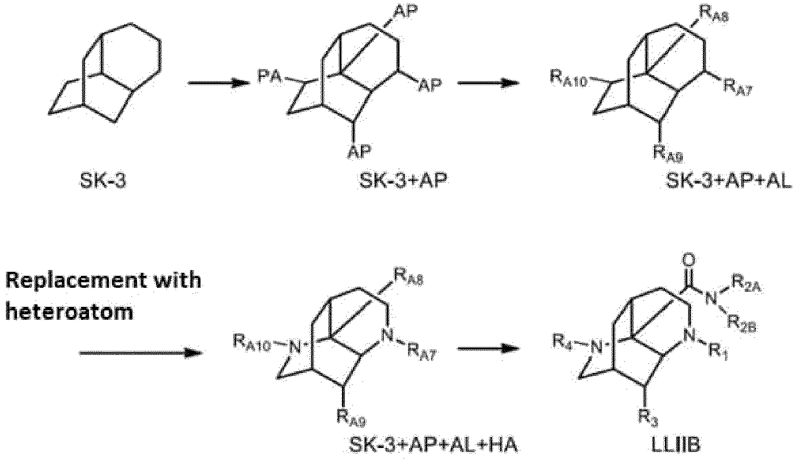

In one embodiment, an AP is generated after generating the backbone (SK-4) of C10H16-0004. Formula "SK-4 + AP" described below is an example of generating APs at three locations. Next, an AP of a carbon backbone with APs is attached to the base of an amino acid-like side chain. When amino acid-like side chains are attached to formula "SK-4 + AP", formula "SK-4 + AP + AL" described below is obtained, wherein R_{A11}, R_{A12}, and R_{A13} are groups having any amino acid-like side chain. Next, fitting is performed for formula "SK-4 + AP + AL", and a synthetic route is considered for those satisfying the criteria for a fitting score. For a compound of formula SK-4 + AP + AL, a compound of formula "SK-4 + AP + AL + HA" with two backbone carbon atoms replaced with nitrogen atoms can be given. A compound of formula LLLI was obtained in the Examples herein.

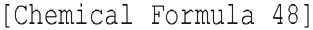

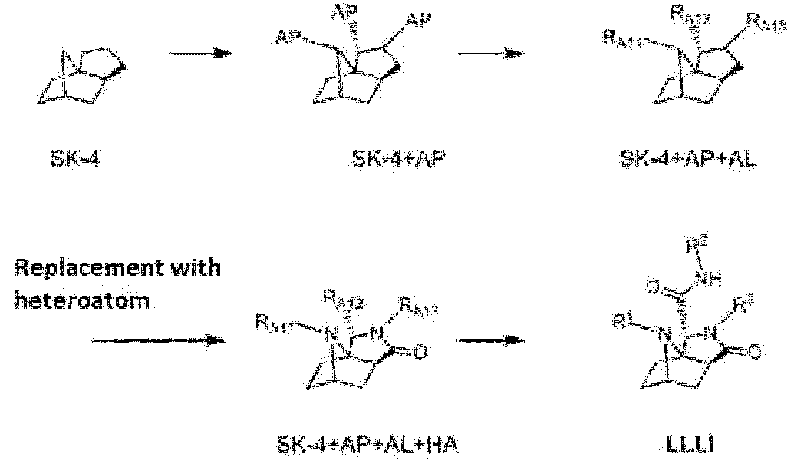

In one embodiment, a compound with the backbone of C10H16-0007 can be manufactured as described in the following Synthesis Schemes 1 to 6.

### (Synthesis Scheme 1)

### Synthesis of compound III

Compound III can be manufactured, for example, by the following manufacturing method.

### (1) Pathway 1-1: Synthesis from compound VII and compound VIII

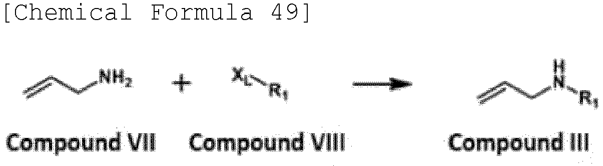

### (2) Pathway 1-2: Synthesis from compound VII and compound VIII via amine protection

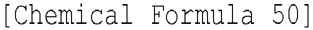

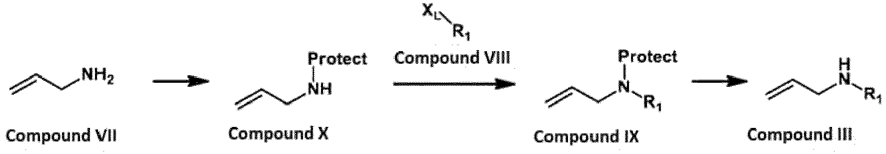

### (3) Pathway 1-3: Synthesis from compound XI and compound XII

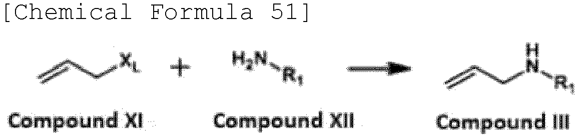

wherein, X_{L} represents a leaving group in a nucleophilic substitution reaction. Examples thereof include halogen (e.g., chlorine, bromine, iodine), sulfate ester (-OSO₃H, etc.), sulfonyl-O- group (e.g., methanesulfonyl-O-, toluenesulfonyl-O-, etc.).

### (4) Pathway 1-4: Synthesis from compound XI and compound XIII

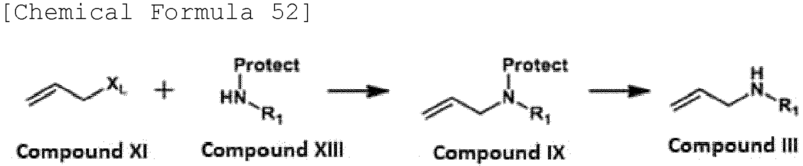

### (5) Route 1-5: synthesis from compound VII and compound XIV

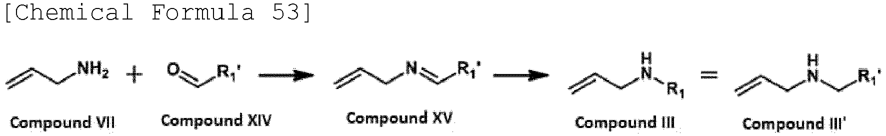

wherein, if R₁ in compound III can be represented by -CH₂-R₁', R₁ in compound III can be replaced with -CH₂-R₁' to represent compound III as compound III'.
wherein R₁ is
hydrogen,
an optionally substituted hydrocarbon group,
an optionally substituted heterocycle,
optionally substituted carbonyl, or
an optionally substituted functional group, or each independently, optionally substituted alkyl, optionally substituted cycloalkyl, or optionally substituted heterocycloalkyl.

"Protect" is a protecting group of an amine group. Examples of protecting groups of an amino group include an ethoxycarbonyl group, tert-butoxycarbonyl group, acetyl group, benzoyl group, trifluoroacetyl group, benzyloxycarbonyl group, 3- or 4-chlorobenzyloxycarbonyl group, triphenylmethyl group, methanesulfonyl group, p-toluenesulfonyl group, trimethylsilyl group, benzyloxycarbonyl group, 3- or 4-chlorobenzyloxycarbonyl group, benzylsulfonyl group, benzyl group, 4-nitrobenzyl group, 4-methoxybenzyl group, methyl group, ethyl group, etc.

A compound that is commercially available or a compound manufactured by a known method can be used as a starting raw material compound.

### Synthesis Scheme 2

### Synthesis of compound V

A compound of formula V can be manufactured by, for example, the following manufacturing method. (1) Route 2-1 Synthesis from compound XVI and compound XVII 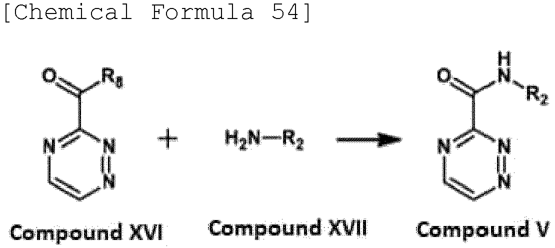 wherein R₂ represents hydrogen, an optionally substituted hydrocarbon group, an optionally substituted heterocycle, optionally substituted carbonyl, or an optionally substituted functional group, and R₈ represents alkoxy, aryloxy, hydroxy, or halogen. This synthesis is achieved by various reactions known to those skilled in the art. If R₂ is aryl, compound V may be synthesized in accordance with the method described in C. W. Cheung, M. L. Ploeger, and X. Hu, Nature Communications 2017, 8, 14878.

### (2) Route 2-2 Synthesis from compound XVIII

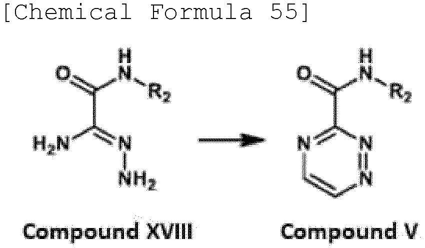

wherein R₂ is the same as defined herein.

A compound that is commercially available or a compound that is manufactured by a known method can be used as the starting raw material compound.

### Synthesis Scheme 3

### Synthesis of compound VI

A compound of formula VI can be manufactured from compound III and compound IV by, for example, the following manufacturing method. 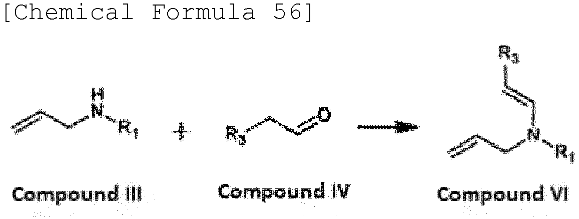 wherein R₁ is the same as defined herein, and R₃ is hydrogen, an optionally substituted hydrocarbon group, an optionally substituted heterocycle, optionally substituted carbonyl, or an optionally substituted functional group.

### Synthesis Scheme 4

Compound of formula IIF and compound of formula IIB-Route 1

A compound of formula IIF and compound of formula IIB can be manufactured, for example, from three components through one-pot synthesis in accordance with a known method (e.g., method described in Bioorg. Med. Chem. 23 (2015) 2629-2635, Tetrahedron 63 (2007) 6004-6014, Eur. J. Org. Chem. 2009, 2185-2189, Eur. J. Org. Chem. 2011, 2354-2359, etc.). 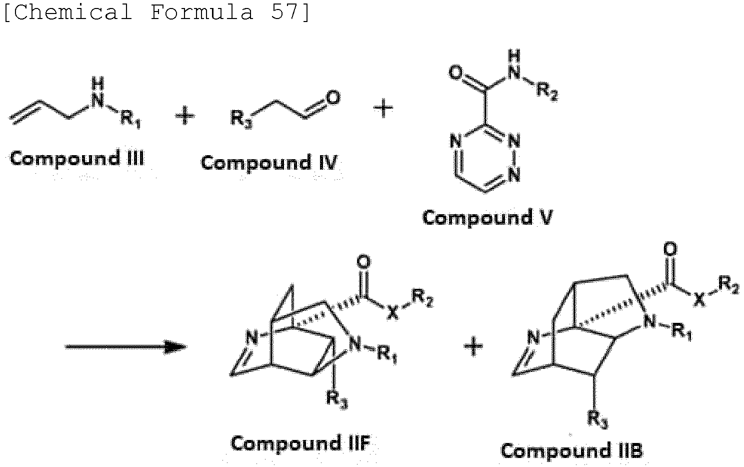 wherein R₁, R₂, and R₃ are the same as defined herein, and X is -NH-.

### Synthesis Scheme 5

### Compound of formula IIF and compound of formula IIB-Route 2

A compound of formula IIF and compound of formula IIB can be manufactured, for example, from two components as described below. 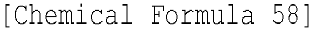 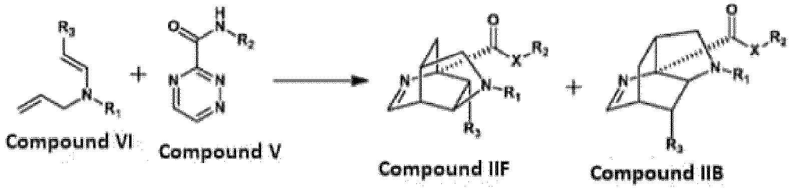 wherein R₁, R₂, and R₃ are the same as defined herein, and X is -NH-.

### Synthesis Scheme 6

### Synthesis of compound IF and compound IB

### Step 6-1

### Oxidation of imine

Compound IF (i.e., compound of formula IF) or compound IB (i.e., compound of formula IB) can be manufactured, for example, from compound IIF (i.e., compound of formula IIF) or compound IIB (i.e., compound of formula IIB) by oxidation as described below in accordance with a known method (e.g., the method described in RSC Advances, 2012, 2, 5536-5538, Tetrahedron Lett. 50 (2009) 3436-3438, etc.). X in the following formulas is -NH-.

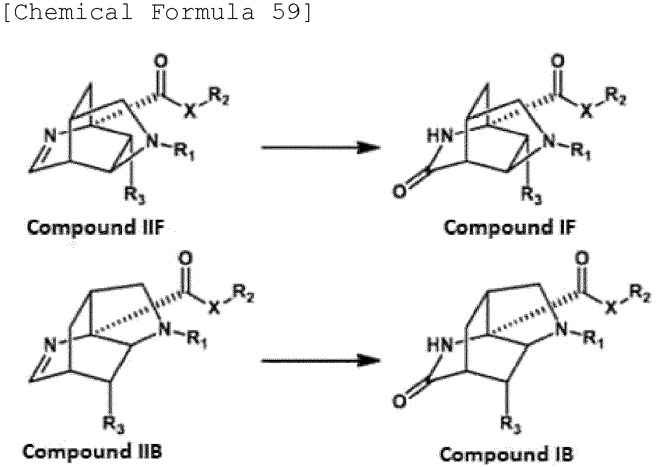

Another example modified to include a double bond is 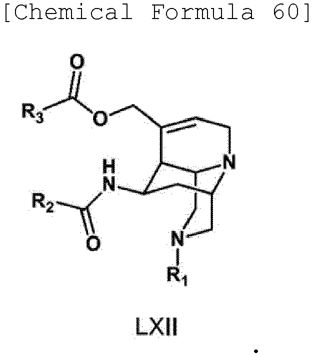 This backbone can be synthesized as described in the following Schemes 7 to 17.

### Synthesis Scheme 7

### Synthesis of compounds V1 and V2

The compounds of formulas V1 and V2 can be manufactured, for example, in accordance with a known method (Fricke, T.; Kopp, N.; Wunsch, B. Synthesis 2010, 5, 791).

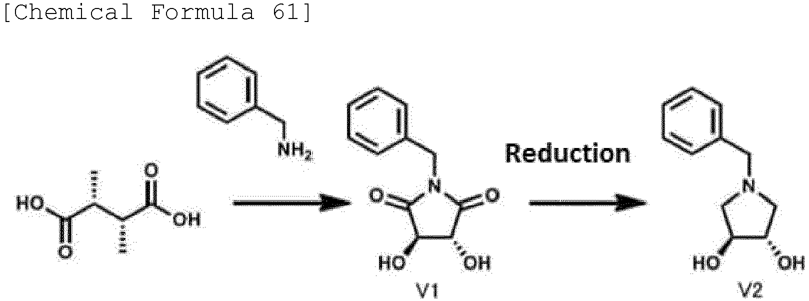

A compound that is commercially available or a compound manufactured by a known method can be used as a starting raw material compound.

### Synthesis Scheme 8

### Synthesis of compound LIII

A compound of formula LIII can be manufactured from compound V2 by, for example, the following manufacturing method. 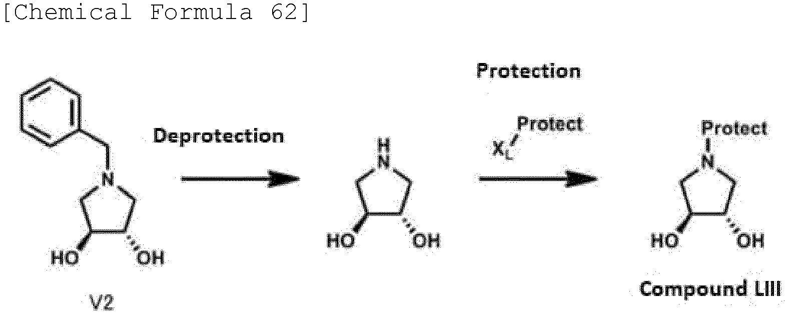 wherein, X_{L} represents a leaving group in a nucleophilic substitution reaction. Examples thereof include halogen (e.g., chlorine, bromine, and iodine), sulfate ester (-OSO₃H, etc.), and sulfonyl-O- group (e.g., methanesulfonyl-O-, toluenesulfonyl-O-, etc.). "Protect" is a protecting group of an amino group. Examples of protecting groups of an amino group include an ethoxycarbonyl group, tert-butoxycarbonyl group, acetyl group, benzoyl group, trifluoroacetyl group, benzyloxycarbonyl group, 3- or 4-chlorobenzyloxycarbonyl group, triphenylmethyl group, methanesulfonyl group, p-toluenesulfonyl group, trimethylsilyl group, benzyloxycarbonyl group, 3- or 4-chlorobenzyloxycarbonyl group, benzylsulfonyl group, methyl group, ethyl group, etc.

### Synthesis Scheme 9

### Synthesis of compound LIV

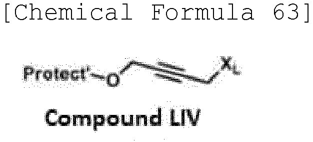

Compound LIV can be manufactured, for example, in accordance with a known method (Billaud, E. F.; Rbah-Vidal, L.; Vidal, A.; Besse, S.; Tarrit, S.; Askienazy, S.; Maisonial, A.; Moins, N.; Madelmont, J. C.; Miot-Noirault, E.; Chezal, J. M.; Auzeloux, P. J. Med. Chem. 2013, 56, 8455.). In this regard, X_{L} represents a leaving group in a nucleophilic substitution reaction. Examples thereof include halogen (e.g., chlorine, bromine, and iodine), sulfate ester (-OSO₃H, etc.), and sulfonyl-O- group (e.g., methanesulfonyl-O-, toluenesulfonyl-O-, etc.). "Protect'" is a protecting group of a hydroxy group. Examples of protecting groups of a hydroxy group include a methoxymethyl group, benzyl group, p-methoxybenzyl group, trimethylsilyl group, triethylsilyl group, t-butyldimethylsilyl group, acetyl group, benzoyl group, trityl group, etc.

### Synthesis Scheme 10

### Synthesis of compound LV

A compound of formula LV can be manufactured from compound LIII and compound LIV by, for example, the following manufacturing method.

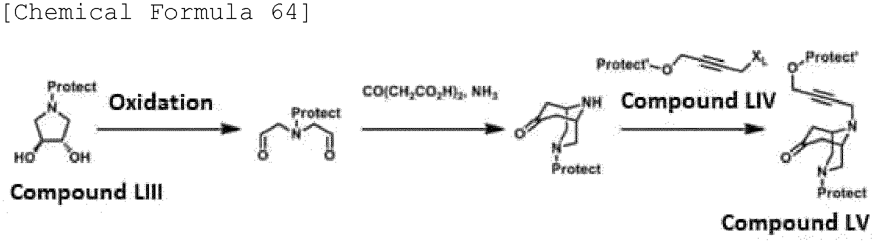

### Synthesis Scheme 11

### Synthesis of compound LVI

A compound of formula LVI can be manufactured from compound LV by, for example, the following manufacturing method.

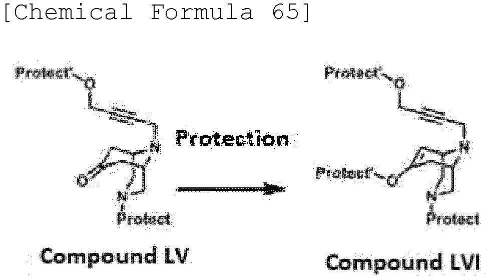

### Synthesis Scheme 12

### Synthesis of compound LVII

A compound of formula LVII can be manufactured from compound LVI by, for example, the following manufacturing method.

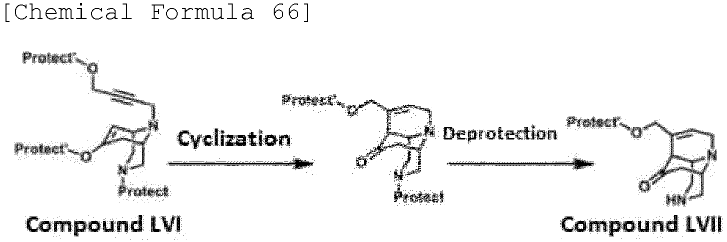

### Synthesis Scheme 13

### Synthesis of compound LVIII

Compound LVIII can be manufactured by, for example, the following manufacturing method.

### (1) Route 1-1: Synthesis from compound LVII and compound LXIII

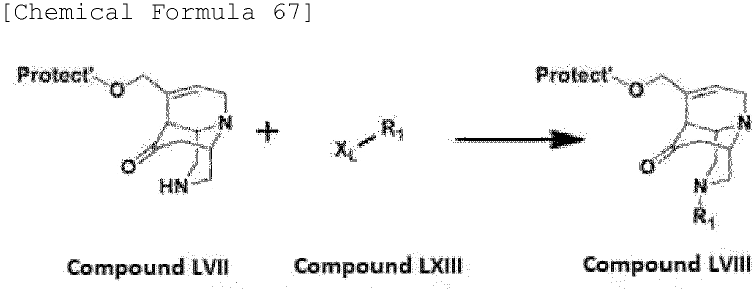

wherein X_{L} represents a leaving group in a nucleophilic substitution reaction. Examples thereof include halogen (e.g., chlorine, bromine, and iodine), sulfate ester (-OSO₃H, etc.), and sulfonyl-O- group (e.g., methanesulfonyl-O-, toluenesulfonyl-O-, etc.).

### (2) Route 1-2: Synthesis from compound LVII and compound LXIV

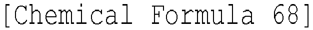

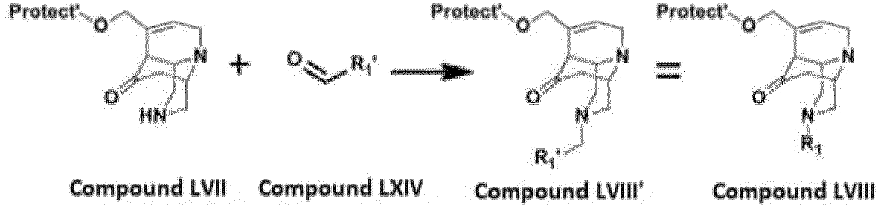

wherein, if R₁ in compound LVIII can be represented by -CH₂-R₁', R₁ in compound LVIII can be replaced with -CH₂-R₁' to represent compound LVIII as compound LVIII'.

R₁ therein is the same as defined herein.

A compound that is commercially available or a compound manufactured by a known method can be used as a starting raw material compound.

### Synthesis Scheme 14

### Synthesis of compound LIX

A compound of formula LIX can be manufactured from compound LVIII by, for example, the following manufacturing method.

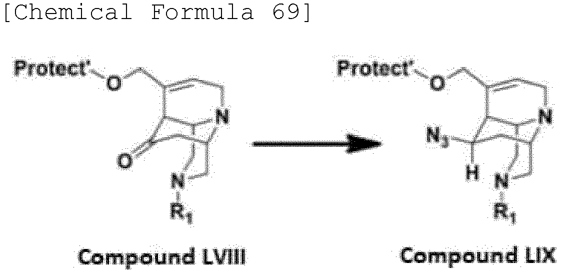

### Synthesis Scheme 15

### Synthesis of compound LX

A compound of formula LX can be manufactured from compound LIX by, for example, the following manufacturing method.

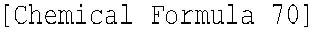

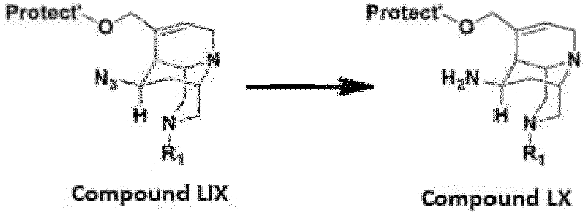

### Synthesis Scheme 16

### Synthesis of compound LXI

Compound LXI can be manufactured by, for example, the following manufacturing method.

### (1) Route 1-1: Synthesis from compound LX and compound LXV

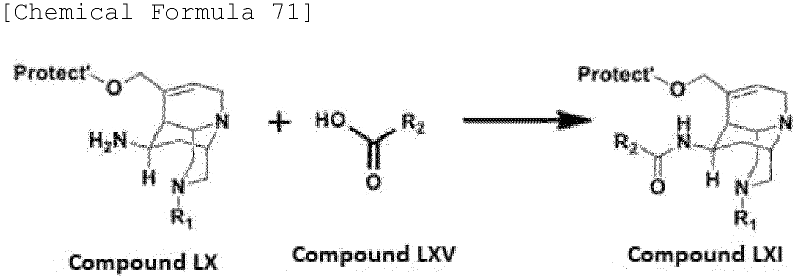

### (2) Route 1-2: Synthesis from compound LX and compound LXVI

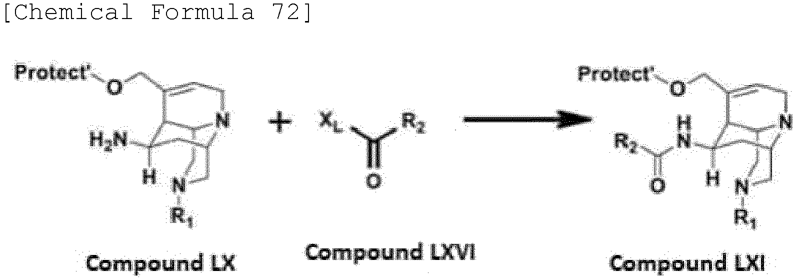

wherein R₁ and R₂ are the same as defined herein,
wherein X_{L} represents a leaving group in a nucleophilic substitution reaction. Examples thereof include halogen (e.g., chlorine, bromine, and iodine), sulfate ester (-OSO₃H, etc.), and sulfonyl-O- group (e.g., methanesulfonyl-O-, toluenesulfonyl-O-, etc.).

### Synthesis Scheme 17

### Synthesis of compound LXII

Compound LXII can be manufactured by, for example, the following manufacturing method.

### (1) Route 1-1: Synthesis from compound LXI and compound LXVII

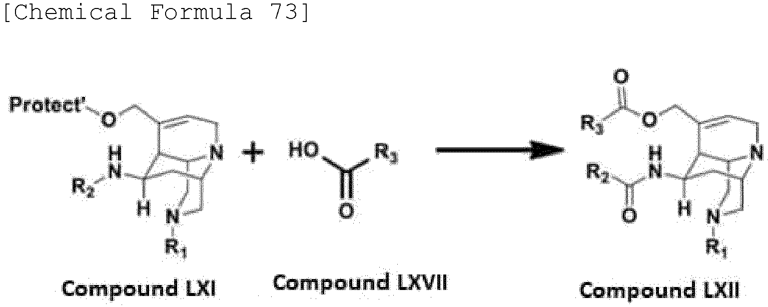

### (2) Route 1-2: Synthesis from compound LXI and compound LXVIII

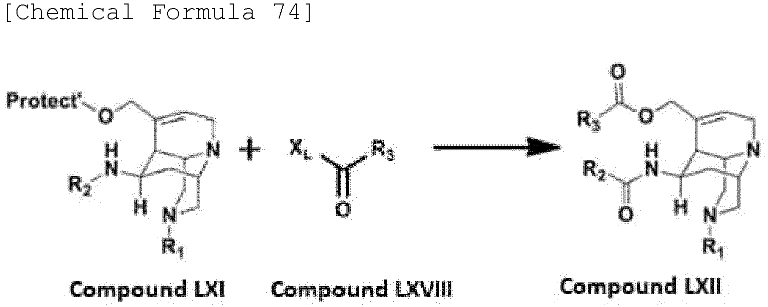

wherein R₁, R₂, and R₃ are the same as defined herein,
wherein X_{L} represents a leaving group in a nucleophilic substitution reaction. Examples thereof include halogen (e.g., chlorine, bromine, and iodine), sulfate ester (-OSO₃H, etc.), and sulfonyl-O- group (e.g., methanesulfonyl-O-, toluenesulfonyl-O-, etc.).
R₁, R₁', R₂, and R₃ of compounds LVIII, LVIII', LIX, LX, LXI, LXII, LXIII, LXIV, LXV, LXVI, LXVII, LXVIII, LXIV, LXVI, LXVII, and LXII are present independently from R₁, R₁', R₂, and R₃ of compounds IF, IB, IIF, IIB, III, III', IV, V, VI, VIII, IX, XII, XIII, XIV, XV, XVI, XVII, and XVIII.

In one embodiment, a compound with a backbone of C11H18-0001 can be manufactured as described in the following Synthesis Schemes 18 to 30.

### Synthesis Scheme 18

### Synthesis of compound LLIII

Compound LLIII can be manufactured by, for example, the following manufacturing method.

### (1) Route 1-1: Synthesis from compound LLVII and compound LLVIII

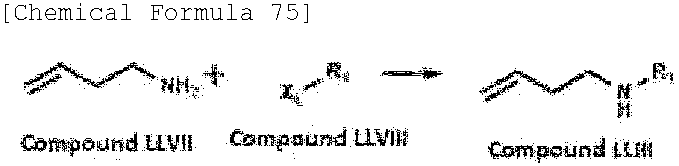

### (2) Route 1-2: Synthesis from compound LLVII and compound LLVIII via amine protection

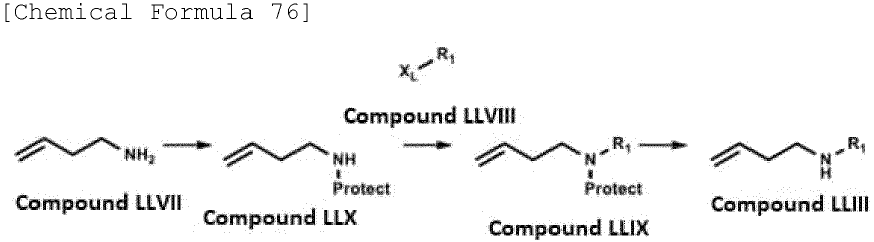

### (3) Route 1-3: Synthesis from compound LLXI and compound LLXII

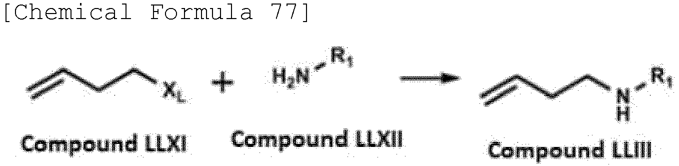

wherein X_{L} represents a leaving group in a nucleophilic substitution reaction. Examples thereof include halogen (e.g., chlorine, bromine, and iodine), sulfate ester (-OSO₃H, etc.), and sulfonyl-O- group (e.g., methanesulfonyl-O-, toluenesulfonyl-O-, etc.).

### (4) Route 1-4: Synthesis from compound LLXI and compound LLXIII

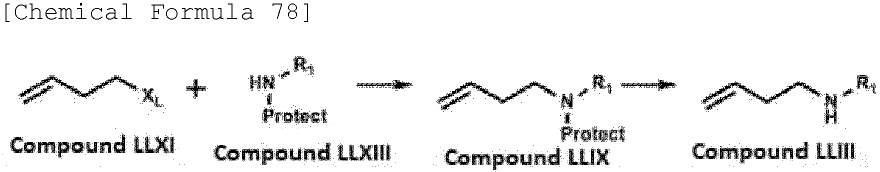

### (5) Route 1-5: Synthesis from compound LLVII and compound LLXIV

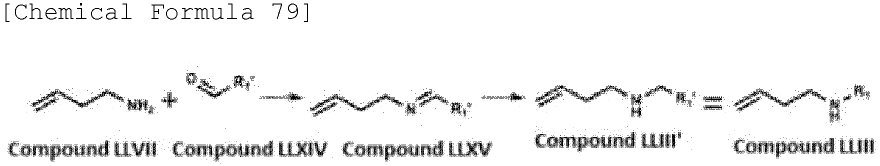

wherein, if R₁ in compound LLIII can be represented by -CH₂-R₁', R₁ in compound LLIII can be replaced with -CH₂-R₁' to represent compound LLIII as compound LLIII'.

R₁ therein is the same as defined herein. "Protect" is a protecting group of an amino group. Examples of protecting groups of an amino group include an ethoxycarbonyl group, tert-butoxycarbonyl group, acetyl group, benzoyl group, trifluoroacetyl group, benzyloxycarbonyl group, 3- or 4-chlorobenzyloxycarbonyl group, triphenylmethyl group, methanesulfonyl group, p-toluenesulfonyl group, trimethylsilyl group, benzyloxycarbonyl group, 3- or 4-chlorobenzyloxycarbonyl group, benzylsulfonyl group, benzyl group, 4-nitrobenzyl group, 4-methoxybenzyl group, methyl group, ethyl group, etc.

A compound that is commercially available or a compound that is manufactured by a known method can be used as the starting raw material compound.

### Synthesis Scheme 19

### Synthesis of compound LLVI

A compound of formula LLVI can be manufactured from compound LLIII and compound LLIV by, for example, the following manufacturing method. 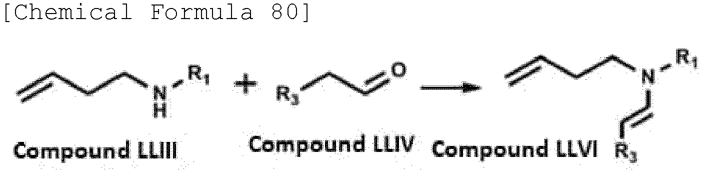 wherein R₁ and R₃ are the same as defined herein.

### Synthesis Scheme 20

### A compound of formula XXIF and a compound of formula XXIB-Route 1

A compound of formula XXIF and a compound of formula XXIB can be manufactured, for example, from three components through one-pot synthesis in accordance with a known method (e.g., method described in Bioorg. Med. Chem. 23 (2015) 2629-2635, Tetrahedron 63 (2007) 6004-6014, Eur. J. Org. Chem. 2009, 2185-2189, Eur. J. Org. Chem. 2011, 2354-2359, etc.). 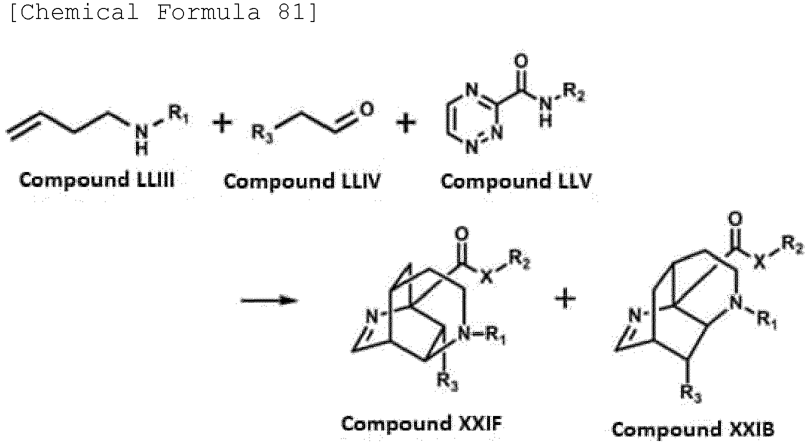 wherein R₁, R₂, and R₃ are the same as defined herein, and X is -NH-.

### Synthesis Scheme 21

### A compound of formula XXIF and a compound of formula XXIB-Route 2

A compound of formula XXIF and a compound of formula XXIB can be manufactured, for example, from two components as described below. 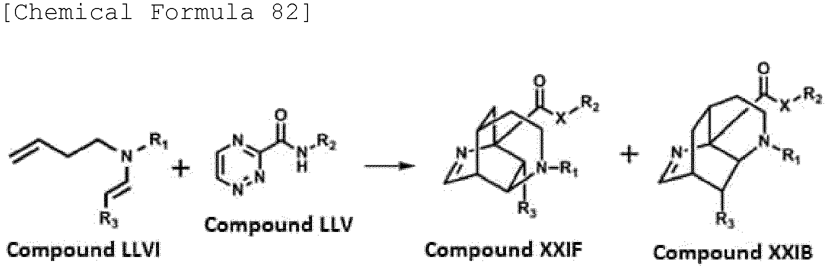 wherein R₁, R₂, and R₂ are the same as defined herein, and X is -NH-.

### Synthesis Scheme 22-1

### Synthesis of compound XXIIF and compound XXIIB

### Step 22-1

### Reduction of imine

Compound XXIIF (i.e., compound of formula XXIIF) or compound XXIIB (i.e., compound of formula XXIIB) can be manufactured from compound XXIF (i.e., compound of formula XXIF) or compound XXIB (i.e., compound of formula XXIB) by, for example, reducing in the following manner. X in the following formulas is -NH-.

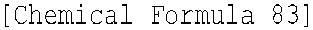

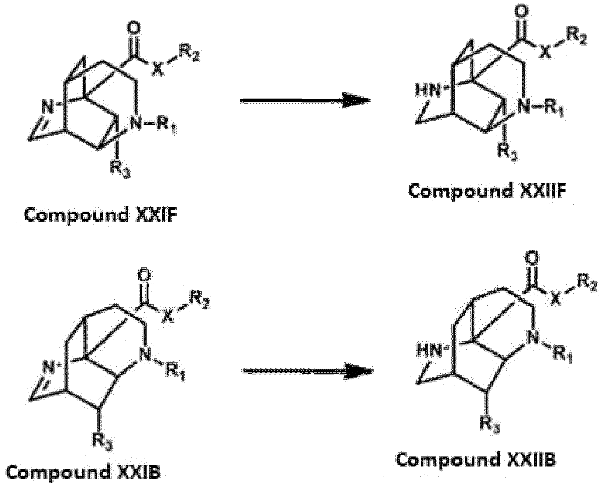

### Synthesis Scheme 22-2

### Synthesis of compound XXIIIF and compound XXIIIB

### Step 22-2

### Oxidation of imine

Compound XXIIIF (i.e., compound of formula XXIIIF) or compound XXIIIB (i.e., compound of formula XXIIIB) can be manufactured from compound XXIF (i.e., compound of formula XXIF) or compound XXIB (i.e., compound of formula XXIB) by, for example, oxidation in the following manner in accordance with a known method (e.g., method described in RSC Advances, 2012, 2, 5536-5538, Tetrahedron Lett. 50 (2009) 3436-3438, etc.). X in the following formulas is -NH-.

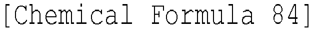

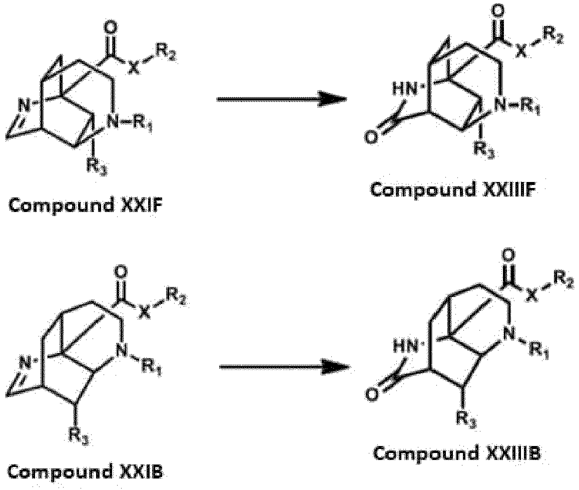

### Synthesis Scheme 23

### Synthesis of compound LLV'

A compound of formula LLV' can be manufactured, for example, by the following manufacturing method.

### (1) Route 23-1: Synthesis from compound LLXVI and compound LLXVII'

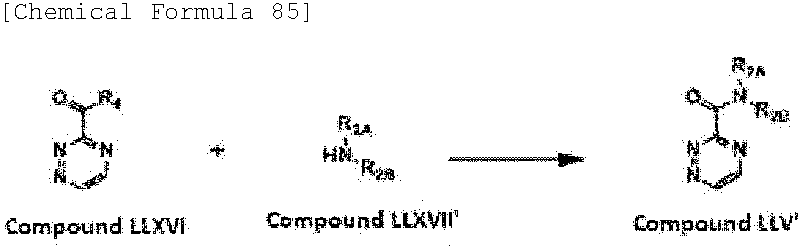

A compound of formula LLV' can be synthesized under the same condition as route 2-1 of Synthesis Scheme 2. R₈ represents alkoxy, aryloxy, hydroxy, or halogen.

### (2) Route 23-2: Synthesis from compound LLXVIII'

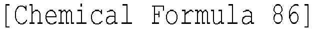

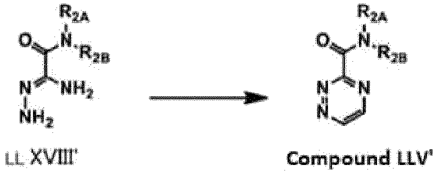

wherein R_{2A} and R_{2B} are each independently
hydrogen,
an optionally substituted hydrocarbon group,
an optionally substituted heterocycle,
optionally substituted carbonyl, or
an optionally substituted functional group, or
R_{2A} and R_{2B}, together with the nitrogen atom to which they are attached, form a heterocycle, wherein the heterocycle is, each independently, optionally substituted.

A compound that is commercially available or a compound manufactured by a known method can be used as a starting raw material compound.

### Synthesis Scheme 24

### A compound of formula LLIF and a compound of formula LLIB-Route 1

A compound of formula LLIF and a compound of formula LLIB can be manufactured through one-pot synthesis from three components in accordance with, for example, a known method (e.g., method described in Bioorg. Med. Chem. 23 (2015) 2629-2635, Tetrahedron 63 (2007) 6004-6014, Eur. J. Org. Chem. 2009, 2185-2189, Eur. J. Org. Chem. 2011, 2354-2359, etc.). 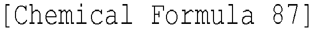 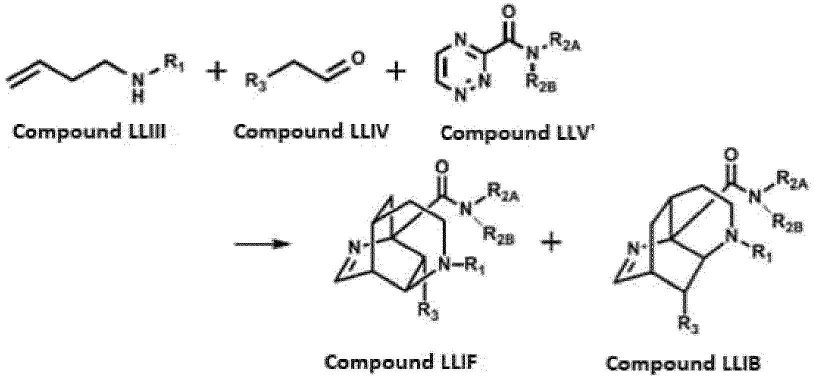 wherein R₂, R_{2A}, R_{2B}, and R₃ are the same as defined herein.

### Synthesis Scheme 25

### A compound of formula LLIF and a compound of formula LLIB-Route 2

A compound of formula LLIF and a compound of formula LLIB can be manufactured, for example, from two components as described below.

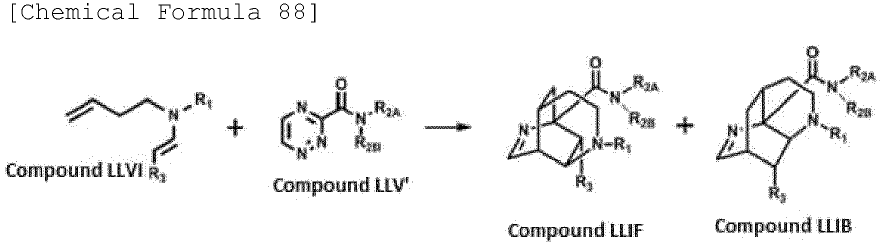

### Synthesis Scheme 26-1

### Synthesis of compound LLIIF and compound LLIIB through reduction of imine

Compound LLIIF (i.e., compound of formula LLIIF) or compound LLIIB (i.e., compound of formula LLIIB) can be manufactured from compound LLIF (i.e., compound of formula LLIF) or compound LLIB (i.e., compound of formula LLIB) by, for example, reduction in the following manner.

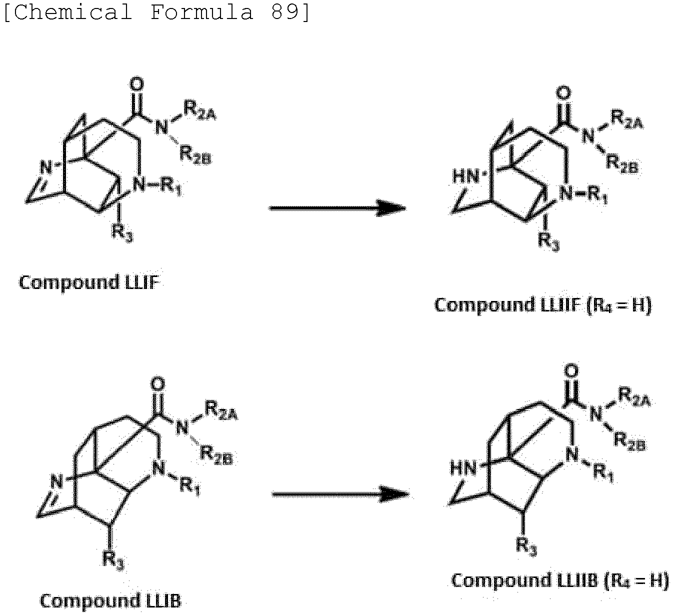

### Synthesis Scheme 27

### Synthesis of compound LLIIIF and compound LLIIIB through oxidation of imine

Compound LLIIIF (i.e., compound of formula LLIIIF) or compound LLIIIB (i.e., compound of formula LLIIIB) can be manufactured from compound LLIF (i.e., compound of formula LLIF) or compound LLIB (i.e., compound of formula LLIB) by, for example, oxidation in the following manner in accordance with a known method (e.g., method described in RSC Advances, 2012, 2, 5536-5538, Tetrahedron Lett. 50 (2009) 3436-3438, etc.).

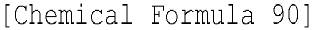

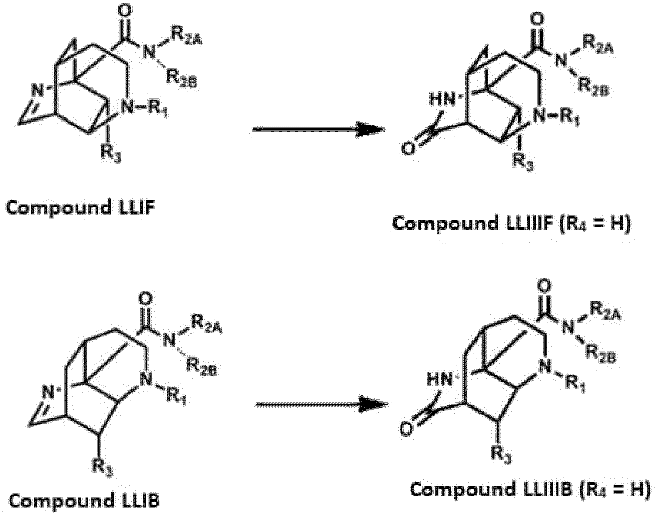

### Synthesis Scheme 28

### Synthesis of intermediates XXXIF and XXXIB

Each of intermediates XXXIF and XXXIB can be manufactured, for example, in the following manner. 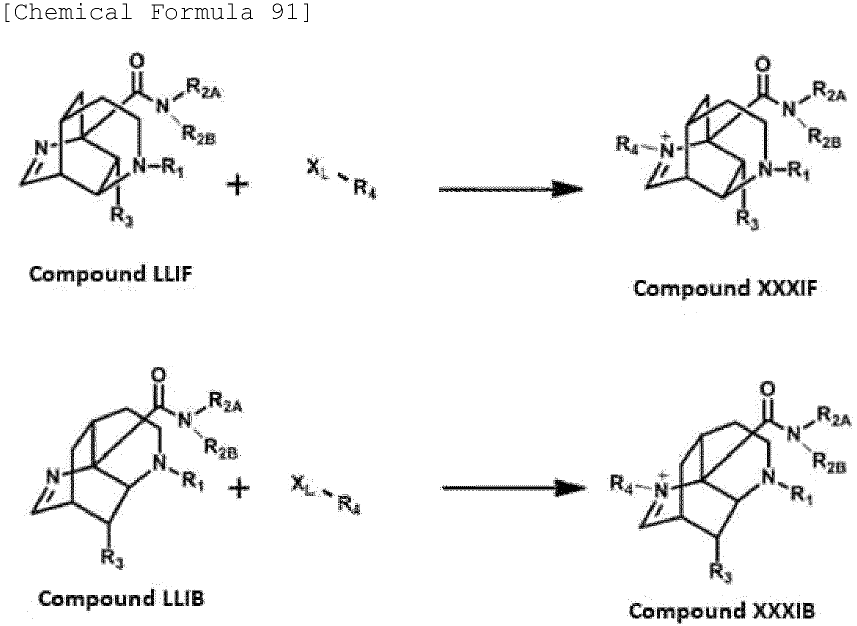 wherein X_{L} represents a leaving group in a nucleophilic substitution reaction. Examples thereof include halogen (e.g., chlorine, bromine, and iodine), sulfate ester (-SO₃H), and sulfonyl-O- group (e.g., methanesulfonyl-O-, toluenesulfonyl-O-, etc.).

### Synthesis Scheme 29

### Synthesis of compounds LLIIF, LLIIB, LLIIIF, and LLIIIB wherein R₄ is not hydrogen

Each of compounds LLIIF, LLIIB, LLIIIF, and LLIIIB wherein R₄ is not hydrogen can be manufactured from intermediate compounds XXXIF and XXXIB, for example, in the following manner.

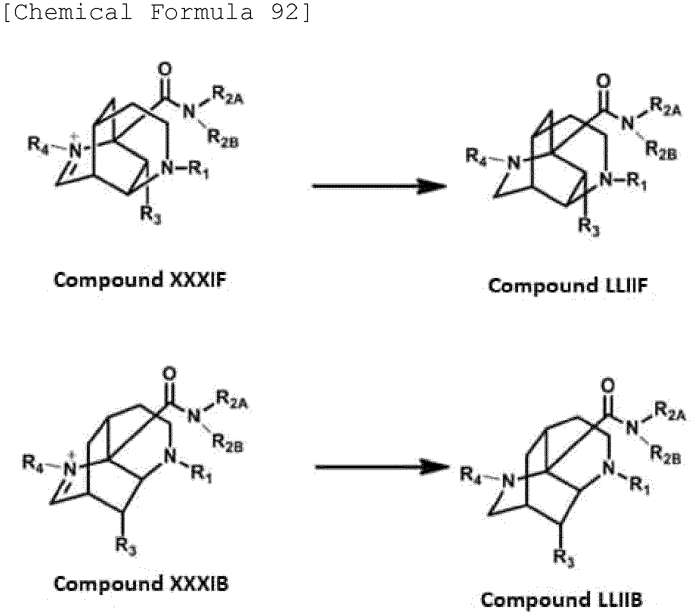

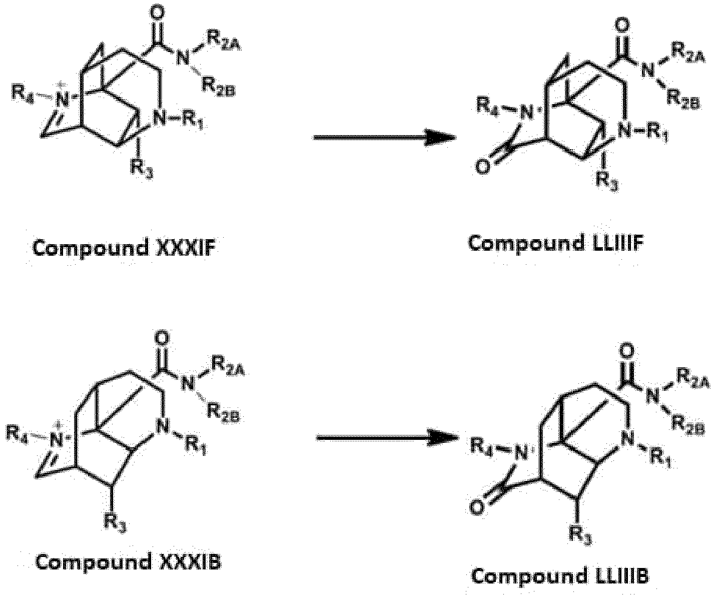

Compounds LLIIF, LLIIB, LLIIIF, and LLIIIB with various groups at R₁, R_{2A}, R_{2B}, R₃, and R₄ can be synthesized through a suitable chemical reaction condition.

### Synthesis Scheme 30

### Synthesis of compounds LLIIF, LLIIB, LLIIIF, and LLIIIB via conversion of R₁

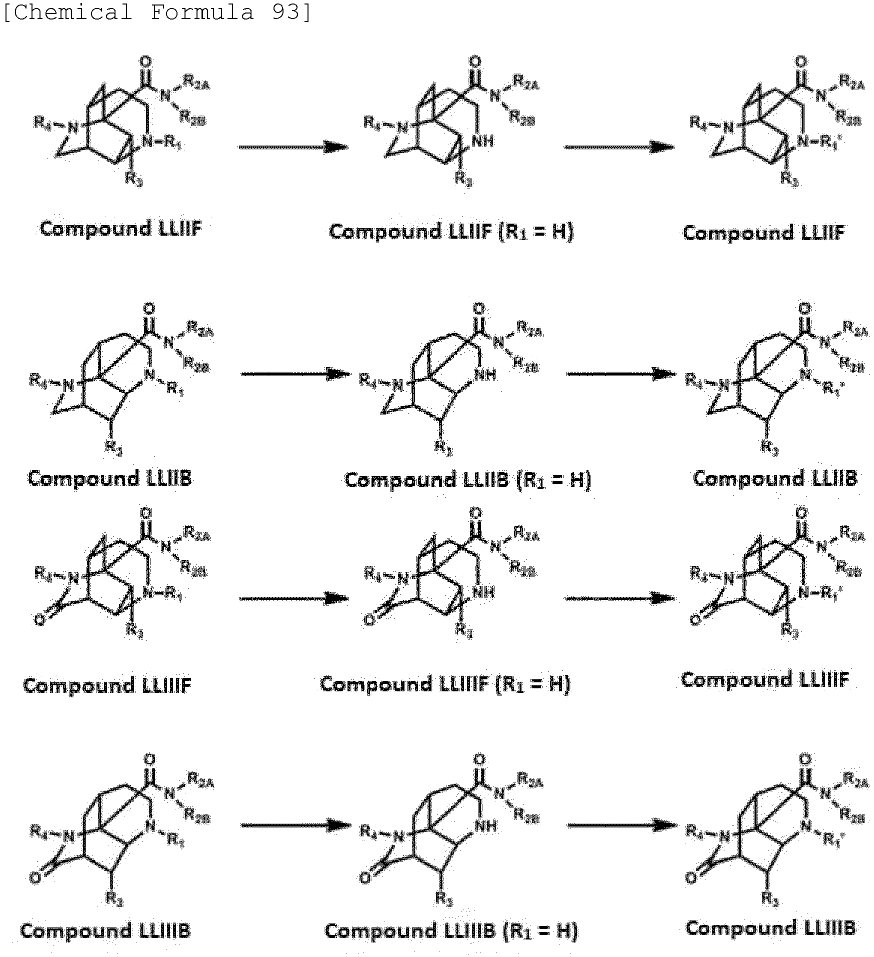

The group at R₁ of compounds LLIIF, LLIIB, LLIIIF, and LLIIIB can be converted to hydrogen through a suitable chemical reaction, and hydrogen can be further converted to different R₁'.
R₁, R_{2A}, R_{2B}, and R₃ are the same as defined herein, and R₄ is hydrogen, an optionally substituted hydrocarbon group, an optionally substituted heterocycle, optionally substituted carbonyl, or an optionally substituted functional group.

In one embodiment, a compound with a backbone of C10H16-0004 can be manufactured as described in the following Synthesis Scheme 31 to 36.

### Synthesis Scheme 31

### Manufacture of intermediate 1

Intermediate 1 can be manufactured from four components through one-pot synthesis in accordance with a known method (e.g., method described in J. Org. Chem. 2014, 1207-1214, etc.). 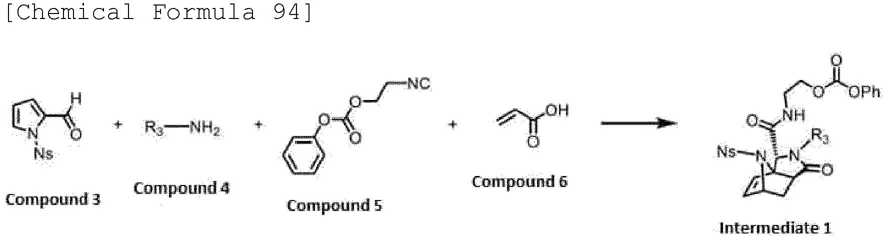 wherein R₃ is the same as defined herein.

### Synthesis Scheme 32

### Manufacture of intermediate 2

Intermediate 2 can be manufactured by subjecting intermediate 1 to a hydrolysis condition. 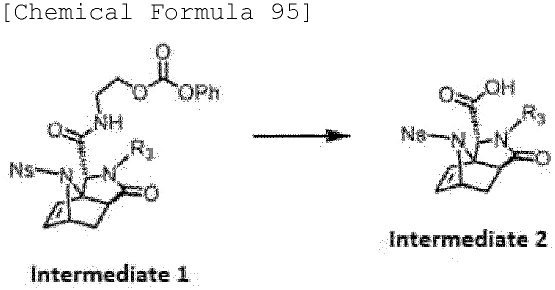 wherein R₃ is the same as defined herein.

### Synthesis Scheme 33

### Manufacture of intermediate 3

Intermediate 3 can be manufactured through a condensation reaction between intermediate 2 and compound 7. 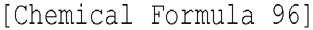 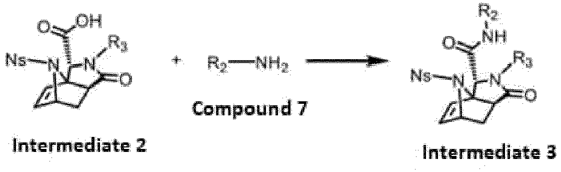 wherein R₂ and R₃ are the same as defined herein. Intermediate 3 can also be manufactured by the same method for intermediate 1 by using the following compounds. 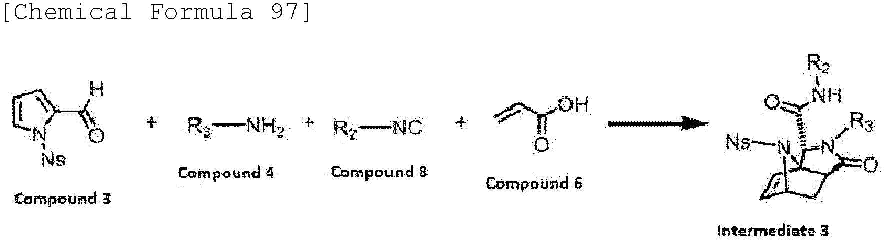 wherein R₂ and R₃ are the same as defined herein.

### Synthesis Scheme 34

### Manufacture of intermediate 4

Intermediate 4 can be manufactured by removing a 2-nitrobenzensulfonyl group (Ns group) of intermediate 3. 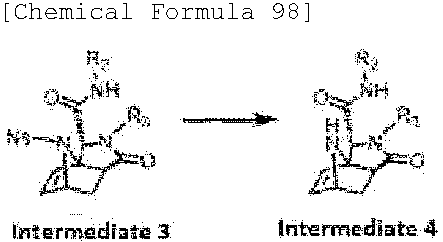 wherein R₂ and R₃ are the same as defined herein.

### Synthesis Scheme 35

### Manufacture of intermediate 5

Intermediate 5 can be manufactured, for example, from intermediate 4 through reduction in the following manner. 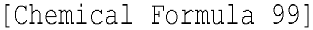 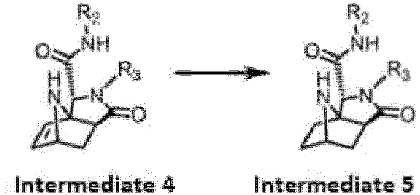 wherein R₂ and R₃ are the same as defined herein.

### Synthesis Scheme 36

### Manufacture of compound 1 or compound 2

Compound 1 or compound 2 can be manufactured by, for example, modifying intermediate 5 or intermediate 4 through an amidation, alkylation, or other reactions. 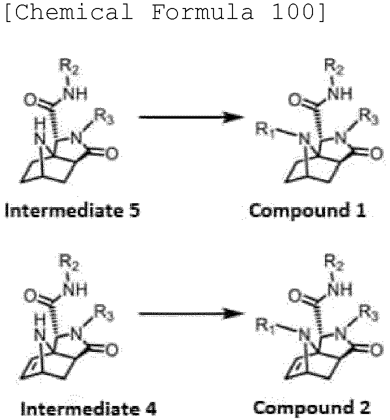 wherein R₁, R₂, and R₃ are the same as defined herein.

Compound 1 can also be manufactured by reducing compound 2. 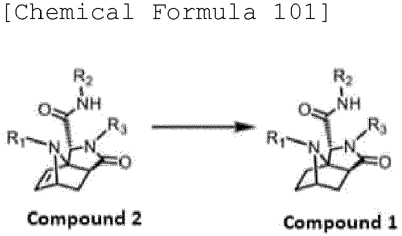 wherein R₁, R₂, and R₃ are the same as defined herein.

After introducing an appropriate heteroatom into a calculated backbone and preparing a backbone that is readily synthesized, a position where a substituent is readily introduced can be determined (may be based on calculation of an attachment point) to introduce an amino acid-like structure to said position. The synthesis examples described above can be practiced by appropriately replacing a hydrogen atom or based on the disclosed Examples.

An amino acid-like structure used in the present disclosure can be designed and added/synthesized through reductive amination using substituted amine and substituted aldehyde, alkylamination using substituted amine and substituted halide, introduction of an allyl group or an alkyl group through a Diels-Alder reaction, amidation using substituted carboxylic acid and substituted amino group, substituted amidation using substituted ester and a substituted amino group, amidation using substituted active ester and a substituted amino group, an Ugi reaction using substituted imine, substituted carboxylic acid, and substituted isonitrile, a Horner-Emmons reaction, synthesis of unsaturated ester through aldol condensation, etc.

### (5. Preferred embodiments)

The preferred embodiments of the present disclosure are described hereinafter. It is understood that the embodiments provided hereinafter are provided for the better understanding of the present disclosure, so that the scope of the present disclosure should not be limited by the following descriptions. Thus, it is apparent that those skilled in the art can refer to the descriptions herein to make appropriate modifications within the scope of the present disclosure. It is also understood that the following embodiments of the present disclosure can be used individually or as a combination.

### (Backbone design)

In one aspect, the present disclosure provides a carbon backbone that is useful in a functional molecule such as a peptidomimetic and a technology for efficiently designing and/or generating such a carbon backbone.

The present disclosure provides a method of designing a compound backbone with a desired structure, comprising the steps of: (1) generating a molecular formula of a compound comprising carbon; (2) randomly generating molecules using an adjacency matrix, etc. to extract a compound falling under the molecular formula among the randomly generated molecules; and (3) selecting a compound satisfying a predefined condition, a program describing a code for implementing said method on a computer, a computer readable recording medium that has said program recorded thereon, and a system, apparatus, or device for implementing the same. The method can be performed by manual calculation or by a computer.

In one specific embodiment, the desired structure of the present disclosure is a cage-like carbon backbone. Examples of a cage-like carbon structure include structures with three or more ring structures that are not on the same plane such as those that are fused, etc.

In a preferred embodiment, a predefined condition can advantageously satisfy at least one, preferably two, more preferably three, and most preferably all four of condition 1: all carbon atoms and bonds are within a 5- or 6- membered ring (in another embodiment, may comprise atoms other than carbon atoms), condition 2: there is no spiro ring; condition 3: there are two or more atoms that are present in common in at least three ring structures within a molecule (ring structure under condition 1 is a 5- or 6-membered ring); and condition 4: energy is 150 kcal/mol or less (this value may be, for example, a numerical range from 50 to 150 kcal/mol, etc.) in an MMFF (Merck Molecular Force Field) when a backbone comprising only a single bond is assumed upon generation of a 3D structure.

In a specific embodiment, the molecular formula is, but not limited to, one selected from the group consisting of C₁₀H₁₆, C₁₁H₁₆, C₁₁H₁₈, C₁₂H₁₈, C₁₂H₂₀, C₁₃H₂₀, and C₁₃H₂₀.

In another aspect, the present disclosure provides a method of generating a compound having a compound backbone with a desired structure, comprising the steps of: (1) causing a computer to generate a molecular formula of a compound comprising carbon; (2) causing a computer to randomly generate molecules using an adjacency matrix, etc. to extract a compound satisfying the molecular formula among the randomly generated molecules; (3) selecting a compound satisfying a predefined condition; and (4) producing a compound having a backbone structure selected in (3), a program describing a code for implementing said method on a computer, a computer readable recording medium that has said program recorded thereon, and a system, apparatus, or device for implementing the same. Such program, etc. may be combined with means for producing a compound with a backbone structure selected in (3).

A compound that can be generated by the present disclosure comprises at least one type of bond selected from a single bond, a double bond, and a triple bond in a backbone forming moiety, preferably a single bond and a double bond in a backbone forming moiety, or only a single bond in a backbone forming moiety. As used herein, "backbone forming moiety" refers to the main backbone of a compound excluding side chains and substituents. A compound that can be generated by the present disclosure, when displayed in SMILES, include, but not limited to, the following backbones.

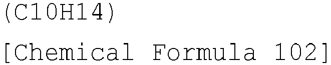

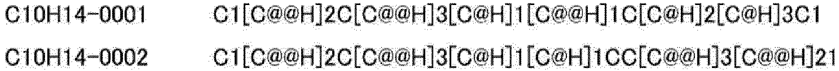

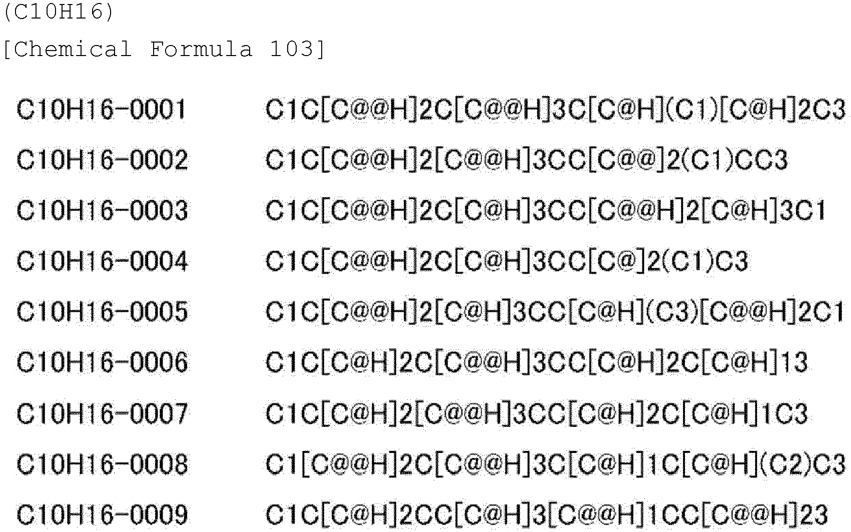

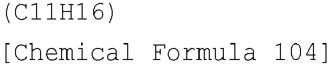

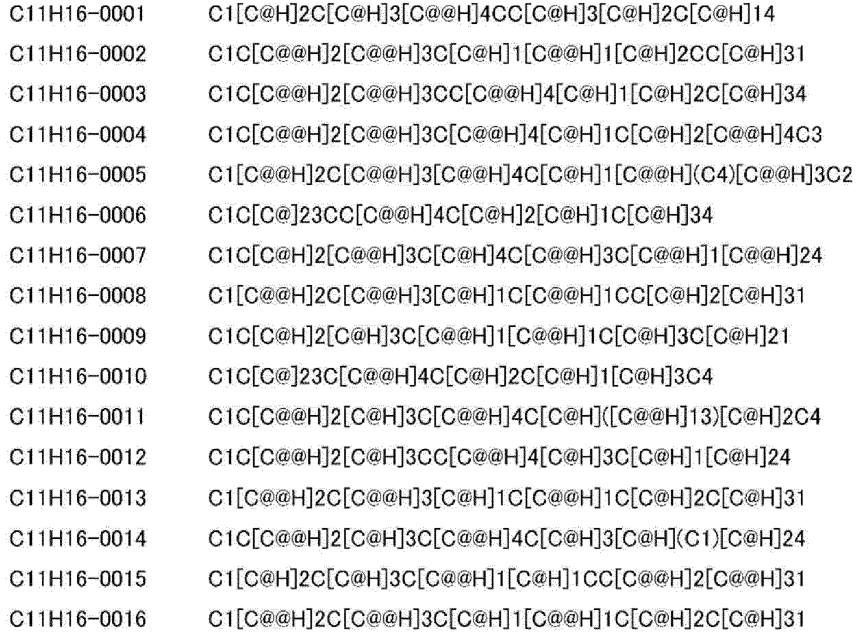

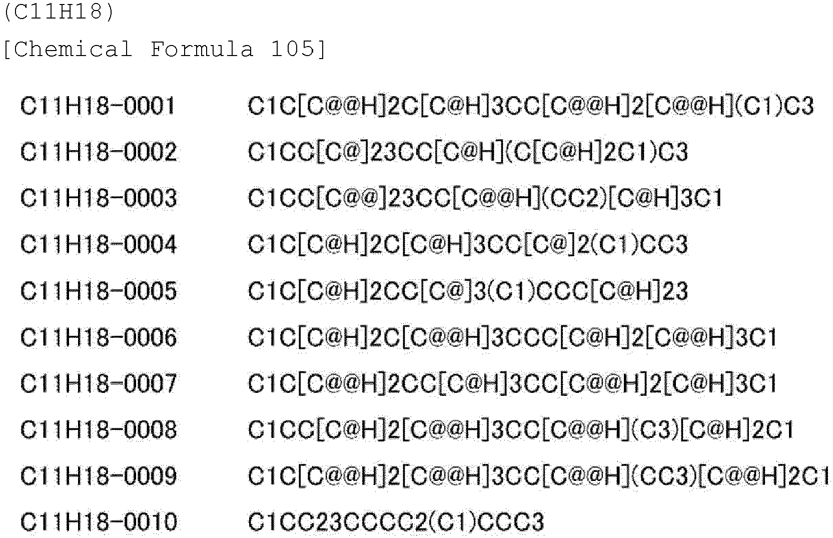

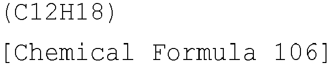

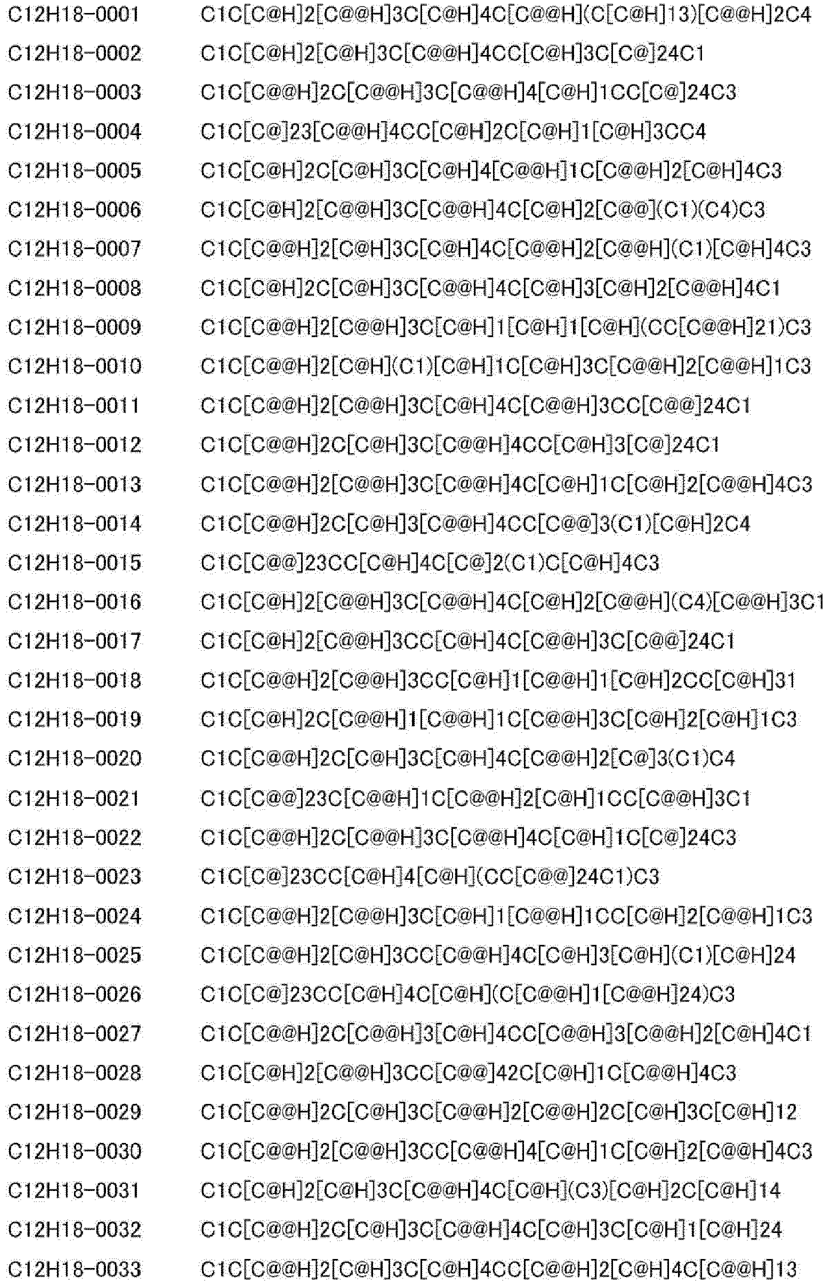

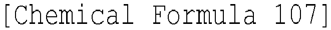

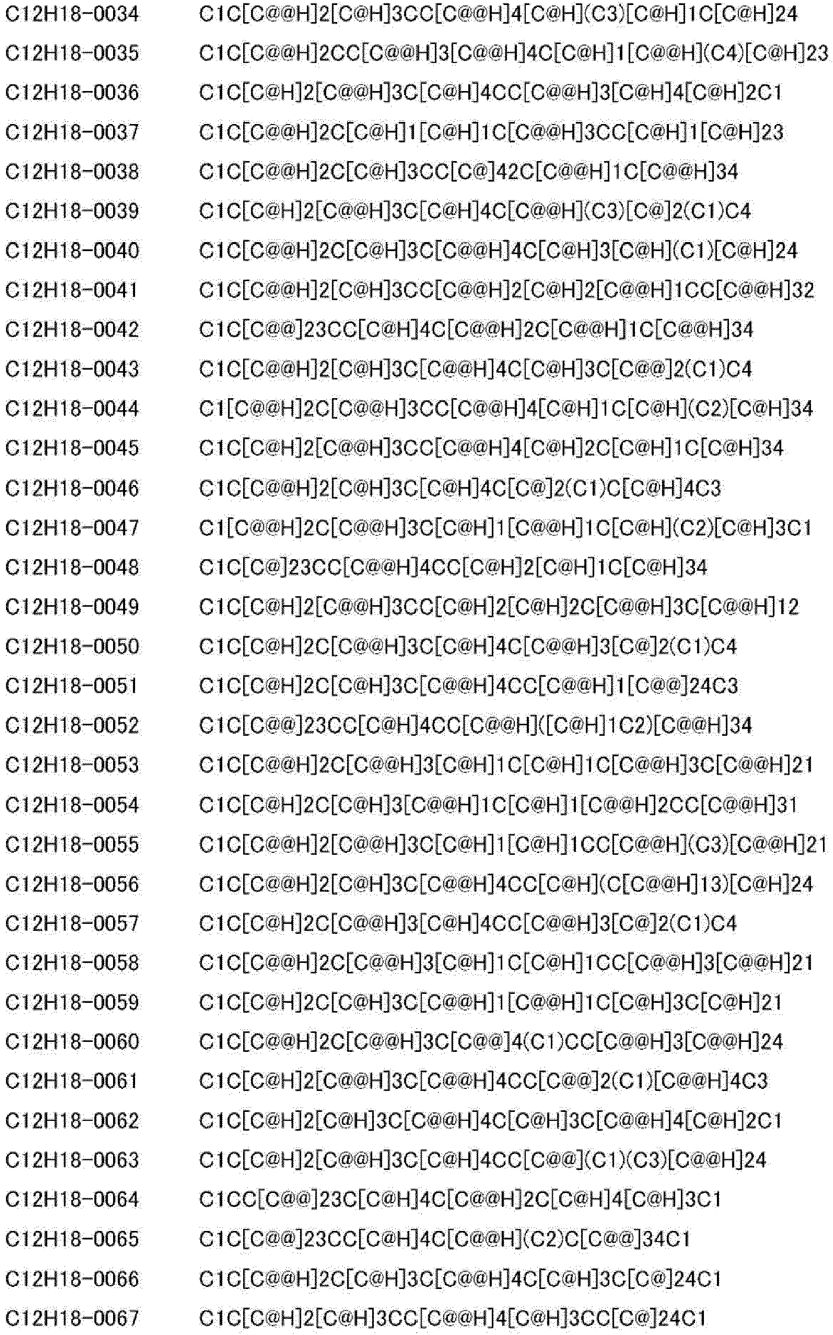

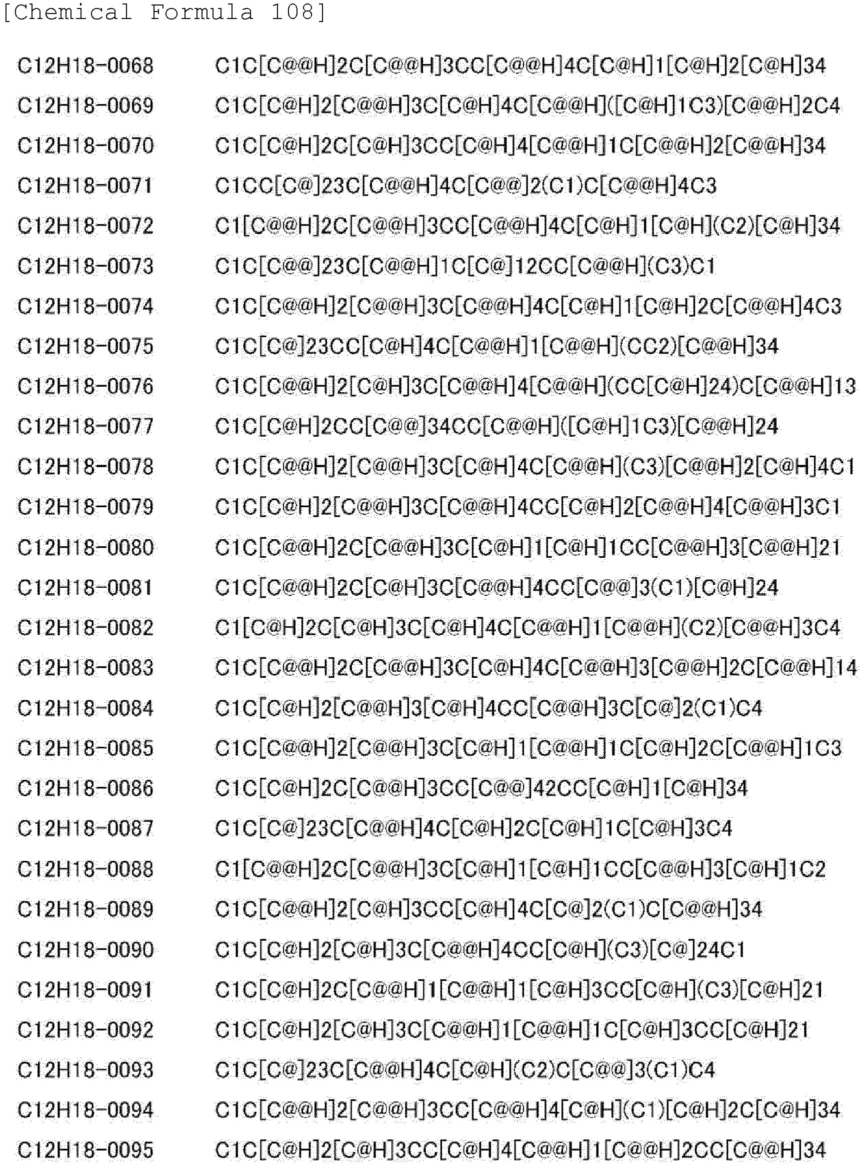

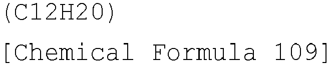

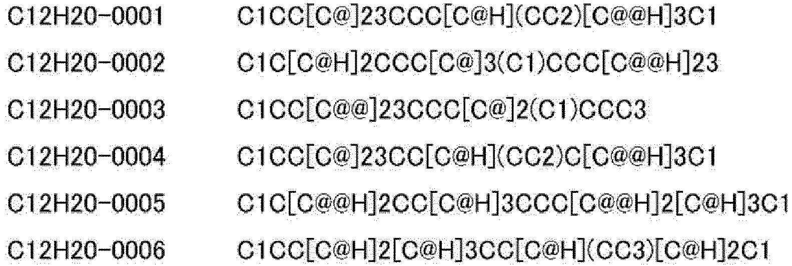

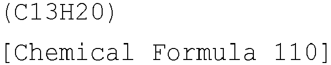

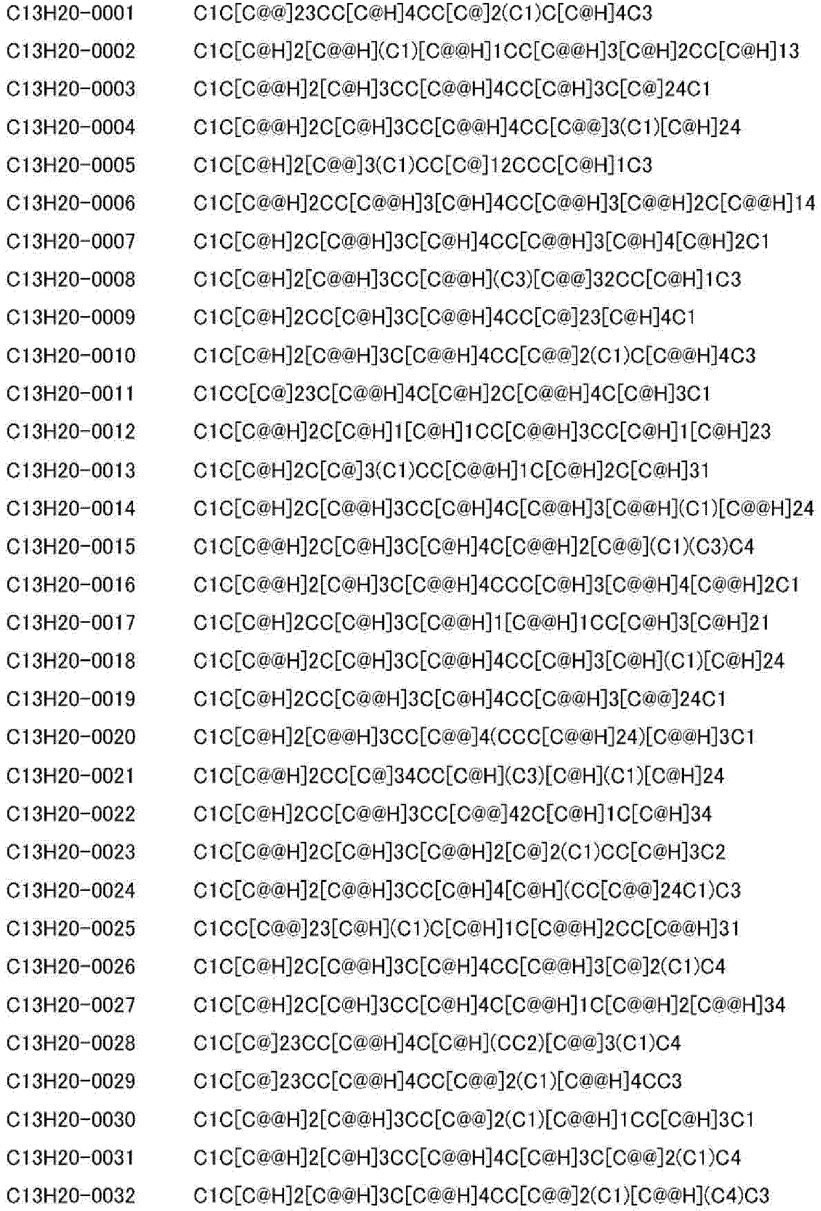

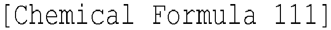

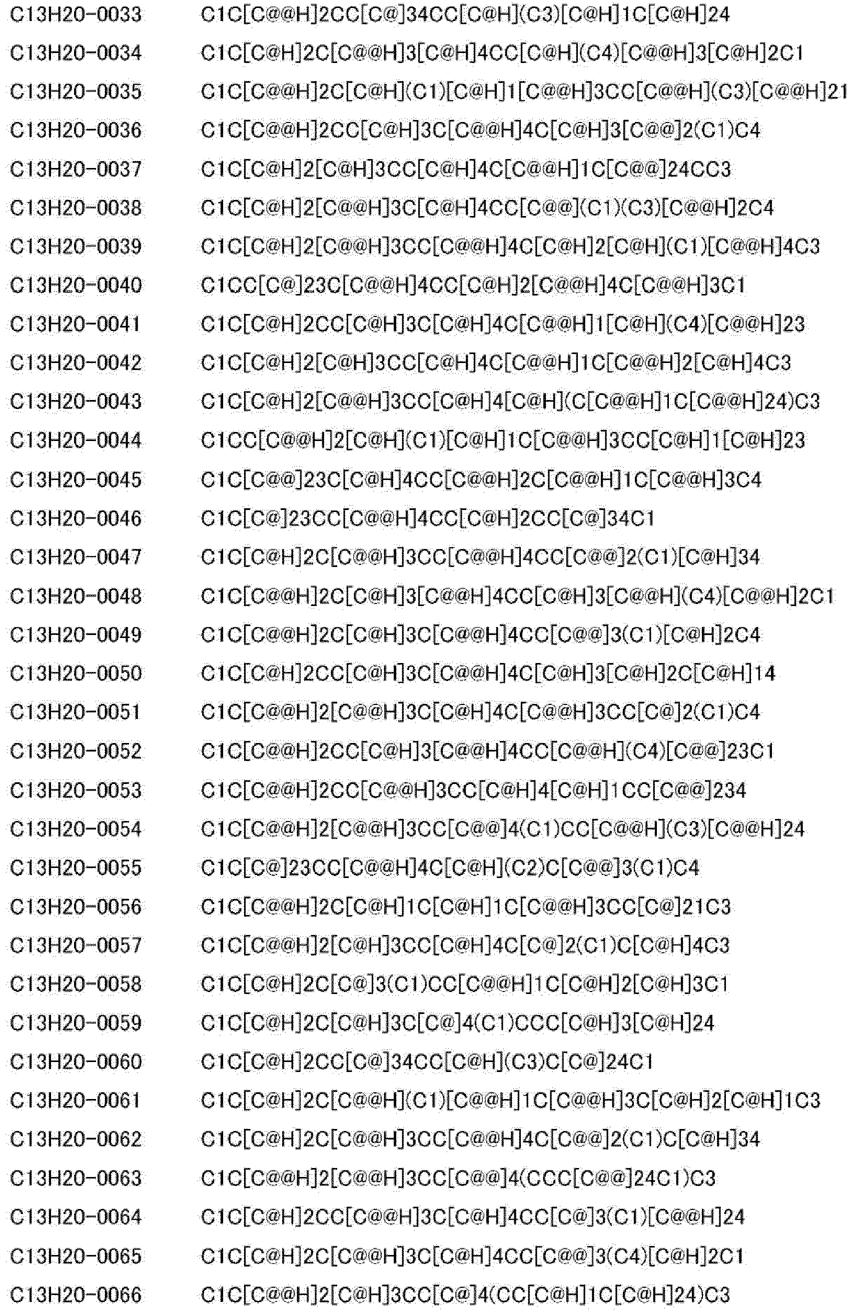

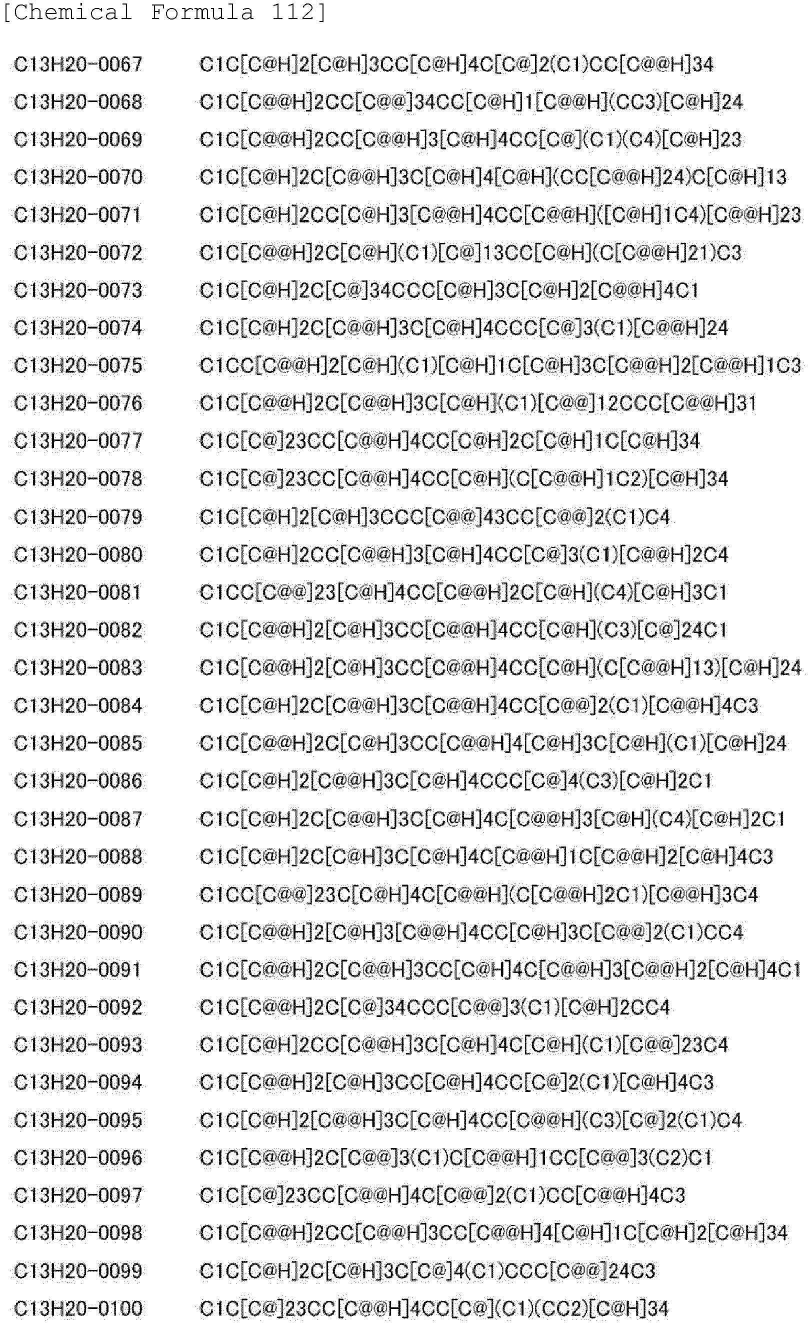

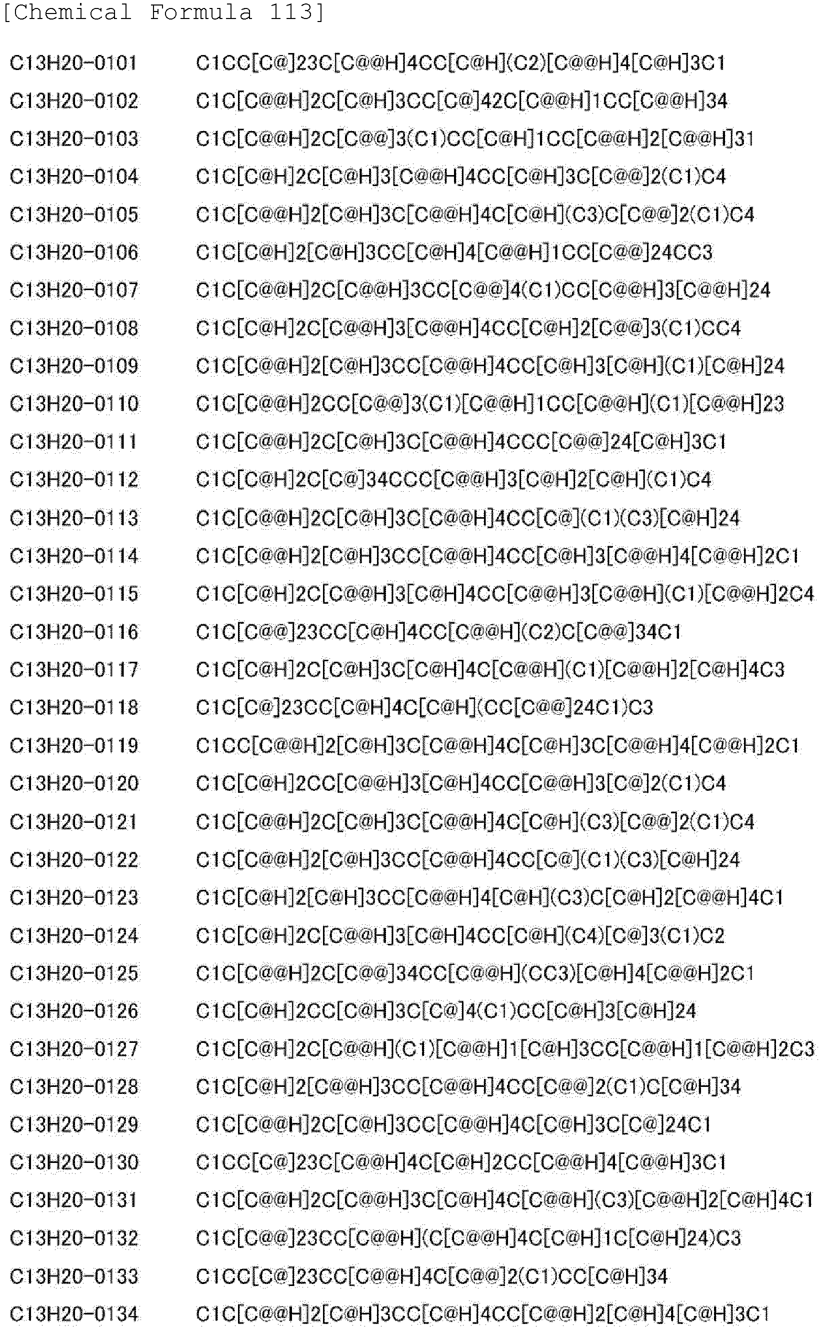

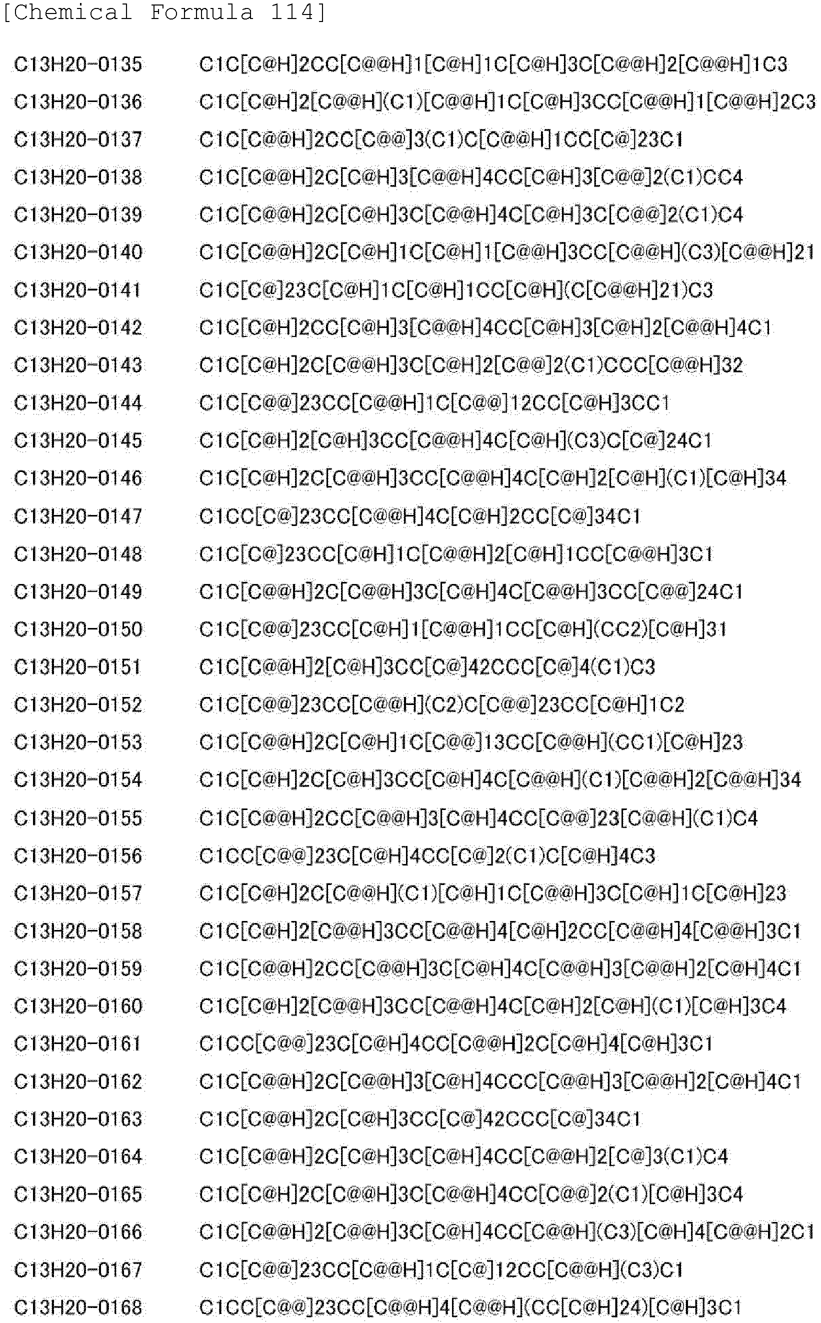

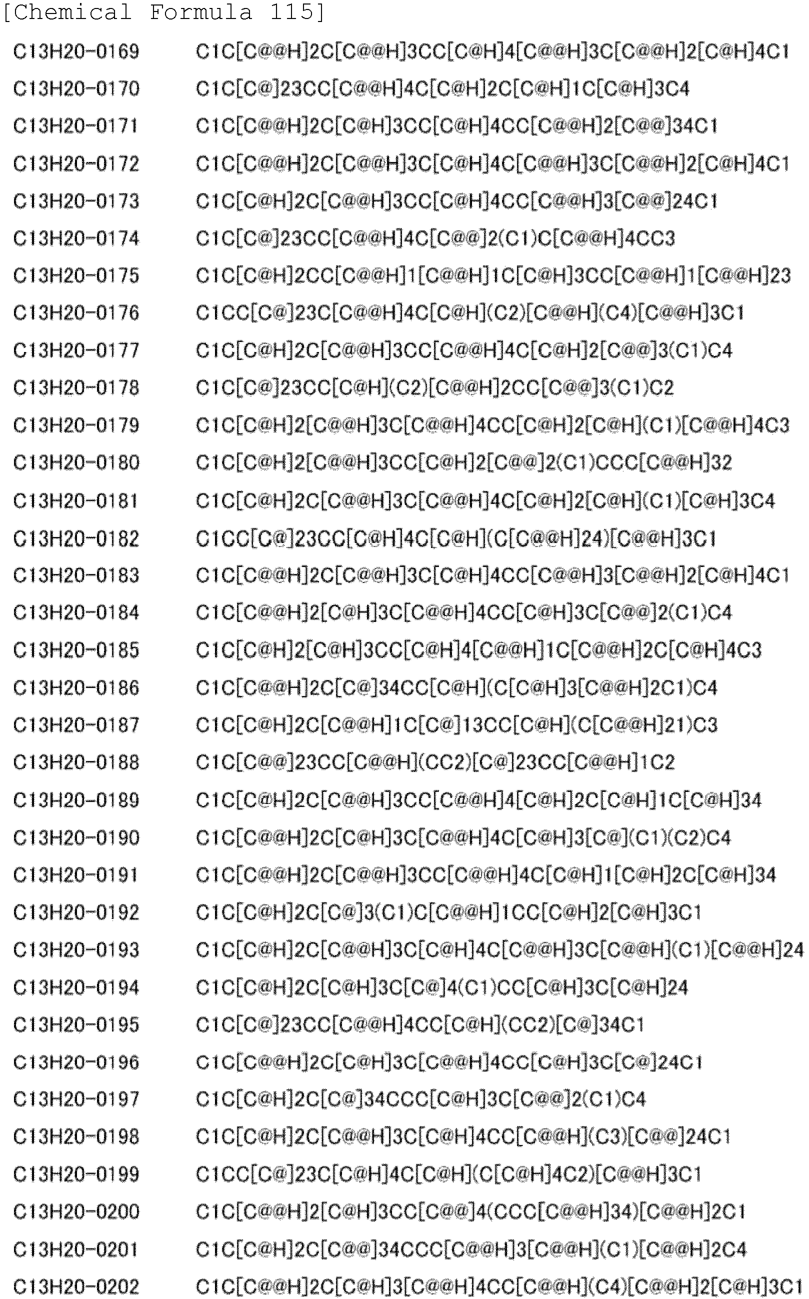

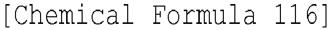

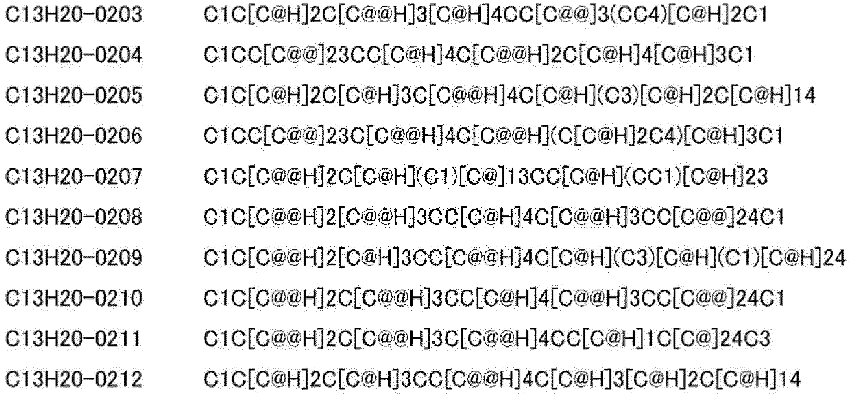

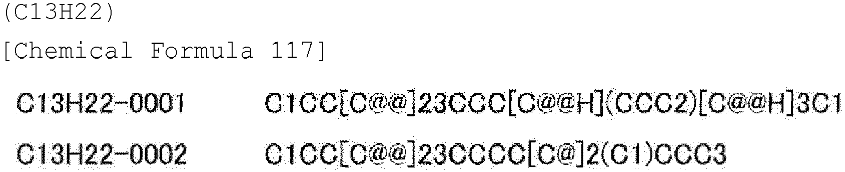

### (Peptidomimetic molecule framework)

In another aspect, the present disclosure provides a peptidomimetic molecule framework and design/generation thereof.

In one embodiment, the present disclosure provides a method of designing a peptidomimetic molecule framework, comprising the steps of causing a computer to: (A) provide structural data (can be provided in a format such as SMILES, but the format is not limited thereto) for a carbon moiety of a predefined carbon backbone (e.g., cage-like carbon backbone); (B) generate an attachment point (AP), which is a position where a side chain structure is given from the structural data for the carbon backbone; (C) randomly select APs at M locations; (D) optionally, generate a carbon backbone with N APs by repeating steps (A) to (C) N times, wherein N is a positive integer; (E) optionally, comprehensively add the same or different amino acid-like structure to each AP at M locations (M is a positive integer with no particular upper limit, but the number of APs itself can be the upper limit) to generate an amino acid-like structure-containing carbon structure; (F) fit the amino acid-like structure-containing carbon structure to a pharmacophore obtained from a three-dimensional structure of a predefined peptide, and select a carbon structure that has fit as a peptidomimetic molecule framework; a program encoding the same, a recording medium with this recorded on a computer readable medium, a system, apparatus, or device materializing the same, etc.

In one embodiment, N is typically 1000 or greater, but can be, for example, at least 5 or greater, 10 or greater, 20 or greater, 50 or greater, 100 or greater, 300 or greater, 500 or greater, 1000 or greater, 3000 or greater, 10000 or greater, 100000 or greater, etc. in step (D). In a specific embodiment, M is an integer from 2 to 6, preferably 2 to 5, and more preferably 2 to 4. Although not wishing to be bound by any theory, this is because 1 is often insufficient in terms of a peptidomimetic, which is often optimized with about 3.

In one embodiment, an amino acid used in step (E) can be, but is not limited to, leucine, phenylalanine, isoleucine, tryptophan, etc. It can be better to use, instead of a naturally-occurring chain (amino acid side chain itself), a longer carbon chain, branched chain, etc. as a result of fitting for an amino acid-like structure used in this regard. Examples of practicing the present disclosure include, when a target is clearly determined, a method of optimizing fitting to a compound, and when a target is not clearly determined, preparing various similar structures as a library as a population for screening, etc. An example of backbone selection and compound optimization is described. A backbone can be selected by pharmacophore screening on pockets of actual target protein after generating a side chain. A synthetic compound can be designed by using a computer when the three-dimensional structure of a target is clear. For the periphery of an active compound obtained by screening a library, a carbon chain can be adjusted, a substituent can be introduced to a benzene ring, or a heteroatom can be introduced into a side chain structure to search for a side chain that interacts therewith. Although not wishing to be bound by any theory, this is because a molecular backbone formed with a peptidomimetic molecule framework mimics a naturally-occurring peptide, but naturally-occurring peptides often do not completely fit with a target, so that a deviation in naturally-occurring peptides is reflected when a naturally-occurring structure is used as an amino acid-like structure. Such a deviation can be refined, or variation can be introduced through fitting by using a framework designed by the present disclosure.

In one embodiment, step (F) comprises causing a computer to select top K carbon backbones with a best fitting score of the pharmacophore (wherein K is a positive integer which is less than N).

A carbon backbone used in designing a peptidomimetic molecule framework herein may be obtained by a method of designing and generating a backbone of the present disclosure or obtained through other means.

In another aspect, the present disclosure provides a method of generating a compound having a peptidomimetic molecule framework, comprising the steps of: (A) providing to a computer structural data (can be represented, for example, in the SMILES format) for a carbon moiety of a predefined carbon backbone (e.g., cage-like carbon backbone); (B) causing a computer to generate an attachment point (AP), which is a position where a side chain structure is given from the structural data for the carbon backbone; (C) causing a computer to randomly select APs at M locations (M is any positive integer); (D) optionally, causing a computer to generate a carbon backbone with N APs by repeating step (A) to (C) N times, wherein N is a positive integer; (E) optionally, causing a computer to comprehensively add the same or different amino acid-like structure to each AP at M locations to generate a carbon backbone with an amino acid side chain structure; (F) causing a computer to fit the carbon structure with the amino acid side chain structure to a pharmacophore obtained from a three-dimensional structure of a predefined peptide, and select a carbon structure that has fit as a peptidomimetic molecule framework; and (G) producing a compound with the peptidomimetic molecule framework selected in (F), a program describing a code for implementing said method on a computer, a computer readable recording medium that has said program recorded thereon, and a system, apparatus, or device for implementing the same. These programs, etc. may be combined with a candidate compound having a peptidomimetic molecule framework selected in (F).

In one embodiment, the compound of the present disclosure comprises at least one type selected from a single bond, a double bond, and a triple bond.

The present disclosure provides a peptidomimetic molecule framework obtained by the method of the present disclosure.

### (System, etc.)

The system of the present disclosure can be provided as a system LSI, etc. A system LSI is an ultramultifunctional LSI manufactured by integrating a plurality of components on a single chip. Specifically, the system can be a computer system comprised of a microprocessor, ROM (read only memory), RAM (random access memory), etc. A computer program is stored in the ROM. A system LSI can accomplish its function by operating the microprocessor in accordance with the computer program.

The system was referred to as system LSI, but may also be referred to as IC, LSI, super LSI, or ultra LSI depending on the difference in the degree of integration. The approach of building an integrated circuit is not limited to LSI. The system may be materialized with a dedicated circuit or a generic processor. After the manufacture of LSI, a programmable FPGA (Field Programmable Gate Array) or reconfigurable processor which allows reconfiguration of the connection or setting of circuit cells inside the LSI may be utilized.

If a technology of building integrated circuits that replaces LSI by advances in semiconductor technologies or other derivative technologies becomes available, functional blocks may be obviously be integrated using such technologies. Application of biotechnology, etc. is also a possibility.

One embodiment of the present disclosure is a system for materializing the method of the present disclosure, or the embodiment may be a computer program for causing a computer to execute each characteristic step contained in each method. One embodiment of the present disclosure may be a computer readable non-transitory recording medium with such a computer program recorded thereon. One embodiment of the present disclosure may also be a computer product or program product for materializing such a computer program. One embodiment of the present disclosure may be a learned model which has learned such a computer program through machine learning, etc., or a program, computer product, or program product for materializing such a learned model. Any information and/or data used in the present disclosure can be provided and utilized as data for the generation of such a learned model.

In each of the embodiments described above, each constituent element may be comprised of a dedicated hardware or materialized by executing a software program that is suited to each constituent element. Each constituent element may be materialized by a program execution unit such as a CPU or a processor reading out and executing a software program recorded on a recording medium such as a hard disk or semiconductor memory. In this regard, software materializing the pain estimation apparatuses of each of the embodiments described above, etc. can be a program described above herein. The storage unit may be a recording medium such as CD-R, DVD, Blueray, USB, SSD, or hard disk, or the storage unit may be stored in a server or configured in a form that appropriately records on the cloud.

Machine learning can be utilized in the method of the present disclosure. As used herein, "machine learning" refers to a technology for imparting a computer with the ability to learn without explicit programming. This is a process of improving a function unit's own performance by acquiring new knowledge/skill or reconstituting existing knowledge/skill. Most of the effort required for programming details can be reduced by programming a computer to learn from experience. In the machine learning field, a method of constructing a computer program that enables automatic improvement from experience has been discussed. Data analysis/machine learning plays a role as elemental technology that is the foundation of intelligent processing along with a field of algorithms. Generally, data analysis/machine learning is utilized in conjunction with other technologies, thus requiring the knowledge in the linked field (domain specific knowledge; e.g., medical field). The range of application thereof includes roles such as prediction (collect data and predict what would happen in the future), search (find a notable feature from collected data), and testing/describing (find relationship of various elements in the data). Machine learning is based on an indicator indicating the degree of achievement of a goal in the real world. The user of machine learning must understand the goal in the real world. An indicator that improves when an objective is achieved needs to be formulated. Machine learning has the opposite problem that is an ill-posed problem for which it is unclear whether a solution is found. The behavior of the learned rule is not definitive but is stochastic (probabilistic). Machine learning requires an innovative operation with the premise that some type of uncontrollable element would remain. The present disclosure can be considered as means for solving such an issue. It is useful for a user of machine learning to successively pick and choose data or information in accordance with the real world goal while observing performance indicators during training and operation.

Linear regression, logistic regression, support vector machine, etc. can be used for machine learning, and cross validation (CV) can be performed to compute differentiation accuracy of each model. After ranking, a feature can be increased one at a time for machine learning (linear regression, logistic regression, support vector machine, etc.) and cross validation to compute the differentiation accuracy of each model. A model with the highest accuracy can be selected thereby. Any machine learning can be used herein. Linear, logistic, support vector machine (SVM), etc. can be used as supervised machine learning.

As used herein, "weighting coefficient" is a coefficient that is set so that an important element is calculated as more important in the calculation of the present disclosure, including approximation coefficients. For example, a coefficient can be obtained by approximating a function to data, but the coefficient itself only has a description indicating the degree of approximation. When coefficients are ranked or chosen/discarded on the basis of the magnitude, etc., a difference in contribution within the model is provided to a specific feature, so that this can be considered a weighting coefficient. A weighting coefficient is used in the same meaning as an approximation index of a differentiation function. Examples thereof include R² value, correlation coefficient, regression coefficient, residual sum of squares (difference in feature from differentiation function), etc. These can be used for fitting.

### (Compound)

In another aspect, the present disclosure provides a cyclic compound having a backbone satisfying, when calculated in terms of hydrocarbon:
condition 1: all carbon atoms and bonds are within a 5- or 6- membered ring;
condition 2: there is no spiro ring;
condition 3: there are two or more atoms that are present in common in at least three ring structures within a molecule (ring structure under condition 1 is a 5- or 6-membered ring); and
condition 4: energy is, for example, 150 kcal/mol or less in an MMFF upon generation of a 3D structure;
wherein the cyclic compound optionally has a heteroatom in place of a carbon atom within a backbone and optionally has one or more substituents.

Condition 1 can be for only a 6-membered ring or for only a 5-membered ring, but it is preferable that both coexist.

Condition 3 may be changed from at least three to four, five, etc., or from two or more to three or more, four or more, etc. within a molecule. These numbers can be appropriately varied in accordance with the objective and circumstance.

Condition 4 may be changed from MMFF of 150 to 100 or less, 75 or less, 50 or less, etc., or an upper limit and a lower limit may be provided (e.g., 50 to 150). Other parameters described herein may also be used.

In one aspect, the present disclosure also provides a peptidomimetic compound having a backbone satisfying when calculated in terms of hydrocarbon:
condition 1: all carbon atoms and bonds are within a 5- or 6- membered ring;
condition 2: there is no spiro ring;
condition 3: there are two or more atoms that are present in common in at least three ring structures within a molecule (ring structure under condition 1 is a 5- or 6-membered ring); and
condition 4: energy is, for example, 150 kcal/mol or less in an MMFF upon generation of a 3D structure;
   and M substituents (wherein M is any integer from 1 to (number of atoms in a backbone moiety that can have a substituent)) corresponding to M amino acid-like structures (amino acid side chain or an equivalent thereof) from the backbone.

A preferred embodiment can also comprise fitting and selecting from a top score.

In a specific embodiment, the present disclosure provides a cyclic compound having any one of the following backbones: 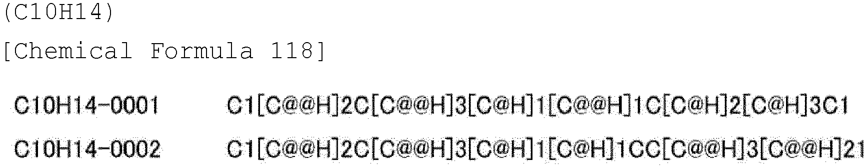 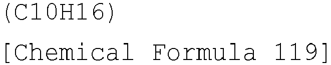 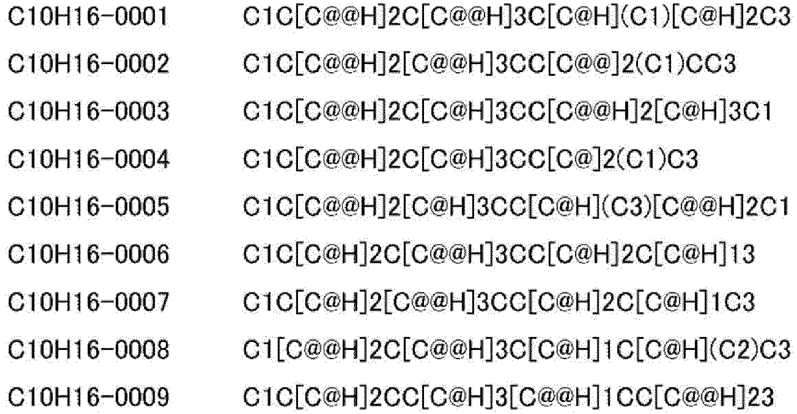 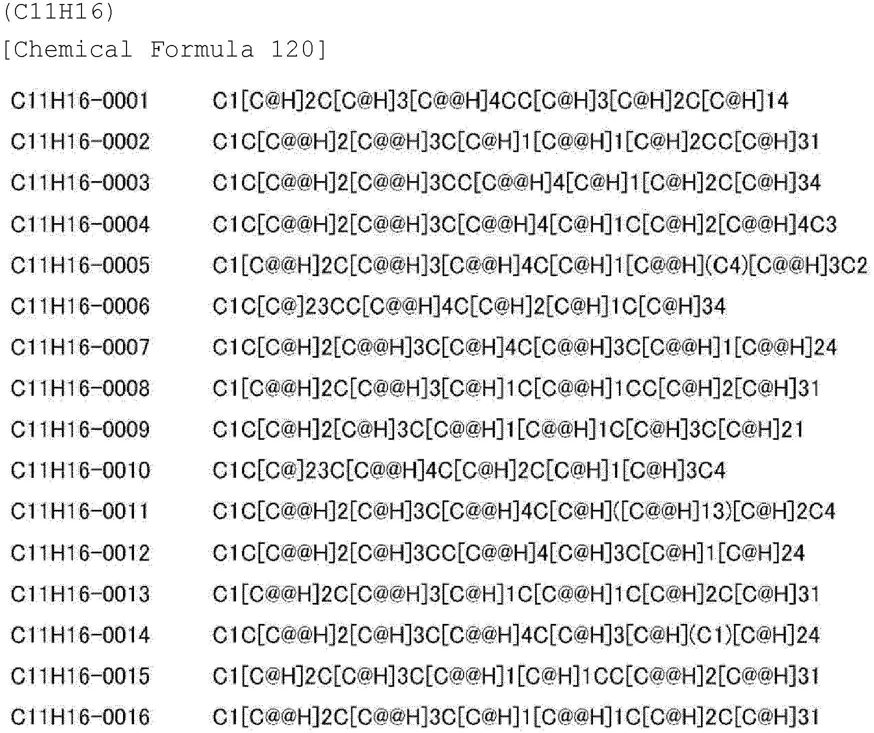 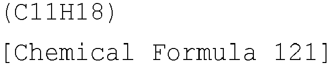 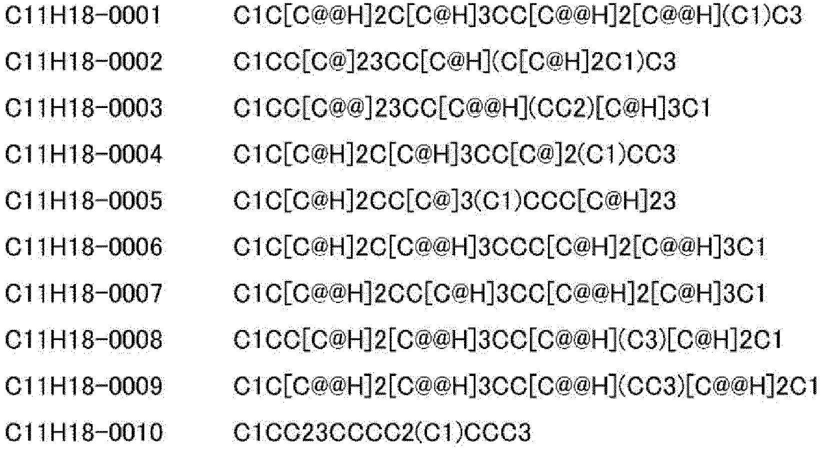 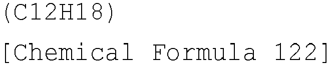 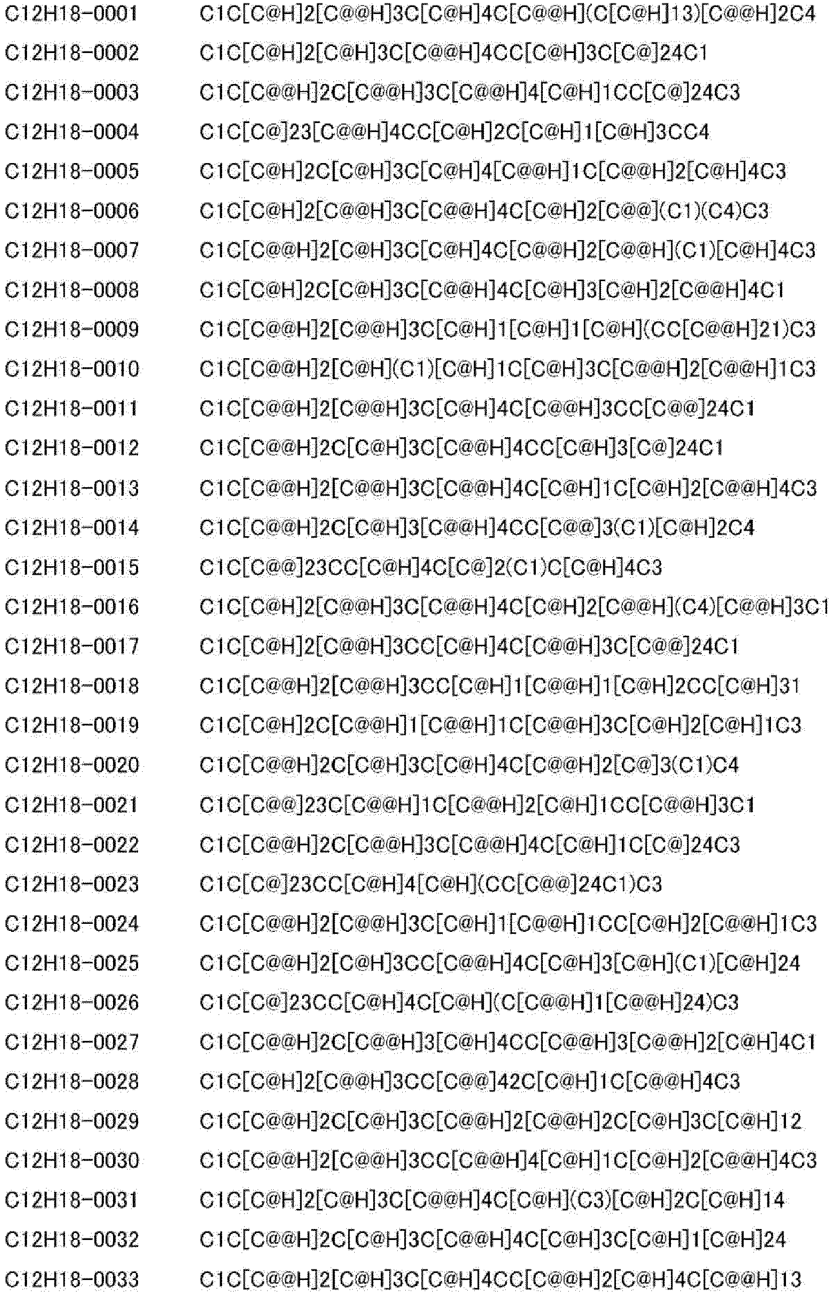 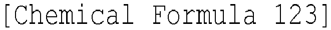 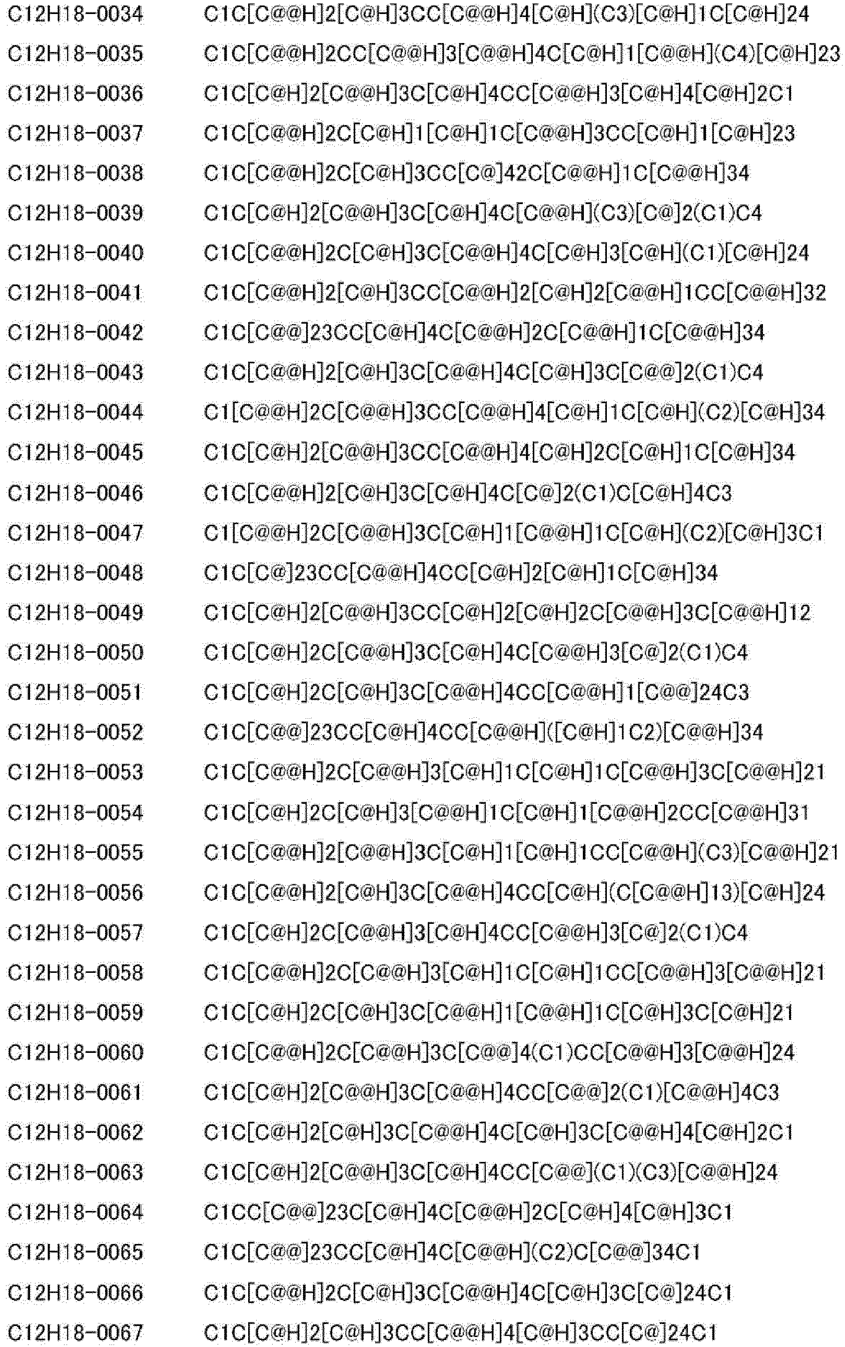 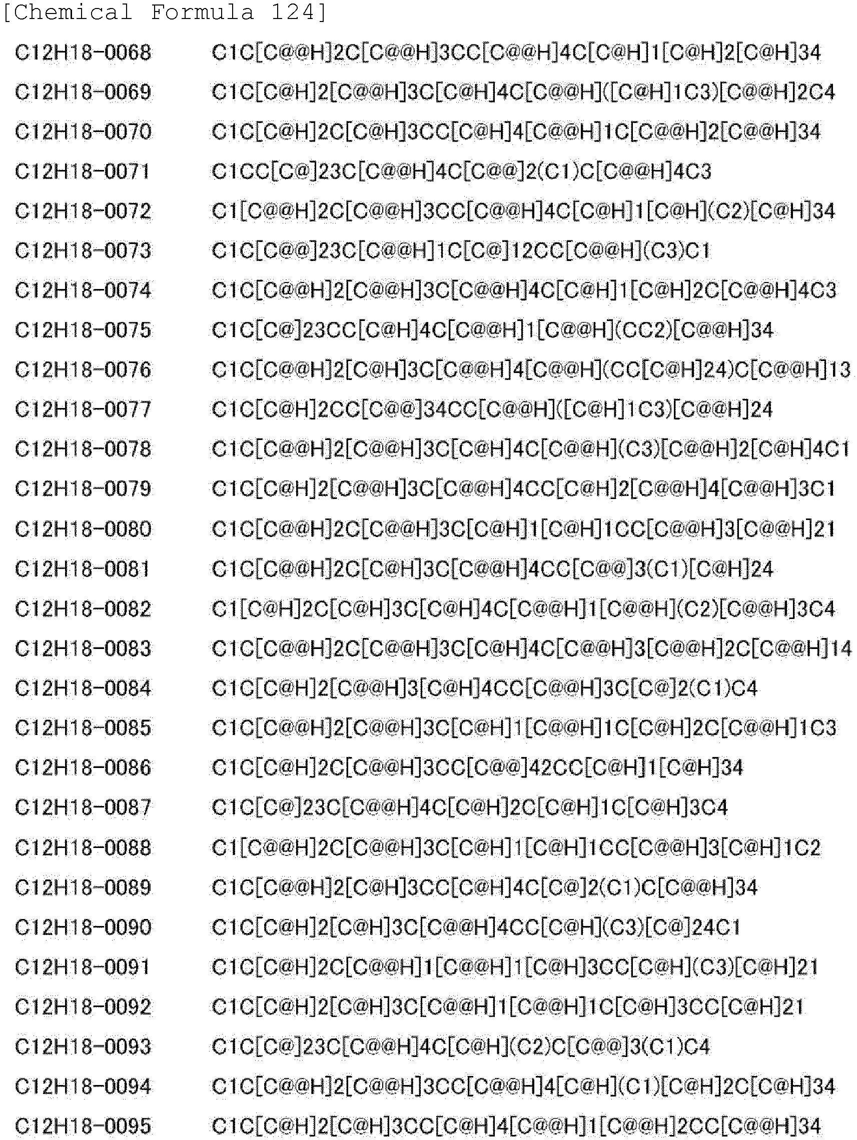 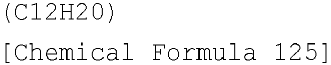 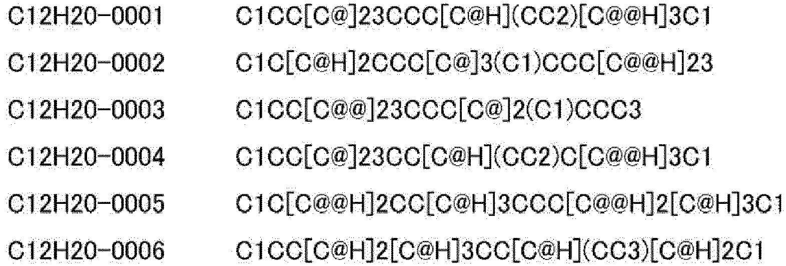 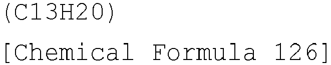 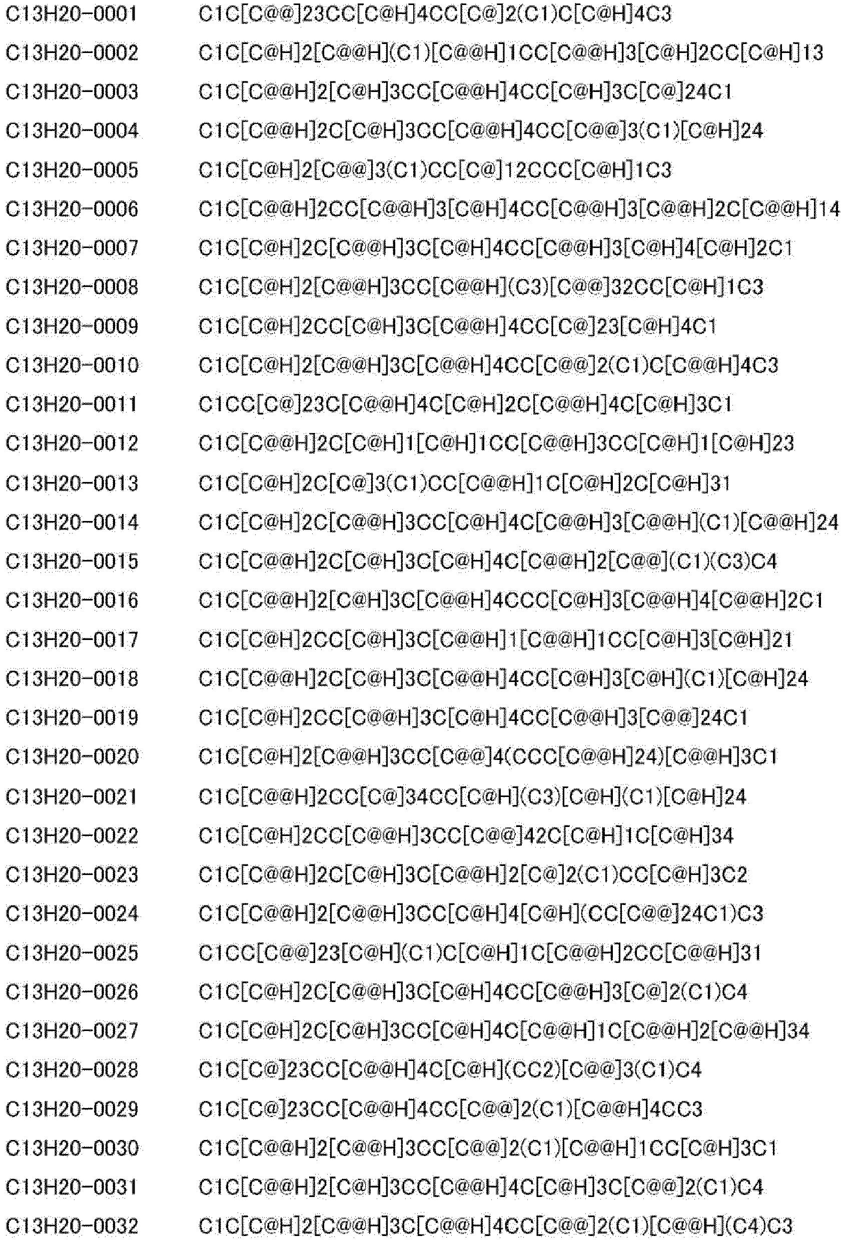 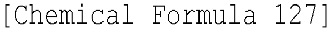 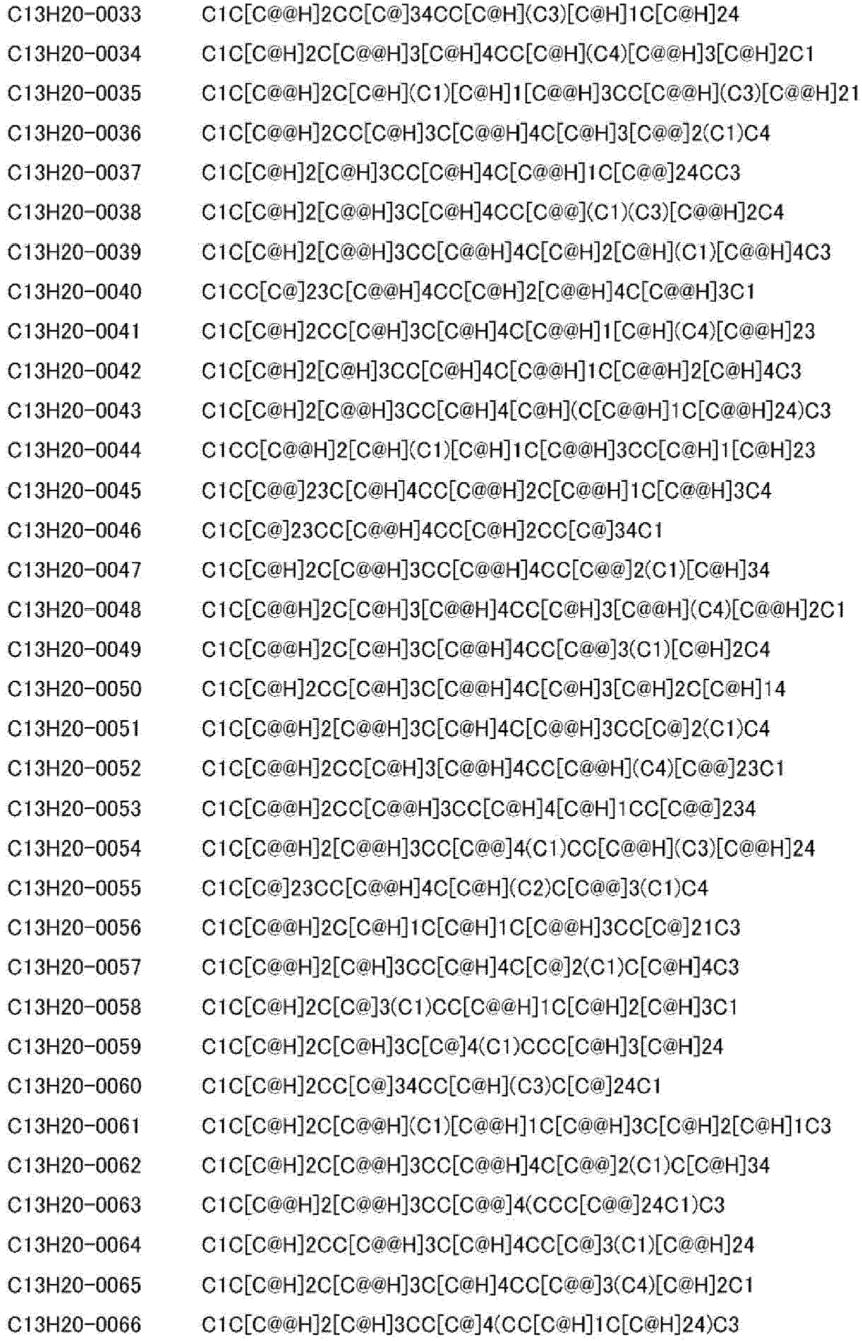 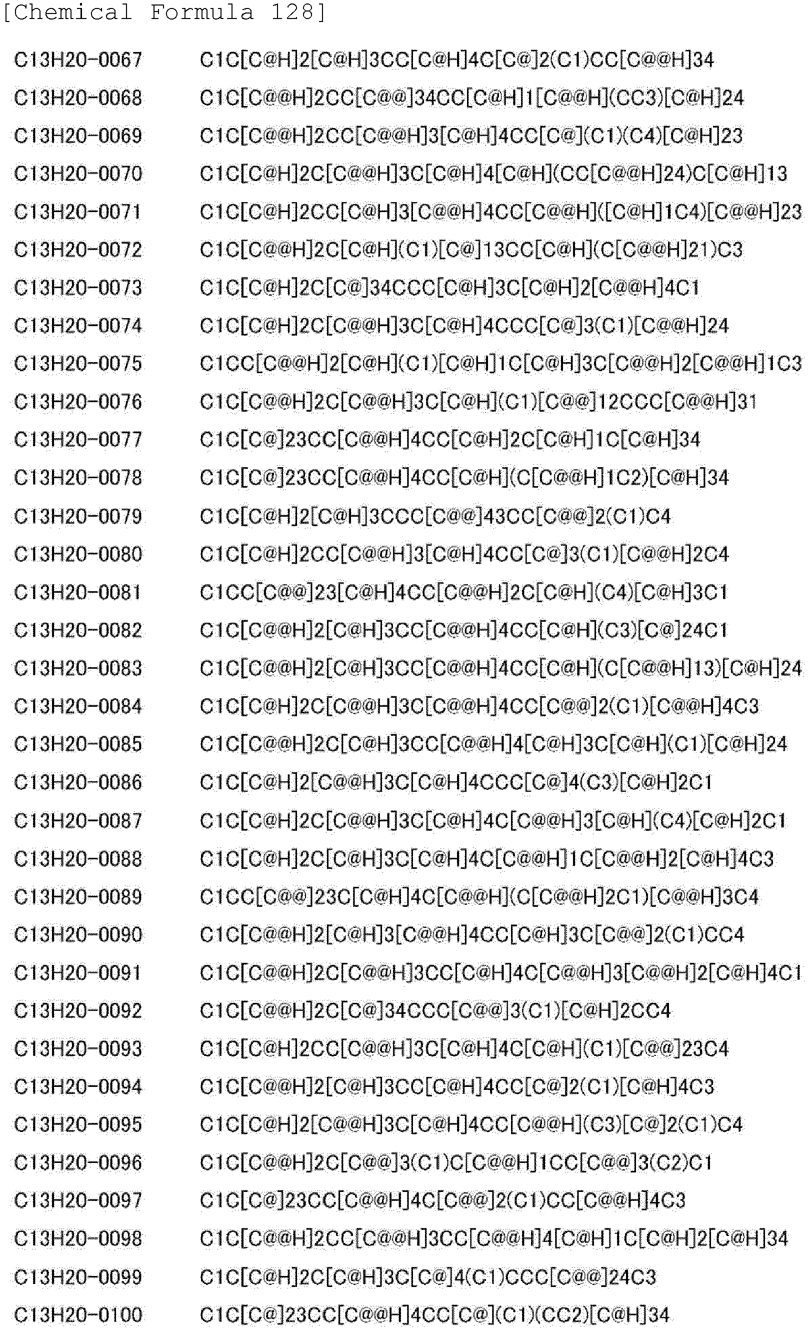 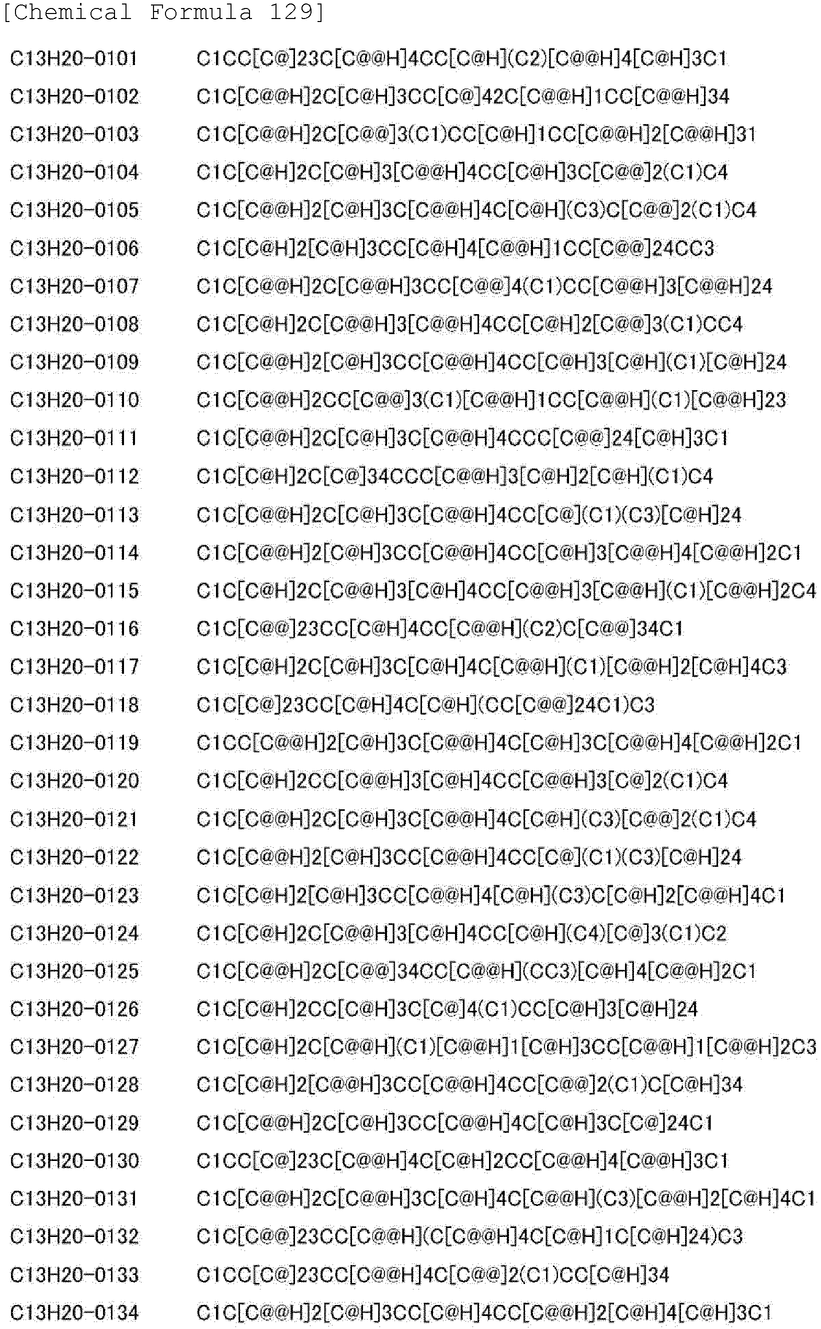 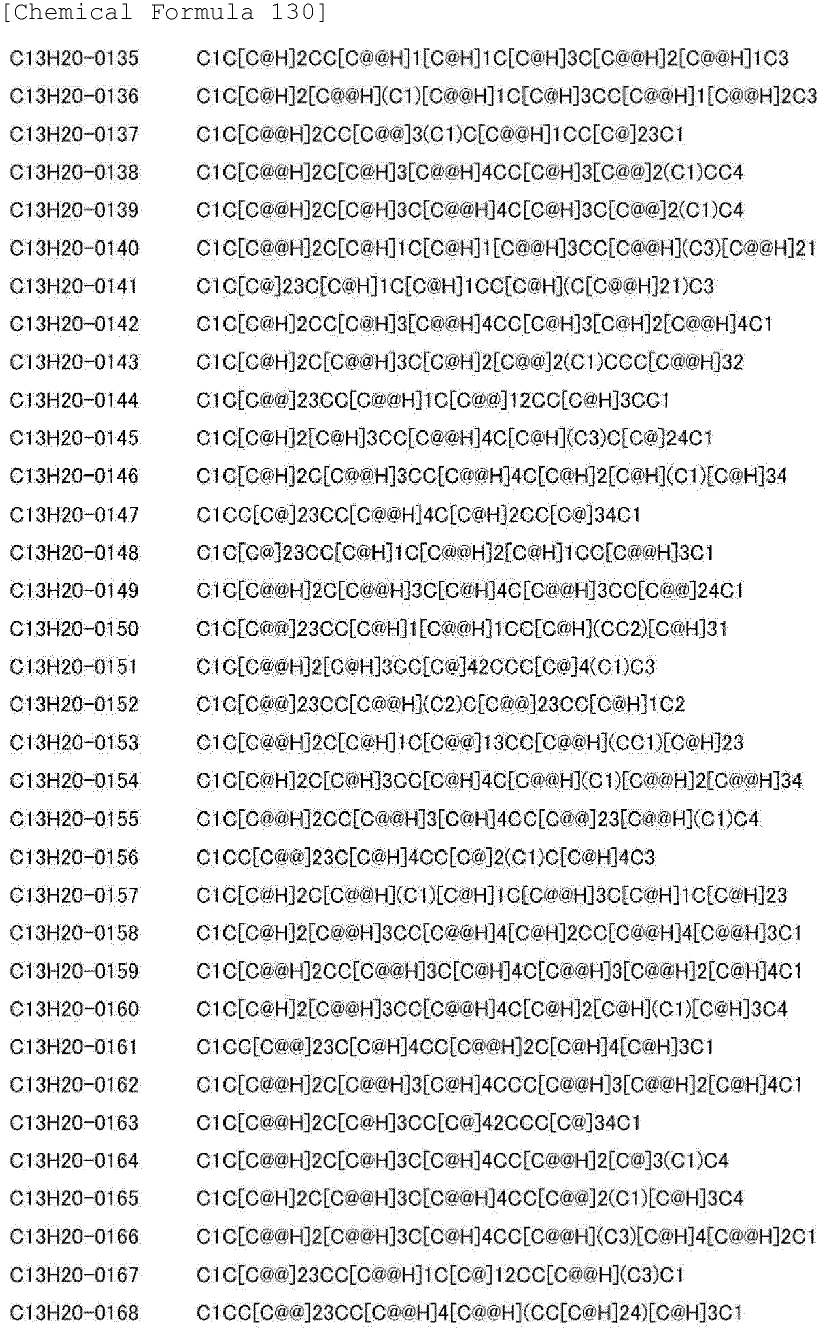 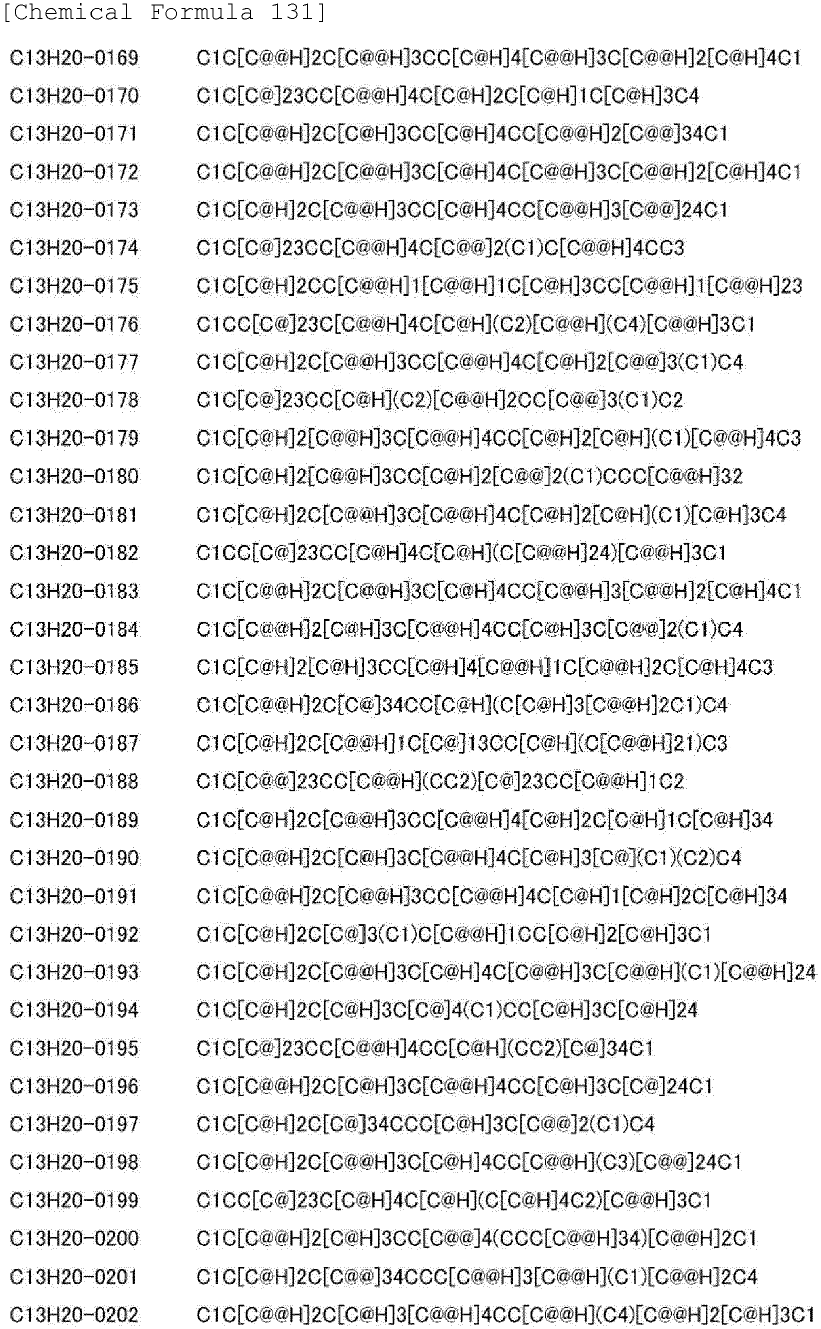 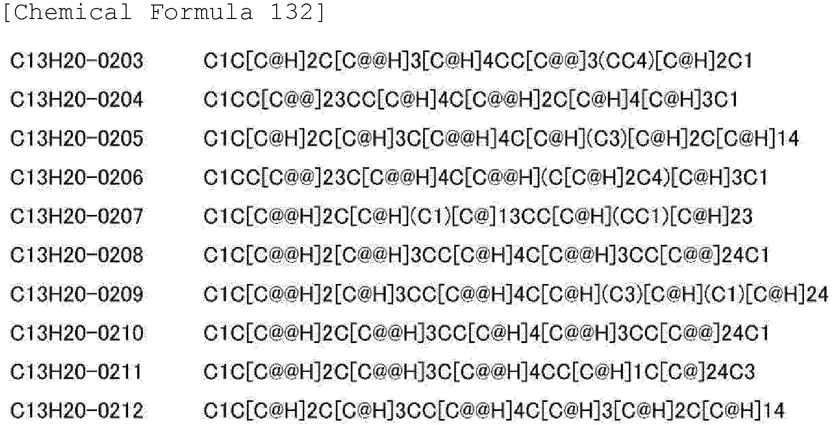 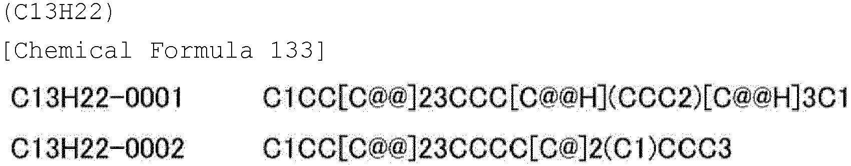 or an enantiomer thereof, optionally having a heteroatom in place of a carbon atom within a backbone and optionally having one or more substituents.

In another specific embodiment, the present disclosure provides a peptidomimetic compound having, when represented in SMILES format, any one of the following backbones: 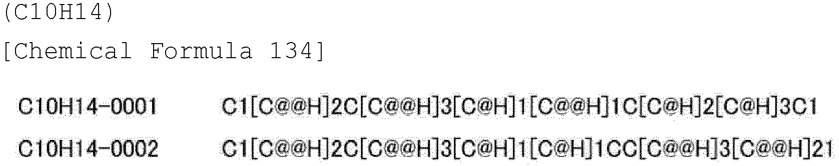 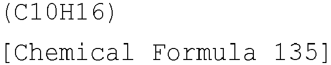 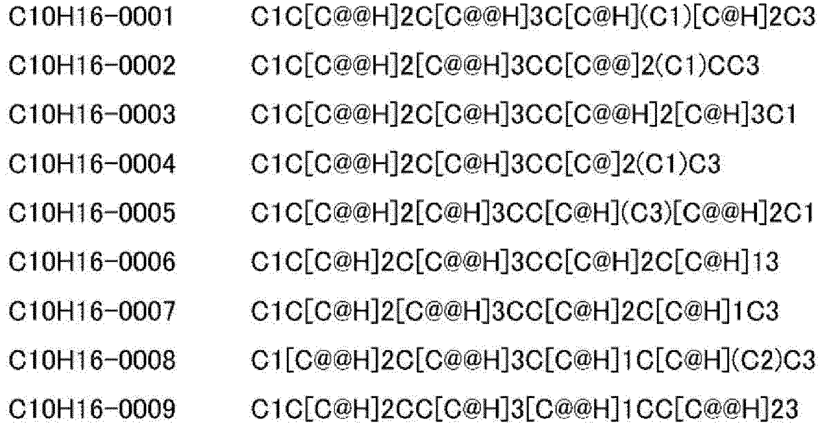 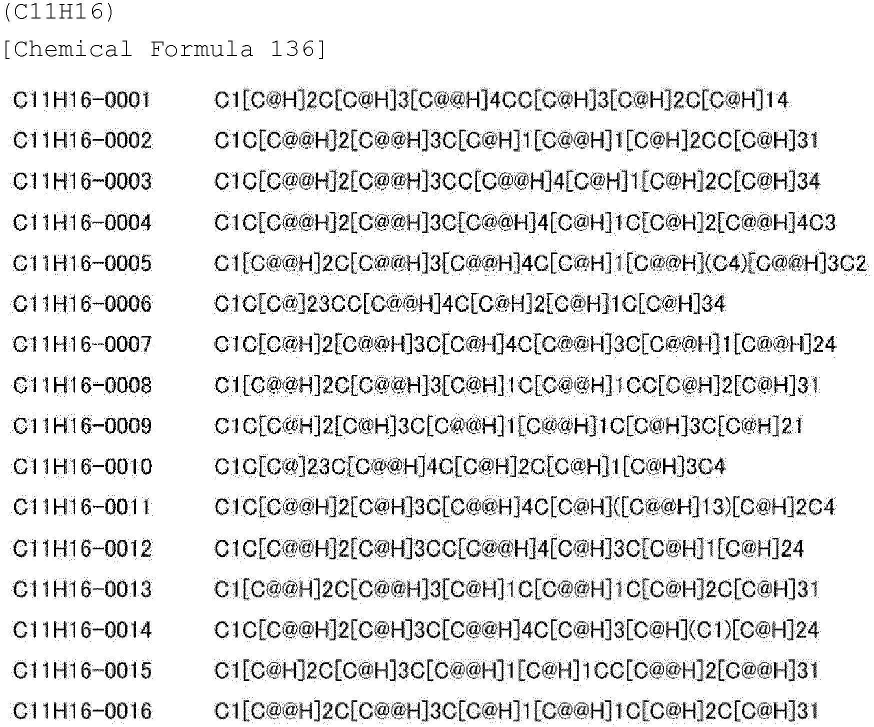 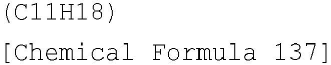 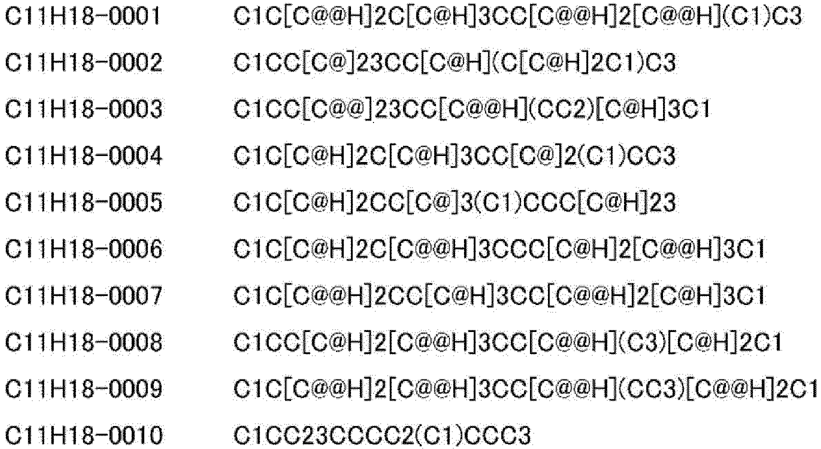 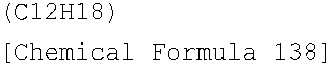 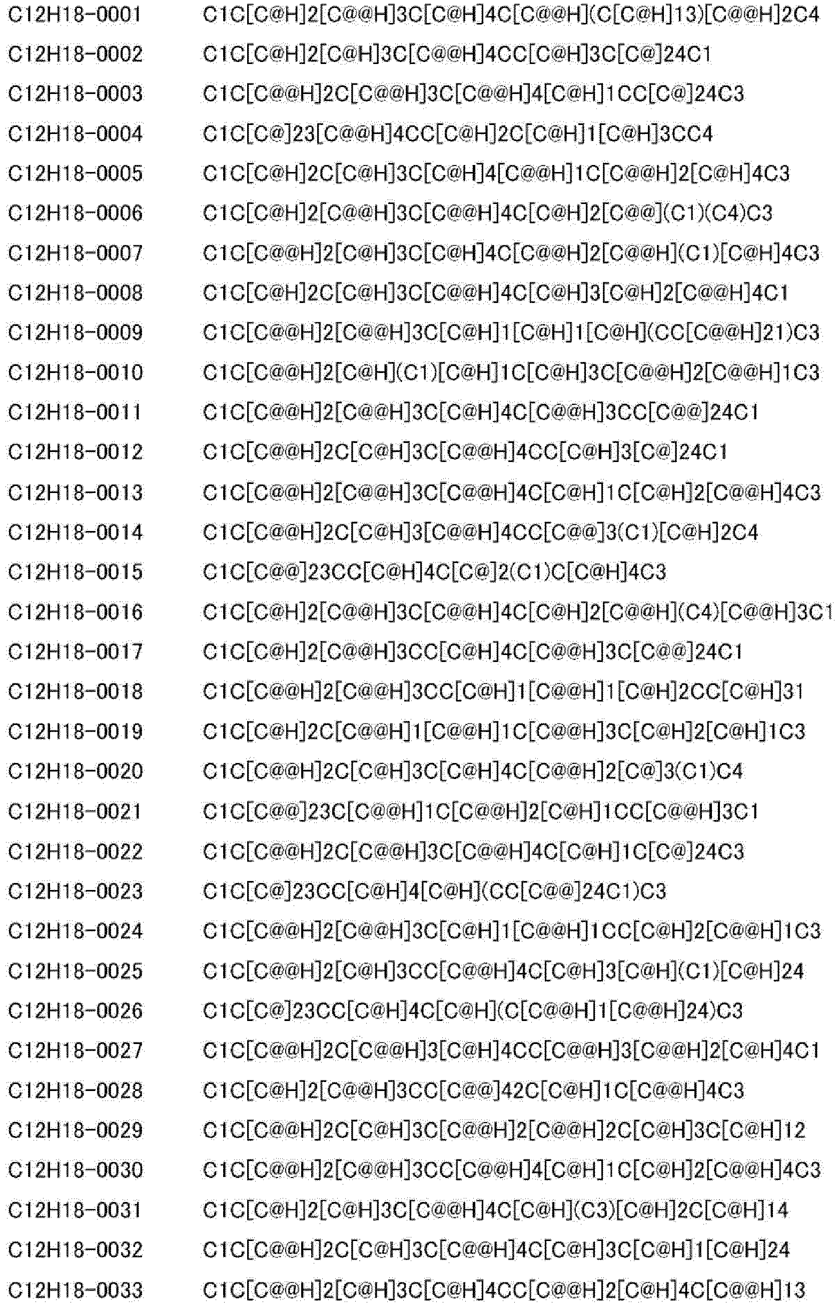 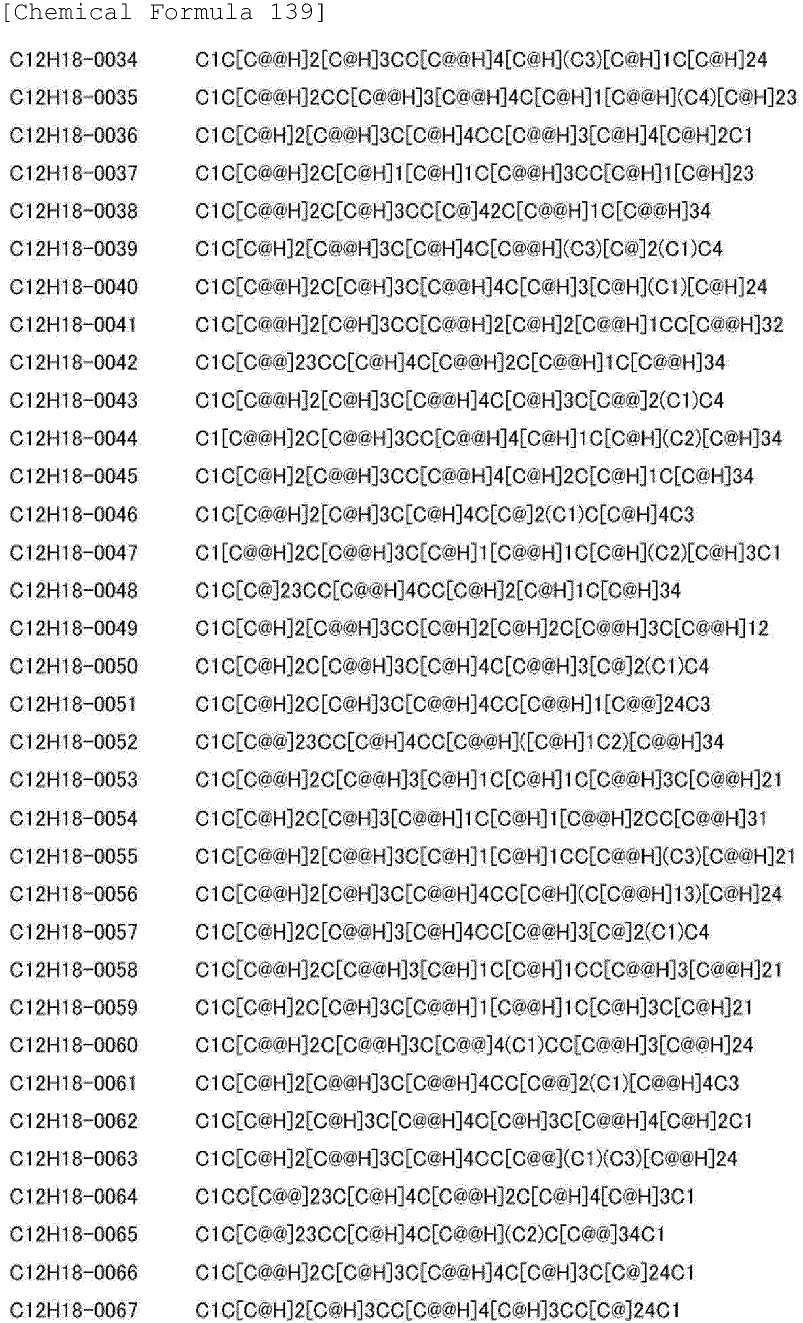 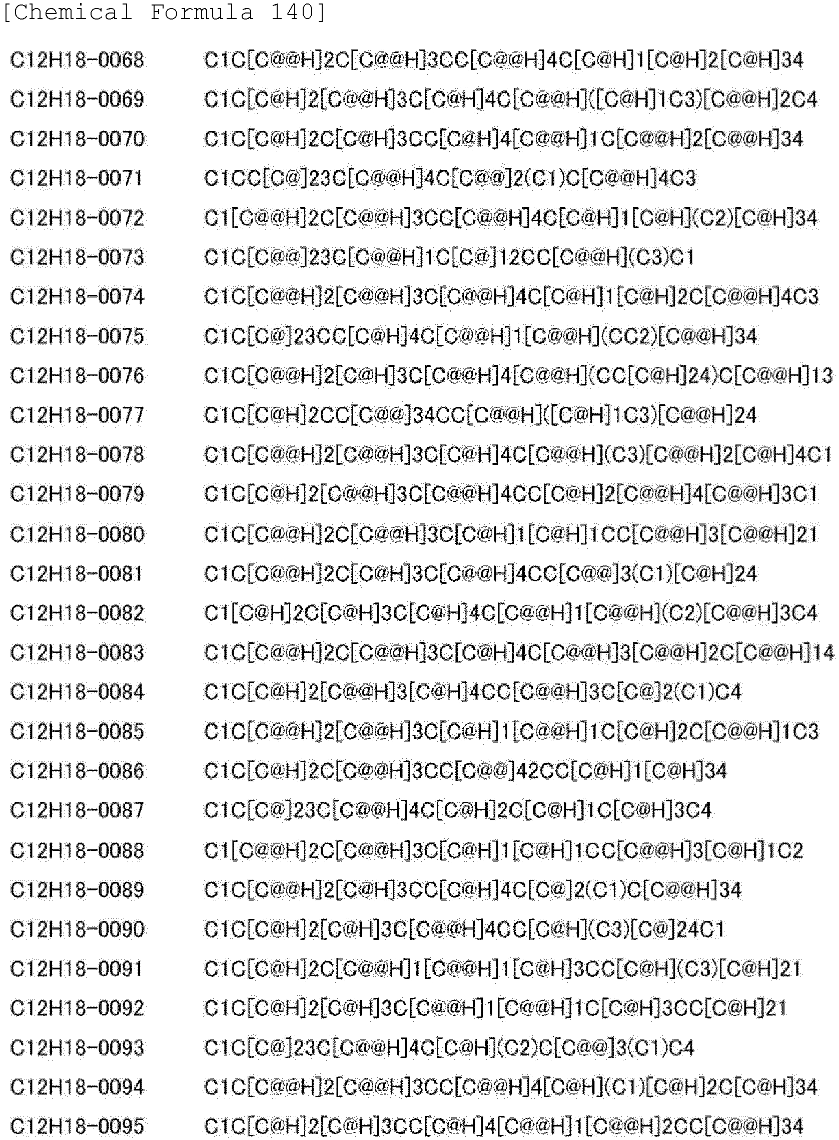 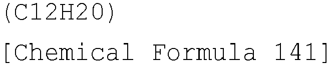 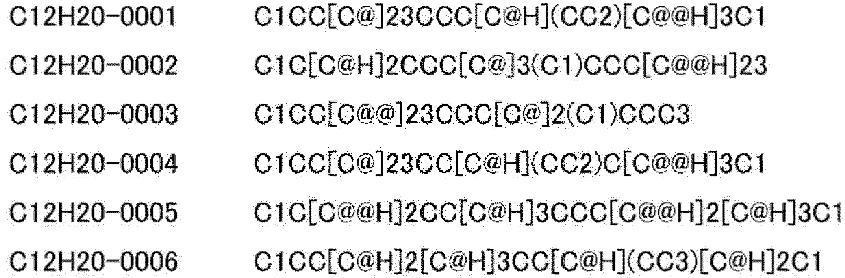 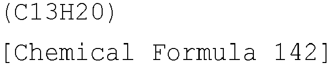 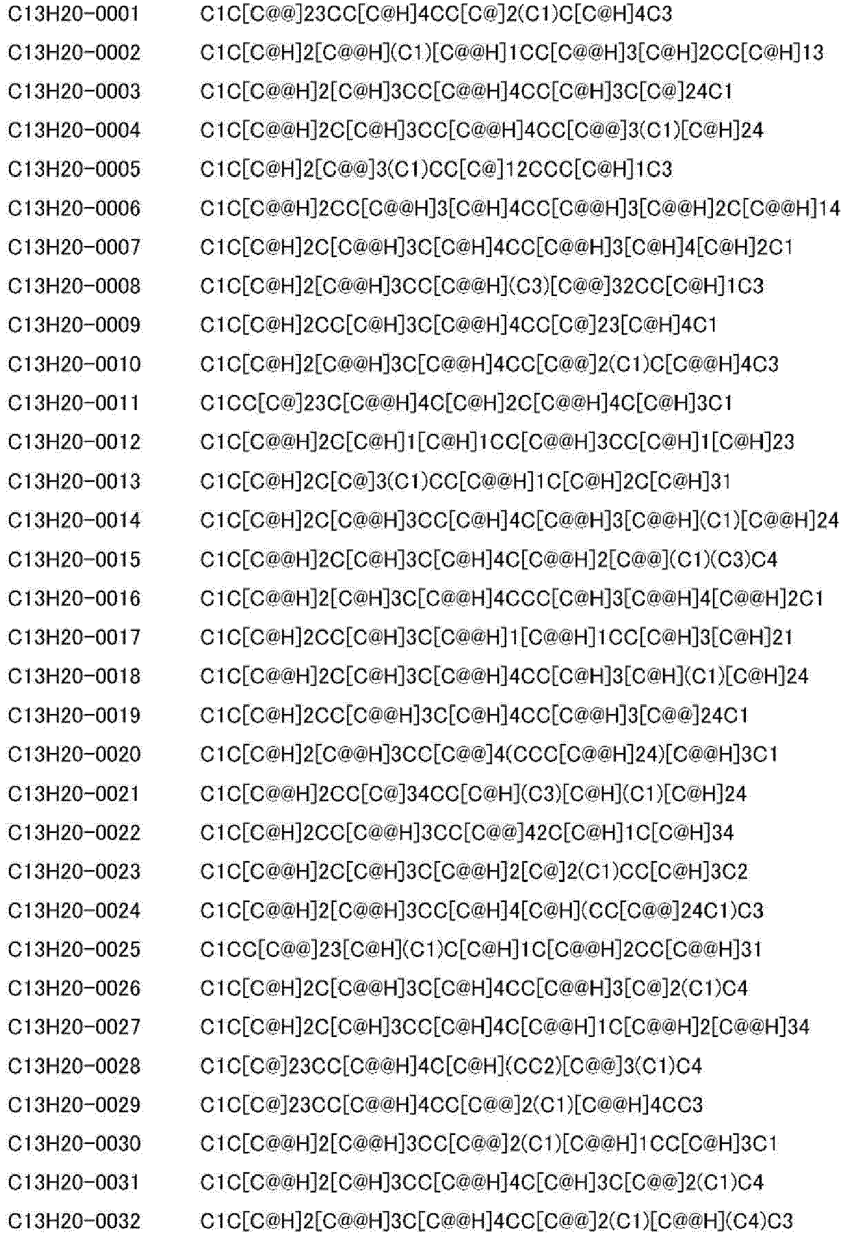 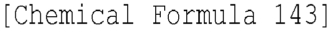 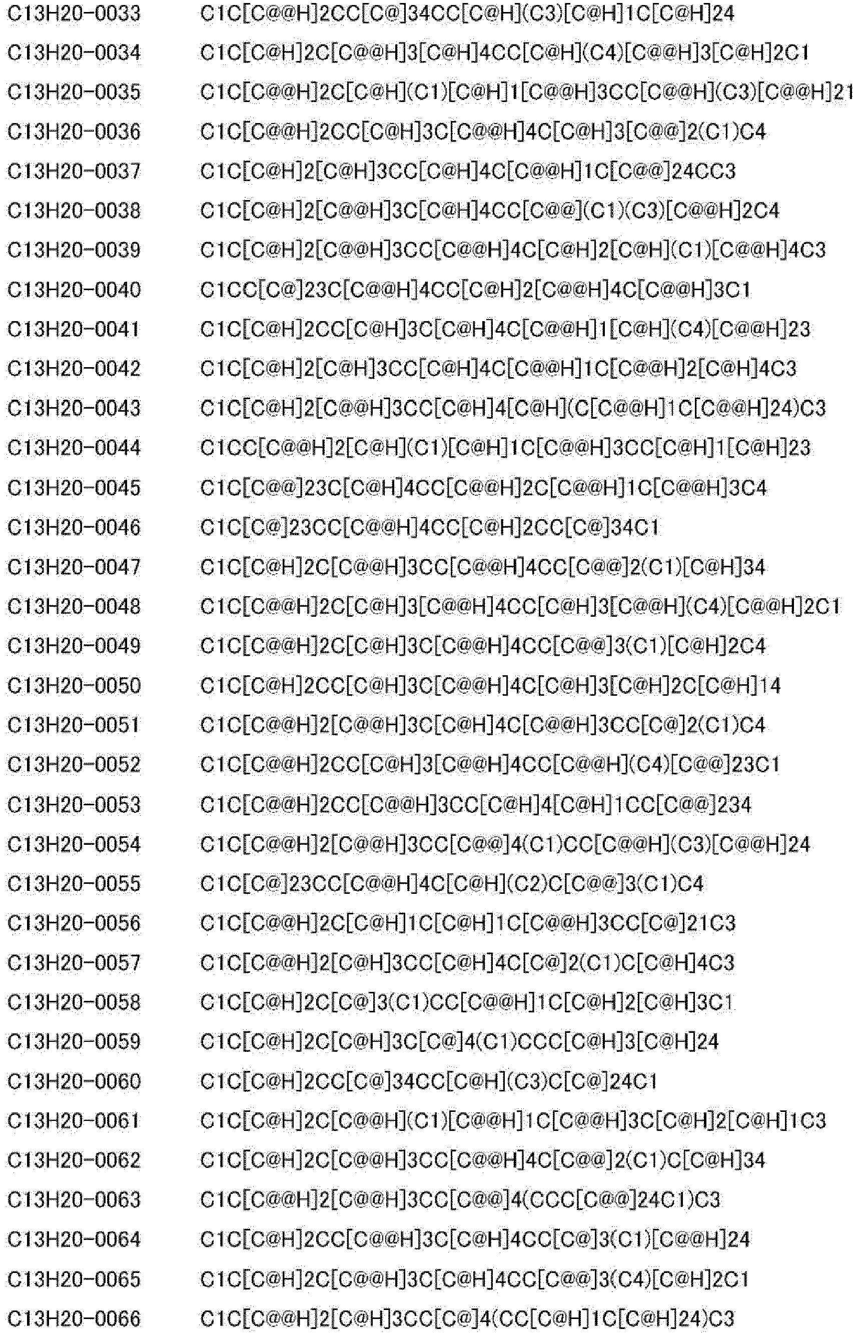 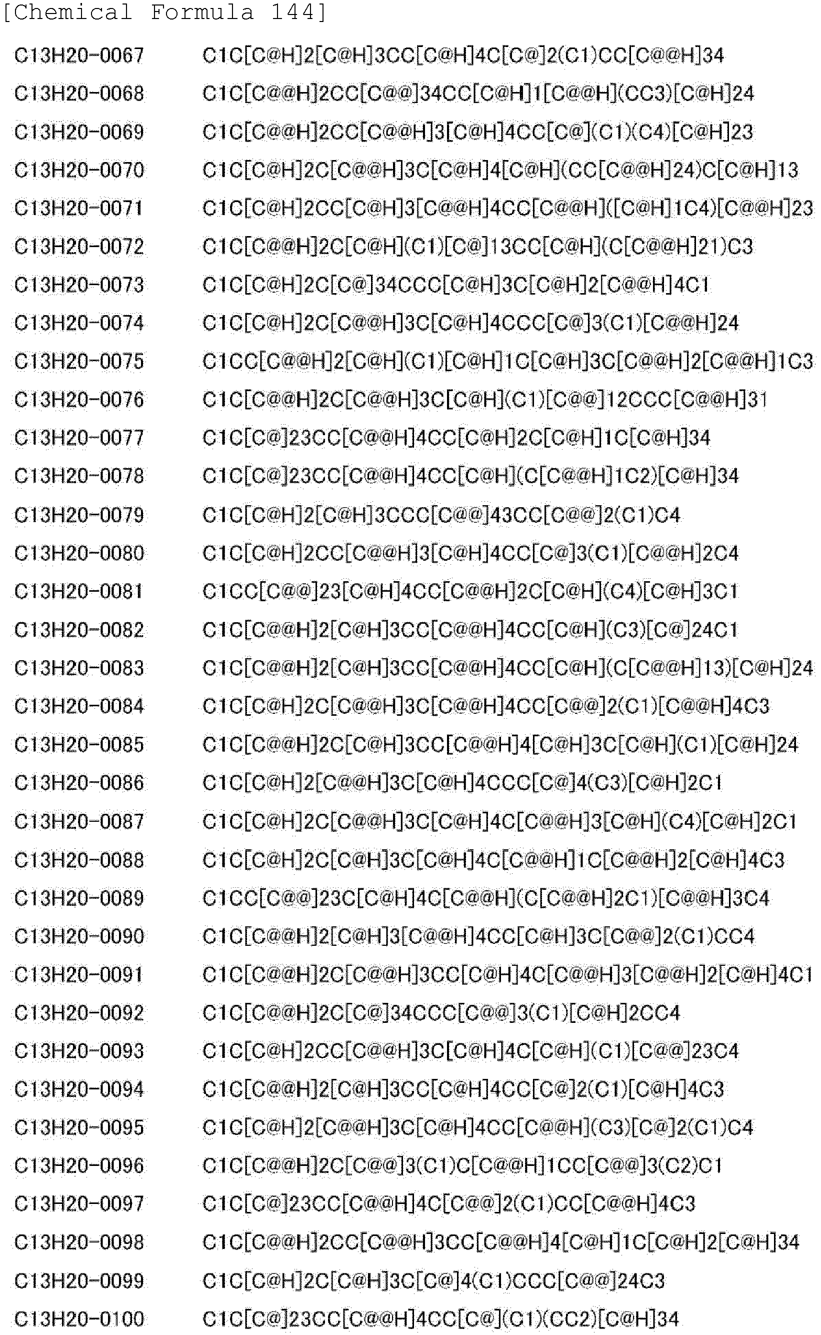 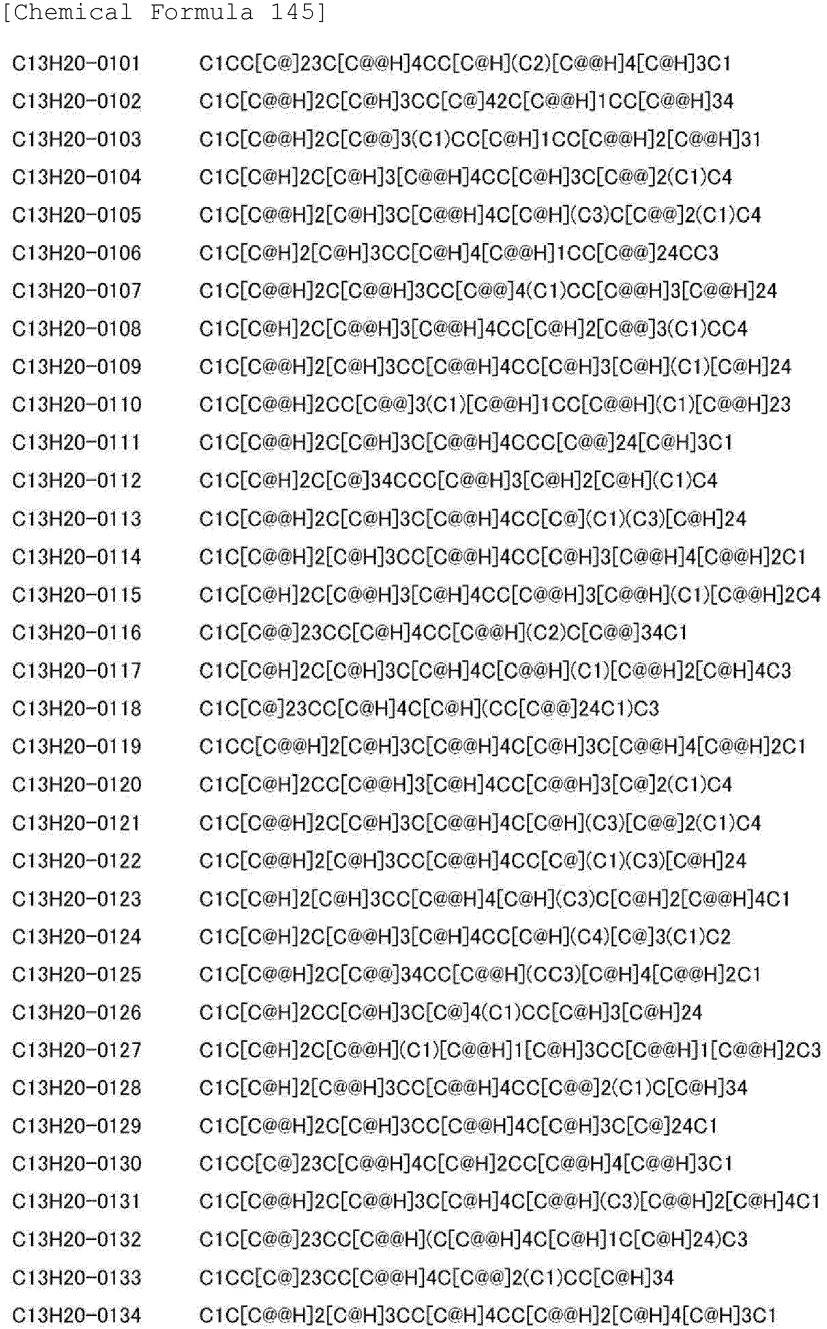 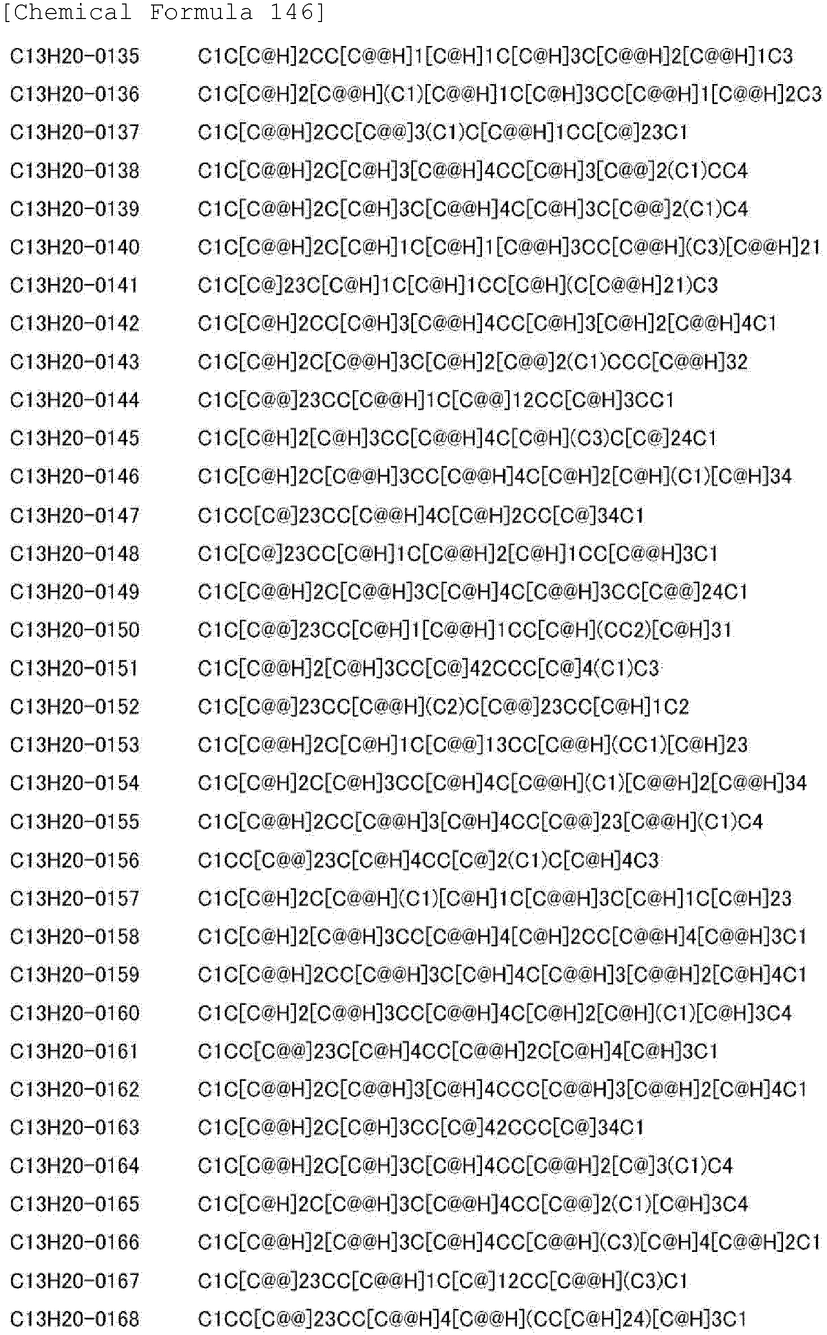 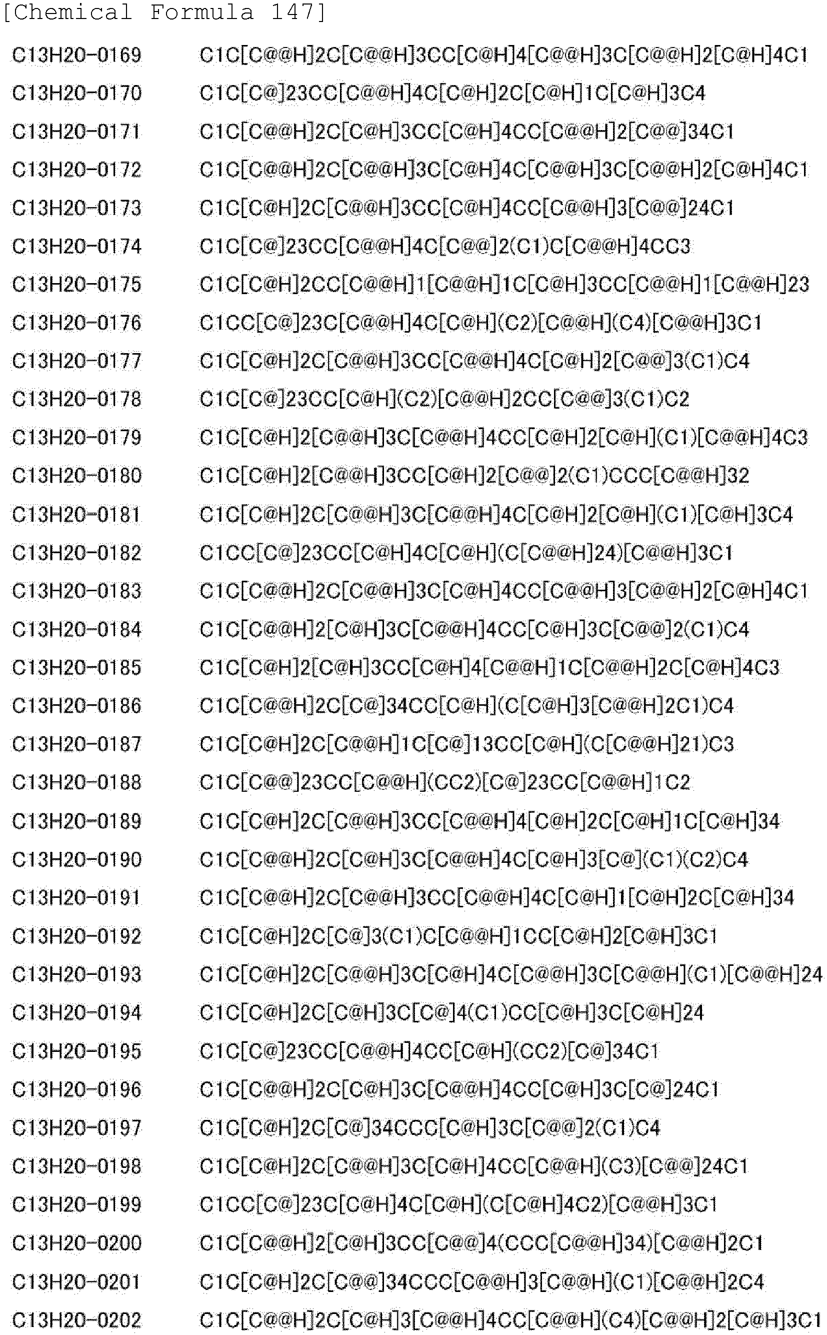 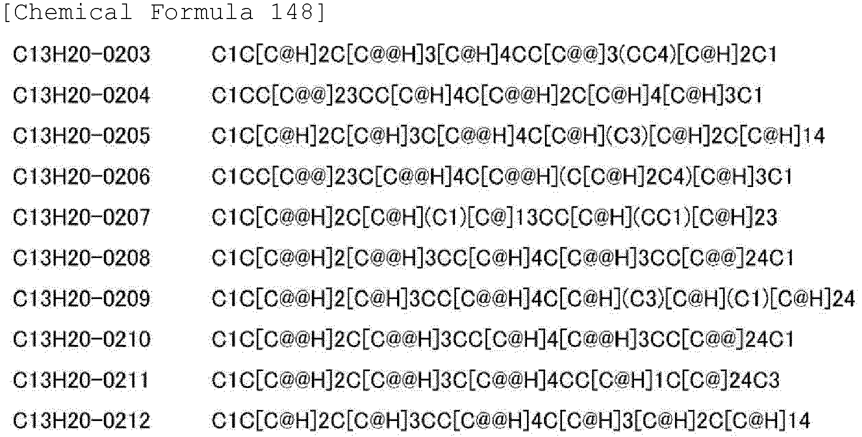 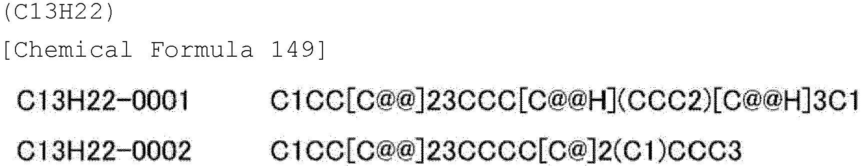 or an enantiomer backbone thereof and M substituents (wherein M is any integer from 1 to (number of atoms in a backbone moiety that can have a substituent)) corresponding to M amino acid-like structures (amino acid side chain or an equivalent thereof) from the backbone, optionally having a heteroatom in place of a carbon atom within a backbone.

In a certain embodiment, a peptidomimetic compound has the following structures. 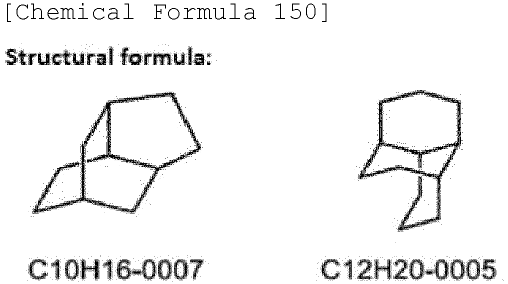 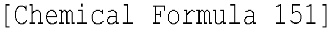 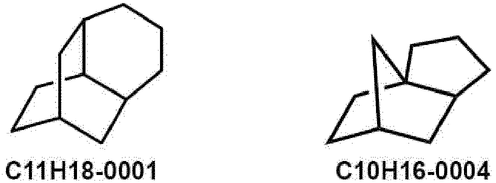 For example, the backbone can be represented as follows.

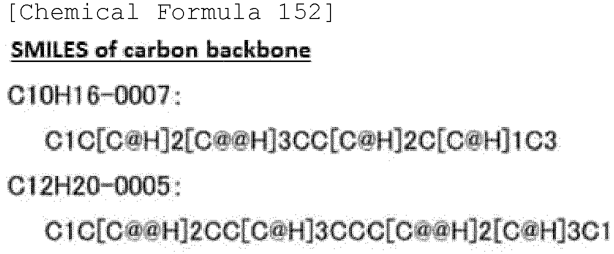

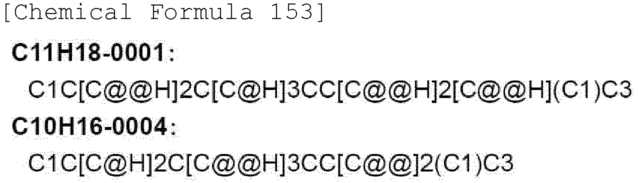

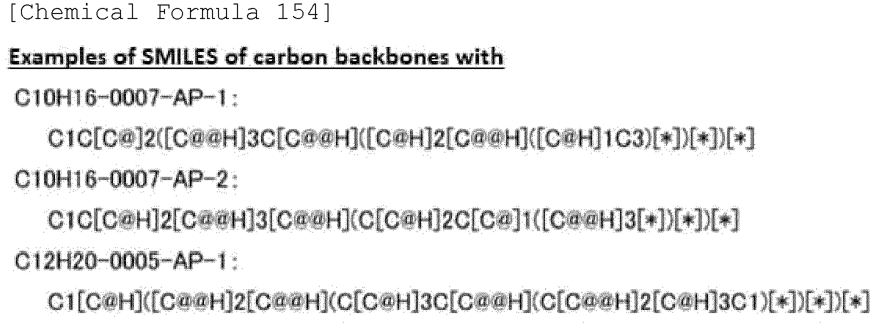

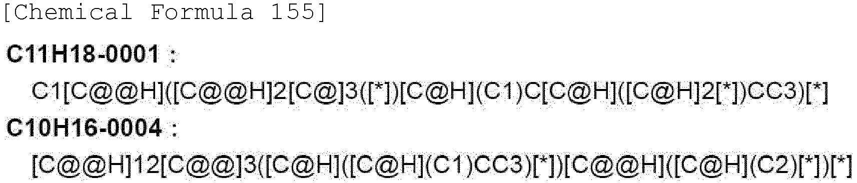

### (Application in interaction of peptide base interaction)

In another aspect, the present disclosure provides a composition for interacting a target molecule and a peptide base, comprising a compound, the compound comprising the peptidomimetic compound of the present disclosure, wherein a substituent of the peptidomimetic compound comprises an amino acid-like structure corresponding to an amino acid contained in the peptide.

The application provided by the present disclosure is provided from being able to rationally design and confirm a peptidomimetic compound of the present disclosure to be usable in an application for interacting a peptide base.

In still another aspect, the present disclosure provides a composition for interacting a target molecule and a peptide base, comprising a compound, the compound comprising a compound backbone based on the carbon backbone of the present disclosure, wherein a substituent of the compound backbone comprises an amino acid-like structure corresponding to an amino acid contained in the peptide.

### (General technology)

The chemical methodology, computer science methodology, biomolecular methodology, biochemical methodology, etc. used herein are well known and conventionally used in the art. Fundamental textbooks to be listed for chemical methodologies include The Fourth Series of Experimental Chemistry: 19. Organic synthesis I, hydrocarbon/halogen compound, 20. Organic synthesis II, alcohol/amine, 21. Organic synthesis III, aldehyde/ketone/quinone, 22. Organic synthesis IV, acid/amino acid/peptide, 23. Organic synthesis V, oxidation reaction, 24. Organic synthesis VI, heteroelement/typical metal element compound, 25. Organic synthesis VII, synthesis with an organic metal reagent, 26. Organic synthesis VIII, asymmetric synthesis/reduction/sugar/label compound, The Fifth Series of Experimental Chemistry: 13. Synthesis of organic compound I, hydrocarbon/halogen compound, 14. Synthesis of organic compound II, alcohol/amine, 15. Synthesis of organic compound III, aldehyde/ketone/quinone, 16. Synthesis of organic compound IV carboxylic acid/amino acid/peptide, 17. Synthesis of organic compound V, oxidation reaction, 18. Synthesis of organic compound VI, organic synthesis using a metal, 19. Synthesis of organic compound VII, asymmetric synthesis/radical reaction, Shin Jikken Kagaku Koza [New Experimental Chemistry Course]: 14-1. Synthesis and reaction of organic compound [1], 14-2. Synthesis and reaction of organic compound [II], 14-3. Synthesis and reaction of organic compound [III], 14-4. Synthesis and reaction of organic compound [IV], 14-5. Synthesis and reaction of organic compound [V], Pepuchido Gosei no Kiso to Jikken [Fundamentals and Experiment for Peptide Synthesis]] Nobuo IZUMIYA, 1985, Greene's Protective Groups in Organic Synthesis, Fourth Edition, 2006, Greene's Protective Groups in Organic Synthesis, 2014, etc. Fundamental textbooks to be listed for computer science methodologies include Kemoinformatikkusu Yosoku to Sekkei no tameno Kagaku Jouhougaku [Chemoinformatics: Chemoinformatics for Prediction and Design], Maruzen, 2005, ISBN4-621-07552-7. Molecular biological/biochemical methodologies are described in, for example, Current Protocols in Molecular Biology(http://onlinelibrary.wiley.com/book/10.1002/0471142 727) and Molecular Cloning: A Laboratory Manual (Fourth Edition) (http://www.molecularcloning.com), etc. Relevant parts of these documents (can be the entire document) are incorporated herein by reference.

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

The present disclosure has been described while showing preferred embodiments to facilitate understanding. While the present disclosure is described hereinafter based on the Examples, the above descriptions and the following Examples are provided for the sole purpose of exemplification, not limitation of the present disclosure. Thus, the scope of the present disclosure is not limited to the embodiments and Examples that are specifically described herein and is limited only by the scope of claims.

### [Examples]

The Examples are described hereinafter. For reagents, the specific products described in the Examples were used. However, the reagents can be substituted with an equivalent product from another manufacturer (Sigma-Aldrich, Wako Pure Chemical, Nacalai Tesque, R & D Systems, USCN Life Science Inc., etc.).

The abbreviations described above and the following abbreviations are used in the Examples in some cases to simplify the description.
s: singlet
d: doublet
t: triplet
q: quadruplet
m: multiplet
dd: double doublet
J: coupling constant
Hz: Hertz
δ: chemical shift
min: minute
RT: retention time
CDCl₃: deuterated chloroform
Me: methyl
Et: ethyl
Pr: propyl
i-Pr: isopropyl
i-Bu: isobutyl
s-Bu and sec-Bu: secondary butyl
^{t}Bu, tBu, and tert-Bu: tertiary butyl
i-Pnt: isopentyl
Hxy: n-hexyl
Ac: acetyl
Bz: benzoyl
Bnzl: benzyl
Cbz: benzyloxycarbonyl
3-Me-Bnzl: 3-methylbenzyl
4-Me-Bnzl: 4-methylbenzyl
4-tBu-Bnzl: 4-(tert-butyl)benzyl
3-MeO-Bnzl: 3-methoxybenzyl
4-MeO-Bnzl: 4-methoxybenzyl
4-OH-Bnzl: 4-hydroxybenzyl
3-F-Bnzl: 3-fluorobenzyl
4-F-Bnzl: 4-fluorobenzyl
3-Cl-Bnzl: 3-chlorobenzyl
4-Cl-Bnzl: 4-chlorobenzyl
3,4-Cl₂-Bnzl: 3,4-dichlorobenzyl
4-tBuO-Bnzl: 4-(tert-butoxy)benzyl
4-Nt-Bnzl: 4-nitrobenzyl
Cbx-E and 2-Cbx-Et: 2-carboxyethyl
Cbm-M: 2-amino-2-oxoethyl, or carbamoylmethyl
Cbm-E: 3-amino-3-oxopropyl, or 2-carbamoylethyl
tBOC-E: 2-(tert-butoxycarbonyl)ethyl, or 3-(tert-butoxy)-3-oxopropyl
Gun-Pr and 3-Gun-Pr: 3-guanidinopropyl
Hdr-M: hydroxymethyl
Hdr-E and 2-OH-Et: 2-hydroxyethyl
tBuO-E and 2-OtBu-Et: 2-(tert-butoxy)ethyl
Ph-Et: 2-phenylethyl
Ph-Pr: 3-phenylpropyl
Ph-Bu: 4-phenylbutyl
Np-M and 1-Npm: naphthalen-1-ylmethyl
2-Npm: naphthalen-2-ylmethyl
Np-E: 2-(naphthalen-1-yl)ethyl
6-Me-Indm: 6-methyl-1H-indol-3-ylmethyl
Boc-6-Me-Indm: 1-tert-butoxycarbonyl-6-methyl-1H-indol-3-ylmethyl
6-F-Indm: 6-fluoro-1H-indol-3-ylmethyl
Boc-6-F-Indm: 1-tert-butoxycarbonyl-6-fluoro-1H-indol-3-ylmethyl
Cpm: cyclopentylmethyl
Chm: cyclohexylmethyl
Chepm: cycloheptylmethyl
DCM: dichloromethane
DMF: dimethylformamide
DIEA: N,N-diisopropylethylamine
EDCI: 1-ethyl-3-(3-dimethylaminopropyl)carbodiimide
Hex: hexane
Boc and tBOC and ^{t}BOC: tert-butoxycarbonyl
TBSO-E and 2-OTBS-Et: 2-(tert-butyldimethylsilyloxy)ethyl
TBS: tert-butyldimethylsilyl
TBSOTf: tert-butyldimethylsilyltrifluoroacetate
Trt: trityl or triphenylmethyl
r.t. or rt: room temperature
eq: equivalent

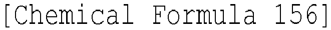

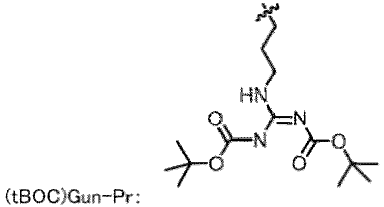

### (Example 1: Example of carbon backbone)

This Example demonstrates an example of a design of a carbon backbone.

### (Example of backbone design)

After designating molecular formula C10H16, an adjacency matrix was randomly generated 10,000,000 times to generate carbon backbones. Nine carbon backbones satisfied a predefined condition. In this regard, C10H16-007 among the nine backbones was selected.

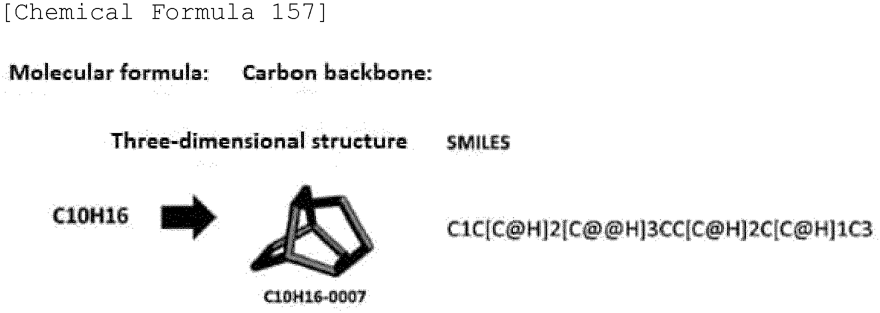

### (Example 2: Example of peptidomimetic molecule)

This Example demonstrate an example of a design of a peptidomimetic molecule (framework).

Since this Example mimics a peptide with FxxFL, one type of Leu-like structure and two types of Phe-like structures shown below were prepared as an amino acid-like structure in the SMILES format.

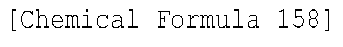

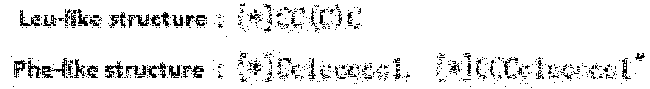

The number (M) of APs was three, and the number (N) of times a carbon backbone with APs was generated was 1000. The total number of peptidomimetic molecule frames was 3 × 3 × 3 × 1,000 = 27,000. The number was 12,447 after eliminating duplicates.

An example of a peptidomimetic molecule frame is shown below.

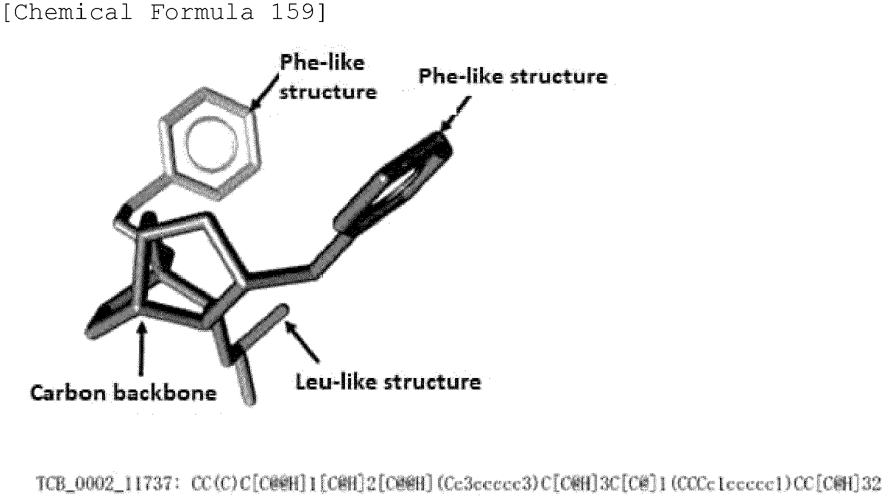

A backbone is generated as described in Examples 1 and 2, and a suitably selected example is designed and synthesized as shown in the following Examples.

### (Example 3: Manufacturing Example of carbon backbone (1))

The backbones generated in the Example described above, (C10H16-0007): 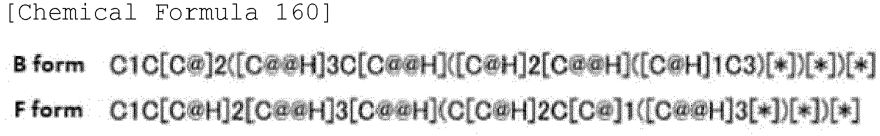 are synthesized in this Example. XXI-5 (or IB-5) was synthesized as a B form, and XXI-6 (or IF-6) was synthesized as an F form. A compound with the same backbone structure, but a variation in a substituent (IF-801) was separated synthesized.

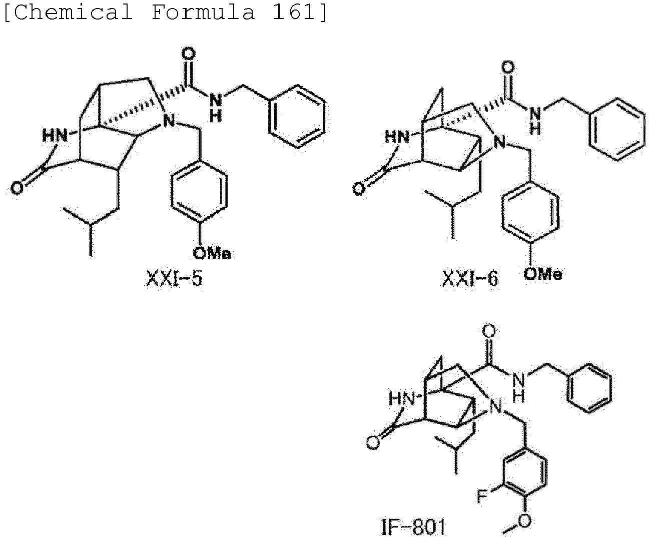

This Example shows actual synthesis example based on an example of a backbone generated in the Examples described above (Synthesis Schemes 1 to 6).

### [Synthesis example for compound XXV]

### Synthesis of N-benzyl-1,2,4-triazine-3-carbozamide

Ethyl 1,2,4-triazine-3-carboxylate (200 mg, 1.3 mmol) was dissolved in methanol (2.5 mL), and benzylamine (139 mg, 1.3 mmol) was added. The mixture was then stirred for 12 hours at room temperature. Methanol was evaporated under reduced pressure, and the resulting residue was purified by column chromatography (column: CHROMATOREX Q-Pack SI30, SIZE 20, eluent: ethyl acetate-methanol (gradient from 0% to 5%)) . A fraction of a compound of interest was concentrated to obtain the title compound (181 mg, yield: 67%) as a brown amorphous solid.

### [Synthesis example for compound XXXV]

### Synthesis of 4-methylpentanal

A methylene chloride (40.0 mL) solution of acetic acid (0.0994 mL, 1.30 mmol) and 4-methylpentan-1-ol (1.49 mL, 11.8 mmol) was slowly added dropwise into a methylene chloride (45.0 mL) solution of 1,1,1-triacetoxy-1,1-dihydro-1,2-benziodoxol-3-(1H)-one (Dess-Martin periodinane) (5.25 g, 12.4 mmol) at room temperature. After completion of the addition, the mixture was stirred for another hour, and then diethyl ether (280 mL) was added. An aqueous 1.30M sodium hydroxide solution (200 mL) was added to the organic layer, and the mixture was stirred for 10 minutes at room temperature. The organic layer was washed with an aqueous 1.30M sodium hydroxide solution (90.0 mL) and water (50.0 mL). After drying with anhydrous sodium magnesium sulfate, the solvent was carefully evaporated to obtain the title compound (1.01 g, 86%).

### [Synthesis example for compound XXIII]

### Synthesis of N-(4-methoxybenzyl)prop-2-en-1-amine

Bromomethyl(4-methoxybenzene) (1.17 mL, 8.35 mmol) was gradually added dropwise to a suspension of anhydrous potassium carbonate (1.39 g, 10.0 mmol) and prop-2-en-1-amine (7.53 mL, 100 mmol) and then the mixture was stirred for 3 hours at room temperature. The solids were filtered and washed with methylene chloride. The combined organic layer was evaporated under reduced pressure. The resulting residue was purified with CHROMATOREX Q-PACK SI30 SIZE 20 (hexane: ethyl acetate = 50%:50% to 0%:100%) to obtain the title compound (902 mg, yield: 71%).

### [Imine Intermediate Synthesis Example: XXII-5 and XXII-6]

### Synthesis of (3S*, 3aS*, 6R*, 7R*, 7aS*) -N-benzyl-7-isobutyl-1-(4-methoxybenzyl)-1,2,3,6,7,7a-hexahydro-3aH-3,6-methanopyrrolo[3,2-b]pyridine-3a-carboxamide (XXII-6) and (3S*, 3aR*, 6S*, 7R*, 7aR*) -N-benzyl-7-isobutyl-1- (4-methoxybenzyl)-1,2,3,3a,7,7a-hexahydro-6H-3,6-methanopyrrolo[3,2-c]pyridine-6-carboxamide (XXII-5)

4A molecular sieves (335 mg), 4-methylpentanal (94.5 mL, 0.93 mmol), and N-(4-methoxybenzyl)prop-2-en-1-amine (165.5 mg, 0.93 mmol) were added to a chloroform (2 mL) solution of N-benzyl-1,2,4-triazine-3-carboxamide (80 mg, 0.37 mmol), and the mixture was heated and refluxed for 10 hours. The molecular sieves were filtered and washed twice with chloroform (5.00 mL). The combined organic layer was evaporated under reduced pressure. The resulting residue was purified with CHROMATOREX Q-PACK SI30 SIZE 20 (hexane:ethyl acetate = 75%:25% to 0%:100%) and subsequently with CHROMATOREX Q-PACK DNH30 SIZE 10 (hexane:ethyl acetate = 100%:0% to 50%:50%) to obtain the title compounds (XXII-5) and (XXII-6) as about a 1:1 mixture (quantified by ¹H NMR) (69.5 mg, yield: 42%, [M+1]⁺ = 446) .

### [Cyclic amide synthesis example: XXI-5 and XXI-6]

### Synthesis of (3S*, 3aS*, 6R*, 7R*, 7aS*) -N-benzyl-7-isobutyl-1-(4-methoxybenzyl)-5-oxooctahydro-3aH-3,6-methanopyrrolo[3,2-b]pyridine-3a-carboxamide (IB-1) and (3S*, 3aR*, 6S*, 7R*, 7aR*)-N-benzyl-7-isobutyl-1- (4-methoxybenzyl)-4-oxooctahydro-6H-3,6-methanopyrrolo[3,2-c]pyridine-6-carboxamide (IF-1)

To a toluene (401 µL) solution of a 1:1 mixture (50.0 mg, 0.100 mmol) of (3S*, 3aR*, 6S*, 7R*, 7aR*) -N-benzyl-7-isobutyl-1-(4-methoxybenzyl)-1,2,3,3a,7,7a-hexahydro-6H-3,6-methanopyrrolo[3,2-c]pyridine-6-carboxamide (XXII-5) and (3S*, 3aS*, 6R*, 7R*, 7aS*) -N-benzyl-7-isobutyl-1- (4-methoxybenzyl)-1,2,3,6,7,7a-hexahydro-3aH-3,6-methanopyrrolo[3,2-b]pyridine-3a-carboxamide (XXII-6), trifluoroacetic acid (15.3 µL, 0.201 mmol), 2,3-dimethyl-2-butene (59.4 µL, 0.501 mmol), aqueous 5 M sodium chlorite solution (30.1 µL, 0.150 mmol), and aqueous 5 M sodium dihydrogen phosphate solution (70.2 µL, 0.351 mmol) were added, and the mixture was vigorously stirred for 1 hour at room temperature. Ethyl acetate (1 mL) was added to the reaction mixture. An organic layer was sequentially washed with water (0.5 mL), aqueous 5% sodium thiosulfate solution (0.5 mL), and saturated salt water (0.5 mL) and dried with anhydrous sodium sulfate, and then filtered. Ethyl acetate was evaporated under reduced pressure from the filtrate. The residue including toluene was purified with CHROMATOREX Q-PACK DNH30 SIZE 10 (hexane: ethyl acetate = 100%:0% to 0%:100%) to obtain the title compounds (XXI-5) (21.4 mg, yield: 46.3%, RT = 1.32 minutes (method B), [M+1]⁺ = 462) and (XXI-6) (22.6 mg, yield: 48.9%, RT = 1.32 minutes (method B), [M+1]⁺ = 462).
XXI-5: ¹H-NMR spectrum (400 MHz, CDCl₃) δ (ppm): 0.51 (3H, d), 0.65 (3H, d), 0.72-0.85 (1H, m), 0.87-0.93 (1H, m), 1.68-1.76 (3H, m), 2.17 (1H, dd), 2.58-2.70 (3H, m), 2.92 (1H, d), 2.94 (1H, d), 3.60 (1H, d), 3.72 (1H, d), 3.79 (3H, s), 4.40 (1H, dd), 4.55 (1H, dd), 5.97 (1H, br), 6.81-6.83 (2H, m), 7.00 (1H, s), 7.19-7.21 (2H, m), 7.25-7.35 (5H, m). XXI-6: ¹H-NMR spectrum (400 MHz, CDCl₃) δ (ppm): 0.91 (3H, d), 0.93 (3H, d), 0.93-1.00 (1H, m), 1.20-1.27 (1H, m), 1.65-1.76 (3H, m), 2.22 (1H, m), 2.28 (1H, t), 2.38 (1H, dd), 2.48 (1H, s), 2.87 (1H, s), 3.24 (1H, dd), 3.58 (1H, d), 3.77 (1H, d), 3.77 (3H, s), 4.46 (2H, m), 6.63 (2H, m), 6.78 (2H, m), 6.90 (1H, s), 7.24-7.38 (5H, m), 9.71 (1H, t).
The structure of the resulting compounds was as described below.

Compound XXI-7 was synthesized by the same method as compound XXI-5 described above.

### (Example 4: Manufacturing example of carbon backbone (3)) Synthesis of compound L3 from compound L2

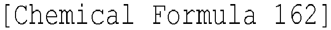

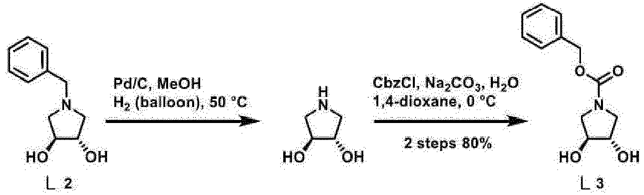

While compound L3 is a known compound, a document was not referred for the experimental procedure, so that the procedure is described below.

Compound L2 (4.04 g, 20.9 mmol) was dissolved in methanol (30 mL), and Pd/C (404 mg) was added. The mixture was then stirred for 2 hours at 50°C under a hydrogen atmosphere. Pd/C (400 mg) was then added, and the mixture was further stirred under the same condition. After two hours, the reaction solution was filtered with Celite^{®} and the filtrate was concentrated under reduced pressure.

The resulting residue was dissolved in a mixture of an aqueous sodium carbonate solution (10 wt%, 63 mL) and 1,4-dioxane (44.2 mL), and cooled with ice to 0°C. Benzyl chloroformate (2.95 mL, 20.9 mmol) was then added dropwise. After the addition, the mixture was stirred for 1 hour at 0°C. A saturated aqueous ammonium chloride solution was added to quench the reaction. The mixture was extracted with ethyl acetate three times. The organic layer was washed with saturated saline and dried with magnesium sulfate. After filtration, the solvent was removed under reduced pressure. The residue was purified by silica gel column chromatography (DCM/MeOH, 98:2 to 9:1) to obtain compound L3 (4.00 g, yield: 80%) as a yellow liquid. Compound data matched the reported values (Chapman, T. M.; Courtney, S.; Hay, P.; Davis, B. G. Chem. Eur. J. 2003, 9 , 3397.)

### Synthesis of compound L4

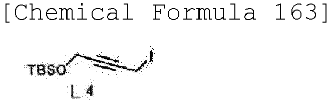

Compound L4 is a known compound, which was synthesized by the method described in a reference (Billaud, E. F. ; Rbah-Vidal, L.; Vidal, A.; Besse, S.; Tarrit, S.; Askienazy, S.; Maisonial, A.; Moins, N.; Madelmont, J. C.; Miot-Noirault, E.; Chezal, J. M.; Auzeloux, P. J. Med. Chem. 2013, 56, 8455.) .

### Synthesis of compound L5 from compound L3

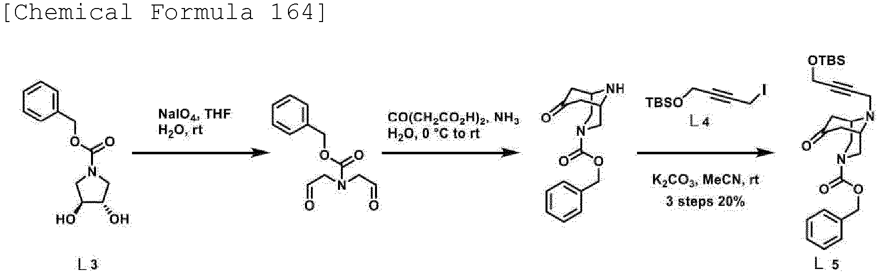

Compound L3 (1.19 g, 5.02 mmol) was dissolved in THF (35 mL), and sodium periodate (1.28 g, 5.98 mmol) dissolved in water (15 mL) was added thereto. The mixture was stirred at room temperature. After 24 hours, the same amount of aqueous sodium periodate solution was added. The mixture was stirred further for 21 hours at room temperature. Since elimination of the raw material could not be confirmed, the same amount of aqueous sodium periodate solution was added again. The mixture was stirred for 7.5 hours. THF was then removed under reduced pressure, and saturated saline was added. The mixture was extracted three times with ethyl acetate. The organic layer was dried with sodium sulfate and dried, filtered, and then the solvent was removed under reduced pressure. The residue was diluted with dichloromethane, further dried with magnesium sulfate, and filtered. The filtrate was then concentrated under reduced pressure.

1,3-acetonedicarboxylic acid (731 mg, 5.00 mmol) was dissolved in water (3.5 mL), and 25% ammonium water (3.5 mL) was added. The mixture was cooled with ice at 0°C. The residue from the operation described above was added thereto as a THF (3.5 mL) solution. The reaction mixture was gradually warmed to room temperature and stirred for 2 days. Saturated saline was then added, and the mixture was extracted three times with ethyl acetate. The organic layer was dried with magnesium sulfate and filtered. The solvent was then removed under reduced pressure. The residue was purified by silica gel column chromatography (DCM/MeOH = 98:2 to 95:5) to obtain a crude purified product as a yellow liquid.

A mixture of the resulting crude purified product from the operation described above, compound 4 (459 mg, 1.48 mmol), and N,N-diisopropylethylamine (367 µL, 2.11 mmol) was stirred for 1.5 hours at room temperature in MeCN (4.3 mL). The reaction mixture was then warmed to 40°C, stirred for 1 hour, and further warmed to 50°C. After 1 hour, the reaction mixture was diluted with water and extracted three times with ethyl acetate. The organic layer was dried with magnesium sulfate and filtered, and the solvent was then removed under reduced pressure. The residue was purified by silica gel column chromatography (Hexane/AcOEt = 6:4 to 5:5) to obtain compound L5 (446 mg, yield: 19%) as a yellow liquid. ¹H NMR (400 MHz, CDCl₃) δ 7.36-7.28 (m, 5H), 5.09 (d, J = 6.9 Hz, 2H), 4.33 (s, 2H), 4.06-3.92 (m, 2H), 3.61 (s, 2H), 3.50 (d, J = 14.7 Hz, 2H), 3.17 (m, 2H), 2.65 (dd, J = 15.8 Hz, J = 5.8 Hz, 2H), 2.25 (m, 2H), 0.90 (s, 9H), 0.10 (s, 6H); HRMS (ESI) calcd for C₂₅H₃₆N₂O₄SiNa⁺ [M+Na⁺] 479.2337, found 479.2338.

### Synthesis of compound L6 from compound L5

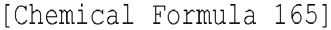

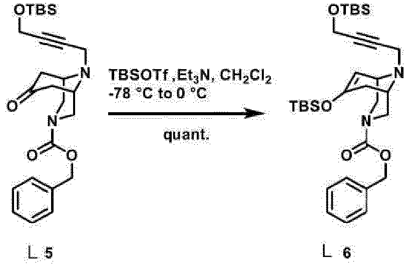

Compound L5 (1.84 g, 4.03 mmol) was dissolved in dichloromethane (20 mL), and triethylamine (840 µL, 6.03 mmol) was added. After cooling the reaction mixture to -78°C, TBSOTf (1.11 mL, 4.83 mmol) was added dropwise. The mixture was warmed to 0°C. After stirring for 10 minutes, an aqueous saturated ammonium chloride solution was added to quench the reaction. The mixture was extracted three times with dichloromethane. The organic layer was washed with saturated saline, dried with magnesium sulfate, and filtered. The solvent was then removed under reduced pressure. The residue was purified by silica gel column chromatography (Hexane/AcOEt = 7:3) to quantitatively obtain compound L6 (2.27 g) as yellow liquid. ¹H NMR (400 MHz, CDCl₃) δ 7.34-7.33 (m, 5H), 5.15-4.99 (m, 2H), 4.65 (dd, J = 17.1 Hz, J = 4.8 Hz , 1H), 4.33 (s, 2H), 4.08 (dd, J = 51.6 Hz, J = 13.2 Hz , 1H), 3.85 (dd, J = 41.4 Hz, J = 12.4 Hz , 1H), 3.47-3.08 (m, 6H), 2.35 (dd, J = 18.3 Hz, J = 7.0 Hz , 1H), 1.79 (dd, J = 34.6 Hz, J = 18.2 Hz , 1H), 0.90 (m, 18H), 0.12-0.10 (m, 12H); HRMS (ESI) calcd for C₃₁H₅₁N₂O₄Si₂⁺ [M+H⁺] 571.3382, found 571.3376.

### Synthesis of compound L6

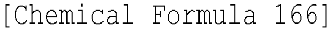

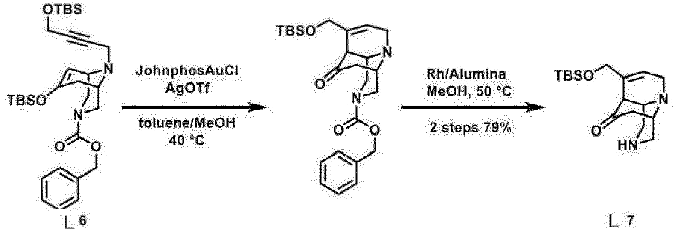

Compound L6 (2.27 g, 3.98 mmol), JohnPhos AuCl (213 mg, 0.397 mmol), and AgOTf (160 mg, 0.623 mmol) were suspended in toluene (20 mL), and methanol (2.0 mL) was added. The reaction solution was stirred for 1.5 hours at 40°C, then filtered with Celite^{®}. The filtrate was concentrated under reduced pressure.

The residue was dissolved in methanol (12 mL), and rhodium-activated alumina (500 mg) was added. After stirring for 11 hours at 50°C under a hydrogen atmosphere, the mixture was filtered with Celite^{®}. The filtrate was concentrated under reduced pressure. The residue was purified by silica gel chromatography (EtOAc = 100% to DCM/MeOH = 95:5) to obtain compound L7 (1.01 g, yield 79%) as a yellow liquid. ¹H NMR (400 MHz, CDCl₃) δ 5.68 (s, 1H), 3.92-3.87 (m, 3H), 3.20-3.26 (m, 2H), 3.01 (s, 1H), 2.95-2.91 (m, 2H), 2.86 (d, J = 11.2 Hz, 1H), 2.71 (d, J = 10.8 Hz, 1H), 2.66-2.61 (m, 2H), 2.03 (d, J = 15.5 Hz, 1H), 1.54 (bs, 1H), 0.76 (s, 9H),

-0.07 (s, 3H), -0.09 (s, 3H); HRMS (ESI) calcd for C₁₇H₃₁N₂O₂Si⁺ [M+H⁺] 323.2149, found 323.2151.

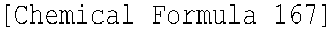

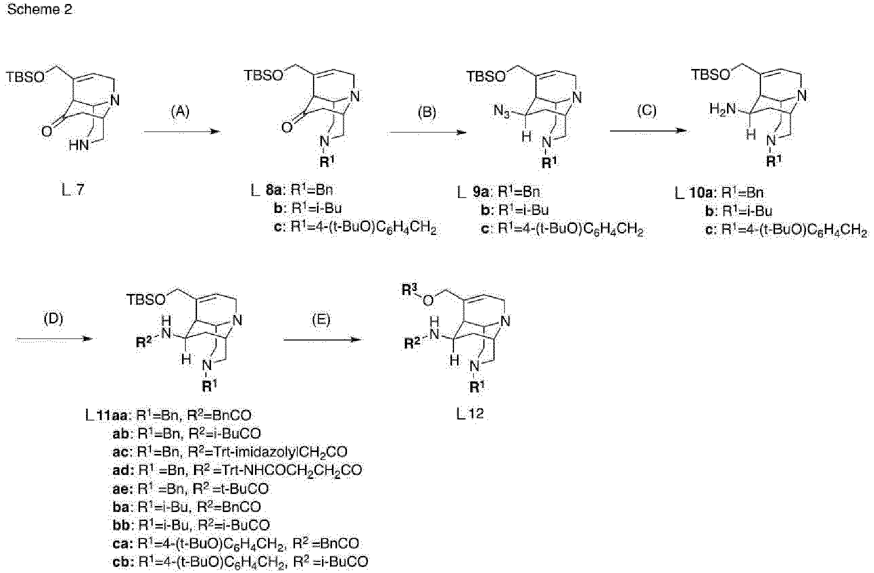

### (A) Synthesis of compound L8a from compound L7 (R¹ = Bn)

Compound L7 (167 mg, 0.517 mmol) and N,N-diisopropylethylamine (130 µL, 0.746 mmol) were dissolved in MeCN (1.5 mL), and benzyl bromide (68 µ L, 0.569 mmol) was added. After stirring for 1.5 hours at room temperature, the reaction solution was diluted with water and extracted three times with ethyl acetate. The organic layer was washed with saturated saline and filtered. The solvent was then removed under reduced pressure. The resulting residue was purified by silica gel chromatography (Hexane/AcOEt = 1:9 to EtOAc = 100%) to obtain compound L8a (191 mg, yield: 90%) as a yellow liquid. ¹H NMR (400 MHz, CDCl₃) δ 7.33-7.29 (m, 2H), 7.23-7.21 (m, 3H), 5.81 (s, 1H), 4.03-4.00 (m, 3H), 3.53 (d, J = 13.2 Hz, 1H), 3.47 (d, J = 13.2 Hz, 1H), 3.34-3.32 (m, 2H), 3.15 (s, 1H), 2.81 (d, J = 11.1 Hz, 1H), 2.75-2.70 (m, 2H), 2.63 (d, J = 10.7 Hz, 1H), 2.45-2.41 (m, 2H), 2.10 (d, J = 15.5 Hz, 1H). 0.89 (s, 9H), 0.05 (s, 3H), 0.04 (s, 3H); HRMS (ESI) calcd for C₂₄H₃₇N₂O₂Si⁺ [M+H⁺] 413.2619, found 413.2619.

### (B) Synthesis of compound L9a from compound L8a (R¹ = Bn)

Compound L8a (57.8 mg, 0.140 mmol) was dissolved in diethyl ether (980 µL) and cooled with ice to 0°C, and LiAlH₄ (13.3 mg, 0.350 mmol) was added. The mixture was stirred for 30 minutes. A saturated aqueous sodium sulfate solution was then added to quench the reaction. The reaction mixture was diluted with methanol then filtered with Celite^{®}. The filtrate was concentrated under reduced pressure. The residue was diluted with dichloromethane and filtered again with Celite^{®}. The solvent was removed under reduced pressure.

The residue was dissolved in dichloromethane (420 µL), and triethylamine (29.3 µL, 0.21 mmol) was added. The reaction mixture was cooled with ice to 0°C, added with mesyl chloride (13.0 µL, 0.168 mmol), then stirred for 1 hour at room temperature. The reaction mixture was then diluted with water and extracted three times with ethyl acetate. The organic layer was washed with saturated saline, dried with sodium sulfate, and filtered. The solvent was then removed under reduced pressure.

The residue was dissolved in N,N-dimethylformamide (560 µL), and sodium azide (18.2 mg, 0.280 mmol) was added. The mixture was stirred for 1 hour at 80°C. The reaction mixture was then diluted with water and extracted three times with ethyl acetate. The organic layer was washed with saturated saline, dried with magnesium sulfate, and filtered. The solvent was then removed under reduced pressure. The residue was purified by silica gel chromatography (EtOAc/Hexane = 4:6) to obtain compound L9a (36.2 mg, yield: 59%) as a yellow liquid. ¹H NMR (400 MHz, CDCl₃) δ 7.36-7.32 (m, 2H), 7.29-7.24 (m, 3H), 5.82 (s, 1 H), 5.41-5.36 (m, 1H), 4.29 (dd, J = 14.0 Hz, J = 1.9 Hz, 1H), 4.05 (dd, J = 14.0 Hz, J = 2.0 Hz, 1H), 3.83 (d, J = 18.9 Hz, 1H), 3.45 (s, 2H), 3.16 (dd, J = 19.2 Hz, J = 2.4 Hz, 1H), 3.00 (s, 1H), 2.92-2.89 (m, 2H), 2.79 (d, J = 10.4 Hz, 1H), 2.49-2.45 (m, 2H), 2.32 (d, J = 5.0 Hz, 1H), 1.87-1.77 (m, 2H), 0.92 (s, 9H), 0.082 (s, 3H), 0.077 (s, 3H); HRMS (ESI) calcd for C₂₄H₃₈N₅OSi⁺ [M+H⁺] 440.2840, found 440.2841.

### (C) Synthesis of compound L10a from compound L9a (R¹ = Bn)

Compound L9a (20.4 mg, 0.0464 mmol) was dissolved in THF (500 µL), and water (50 µL) and triphenylphosphine (18.3 mg, 0.0696 mmol) were added. The reaction mixture was stirred for 2.5 hours at 70°C, then the solvent was removed under reduced pressure. The residue was purified by silica gel chromatography (DCM/MeOH/Et₃N = 92:8:1) to obtain compound L10a (16.2 mg, yield: 84%) as a yellow liquid. ¹H NMR (400 MHz, CDCl₃) δ 7.28-7.26 (m, 4H), 7.24-7.18 (m, 1H), 5.80 (s, 1H), 4.50-4.44 (m, 1H), 4.23 (dd, J = 14.1 Hz, J = 1.8 Hz, 1H), 4.04 (dd, J = 14.1 Hz, J = 2.0 Hz, 1H), 3.79 (dd, J = 19.1 Hz, J = 2.5 Hz, 1H), 3.41 (d, J = 13.1 Hz, 1H), 3.37 (d, J = 13.2 Hz, 1H), 3.14 (dd, J = 19.2 Hz, J = 2.3 Hz, 1H), 2.90-2.88 (m, 2H), 2.82 (d, J = 1.6 Hz, 1H), 2.77 (d, J = 10.6 Hz, 1H), 2.43-2.40 (m, 2H), 2.18 (d, J = 4.7 Hz, 1H), 1.64 (dd, J = 13.2 Hz, J = 5.3 Hz, 1H), 1.49-1.42 (m, 1H), 1.33 (bs, 2H), 0.89 (s, 9H), 0.053 (s, 3H), 0.048 (s, 3H); HRMS (ESI) calcd for C₂₄H₄₀N₃OSi⁺ [M+H⁺] 414.2935, found 414.2943.

### (D) Synthesis of compound L11aa from compound L10a (R¹ = Bn, R² = Bn)

Compound 10a (25.3 mg, 0.0612 mmol) was dissolved in a mixture of dichloromethane (250 µL) and N,N-dimethylformamide (60 µL) and cooled with ice to 0°C. Phenyl acetic acid (10.3 mg, 0.0757 mmol), 4-methylmorpholine (20.3 µL, 0.185 mmol), 1-hydroxybenzotriazole monohydrate (10.4 mg, 0.679 mmol), and EDCI·HCl (16.9 mg, 0.0882 mmol) were added in this order and stirred for 2 hours at room temperature. A saturated aqueous sodium hydrogen carbonate solution was then added to quench the reaction. The mixture was extracted three times with ethyl acetate. The organic layer was washed with saturated saline, dried with magnesium sulfate, and filtered. The solvent was then removed under reduced pressure. The residue was purified by silica gel chromatography (AcOEt/Hexane = 8:2 to 9:1) to obtain compound L11aa (27.6 mg, yield: 85%) as a yellow liquid. ¹H NMR (400 MHz, CDCl₃) δ 7.45-7.43 (m, 2H), 7.36-7.32 (m, 4H), 7.28-7.26 (m, 2H), 7.24-7.21 (m, 2H), 5.90-5.82 (m, 2H), 5.63 (d, J = 8.9 Hz, 1H), 3.90-3.76 (m, 3H), 3.56-3.42 (m, 4H), 3.13 (dd, J = 19.3 Hz, J = 2.0 Hz, 1H), 2.98 (d, J = 11.2 Hz, 1H), 2.88 (s, 1H), 2.82-2.79 (m, 2H), 2.46-2.42 (m, 2 H), 2.32 (d, J = 4.6 Hz, 1H), 1.65 (dd, J = 13.0 Hz, J = 5.5 Hz, 1H), 1.47-1.40 (m, 1H), 0.91 (s, 9H), 0.07 (s, 6H); HRMS (ESI) calcd for C₃₂H₄₆N₃O₂Si⁺ [M+H⁺] 532.3354, found 532.3360.

### (D) Synthesis of compound L11ab from compound L10a (R¹ = Bn, R² = i-Bu)

Compound L10a (60.7 mg, 0.147 mmol) and isovaleric acid were used as a starting material and purified by silica gel chromatography (DCM/MeOH = 95:5 to 9:1) by the same method described above to obtain compound L11ab (43.4 mg, yield: 59%) as a white solid. ¹H NMR (400 MHz, CDCl₃) δ 7.45 (d, J = 7.4 Hz, 2H), 7.33 (dd, J = 7.4 Hz, J = 7.4 Hz, 2H), 7.21 (t, J = 7.4 Hz, 1H), 5.95-5.84 (m, 3H), 4.07 (d, J = 12.9 Hz, 1H), 3.99 (d, J = 13.4 Hz, 1H), 3.81 (d, J = 19.1 Hz, 1H), 3.49 (d, J = 13.1 Hz, 1H), 3.45 (d, J = 13.1 Hz, 1H), 3.17 (d, J = 19.0 Hz, 1H), 2.99 (d, J = 11.0 Hz, 1H), 2.90 (s, 1H), 2.85-2.80 (m, 2H), 2.48-2.42 (m, 2H), 2.29-2.26 (m, 1H), 2.21-2.11 (m, 1H), 2.05-1.93 (m, 2H), 1.73 (dd, J = 13.2 Hz, J = 5.0 Hz, 1H), 1.54-147 (m, 1H), 0.96 (d, J = 6.7 Hz, 3H), 0.94 (d, J = 7.0 Hz, 3H), 0.92 (s, 9H), 0.10 (s, 6H); HRMS (ESI) calcd for C₂₉H₄₈N₃O₂Si⁺ [M+H⁺] 498.3510, found 498.3519.

### (E) Synthesis of compound L12aab from compound L11aa (R¹ = Bn, R² = Bn, R³ = i-Bu)

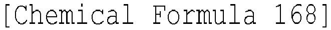

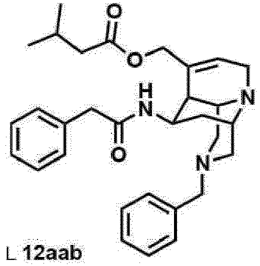

Compound L11aa (29.3 mg, 0.0551 mmol) and isovaleric acid were used as a starting material and purified by silica gel chromatography (EtOAc = 100% from DCM/MeOH=95:5) by the same method as the method described above to obtain compound L12aab (9.4 mg, yield: 34%) as a yellow liquid. ¹H NMR (500 MHz, MeOD) δ 7.61-7.19 (m, 10H), 5.93-5.87 (m, 2H), 4.43 (d, J = 13.6 Hz, 1H), 4.32 (d, J = 13.7 Hz, 1H), 3.72 (d, J = 19.3 Hz, 1H), 3.55-3.50 (m, 3H), 3.43 (d, J = 13.1 Hz, 1H), 3.23 (d, J = 19.2 Hz, 1H), 3.04 (d, J = 11.5 Hz, 1H), 2.96 (s, 1H), 2.81 (m, 2H), 2.47-2.44 (m, 3H), 2.22 (d, J = 7.3 Hz, 2H), 2.11-2.05 (m, 1H), 1.78-1.72 (m, 1H), 1.56 (dd, J = 13.3 Hz, J = 5.3 Hz, 1H), 0.96-0.95 (m, 6H); HRMS (ESI) calcd for C₃₁H₄₀N₃O₃⁺ [M+H⁺] 502.3064, found 502.3072.

### (Example 5: Manufacturing example of carbon backbone (4))

### Synthesis of compound L22

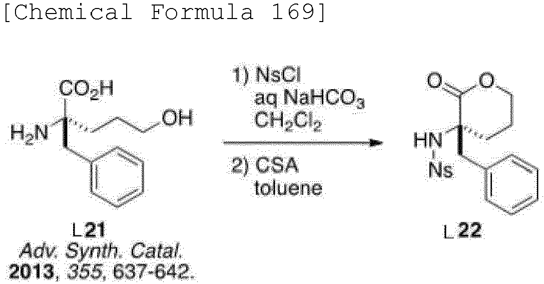

Compound L21 is synthesized in accordance with the method described in (document: Adv. Synth. Catal. 2013, 355, 637). 2-nitrobenzenesulfonyl chloride (1.1 eq.) is added to a mixture of compound 21 (1 eq.), potassium carbonate (2 eq.), 1,4-dioxane (substrate concentration: 0.5 M), and water (1/3 of the volume of dioxane). After confirming completion of a reaction with TLC, hydrochloric acid is added to the reaction solution until the aqueous layer is acidic. After separating the organic layer, the aqueous layer is extracted twice with dichloromethane. The combined organic layer is washed with saturated saline and dried with sodium sulfate. After filtration, the filtrate is concentrated under reduced pressure. The residue is dissolved in toluene, added with CSA, and heated and refluxed. After confirming the completion of the reaction with TLC, the reaction is quenched by adding saturated sodium bicarbonate water at room temperature. After stirring thoroughly, the organic layer is separated. The organic layer is washed with saturated saline and dried with sodium sulfate. After filtration, the filtration is concentrated under reduced pressure. The residue is purified by silica gel column chromatography to obtain compound L22.

### Synthesis of compound L24

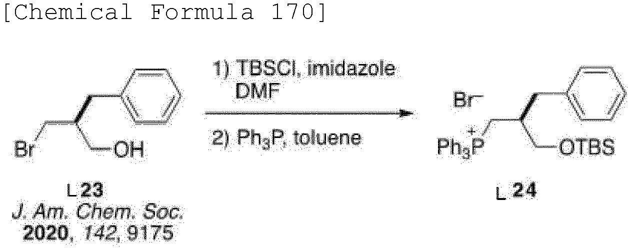

A method of synthesizing an enantiomer of compound L23 is described in (J. Am. Chem. Soc. 2020, 142, 9175). An enantiomer is obtained through synthesis. t-butylchlorodimethylsilane (1.1 eq.) is added to a mixture of compound 23 (1 eq.), imidazole (1.2 eq.), and DMF (substrate concentration: 0.5 M). After confirming completion of the reaction with TLC, the reaction solution is separated with diethyl ether and water. After extracting the aqueous layer with diethyl ether, the combined organic layer is washed with saturated saline, dried with sodium sulfate, and filtered. The filtrate is concentrated under reduced pressure. The resulting residue is purified by silica gel column chromatography. The resulting compound (TBS ether, 1 eq) is dissolved in toluene (substrate concentration: 0.5 M), and triphenylphosphine (1 eq) is added. After stirring for a certain period of time, the resulting solid is filtered out and concentrated under reduced pressure to obtain compound L24.

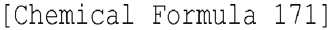

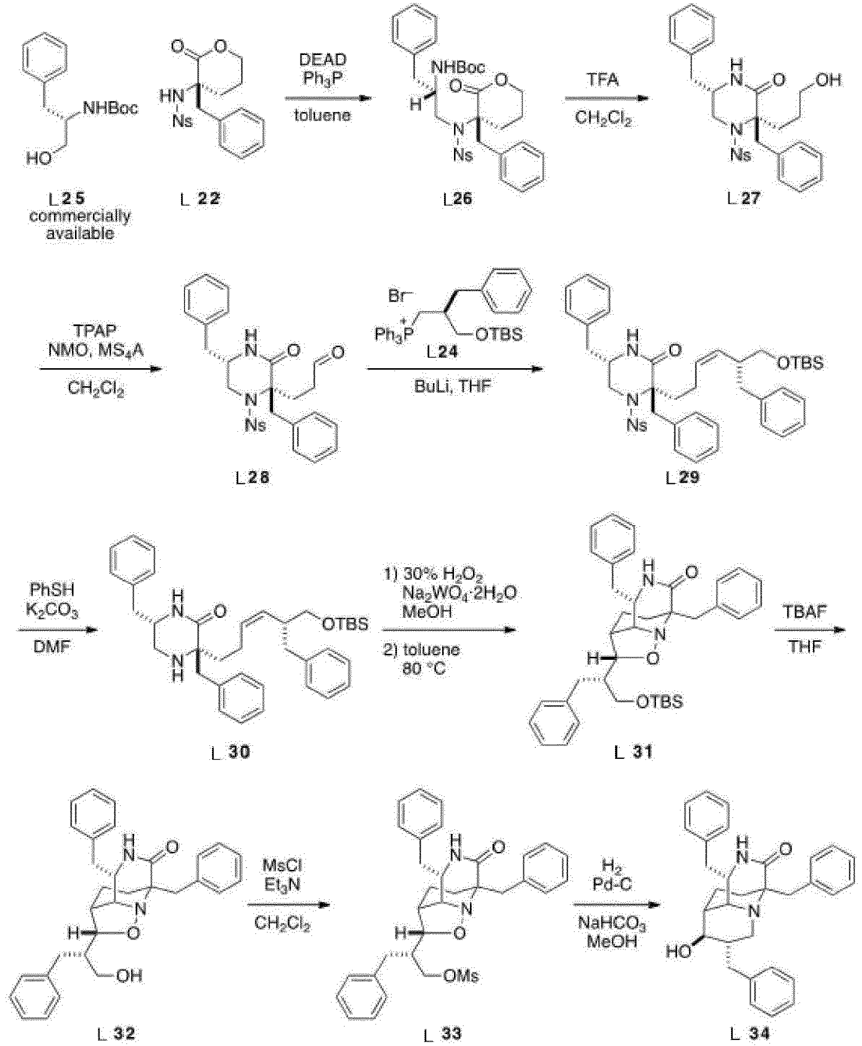

### Synthesis of compound L26

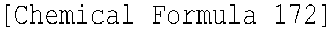

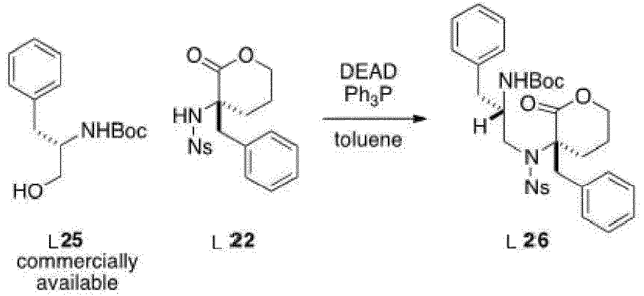

Compound L25 is commercially available. A 40% toluene solution (1.5 eq) of DEAD is added dropwise to a toluene solution (substrate concentration: 0.5 M) of compound 25 (1.2 eq), compound 22 (1 eq), and triphenylphosphine (1.5 eq). After confirming completion of the reaction with TLC, solids deposited in the reaction solution is removed by Celite filtration and then concentrated under reduced pressure. The residue is purified by silica gel column chromatography to obtain compound L26.

### Synthesis of compound L27

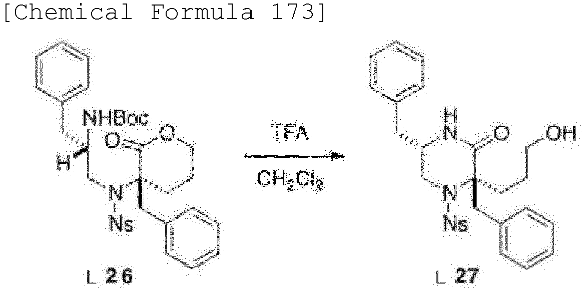

Compound L26 is dissolved in dichloromethane (substrate concentration: 0.2 M), and trifluoroacetic acid (1/5 of the volume of dichloromethane) is added dropwise. After confirming completion of the reaction with TLC, the reaction solution is concentrated under reduced pressure. The residue is purified by silica gel column chromatography to obtain compound L27.

### Synthesis of compound L28

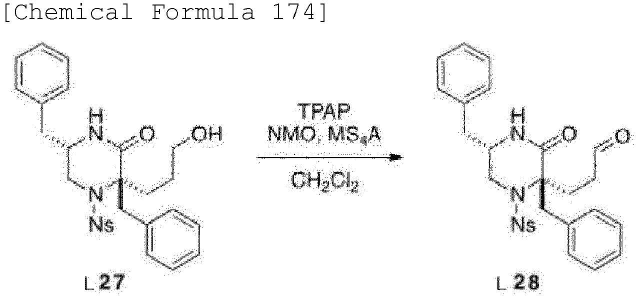

TPAP (0.05 eq) is added to a mixture consisting of compound L27 (1 eq), NMO (1.5 eq), MS4A (same weight as substrate), and dichloromethane (substrate concentration: 0.2 M). After confirming completion of the reaction with TLC, the reaction solution is concentrated under reduced pressure. The residue is filtered using a silica gel column. The filtrate is concentrated to obtain compound 28.

### Synthesis of compound L29

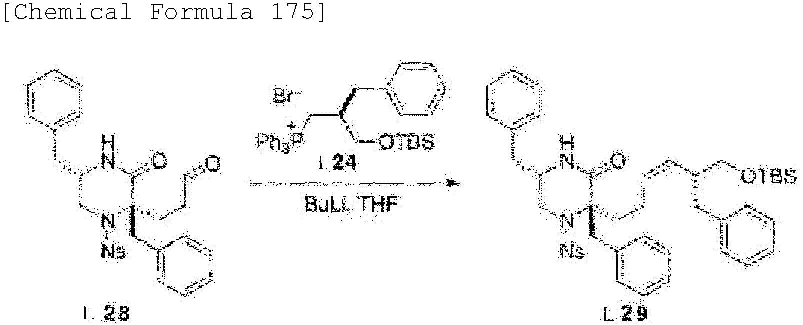

n-butyllithium (1.5 eq) is added at 0°C to a mixture of compound L24 (1.6 eq) and THF (concentration of compound 24: 0.2 M). After stirring for 30 minutes at 0°C, a THF solution of compound 28 (1 eq) is added dropwise. After confirming completion of the reaction with TLC, diluted hydrochloric acid is added to quench the reaction. The mixture is extracted three times with ethyl acetate. The combined organic layer is washed with saturated sodium bicarbonate water and saturated saline, and dried with sodium sulfate. After filtration, the filtration is concentrated under reduced pressure. The residue is purified by silica gel column chromatography to obtain compound L29.

### Synthesis of compound L30

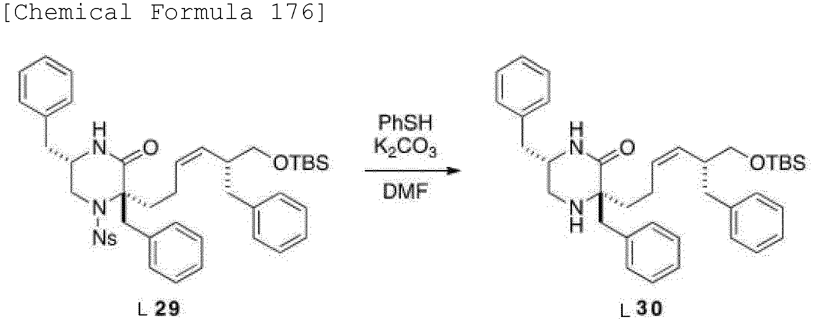

Benzenethiol (1.2 eq) is added to a mixture of compound L29 (1 eq), potassium carbonate (1.5 eq), and DMF (substrate concentration: 0.5 M). After confirming completion of the reaction with TLC, water is added to quench the reaction. The reaction mixture is extracted three time with diethyl ether. The combined organic layer is washed with water and saturated saline and dried with sodium sulfate. After filtration, the filtrate is concentrated under reduced pressure. The residue is purified by silica gel column chromatography to obtain compound L30.

### Synthesis of compound L31

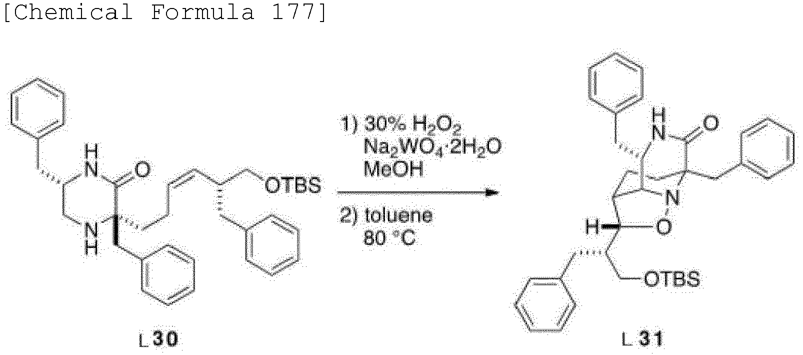

30% hydrogen peroxide water (3 eq) is added dropwise at 0°C to a mixture of compound L30 (1 eq), sodium tungstate (0.04 eq), and methanol (substrate concentration: 0.5 M). After completion of the addition, the reaction solution is stirred at room temperature. After confirming completion of the reaction with TLC, the reaction mixture is concentrated under reduced pressure. The residue is separated with dichloromethane and saturated saline. The organic layer is dried with sodium sulfate. After filtration, the filtrate is concentrated under reduced pressure. The residue is dissolved in toluene, and heated at 80°C. After concentration under reduced pressure, the residue is purified by silica gel column chromatography to obtain compound L31.

### Synthesis of compound L32

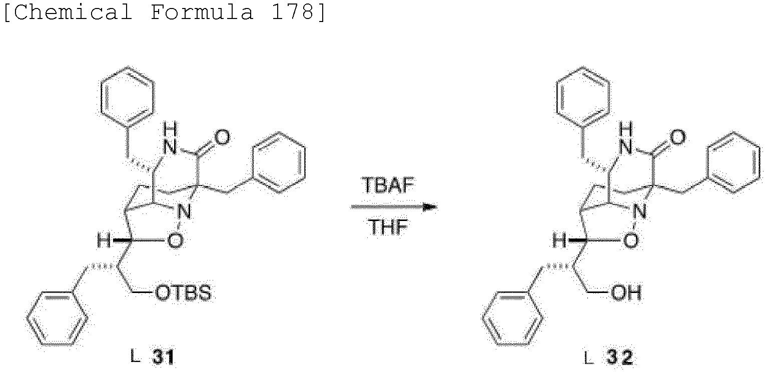

A THF solution (1.5 eq) of TBAF is added to a THF solution (substrate concentration: 0.2 M) of compound L31 (1 eq). After confirming completion of the reaction with TLC, the reaction solution is separated with water and ethyl acetate. The aqueous layer is further extracted twice with ethyl acetate, and then the combined organic layer is washed with saturated saline. The organic layer is dried with sodium sulfate and filtered. The filtrate is then concentrated under reduced pressure. The residue is purified by silica gel column chromatography to obtain compound L32.

### Synthesis of compound L33

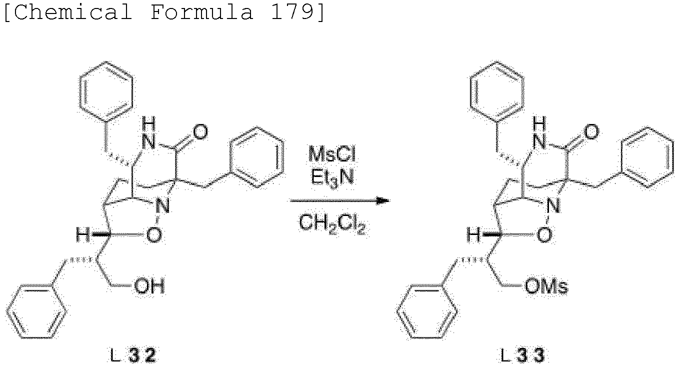

Triethylamine (1.5 eq) and methanesulfonyl chloride (1.2 eq) are sequentially added at 0°C to a dichloromethane solution (substrate concentration: 0.2 M) of compound L32 (1 eq). After confirming completion of the reaction with TLC, saturated sodium bicarbonate water is used to quench the reaction. The reaction mixture is extracted three times with ethyl acetate, and the combined organic layer is washed with saturated saline. The organic layer is dried with sodium sulfate and filtered. The filtrate is then concentrated under reduced pressure. The residue is purified by silica gel column chromatography to obtain compound L33.

### Synthesis of compound L34

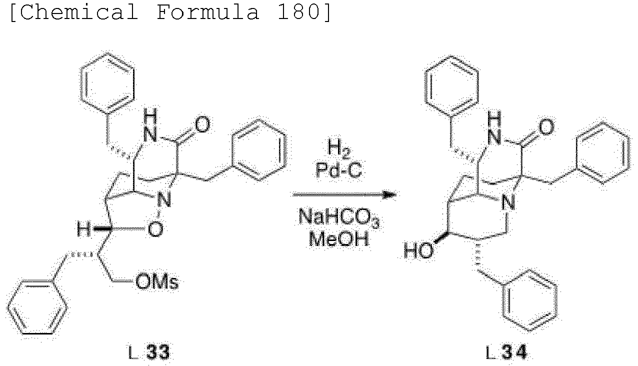

A mixture of compound L33 (1 eq), palladium on carbon (0.05 eq), sodium hydrogen carbonate (5 eq), and methanol (substrate concentration: 0.5 M) is stirred under a hydrogen atmosphere. After confirming completion of the reaction with TLC, the reaction solution is filtered by using Celite, and the filtrate is concentrated under reduced pressure. The residue is purified by silica gel column chromatography to obtain compound L34.

### (Example 6: Manufacturing example of carbon backbone (5))

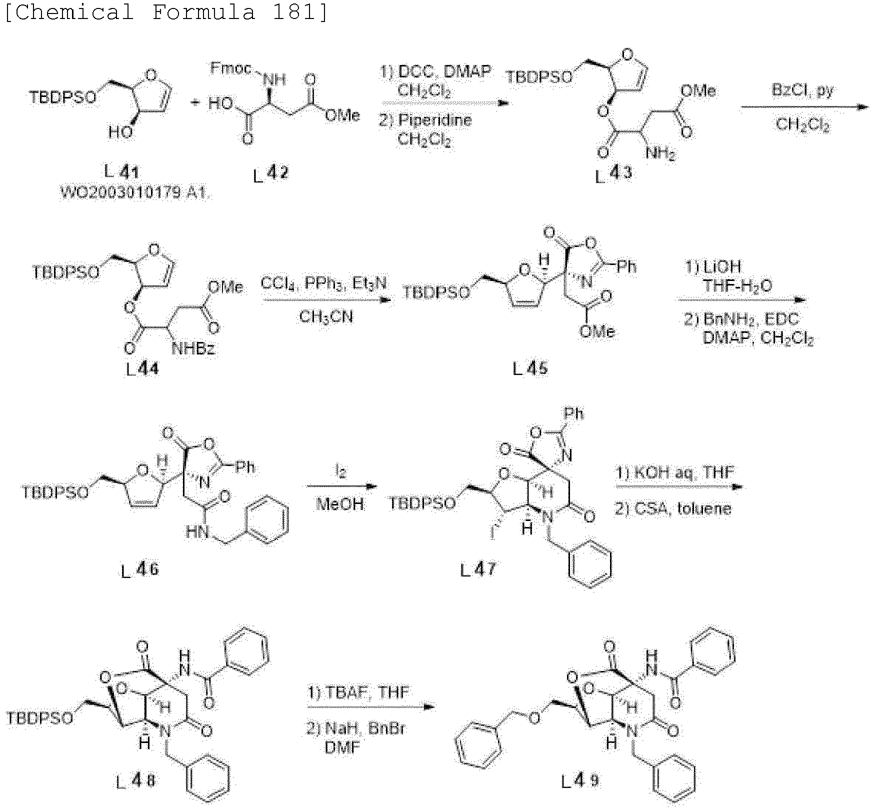

### Synthesis of compound L43

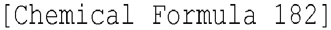

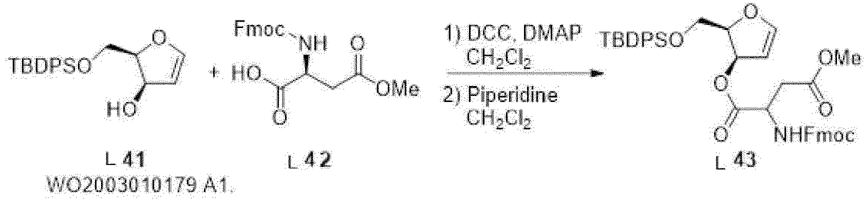

Compound L41 is synthesized in accordance with the method described in (document: WO 2003010179 A1). Dicyclohexylcarbodiimide (1.1 eq) is added to a mixture of compound L41 (1 eq), compound L42 (purchasable, 1 eq), 4-dimethylaminopyridine (small amount), and dichloromethane (substrate concentration: 0.5 M). After confirming completion of the reaction with TLC, hydrochloric acid is added to the reaction solution until the aqueous layer is acidic. After separating the organic layer, the aqueous layer is extracted twice with dichloromethane. The combined organic layer is washed with saturated saline and dried with sodium sulfate. After filtration, the filtrate is concentrated under reduced pressure. The residue is dissolved in dichloromethane, and piperidine (half the amount of dichloromethane) is added. After confirming completion of the reaction with TLC, the reaction solution is concentrated under reduced pressure. The residue is purified by silica gel column chromatography to obtain compound L43.

### Synthesis of compound L44

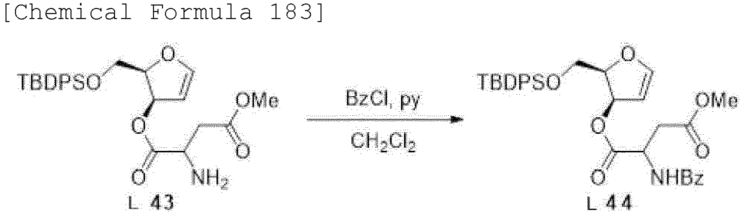

Benzoyl chloride (1.1 eq) is added to a mixture of compound L43 (1 eq), pyridine (5 eq), and dichloromethane (substrate concentration: 0.5 M). After confirming completion of the reaction with TLC, the reaction solution is diluted with ethyl acetate and saturated sodium bicarbonate water. After separation of the solution, the aqueous layer is extracted with ethyl acetate, then the combined organic layer is washed with saturated saline, dried with sodium sulfate, and filtered. The filtrate is concentrated under reduced pressure, and the resulting residue is purified by silica gel column chromatography to obtain compound L44.

### Synthesis of compound L45

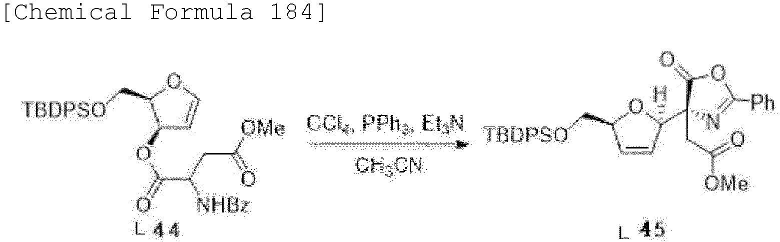

Triphenylphosphine (1.5 eq) is added to an acetonitrile solution (substrate concentration: 0.5 M) of compound L44 (1 eq), carbon tetrachloride (1.5 eq), and triethylamine (1.5 eq). After confirming completion of the reaction with TLC, the solution is concentrated under reduced pressure. The residue is purified by silica gel column chromatography to obtain compound L45.

### Synthesis of compound L46

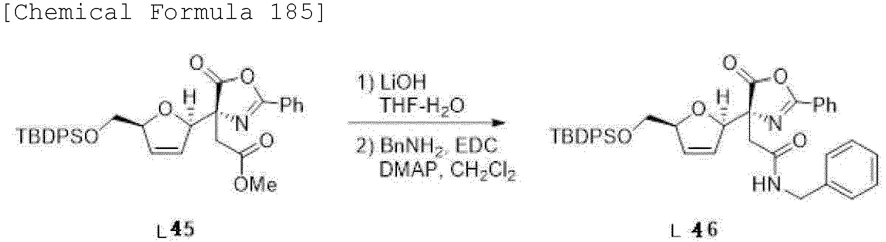

Compound L45 (1 eq) is dissolved in a mixture solvent of tetrahydrofuran and water (substrate concentration: 0.2 M), and lithium hydroxide monohydrate (1.2 eq) is added. After confirming completion of the reaction with TLC, an aqueous hydrochloric acid solution is added until the aqueous phase is weakly acidic, which is extracted with dichloromethane. The organic layer is dried with sodium sulfate and filtered, and the filtrate is concentrated under reduced pressure. Benzylamine (1.5 eq), 1-ethyl-3-(3-dimethylaminopropyl) carbodiimide hydrochloride (1.2 eq), 4-dimethylaminopyridine (small amount), and triethylamine (1.5 eq) are added to a dichloromethane solution (substrate concentration: 0.2 M) of a residue. After confirming completion of the reaction with TLC, an aqueous hydrochloric acid solution is added until the aqueous phase is weakly acidic, which is extracted with dichloromethane. The organic layer is dried with sodium sulfate and filtered. The filtrate is concentrated under reduced pressure. The residue is purified by silica gel column chromatography to obtain compound L46.

### Synthesis of compound L47

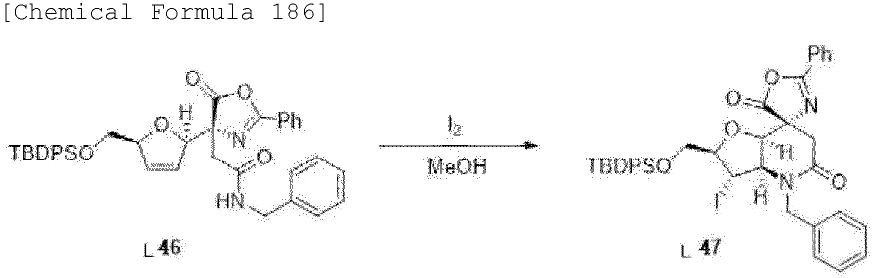

Iodine (1.5 eq) is added to a solution consisting of compound L46 (1 eq) and dichloromethane (substrate concentration: 0.2 M). After confirming completion of the reaction with TLC, the reaction solution is concentrated under reduced pressure. The residue is filtered by using a silica gel column. The filtrate is concentrated to obtain compound L47.

### Synthesis of compound L48

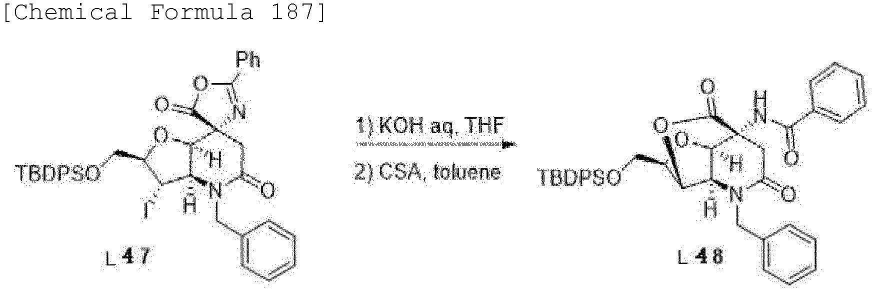

An aqueous potassium hydroxide solution is added to a mixture of THF (substrate concentration: 0.2 M) of compound L47 (1 eq). After confirming completion of the reaction with TLC, diluted hydrochloric acid is added to quench the reaction. The mixture is extracted three times with dichloromethane. The combined organic layer is dried with sodium sulfate. After filtration, the filtrate is concentrated under reduced pressure. Camphorsulfonic acid is added to a toluene solution of the residue, and the mixture is heated. After confirming completion of the reaction with TLC, the solution is concentrated under reduced pressure. The residue is purified by silica gel column chromatography to obtain compound L48.

### Synthesis of compound L49

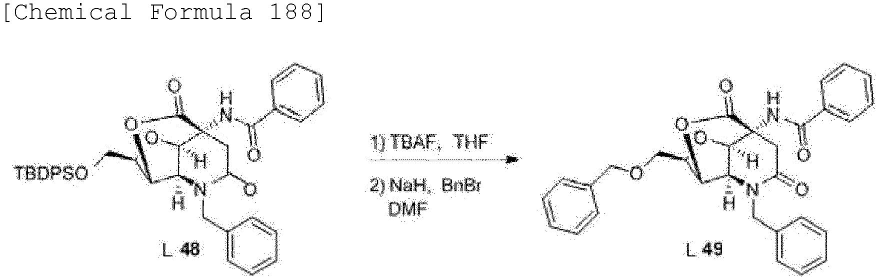

A tetrahydrofuran solution (1.5 eq) of n-tetrabutylammonium fluoride is added to compound L48 (1 eq) . After confirming completion of the reaction with TLC, the mixture is concentrated under reduced pressure. The residue is purified by silica gel column chromatography, and the residue is dissolved in dimethylformamide. Benzyl bromide (1.5 eq) and sodium hydride (2 eq) are added thereto. After confirming completion of the reaction with TLC, an aqueous hydrochloric acid solution is added until the aqueous phase is weakly acidic, which is extracted three times with dichloromethane. The combined organic layer is dried with sodium sulfate. After filtration, the filtrate is concentrated under reduced pressure. The residue is purified by silica gel column chromatography to obtain compound L49.

### (Example 7: Manufacturing example of carbon backbone (6))

### Synthesis of N-benzylbut-3-en-1-amine

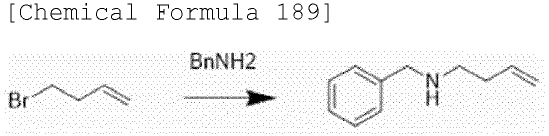

Benzylamine (2.60 ml, 23.93 mmol) and 4-bromo-1-butene (0.486 mL, 4.785 mmol) were added to a round bottom flask and dissolved in anhydrous ethanol (10 ml). Sodium iodide (72 mg, 0.48 mmol) was added thereto, and the mixture was heated and refluxed for 4 hours at 90°C. After evaporating the solvent, an aqueous 1M potassium hydroxide solution (10 ml) was added, and the mixture was extracted with dichloromethane. The organic layer was washed with water and saturated saline, and the solvent was evaporated under reduced pressure. The resulting residue was purified with silica gel column SNAP ultra 25g (chloroform:methanol = 91:9 to 75:25) to obtain the title compound (646 mg, yield: 84%).

### Synthesis of N-isobutylbut-3-en-1-amine

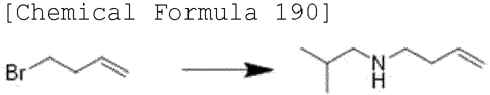

Isobutylamine (3.71 ml, 37.0 mmol) and 4-bromo-1-butene (0.752 mL, 7.41 mmol) were added to a round bottom flask and dissolved in anhydrous ethanol (15 ml). Sodium iodide (111 mg, 0.74 mmol) was added thereto, and the mixture was heated and refluxed for 4 hours at 90°C. After evaporating the solvent, an aqueous 1M potassium hydroxide solution (15 ml) was added, and the mixture was extracted with dichloromethane. The organic layer was washed with water and saturated saline, and the solvent was then evaporated under reduced pressure. The resulting residue was purified with silica gel column SNAP ultra 25g (chloroform:methanol = 91:9 to 75:25) to obtain the title compound (424 mg, yield: 45%) .

### Imine synthesis example: (1S*, 2R*, 3R*, 7S*, 8R*) -4-benzyl-1-benzylaminocarbonyl-2-isobutyl-4,10-diazatricyclo[5.3.1.0^{3,8}]undeca-9-ene (X1F) and (1R*,2R*,3S*,7S*,8S*) -4-benzyl-8-benzylaminocarbonyl-2-isobutyl-4,9-diazatricyclo[5.3.1.0^{3,8}]undeca-9-ene (X1B)

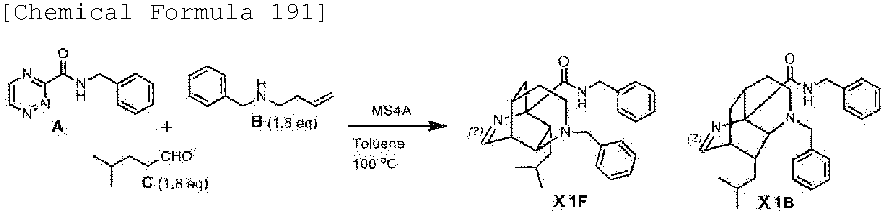

4A molecular sieves (50 mg), 4-methylpentanal (8.4 mg, 0.084 mmol), and N-(4-benzyl)but-3-en-1-amine (13.5 mg, 0.084 mmol) were added to a toluene (0.593 mL) solution of N-benzyl-1,2,4-triazine-3-carboxamide (10 mg, 0.047 mmol), and the mixture was stirred for 17 hours at 100°C. The molecular sieves were filtered and washed with chloroform. The combined organic layer was evaporated under reduced pressure. The resulting residue was purified with CHROMATOREX Q-PACK DNH30 SIZE 10 (hexane:ethyl acetate = 100:0 to 0:100) to obtain X1F (7.0 mg, yield: 35%) and X1B (2.4 mg, yield: 12%) ([M+1]⁺ = 430).

### Imine synthesis example: (1S*,2R*,3R*,7S*,8R*)-4-benzyl-1-isobutylaminocarbonyl-2-isobutyl-4,10-diazatricyclo[5.3.1.0^{3,8}]undeca-9-ene (X2F) and (1R*,2R*,3S*,7S*,8S*)-4-benzyl-8-isobutylaminocarbonyl-2-isobutyl-4,9-diazatricyclo[5.3.1.0^{3,8}]undeca-9-ene (X2B)

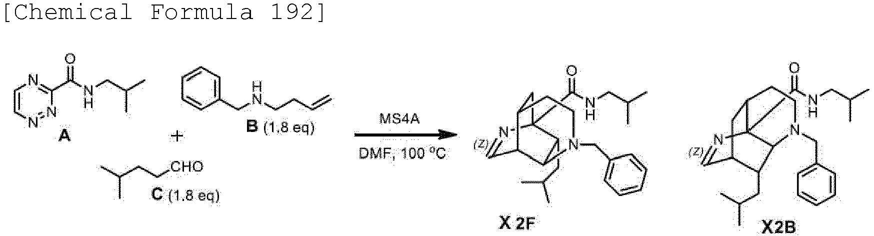

4A molecular sieves (311 mg), 4-methylpentanal (62.2 mg, 0.621 mmol), and N-(4-benzyl)but-3-en-1-amine (100 mg, 0.621 mmol) were added to a DMF (4.38 mL) solution of N-isobutyl-1,2,4-triazine-3-carboxamide (62 mg, 0.345 mmol), and the mixture was stirred for 18 hours at 100°C. The molecular sieves were filtered and washed with chloroform. The combined organic layer was evaporated under reduced pressure. The resulting residue was purified with CHROMATOREX Q-PACK DNH60 SIZE 20 (hexane:ethyl acetate = 100:0 to 0:100) and CHROMATOREX Q-PACK SI30 SIZE 20 (chloroform:methanol = 100:0 to 98:2) to obtain X2F (17.8 mg, yield: 13%) and X2B (5.5 mg, yield: 4%) ([M+1]⁺ = 396) .

Imine synthesis example: (1S*, 2R*, 3R*, 7S*, 8R*) -4-benzyl-1-isobutylaminocarbonyl-2-benzyl-4,10-diazatricyclo[5.3.1.0^{3,8}]undeca-9-ene (X3F) and (1R*,2R*,3S*,7S*,8S*)-4-benzyl-8-isobutylaminocarbonyl-2-benzyl-4,9-diazatricyclo[5.3.1.0^{3,8}]undeca-9-ene (X3B)

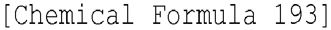

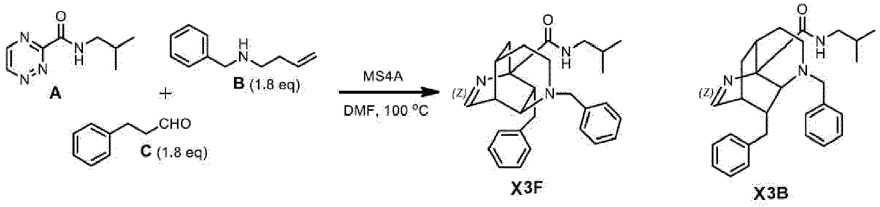

4A molecular sieves (311 mg), 3-phenylpropanal (83.3 mg, 0.621 mmol), and N-(4-benzyl)but-3-en-1-amine (100 mg, 0.621 mmol) were added to a DMF (4.38 mL) solution of N-isobutyl-1,2,4-triazine-3-carboxamide (62 mg, 0.345 mmol), and the mixture was stirred for 18 hours at 100°C. The molecular sieves were filtered and washed with chloroform. The combined organic layer was evaporated under reduced pressure. The resulting residue was purified with CHROMATOREX Q-PACK DNH60 SIZE 20 (hexane:ethyl acetate = 100:0 to 0:100) and CHROMATOREX Q-PACK SI20 SIZE 10 (chloroform:methanol = 100:0 to 95:5) to obtain X3B (5.5 mg, yield: 4%) ([M+1]⁺ = 430). Further, preparative TLC resulted in X3F (24.1 mg, yield: 16%) ([M+1]⁺ = 430) .

### Imine synthesis example: (1S*, 2R*, 3R*, 7S*, 8R*) -4-isobutyl-1-benzylaminocarbonyl-2-isobutyl-4,10-diazatricyclo[5.3.1.0^{3,8}]undeca-9-ene (X4F) and (1R*,2R*,3S*,7S*,8S*)-4-isobutyl-8-benzylaminocarbonyl-2-isobutyl-4,9-diazatricyclo[5.3.1.0^{3,8}]undeca-9-ene (X4B)

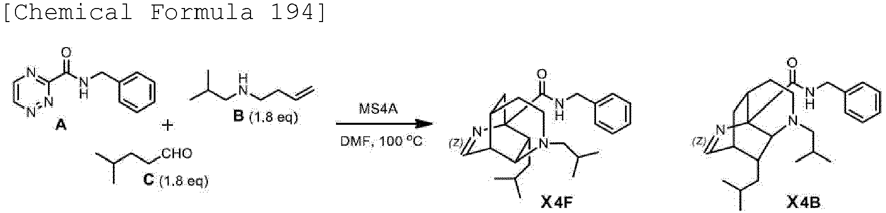

4A molecular sieves (468 mg), 4-methylpentanal (78.7 mg, 0.786 mmol), and N-(4-isobutyl)but-3-en-1-amine (100 mg, 0.786 mmol) were added to a DMF (5.55 mL) solution of N-benzyl-1,2,4-triazine-3-carboxamide (93.5 mg, 0.437 mmol), and the mixture was stirred for 18 hours at 100°C. The molecular sieves were filtered and washed with chloroform. The combined organic layer was evaporated under reduced pressure. The resulting residue was purified with CHROMATOREX Q-PACK DNH60 SIZE 20 (hexane:ethyl acetate = 100:0 to 0:100) and CHROMATOREX Q-PACK DNH30 SIZE 10 (hexane:ethyl acetate = 100:0 to 0:100) to obtain X4F (8.5 mg, yield: 5%) and X4B (17.1 mg, yield: 10%) ([M+1]⁺ = 396) .

### Imine synthesis example: (1S*, 2R*, 3R*, 7S*, 8R*) -4-isobutyl-1-benzylaminocarbonyl-2-benzyl-4,10-diazatricyclo[5.3.1.0^{3,8}]undeca-9-ene (X5F)

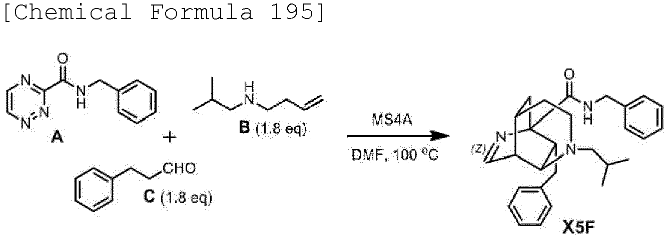

4A molecular sieves (468 mg), 3-phenylpropanal (113.4 mg, 0.786 mmol), and N-(4-isobutyl)but-3-en-1-amine (100 mg, 0.786 mmol) were added to a DMF (5.55 mL) solution of N-benzyl-1,2,4-triazine-3-carboxamide (93.5 mg, 0.437 mmol), and the mixture was stirred for 18 hours at 100°C. The molecular sieves were filtered and washed with chloroform. The combined organic layer was evaporated under reduced pressure. The resulting residue was purified with CHROMATOREX Q-PACK DNH60 SIZE 20 (hexane:ethyl acetate = 100:0 to 0:100) and preparative TLC to obtain X5F (43.5 mg, yield: 23%) ([M+1]⁺ = 430) .

Imine synthesis example: (1S*, 2R*, 3R*, 7S*, 8R*) -4-isobutyl-1-isobutylaminocarbonyl-2-benzyl-4,10-diazatricyclo[5.3.1.0^{3,8}]undeca-9-ene (X6F) and (1R*,2R*,3S*,7S*,8S*)-4-isobutyl-8-isobutylaminocarbonyl-2-benzyl-4,9-diazatricyclo[5.3.1.0^{3,8}]undeca-9-ene (X6B)

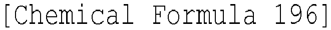

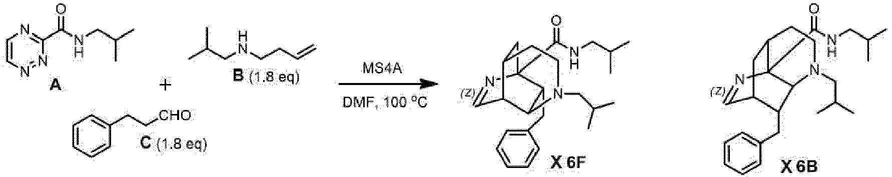

4A molecular sieves (393 mg), 3-phenylpropanal (113.4 mg, 0.786 mmol), and N-(4-isobutyl)but-3-en-1-amine (100 mg, 0.786 mmol) were added to a DMF (5.55 mL) solution of N-isobutyl-1,2,4-triazine-3-carboxamide (78.7 mg, 0.437 mmol), and the mixture was stirred for 18 hours at 100°C. The molecular sieves were filtered and washed with chloroform. The combined organic layer was evaporated under reduced pressure. The resulting residue was purified with CHROMATOREX Q-PACK DNH60 SIZE 20 (hexane:ethyl acetate = 100:0 to 0:100) to obtain X6B (7.3 mg, yield: 4%) ([M+1]⁺ = 396). Further, preparative TLC resulted in X6F (31.4 mg, yield: 18%) ([M+1]⁺ = 396) .

### Imine synthesis example: (1S*, 2R*, 3R*, 7S*, 8R*) -4-benzyl-1-benzylaminocarbonyl-2-benzyl-4,10-diazatricyclo[5.3.1.0^{3,8}]undeca-9-ene (X7F) and (1R*,2R*,3S*,7S*,8S*)-4-benzyl-8-benzylaminocarbonyl-2-benzyl-4,9-diazatricyclo[5.3.1.0^{3,8}]undeca-9-ene (X7B)

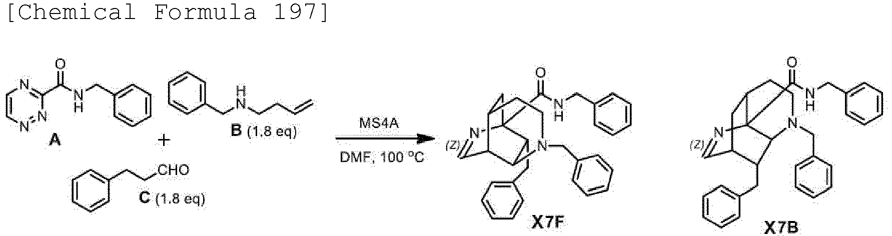

4A molecular sieves (177 mg), 3-phenylpropanal (39.9 mg, 0.297 mmol), and N-(4-benzyl)but-3-en-1-amine (47.9 mg, 0.297 mmol) were added to a DMF (2.1 mL) solution of N-benzyl-1,2,4-triazine-3-carboxamide (35.3 mg, 0.165 mmol), and the mixture was stirred for 17 hours at 100°C. The molecular sieves were filtered and washed with chloroform. The combined organic layer was evaporated under reduced pressure. The resulting residue was purified with CHROMATOREX Q-PACK DNH60 SIZE 20 (hexane:ethyl acetate = 100:0 to 0:100) to obtain X7B (7.5 mg, yield: 10%) ([M+1]⁺ = 464). Further, preparative TLC resulted in X7F (6.6 mg, yield: 9%) ([M+1]⁺ = 464) .

### Imine synthesis example: (1S*,2R*,3R*,7S*,8R*)-4-isobutyl-1-isobutylaminocarbonyl-2-isobutyl-4,10-diazatricyclo[5.3.1.0^{3,8}]undeca-9-ene (X8F) and (1R*,2R*,3S*,7S*,8S*)-4-isobutyl-8-isobutylaminocarbonyl-2-isobutyl-4,9-diazatricyclo[5.3.1.0^{3,8}]undeca-9-ene (X8B)

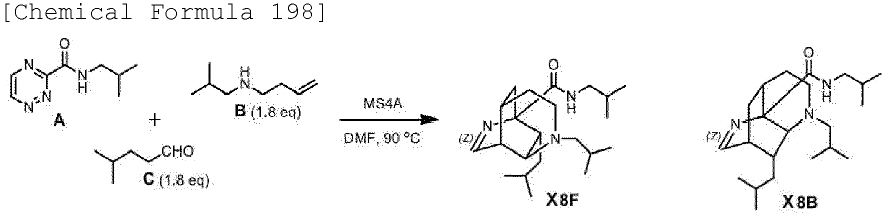

4A molecular sieves (315 mg), 4-methylpentanal (63.1 mg, 0.630 mmol), and N-(4-isobutyl)but-3-en-1-amine (80.2 mg, 0.630 mmol) were added to a DMF (4.45 mL) solution of N-isobutyl-1,2,4-triazine-3-carboxamide (63.1 mg, 0.350 mmol), and the mixture was stirred for 21 hours at 90°C. The molecular sieves were filtered and washed with chloroform. The combined organic layer was evaporated under reduced pressure. The resulting residue was purified with CHROMATOREX Q-PACK DNH60 SIZE 20 (hexane:ethyl acetate = 100:0 to 0:100) to obtain X8B (3.8 mg, yield: 3%) ([M+1]⁺ = 362). Further, preparative TLC resulted in X8F (6.6 mg, yield: 5%) ([M+1]⁺ = 362).

### Diamine synthesis example: (1S*,2R*,3R*,7S*,8R*) -4-isobutyl-1-benzylaminocarbonyl-2-benzyl-4,10-diazatricyclo[5.3.1.0^{3,8}]undecane (X5F-DA)

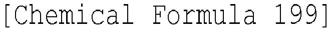

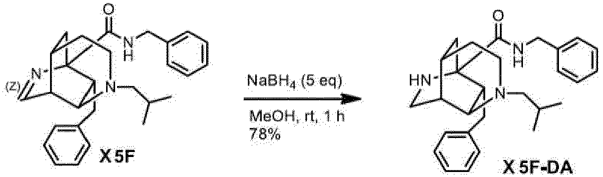

Sodium borohydride (2.6 mg, 0.069 mmol) was added to a methanol (0.25 mL) solution of imine X5F (5.9 mg, 0.014 mmol), and the mixture was stirred for 1 hour at 25°C. The reaction was quenched with a saturated aqueous sodium hydrogen carbonate solution. The mixture was extracted twice with ethyl acetate. The combined organic layer was evaporated under reduced pressure. The resulting residue was purified with CHROMATOREX Q-PACK DNH30 SIZE 10 (hexane:ethyl acetate = 100:0 to 0:100) to obtain X5F-DA (4.6 mg, yield: 78%) ([M+1]⁺ = 432) .

### Diamine synthesis example: (1S*,2R*,3R*,7S*,8R*)-4-isobutyl-1-isobutylaminocarbonyl-2-benzyl-4,10-diazatricyclo[5.3.1.0^{3,8}]undecane (X6F-DA)

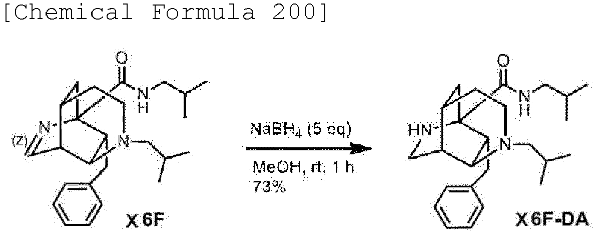

Sodium borohydride (2.8 mg, 0.075 mmol) was added to a methanol (0.25 mL) solution of imine X6F (5.9 mg, 0.015 mmol), and the mixture was stirred for 1 hour at 25°C. The reaction was quenched with a saturated aqueous sodium hydrogen carbonate solution. The mixture was extracted twice with ethyl acetate. The combined organic layer was evaporated under reduced pressure. The resulting residue was purified with CHROMATOREX Q-PACK DNH30 SIZE 10 (hexane:ethyl acetate = 100:0 to 0:100) to obtain X6F-DA (4.3 mg, yield: 73%) ([M+1]⁺ = 398).

### Diamine synthesis example: (1R*, 2R*, 3S*, 7S*, 8S*) -4-isobutyl-8-benzylaminocarbonyl-2-isobutyl-4,9-diazatricyclo[5.3.1.0^{3,8}]undecane (X4B-DA)

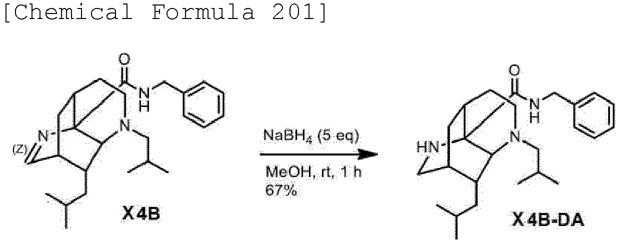

Sodium borohydride (1.9 mg, 0.049 mmol) was added to a methanol (0.25 mL) solution of imine X4B (3.9 mg, 0.010 mmol), and the mixture was stirred for 1 hour at 25°C. The reaction was quenched with a saturated aqueous sodium hydrogen carbonate solution. The mixture was extracted twice with ethyl acetate. The combined organic layer was evaporated under reduced pressure. The resulting residue was purified with CHROMATOREX Q-PACK SI30 SIZE 10 (chloroform:methanol = 100:0 to 95:5) to obtain X4B-DA (2.6 mg, yield: 67%) ([M+1]⁺ = 398) .

### Cyclic amide synthesis example: (1S*, 2R*, 3R*, 7S*, 8R*) - 4-isobutyl-1-benzylaminocarbonyl-2-benzyl-4,10-diaza-9-oxo-tricyclo[5.3.1.0^{3,8}]undecane (X5F-CA)

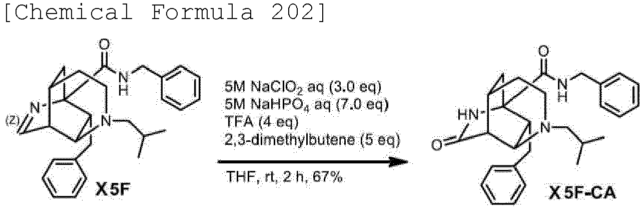

2,3-dimethylbut-2-ene (8 pl, 0.068 mmol), an aqueous 5M sodium dihydrogenphosphate solution (18.9 pl, 0.095 mmol), trifluoroacetic acid (4.1 pl, 0.054 mmol), and an aqueous 5M sodium chlorite solution (8.1 pl, 0.041 mmol) were added to a THF (0.195 mL) solution of imine X5F (5.8 mg, 0.014 mmol), and the mixture was stirred for 2 hours at 25°C. The reaction was quenched with water, and the mixture was extracted with ethyl acetate then washed with an aqueous 10% sodium thiosulfate solution and saturated saline. After the mixture was extracted again with ethyl acetate, the combined organic layer was evaporated under reduced pressure. The resulting residue was purified with CHROMATOREX Q-PACK DNH30 SIZE 10 (hexane:ethyl acetate = 100:0 to 0:100) to obtain X5F-CA (4.0 mg, yield: 67%) ([M+1]⁺ = 446).

### Cyclic amide synthesis example: (1S*, 2R*, 3R*, 7S*, 8R*) - 4-isobutyl-1-isobutylaminocarbonyl-2-benzyl-4,10-diaza-9-oxo-tricyclo[5.3.1.0^{3,8}]undecane (X6F-CA)

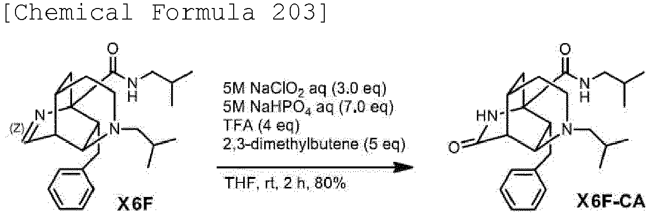

2,3-dimethylbut-2-ene (8.7 ul, 0.073 mmol), aqueous 5M sodium dihydrogenphosphate solution (20.5 pl, 0.103 mmol), trifluoroacetic acid (4.5 pl, 0.059 mmol), and aqueous 5M sodium chlorite solution (8.8 pl, 0.044 mmol) were added to a THF (0.212 mL) solution of imine X6F (5.8 mg, 0.0147 mmol), and the mixture was stirred for 2 hours at 25°C. The reaction was quenched with water, and the mixture was extracted with ethyl acetate then washed with an aqueous 10% sodium thiosulfate solution and saturated saline. After the mixture was extracted again with ethyl acetate, the combined organic layer was evaporated under reduced pressure. The resulting residue was purified with CHROMATOREX Q-PACK SI30 SIZE 10 (chloroform:methanol = 100:0 to 95:5) to obtain X6F-CA (4.6 mg, yield: 80%) ([M+1]⁺ = 412).

### Cyclic amide synthesis example: (1R*, 2R*, 3S*, 7S*, 8S*) - 4-isobutyl-8-benzylaminocarbonyl-2-isobutyl-4,9-diaza-10-oxo-tricyclo [5.3.1.0^{3,8}]undecane (X4B-CA)

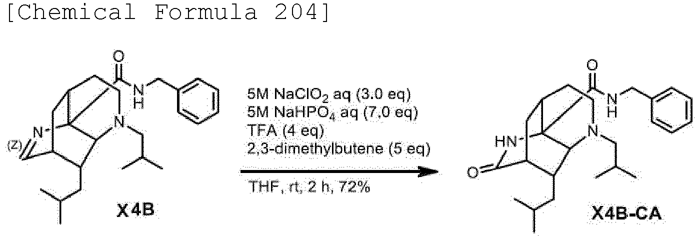

2,3-dimethylbut-2-ene (6.0 ul, 0.051 mmol), aqueous 5M sodium dihydrogenphosphate solution (14.2 pl, 0.071 mmol), trifluoroacetic acid (3.1 pl, 0.040 mmol), and aqueous 5M sodium chlorite solution (6.1 pl, 0.030 mmol) were added to a THF (0.149 mL) solution of imine X4B (4.0 mg, 0.010 mmol), and the mixture was stirred for 2 hours at 25°C. The reaction was quenched with water, and the mixture was extracted with ethyl acetate, then washed with an aqueous 10% sodium thiosulfate solution and saturated saline. After the mixture was extracted again with ethyl acetate, the combined organic layer was evaporated under reduced pressure. The resulting residue was purified with CHROMATOREX Q-PACK DNH30 SIZE 10 (hexane:ethyl acetate = 100:0 to 0:100) to obtain X4B-CA (3.0 mg, yield: 72%) ([M+1]⁺ = 412).

**[Table 1-1]**

| Compound No. | Compound name | LCMS t_{R} (min) | Mass (M+H)⁺ | Analysis method | Yield (%) |
|---|---|---|---|---|---|
| X1F | (1S*,2R*,3R*,7S*,8R*)-4-benzyl-1-benzylaminocarbonyl-2-isobutyl-4,10-diazatricyclo[5.3.1.0^{3,8}]undeca-9-ene | 2.55 | 430 | A | 35 |
| X1B | (1R*,2R*,3S*,7S*,8S*)-4-benzyl-8-benzylaminocarbonyl-2-isobutyl-4,9-diazatricyclo[5.3.1.0^{3,8}]undeca-9-ene | 2.86 | 430 | A | 12 |
| X2F | (1S*,2R*,3R*,7S*,8R*)-4-benzyl-1-isobutylaminoearbonyl-2-isobutyl-4,10-diazatricyclo[5.3.1.0^{3,8}]undeca-9-ene | 1.31 | 396 | B | 13 |

**[Table 1-2]**

| | | | | | |
|---|---|---|---|---|---|
| X2B | (1R*,2R*,3S*,7S*,8S*)-4-benzyl-8-isobutylaminocarbonyl-2-isobutyl-4,9-diazatricyclo[5.3.1.0^{3,8}]undeca-9-ene | 1.31 | 396 | B | 4 |
| X3F | (1S*,2R*,3R*,7S*,8R*)-4-benzyl-1-isobutylaminocarbonyl-2-benzyl-4,10-diazatricyclo[5.3.1.0^{3,8}]undeca-9-ene | 1.30 | 430 | B | 16 |
| X3B | (1R*,2R*,3S*,7S*,8S*)-4-benzyl-8-isobutylaminocarbonyl-2-benzyl-4,9-diazatricyclo[5.3.1.0^{3,8}]undeca-9-ene | 1.32 | 430 | B | 4 |
| X5 | (1S*,2R*,3R*,7S*,8R*)-4-isobutyl-1-benzylaminocarbonyl-2-isobutyl-4,10-diazatricyclo[5.3.1.0^{3,8}]undeca-9-ene | 1.27 | 396 | B | 5 |
| X4B | (1R*,2R*,3S*,7S*,8S*)-4-isobutyl-8-benzylaminocarbonyl-2-isobutyl-4,9-diazatricyclo[5.3.1.0^{3,8}]undeca-9-ene | 1.31 | 396 | B | 10 |
| X5F | (1S*,2R*,3R*,7S*,8R*)-4-isobutyl-1-benzylaminocarbonyl-2-benzyl-4,10-diazatricyclo[5.3.1.0^{3,8}]undeca-9-ene | 1.28 | 430 | B | 23 |
| X6F | (1S*,2R*,3R*,7S*,8R*)-4-isobutyl-1-isobutylaminocarbonyl-2-benzyl-4,10-diazatricyclo[5.3.1.0^{3,8}]undeca-9-ene | 1.27 | 396 | B | 18 |
| X6B | (1R*,2R*,3S*,7S*,8S*)-4-isobutyl-8-isobutylaminocarbonyl-2-benzyl-4,9-diazatricyclo[5.3.1.0^{3,8}]undeca-9-ene | 1.30 | 396 | B | 4 |
| X7F | (1S*,2R*,3R*,7S*,8R*)-4-benzyl-1-benzylaminocarbonyl-2-benzyl-4,10-diazatricyclo[5.3.1.0^{3,8}]undeca-9-ene | 1.31 | 464 | B | 9 |
| X7B | (1R*,2R*,3S*,7S*,8S*)-4-benzyl-8-benzylaminocarbonyl-2-benzyl-4,9-diazatricyclo[5.3.1.0^{3,8}]undeca-9-ene | 1.33 | 464 | B | 10 |
| X8F | (1S*,2R*,3R*,7S*,8R*)-4-isobutyl-1-isobutylamiocarbonyl-2-isobutyl-4,10-diazatricyclo[5.3.1.0^{3,8}]undeca-9-ene | 1.26 | 362 | B | 5 |
| X8B | (1R*,2R*,3S*,7S*,8S*)-4-isobutyl-8-isobutylaminocarbonyl-2-isobutyl-4,9-diazatricyclo[5.3.1.0^{3,8}]undeca-9-ene | 1.29 | 362 | B | 3 |

**[Table 1-3]**

| | | | | | |
|---|---|---|---|---|---|
| X5F-DA | (1S*,2R*,3R*,7S*,8R*)-4-isobutyl-1-benzylaminocarbonyl-2-benzyl-4,10-diazatricyclo[5.3.1.0^{3,8}]undecane | 1.15 | 432 | B | 78 |
| X6F-DA | (1S*,2R*,3R*,7S*,8R*)-4-isobutyl-1-isobutylaminocarbonyl-2-benzyl-4,10-diazatricyclo[5.3.1.0^{3,8}]undecane | 1.12 | 398 | B | 73 |
| X4B-DA | (1R*,2R*,3S*,7S*,8S*)-4-isobutyl-8-benzylaminocarbonyl-2-isobutyl-4,9-diazatricyclo[5.3.1.0^{3,8}]undecane | 1.33 | 398 | B | 67 |
| X5F-CA | (1S*,2R*,3R*,7S*,8R*)-4-isobutyl-1-benzylaminocarbonyl-2-benzyl-4,10-diaza-9-oxo-tricyclo[5.3.1.0^{3,8}]undecane | 1.24 | 446 | B | 67 |
| X6F-CA | (1S*,2R*,3R*,7S*,8R*)-4-isobutyl-1-isobutylaminocarbonyl-2-benzyl-4,10-diaza-9-oxo-tricyclo[5.3.1.0^{3,8}]undecane | 1.23 | 412 | B | 80 |
| X4B-CA | (1R*,2R*,3S*,7S*,8S*)-4-isobutyl-8-benzylaminocarbonyl-2-isobutyl-4,9-diaza-10-oxo-tricyclo[5.3.1.0^{3,8}]undecane | 1.40 | 412 | B | 72 |

[Analysis method A] Elution condition: flow rate 1.0 mL/min, mobile phase a = 0.05% (v/v) aqueous formic acid solution, mobile phase b = 0.05% (v/v) formic acid-containing acetonitrile; 0.1-2 minutes, linear gradient, A:B (95:5)-A:B (60:40), 2-3 minutes, linear gradient, A:B (60:40)-A:B (10:90), 3-3.5 minutes, A:B (10:90)
[Analysis method B] Elution condition: flow rate 0.9 mL/min, mobile phase a = 0.05% (v/v) aqueous formic acid solution, mobile phase b = 0.05% (v/v) formic acid-containing acetonitrile; 0-0.9 minutes, linear gradient, A:B (95:5)-A:B (10:90), 0.9-3 minutes A:B (10:90).

### (Example 8: Manufacturing example of carbon backbone (7))

### [Synthesis Example 1: Ugi reaction/intramolecular Diels-Alder reaction]

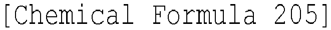

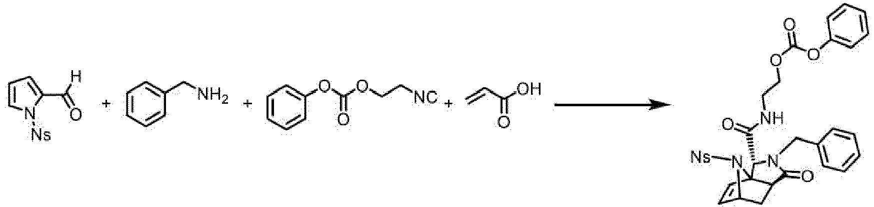

### Synthesis of 2-{(3R*,3aR*,6S*,7aS*)-2-benzyl-8-[(2-nitrophenyl)sulfonyl]-1-oxo-1,2,3,6,7,7a-hexahydro-3a,6-epiiminoisoindole-3-carboxamide}ethylphenyl carbonate (intermediate 1-1)

1-[(2-nitrophenyl)sulfonyl)-1H-pyrrole-2-carbaldehyde (0.50 g) was suspended in 2,2,2-trifluoroethanol (3.6 mL), and then benzylamine (215 µL) was added. The mixture was stirred for 1.5 hours at room temperature. 2-isocyanoethylphenylcarbamate (409 mg) and acrylic acid (147 µL) were added to the reaction solution, and the reaction solution was stirred further for three days. The reaction solution was concentrated under reduced pressure. The resulting residue was dissolved in N,N-dimethylacetamide (3.6 mL) and stirred for 25 hours at 120°C (oil bath set temperature). After cooling the reaction solution to room temperature, ethyl acetate and 1 mol/L aqueous hydrochloric acid solution were added, and the organic layer was separated. The resulting organic layer was washed with a saturated aqueous sodium hydrogen carbonate solution and saturated saline, then dried with anhydrous sodium sulfate. The residue that has been obtained from filtration and evaporation of the solvent under reduced pressure was purified by silica gel column chromatography (CHROMATOREX Q-PACK SI30 SIZE 20, eluent: hexane:ethyl acetate 4:1-0:1) to obtain the title compound (775 mg, yield: 69%, RT = 1.08 minutes, [M+H]+ = 633) as a yellow amorphous substance.
¹H-NMR (300 MHz, CDCl₃) : δ 7.92-7.88 (m, 1H), 7.68-7.63 (m, 2H), 7.54-7.50 (m, 1H), 7.44-7.39 (m, 2H), 7.34-7.26 (m, 6H), 7.20-7.16 (m, 2H), 6.42 (t, J = 5.5 Hz, 1H), 6.18 (d, J = 5.7 Hz, 1H), 6.00 (dd, J = 5.7, 2.1 Hz, 1H), 4.98 (dd, J = 4.0, 2.1 Hz, 1H), 4.95 (s, 1H), 4.59 (d, J = 15.0 Hz, 1H), 4.45 (d, J = 15.0 Hz, 1H), 4.27-4.11 (m, 2H), 3.55-3.47 (m, 2H), 2.90 (dd, J = 9.4, 4.0 Hz, 1H), 2.43 (td, J = 13.0, 4.0 Hz, 1H), 1.55 (dd, J = 13.0, 9.4 Hz, 1H) .

Intermediate 1 synthesized by changing the amine compound used is summarized in the following tables.

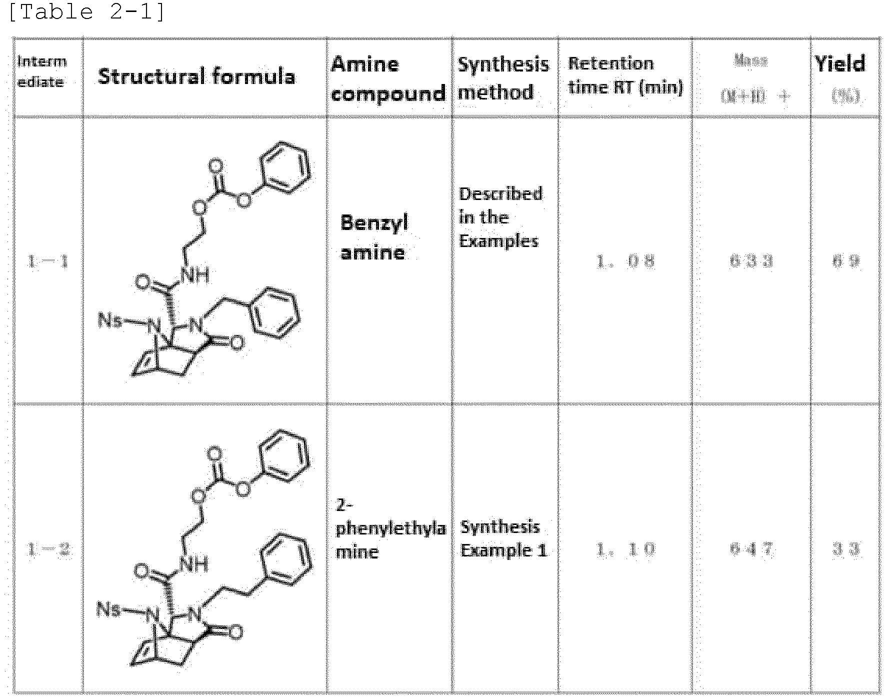

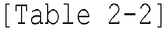

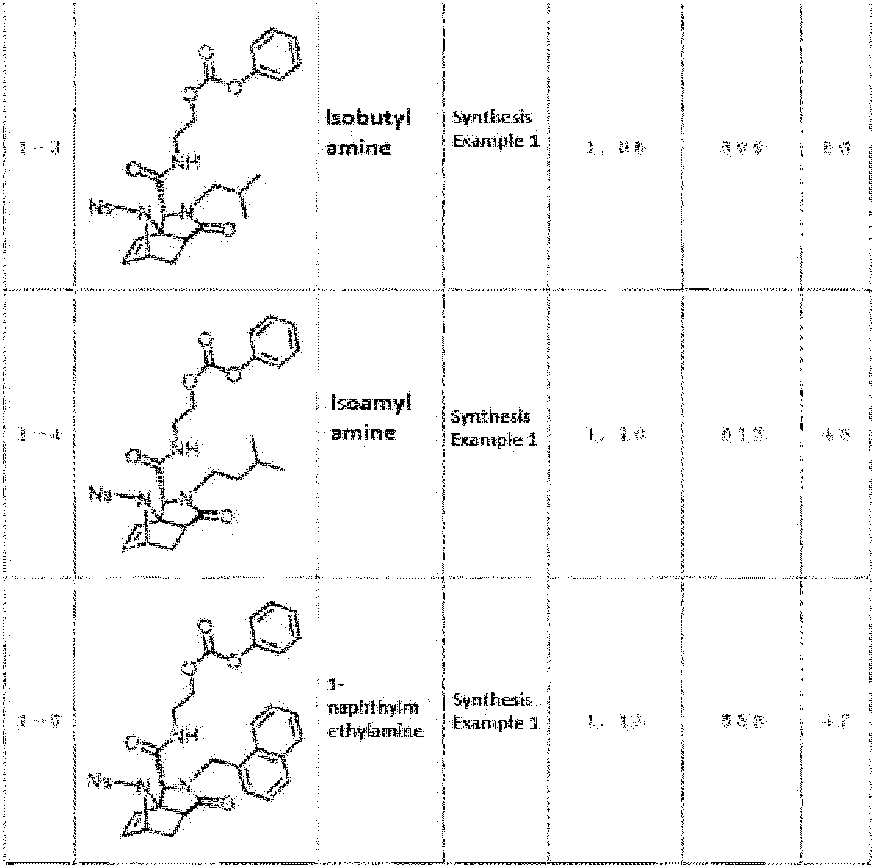

### [Synthesis Example 2: Hydrolysis reaction]

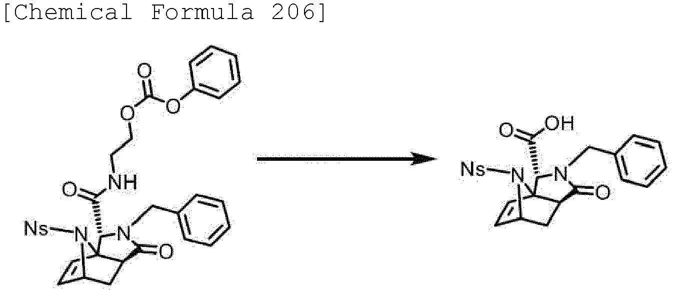

### Synthesis of (3R*,3aR*,6S*,7aS*)-2-benzyl-8-[(2-nitrophenyl)sulfonyl]-1-oxo-1,2,3,6,7,7a-hexahydro-3a,6-epiiminoisoindole-3-carboxylic acid (intermediate 2-1)

Intermediate 1-1 (775 mg) was suspended in a mixture of acetonitrile (5.7 mL) and water (0.3 mL), and then cesium carbonate (1.20 g) was added. The mixture was stirred for 16 hours at room temperature. While cooling with ice, 1 mol/L aqueous hydrochloric acid solution was added to adjust the pH of the reaction solution to 1 to 2, and the mixture was extracted with ethyl acetate. The resulting organic layer was dried with anhydrous sodium sulfate, then filtered. The solvent was evaporated under reduced pressure. The resulting residue was purified by silica gel column chromatography (CHROMATOREX Q-PACK SI30 SIZE 20, eluent: hexane:ethyl acetate 4:1-0:1, then ethyl acetate:methanol 1:0-3:2) to obtain the title compound (445 mg, yield 77%, RT = 0.95 minutes, [M+H]+ = 470) as a yellow amorphous substance.
¹H-NMR (300 MHz, CDCl₃) : δ 7.78 (dd, J = 7.7, 1.3 Hz, 1H), 7.72-7.56 (m, 3H), 7.31-7.26 (m, 5H), 6.17 (dd, J = 5.9, 2.1 Hz, 1H), 6.11 (d, J = 5.9 Hz, 1H), 5.23 (s, 1H), 5.06 (dd, J = 4.0, 2.1 Hz, 1H), 4.59 (d, J = 15.5 Hz, 1H), 4.47 (d, J = 15.5 Hz, 1H), 2.78 (dd, J = 9.4, 4.0 Hz, 1H), 2.40 (td, J = 11.7, 4.0 Hz, 1H), 1.53 (dd, J = 11.7, 9.4 Hz, 1H).

Intermediate 2 synthesized by changing intermediate 1 used as a raw material is summarized in the following tables.

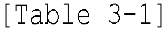

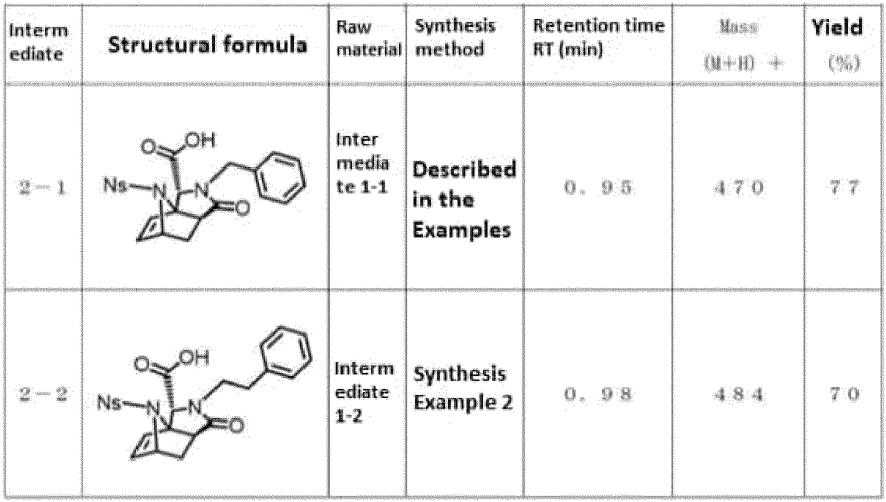

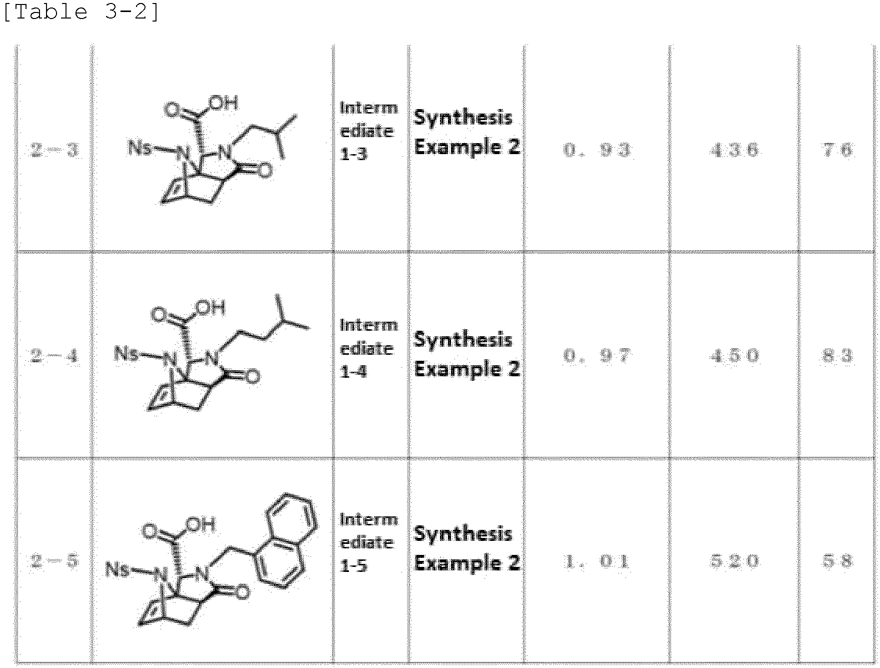

### [Synthesis Example 3: Condensation reaction]

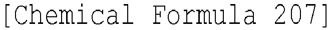

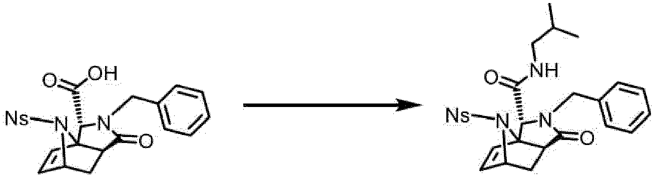

### Synthesis of (3R*,3aR*,6S*,7aS*)-2-benzyl-N-isobutyl-8-[(2-nitrophenyl)sulfonyl]-1-oxo-1,2,3,6,7,7a-hexahydro-3a,6-epiiminoisoindole-3-carboxamide (intermediate 3-1)

After dissolving intermediate 2-1 (222 mg) in N,N-dimethylformamide (1.0 mL), isobutylamine (0.57 mL), HATU (270 mg), and N,N,-diisopropylethylamine (0.24 mL) were sequentially added. The mixture was stirred for 1 hour at room temperature. The reaction was quenched by adding 1 mol/L aqueous hydrochloric acid solution to the reaction solution, and the mixture was extracted with ethyl acetate. The resulting organic phase was dried with sodium sulfate. The residue obtained by filtration and evaporation of the solvent under reduced pressure was purified by silica gel column chromatography (CHROMATOREX Q-PACK NH30 SIZE 20, eluent: hexane:ethyl acetate 4:1-0:1) to obtain the title compound (214 mg, yield 87%, RT = 1.07 minutes, [M+H]+ = 525) as a white solid.

¹H-NMR (300 MHz, CDCl₃) : δ 7.93-7.89 (m, 1H), 7.71-7.65 (m, 2H), 7.61-7.58 (m, 1H), 7.32-7.27 (m, 5H), 6.12-6.02 (m, 3H), 5.00 (dd, J = 4.0, 2.1 Hz, 1H), 4.93 (s, 1H), 4.58 (d, J = 14.7 Hz, 1H), 4.46 (d, J = 14.7 Hz, 1H), 3.03-2.84 (m, 3H), 2.43 (td, J = 7.9, 3.9 Hz, 1H), 1.64-1.52 (m, 2H), 0.85 (d, J = 6.8 Hz, 6H).

### [Synthesis Example 4: Ugi reaction/intramolecular Diels-Alder reaction]

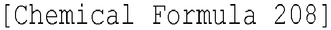

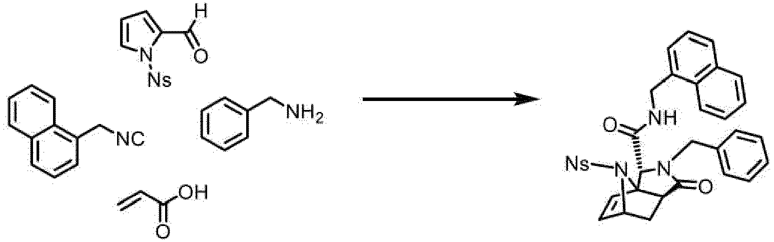

### Synthesis of (3R*,3aR*,6S*,7aS*)-2-benzyl-N-(naphthalen-1-ylmethyl)-8-[(2-nitrophenyl)sulfonyl]-1-oxo-1,2,3,6,7,7a-hexahydro-3a,6-epiiminoisoindole-3-carboxamide (intermediate 3-10)

1-[(2-nitrophenyl)sulfonyl)-1H-pyrrole-2-carbaldehyde (1.00 g) was suspended in 2,2,2-trifluoroethanol (7.0 mL), then benzylamine (0.47 mL) was added. The mixture was stirred for 1 hour at room temperature. 1-(isocyanomethyl)naphthalene (0.78 g) and acrylic acid (0.32 mL) were added to the reaction solution, and the mixture was further stirred for 26 hours. The reaction solution was concentrated under reduced pressure, and the resulting residue was dissolved in N,N-dimethylacetamide (7.0 mL). The mixture was stirred for 17 hours at 120°C (oil bath set temperature). After cooling the reaction solution to room temperature, ethyl acetate and 1 mol/L aqueous hydrochloric acid solution was added, and the organic phase was separated. The resulting organic phase was washed with a saturated aqueous sodium hydrogen carbonate solution and saturated saline, then dried with anhydrous sodium sulfate. The residue obtained by filtration and evaporation of the solvent under reduced pressure was purified by silica gel column chromatography (CHROMATOREX Q-PACK SI30 SIZE 60, eluent: hexane:ethyl acetate 4:1-0:1). The resulting solid was washed with methanol and dried at 40°C under reduced pressure to obtain the title compound (1.12 g, yield 52%, RT = 1.14 minutes, [M+H]+ = 609) as a gray solid.
¹H-NMR (300 MHz, CDCl₃) : δ 7.93-7.84 (m, 4H), 7.70-7.58 (m, 2H), 7.56-7.48 (m, 3H), 7.44 (dd, J = 8.1, 7.0 Hz, 1H), 7.32-7.30 (m, 1H), 7.22-7.13 (m, 5H), 6.30 (t, J = 5.7 Hz, 1H), 6.07 (d, J = 5.7 Hz, 1H), 6.02 (dd, J = 5.7, 2.1 Hz, 1H), 4.98 (t, J = 2.1 Hz, 1H), 4.96 (s, 1H), 4.87 (dd, J = 14.5, 5.7 Hz, 1H), 4.62 (dd, J = 14.5, 5.7 Hz, 1H), 4.53 (d, J = 14.7 Hz, 1H), 4.40 (d, J = 14.7 Hz, 1H), 2.78 (dd, J = 9.4, 4.0 Hz, 1H), 2.38 (td, J = 12.1, 4.0 Hz, 1H), 1.51 (dd, J = 12.1, 9.4 Hz, 1H).

Intermediate 3 synthesized by changing intermediate 2 and amine compound used in Synthesis Example 3 are summarized in the following tables. Intermediate 3 synthesized by changing the amine compound and isonitrile compound used in Synthesis Example 4 are also summarized in the following tables.

**[Table 4-1]**

| Intermediate | Raw material | Amino compound | Isonitrile compound | Synthesis method |
|---|---|---|---|---|
| 3-1 | Intermediate 2-1 | Isobutylamine | - | Described in the Examples |
| 3-2 | Intermediate 2-1 | Phenethylamine | - | Synthesis Example 3 |
| 3-3 | Intermediate 2-2 | Isobutylamine | - | Synthesis Example 3 |
| 3-4 | Intermediate 2-3 | Isobutylamine | - | Synthesis Example 3 |
| 3-5 | Intermediate 2-3 | Isobutylamine | - | Synthesis Example 3 |
| 3-6 | Intermediate 2-4 | Isobutylamine | - | Synthesis Example 3 |
| 3-7 | Intermediate 2-4 | Isobutylamine | - | Synthesis Example 3 |

**[Table 4-2]**

| | | | | |
|---|---|---|---|---|
| 3-8 | Intermediate 2-4 | N-(tert-butoxycarbonyl)-1,3-diaminopropane | - | Synthesis Example 3 |
| 3-9 | Intermediate 2-5 | [4-(tert-butoxy)phenyl]methamine | - | Synthesis Example 3 |
| 3-10 | - | Benzylamine | 1-(isocyanomethyl)naphthalene | Described in the Examples |
| 3-11 | - | Benzylamine | tert-butyl isocyanoacetate | Synthesis Example 4 |
| 3-12 | - | 4-fluorobenzylamine | Phenethylisocyanide | Synthesis Example 4 |
| 3-13 | - | 3-fluorobenzylamine | 4-fluorophenethylisocyanide | Synthesis Example 4 |
| 3-14 | - | [4-(tert-butoxy)phenyl]methamine | Benzylisocyanide | Synthesis Example 4 |
| 3-15 | - | 4-methoxybenzylamine | tert-butyl isocyanoacetate | Synthesis Example 4 |
| 3-16 | - | 1-naphthylmethylamine | tert-butyl isocyanoacetate | Synthesis Example 4 |
| 3-17 | - | 2-(aminomethyl)thiophene | tert-butyl isocyanoacetate | Synthesis Example 4 |
| 3-18 | - | Isoamylamine | Benzylisocyanide | Synthesis Example 4 |
| 3-19 | - | Isoamylamine | 1-(isocyanomethyl)naphthalene | Synthesis Example 4 |
| 3-20 | - | 2-phenylethylamine | Benzylisocyanide | Synthesis Example 4 |
| 3-21 | - | (2-tert-butoxyethyl)amine | Benzylisocyanide | Synthesis Example 4 |
| 3-22 | - | (2-tert-butoxyethyl)amine | Phenethylisocyanide | Synthesis Example 4 |
| 3-23 | - | Cyclohexylamine | tert-butyl isocyanoacetate | Synthesis Example 4 |
| 3-24 | - | Cyclohexylmethylamine | 4-fluorophenethylisocyanide | Synthesis Example 4 |

**[Table 4-3]**

| | | | | |
|---|---|---|---|---|
| 3-25 | - | B-alanine tert-butyl ester | 1-(isocyanomethyl)naphthalene | Synthesis Example 4 |
| 3-26 | - | N-(tert-butoxycarbonyl)-1,3-diaminopropane | Phenethylisocyanide | Synthesis Example 4 |
| 3-27 | - | Isoamylamine | Phenethylisocyanide | Synthesis Example 4 |

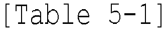

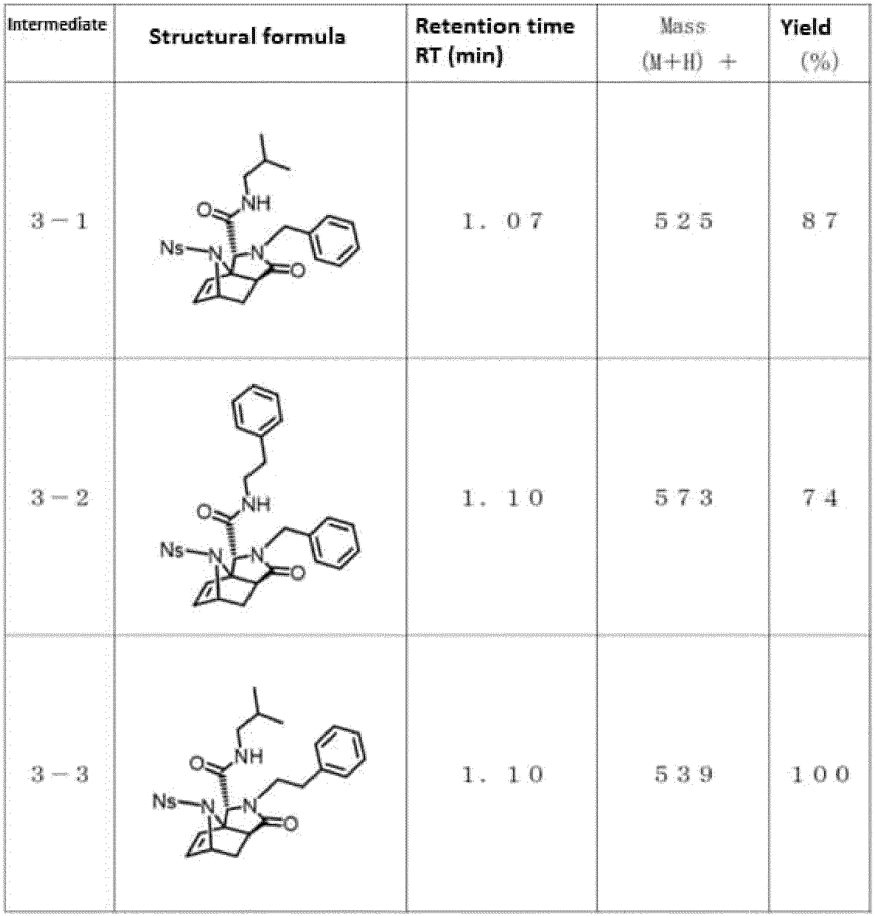

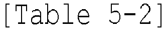

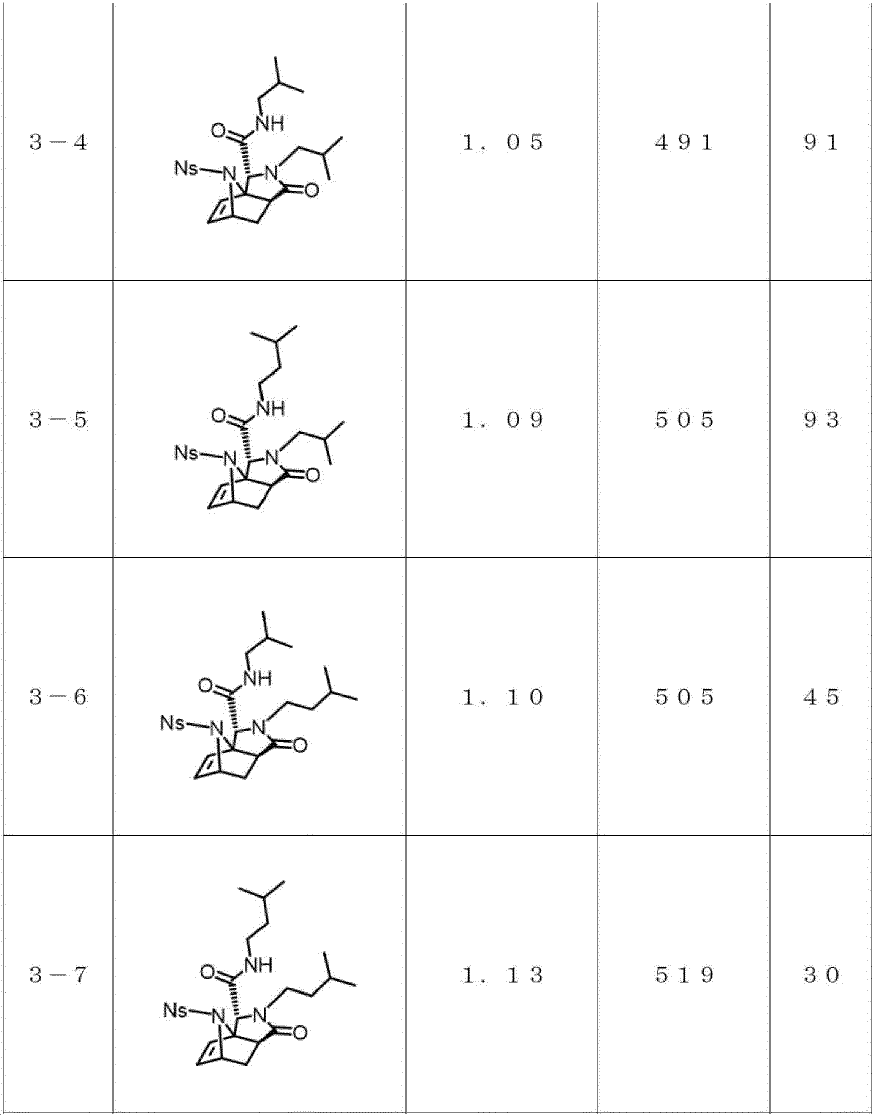

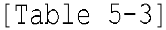

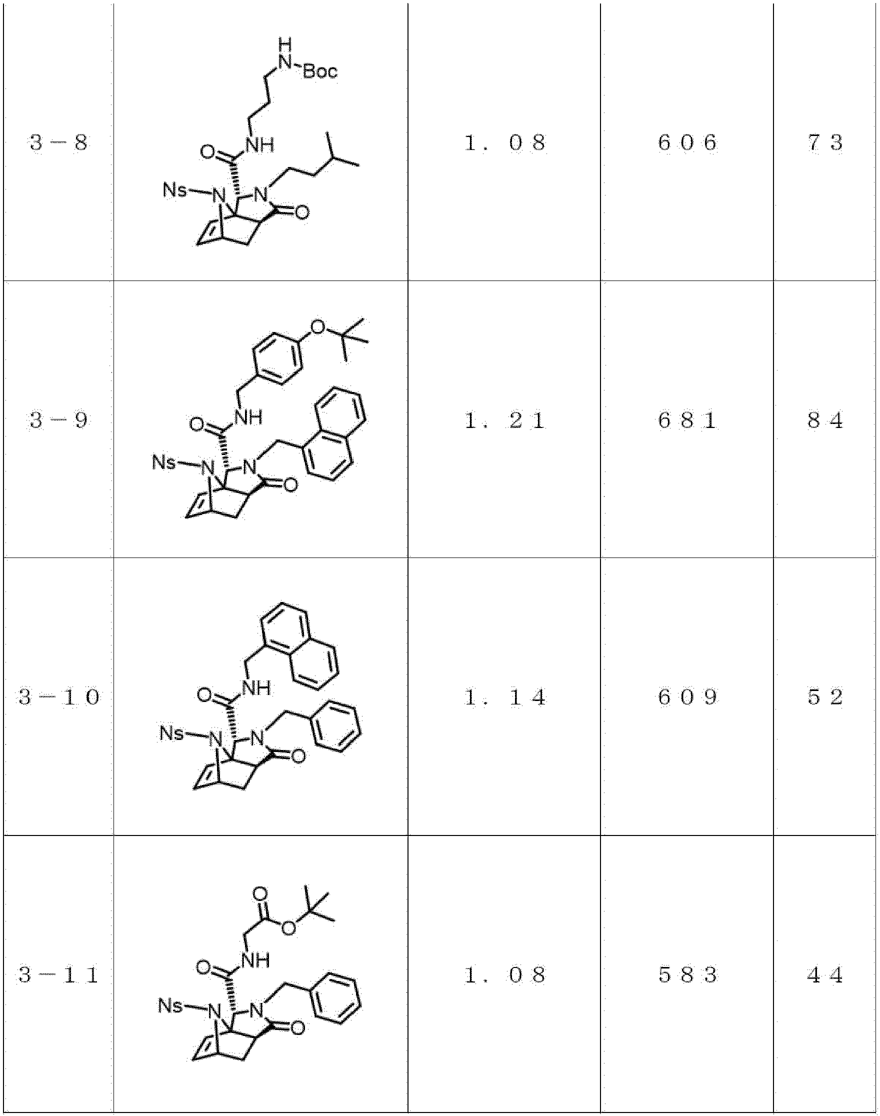

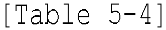

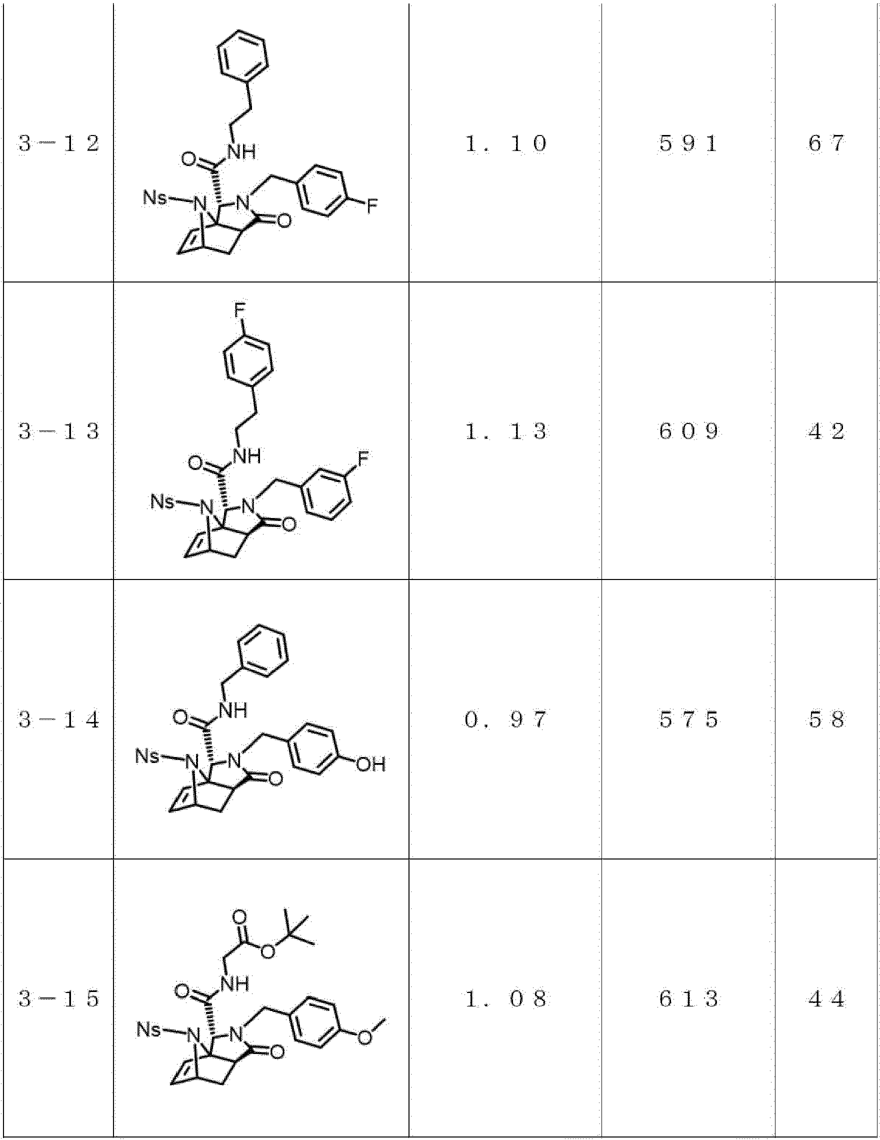

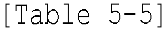

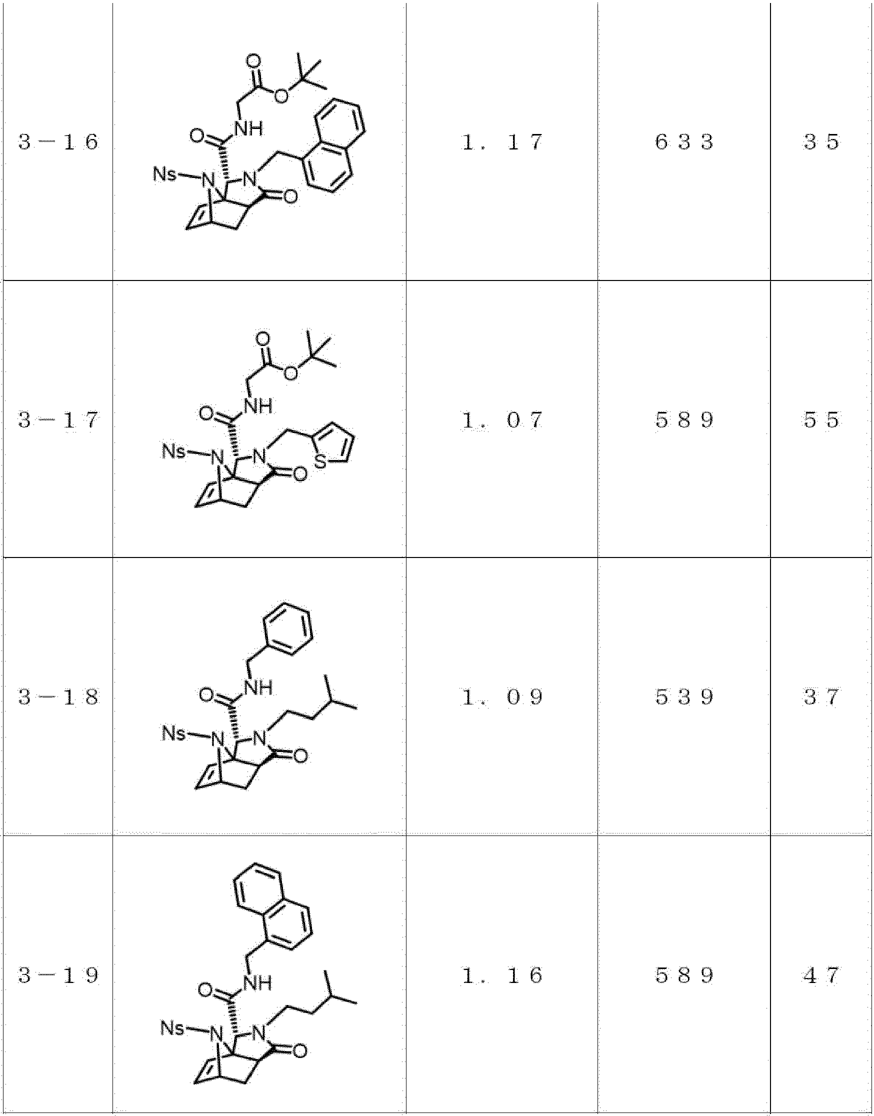

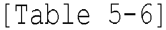

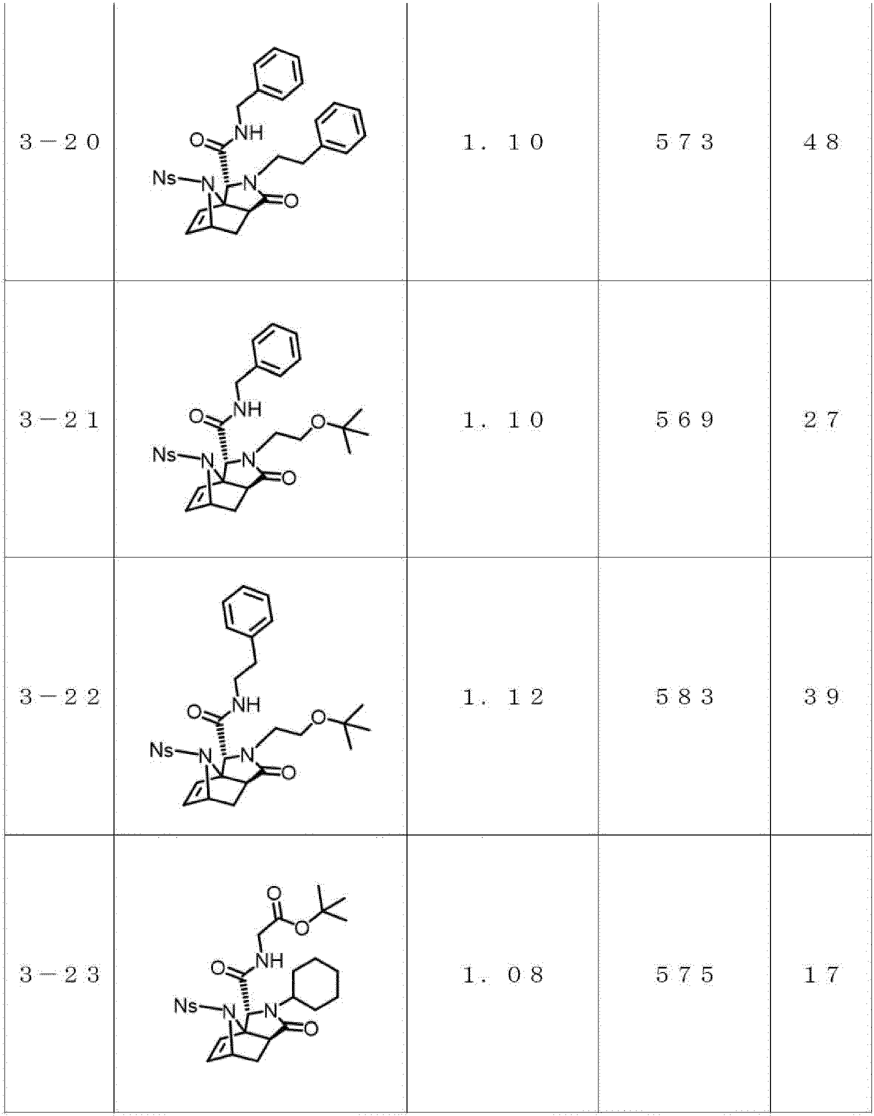

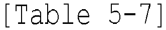

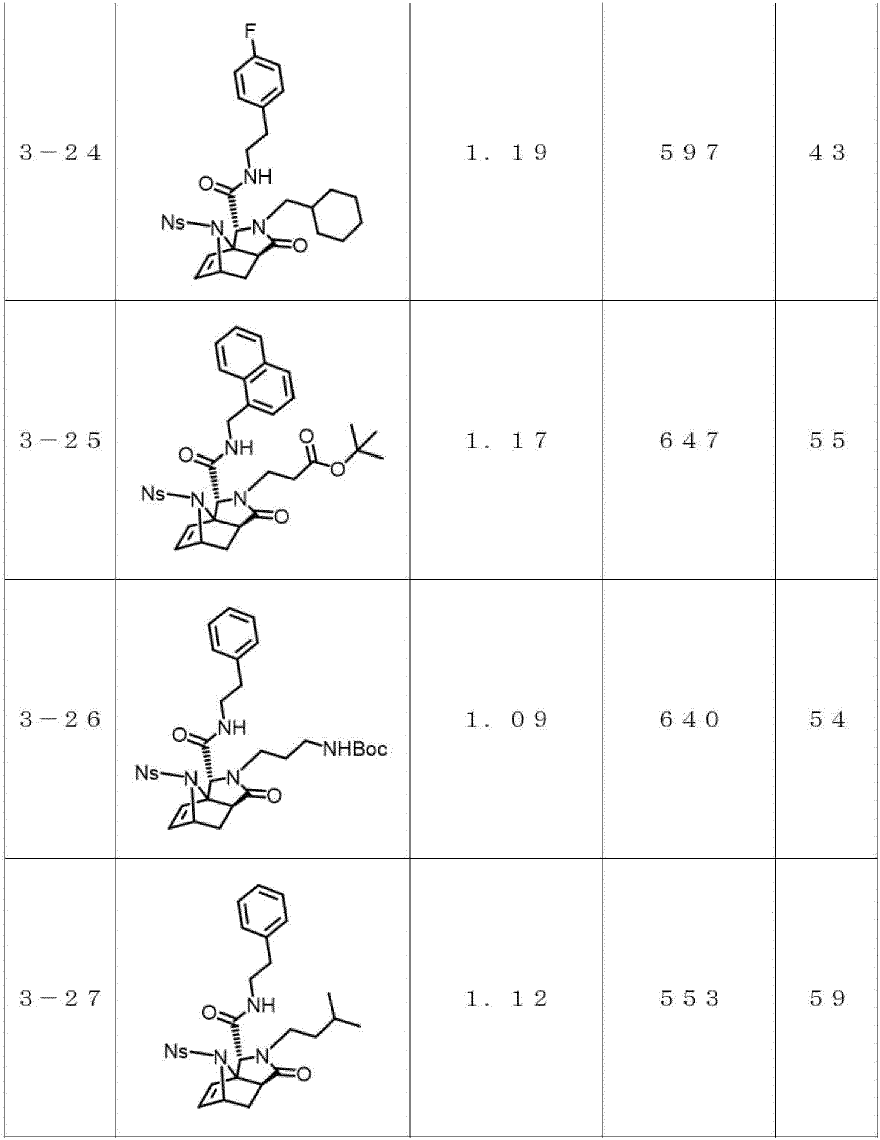

### [Synthesis Example 5: Deprotection reaction of Ns group]

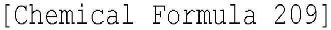

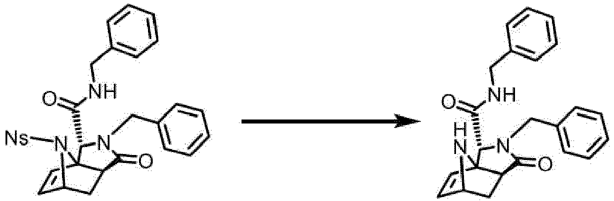

### Synthesis of (3R*,3aR*,6S*,7aS*)-N,2-dibenzyl-1-oxo-1,2,3,6,7,7a-hexahydro-3a,6-epiiminoisoindole-3-carboxamide (intermediate 4-1)

(3R*,3aR*,6S*,7aS*)-N,2-dibenzyl-8-[(2-nitrophenyl)sulfonyl]-1-oxo-1,2,3,6,7,7a-hexahydro-3a,6-epiiminoisoindole-3-carboxamide (compound described in J. Org. Chem. 2014, 1207-1214, 95.2 mg) was suspended in acetonitrile (1.7 mL), and cesium carbonate (111 mg) and 1-thioglycerl (29.0 uL) were sequentially added. The mixture was then stirred for 19 hours at 50°C (oil bath set temperature). After cooling the reaction solution to room temperature, insoluble matters were removed by filtration, and the solvent was concentrated under reduced pressure. The resulting residue was purified by silica gel column chromatography (CHROMATOREX Q-PACK NH30 SIZE 10, eluent: hexane:ethyl acetate 9:1-0:1) to obtain the title compound (43.1 mg, yield 68%, RT = 0.77 minutes, [M+H]+ = 374) as a yellow oily substance.
¹H-NMR (300 MHz, CDCl₃) : δ 7.37-7.21 (m, 10H), 6.70 (t, J = 5.9 Hz, 1H), 6.37 (dd, J = 5.3, 1.9 Hz, 1H), 6.28 (d, J = 5.3 Hz, 1H), 4.91 (d, J = 15.1 Hz, 1H), 4.51 (dd, J = 14.5, 5.9 Hz, 1H), 4.31 (dd, J = 14.5, 5.9 Hz, 1H), 4.22 (dd, J = 3.8, 1.9 Hz, 1H), 4.11 (d, J = 15.1 Hz, 1H), 4.00 (s, 1H), 2.44 (dd, J = 9.1, 3.8 Hz, 1H), 2.19 (td, J = 11.7, 3.8 Hz, 1H), 1.42 (dd, J = 11.7, 9.1 Hz, 1H).

Intermediate 4 synthesized by changing intermediate 3 used as a raw material is summarized in the following tables.

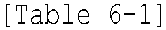

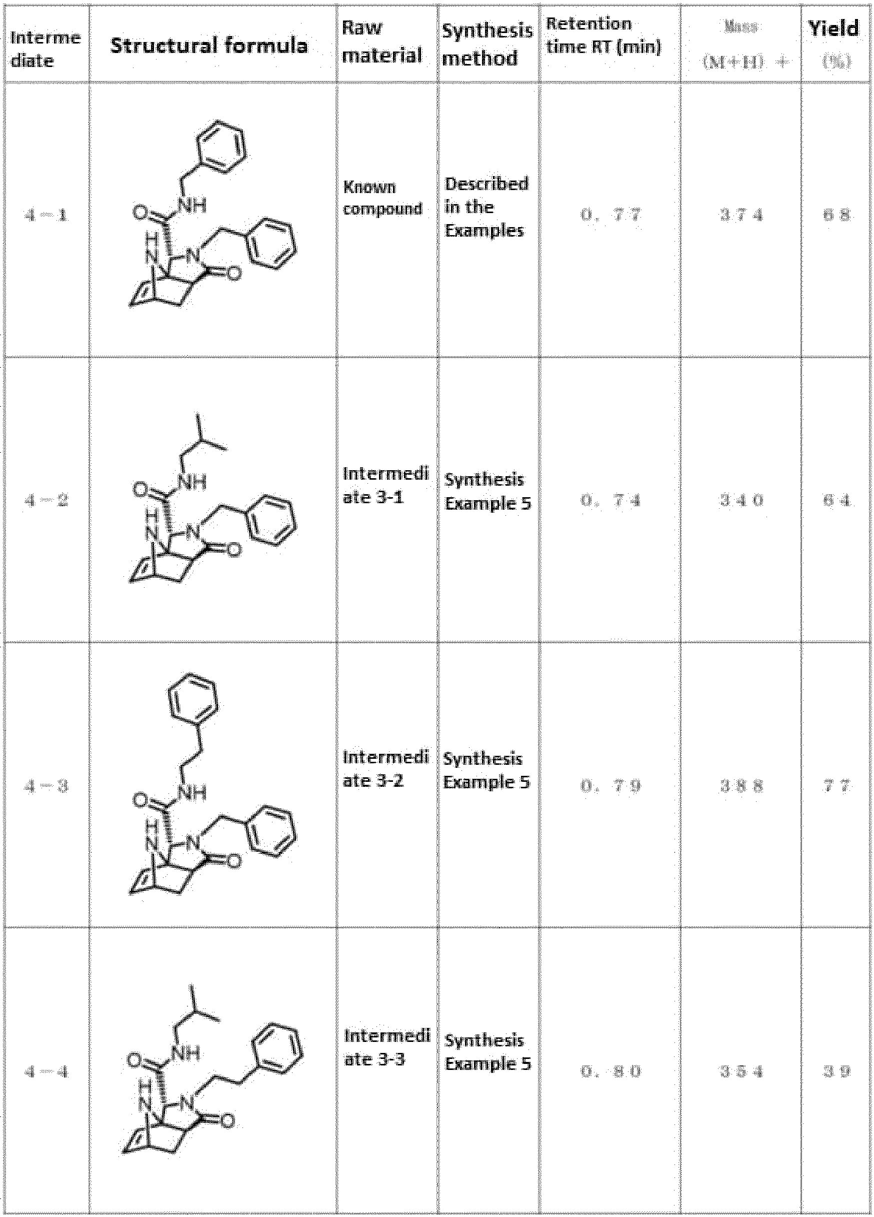

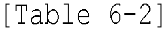

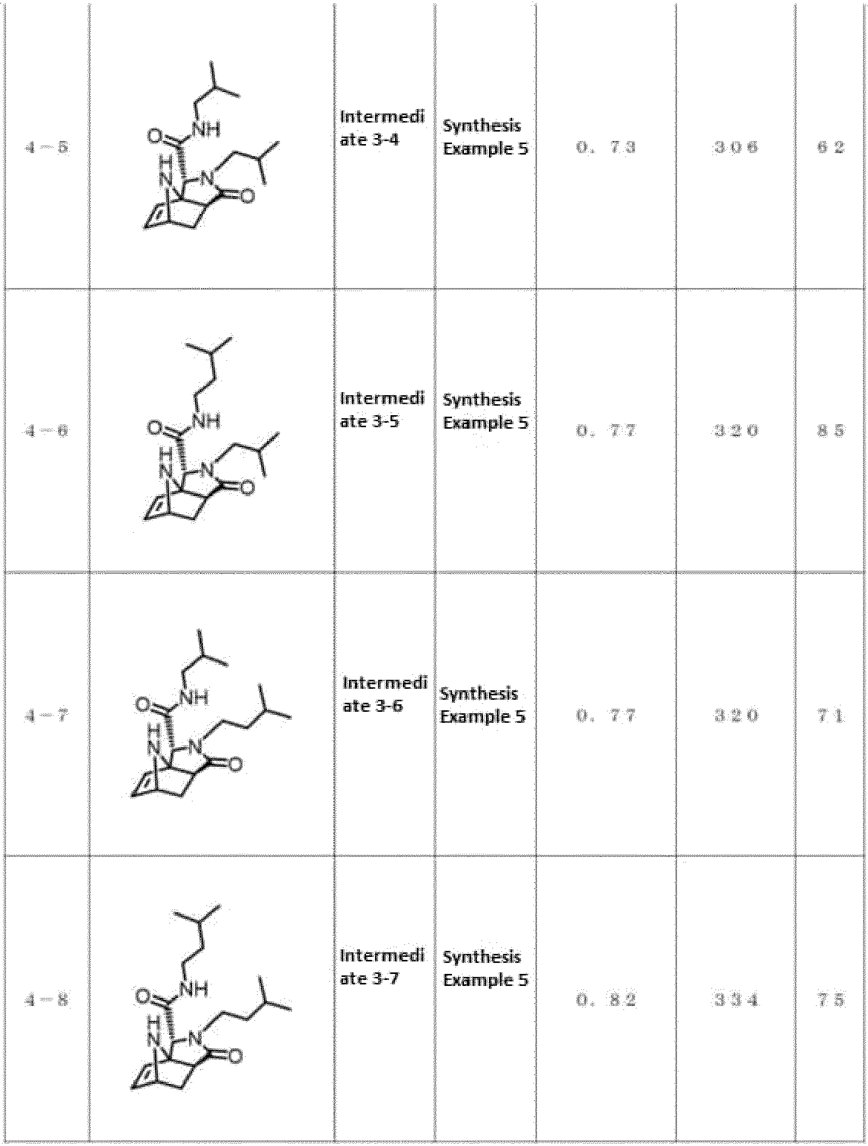

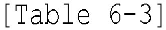

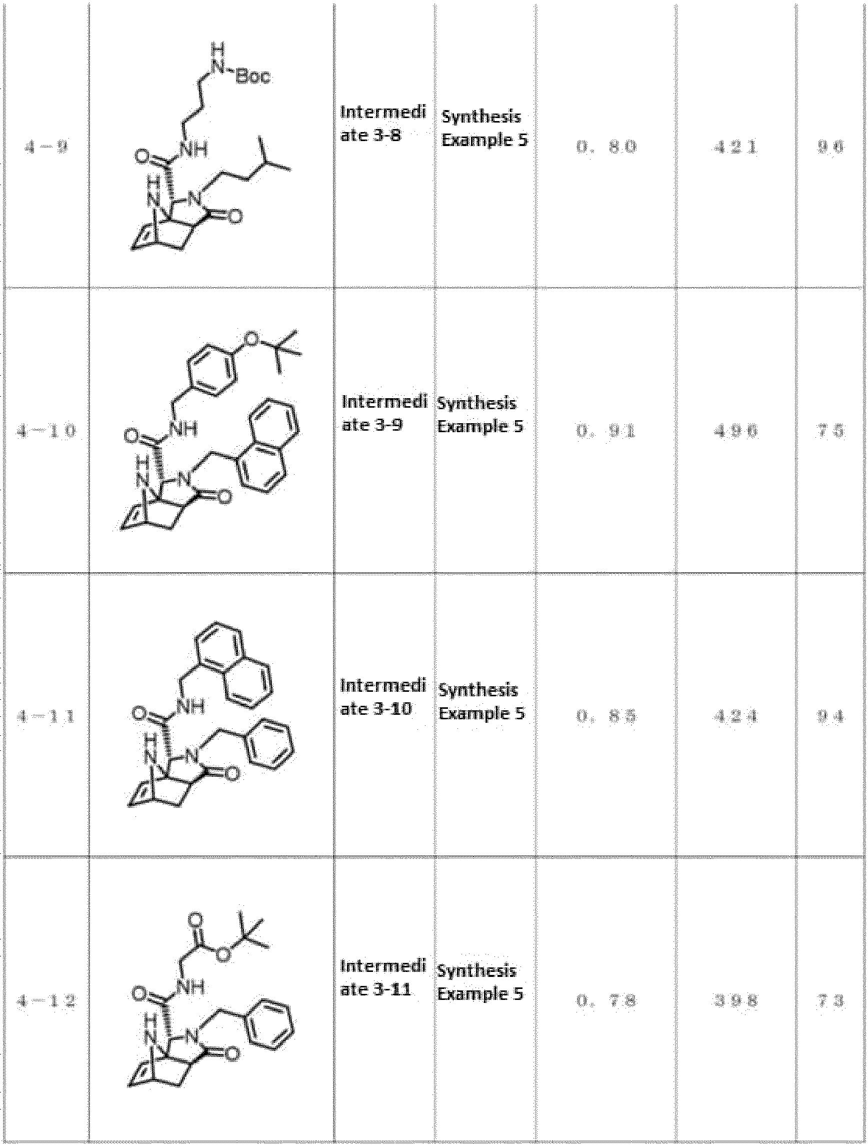

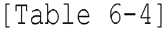

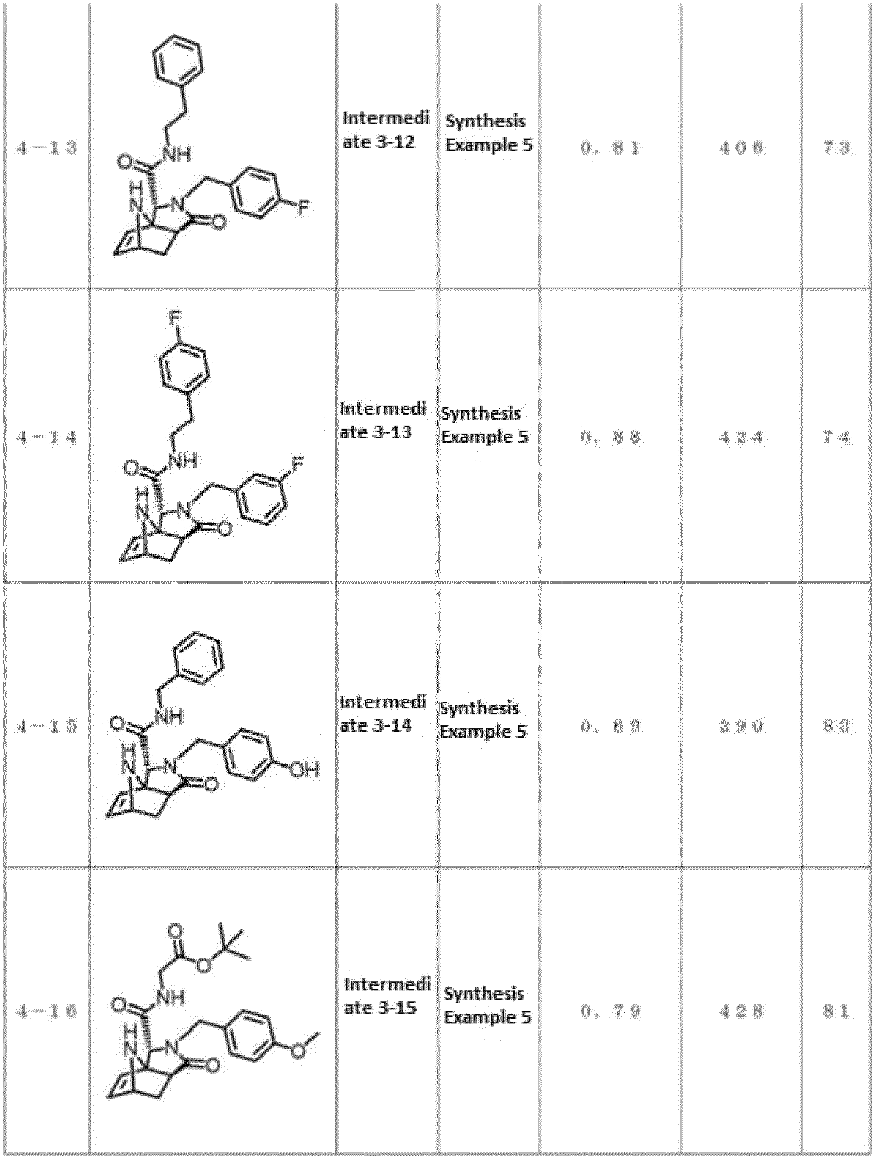

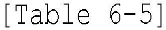

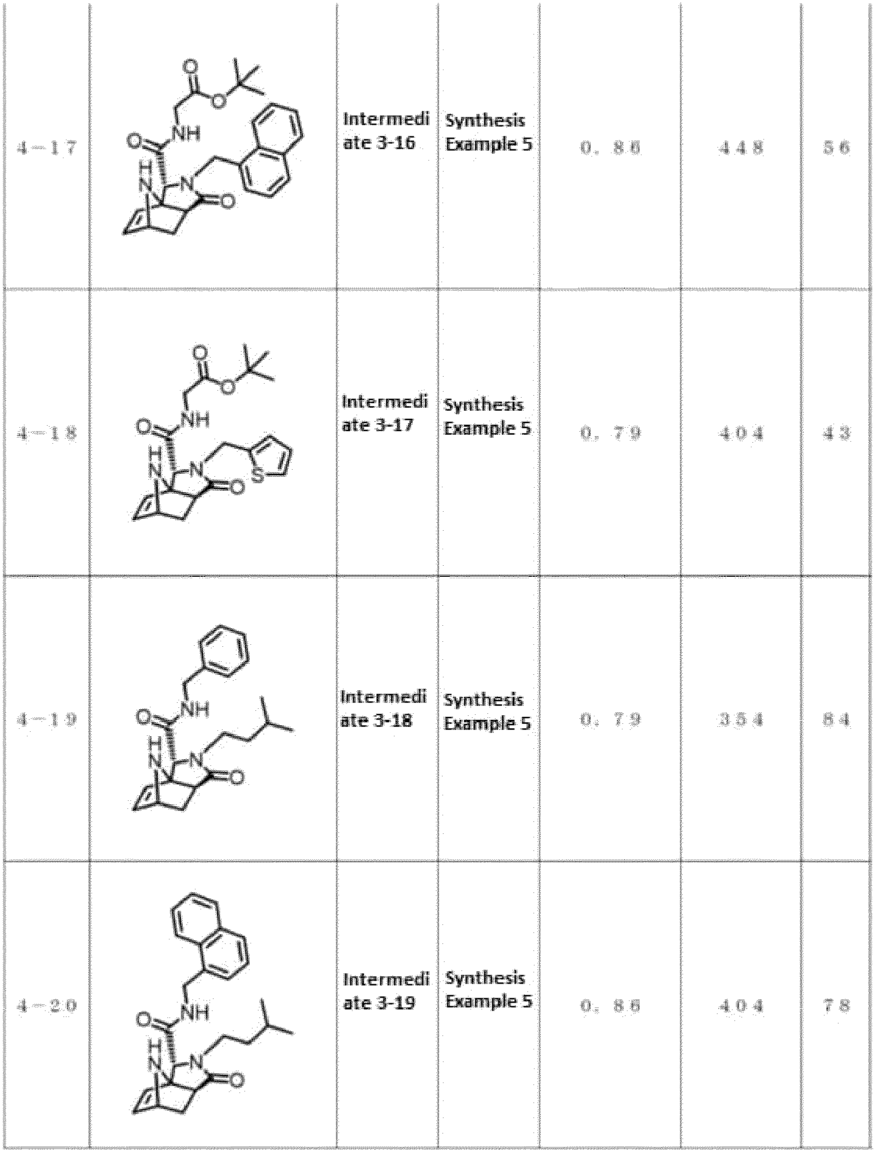

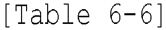

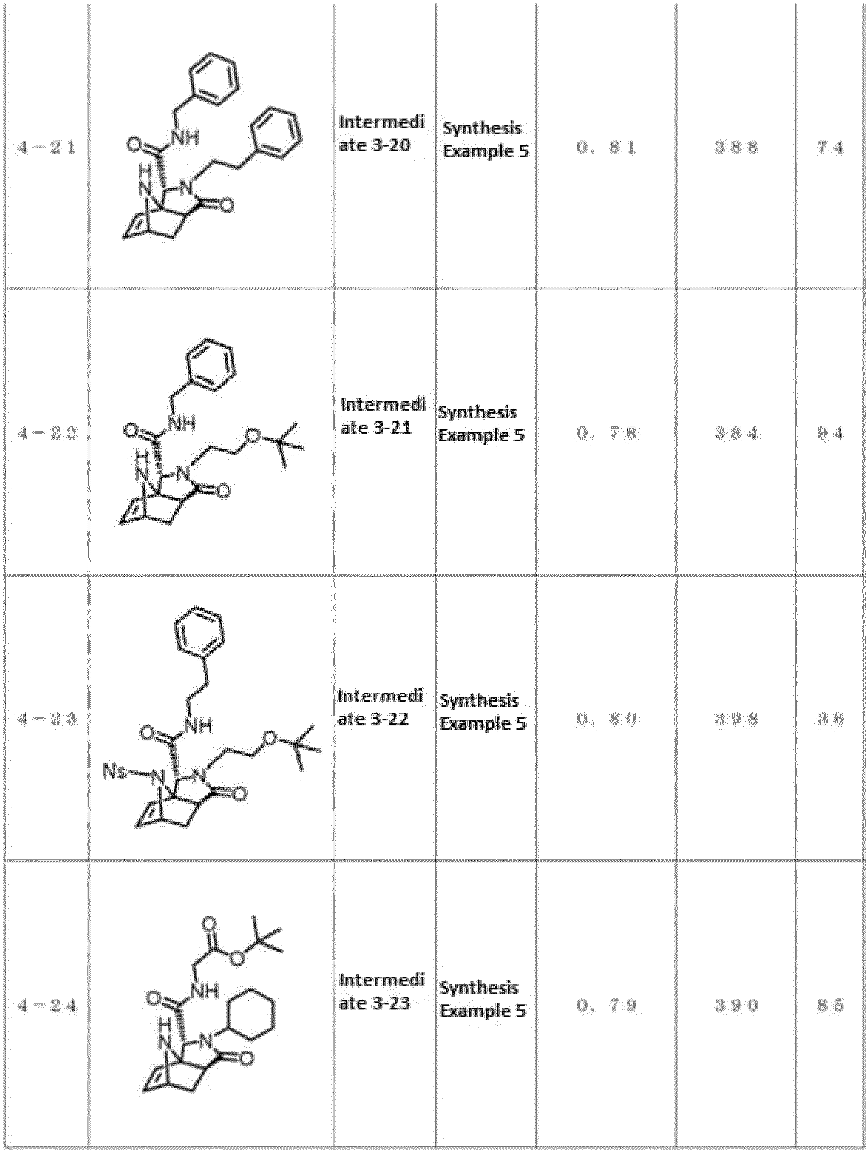

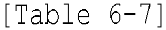

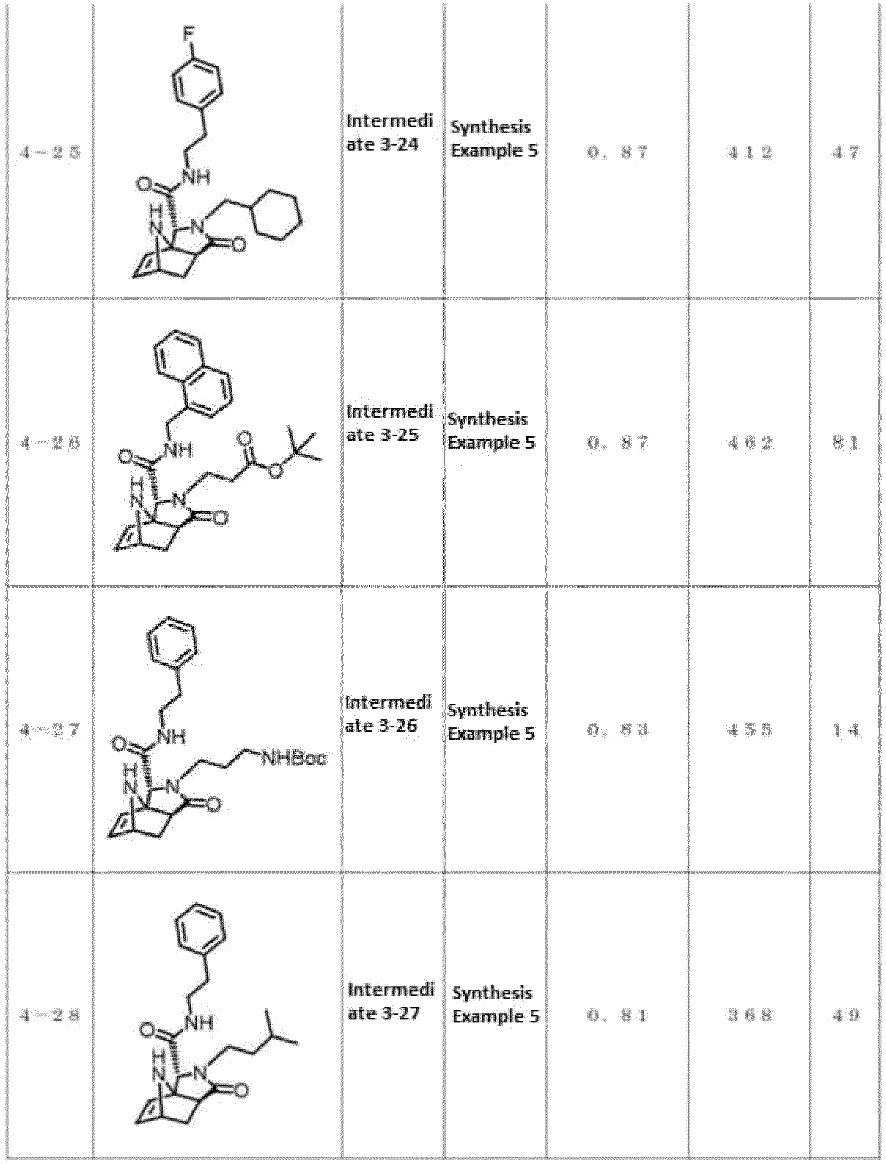

### [Synthesis Example 6: Catalytic hydrogenation reaction]

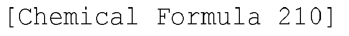

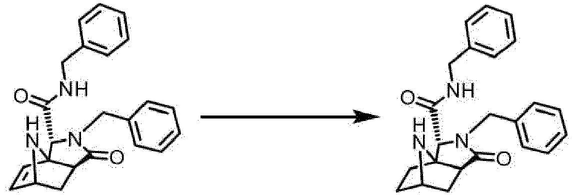

### Synthesis of (3R*, 3aR*, 6S*, 7aS*) -N, 2-dibenzyl-1-oxooctahydro-3a,6-epiiminoisoindole-3-carboxamide (intermediate 5-1)

Intermediate 4-1 (217 mg) was dissolved in methanol (3.0 mL), and palladium on carbon (Pd 10%) (31 mg) was added. The mixture was then stirred for 6 hours at room temperature under a hydrogen atmosphere. After filtering the reaction solution with Celite, the solvent was evaporated under reduced pressure. The resulting residue was purified by silica gel column chromatography (CHROMATOREX Q-PACK NH30 SIZE 10, eluent: hexane:ethyl acetate 4:1-0:1). The resulting solid was washed with hexane, then dried at 40°C under reduced pressure to obtain the title compound (170 mg, yield 78%, RT = 0.76 minutes, [M+H] + = 376) as a white solid. ¹H-NMR (300 MHz, CDCl₃): δ 7.40-7.20 (m, 10H), 6.04-6.02 (m, 1H), 4.93 (d, J = 15.1 Hz, 1H), 4.47 (dd, J = 14.7, 5.3 Hz, 1H), 4.26 (dd, J = 14.7, 5.3 Hz, 1H), 4.06 (d, J = 15.1 Hz, 1H), 3.96 (s, 1H), 3.70 (t, J = 4.5 Hz, 1H), 2.64 (dd, J = 9.4, 4.5 Hz, 1H), 2.01-1.94 (m, 1H), 1.84-1.66 (m, 3H), 1.52-1.48 (m, 2H).

Intermediate 5 synthesized by changing intermediate 4 used as a raw material is summarized in the following tables.

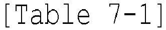

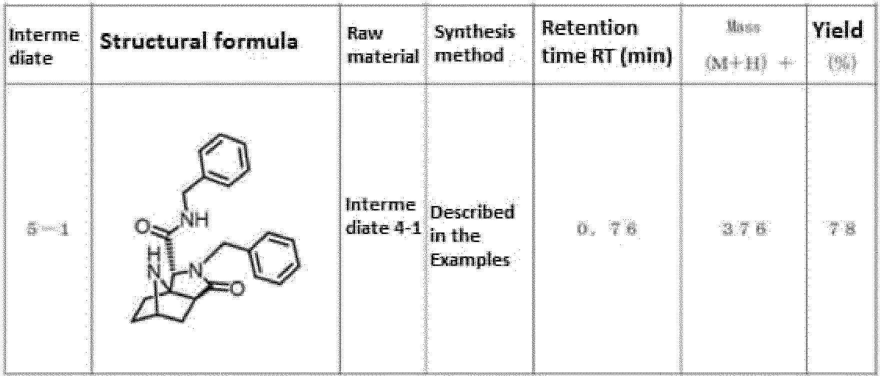

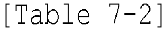

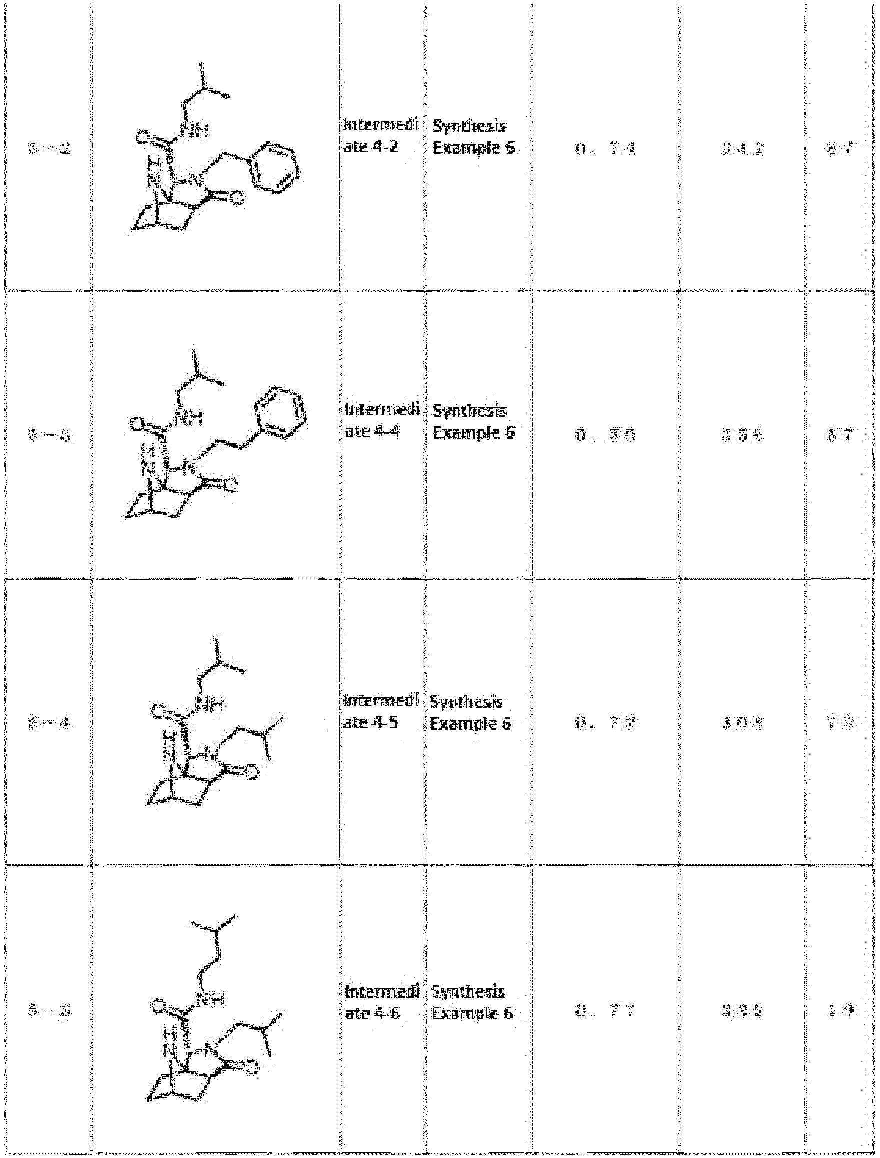

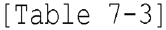

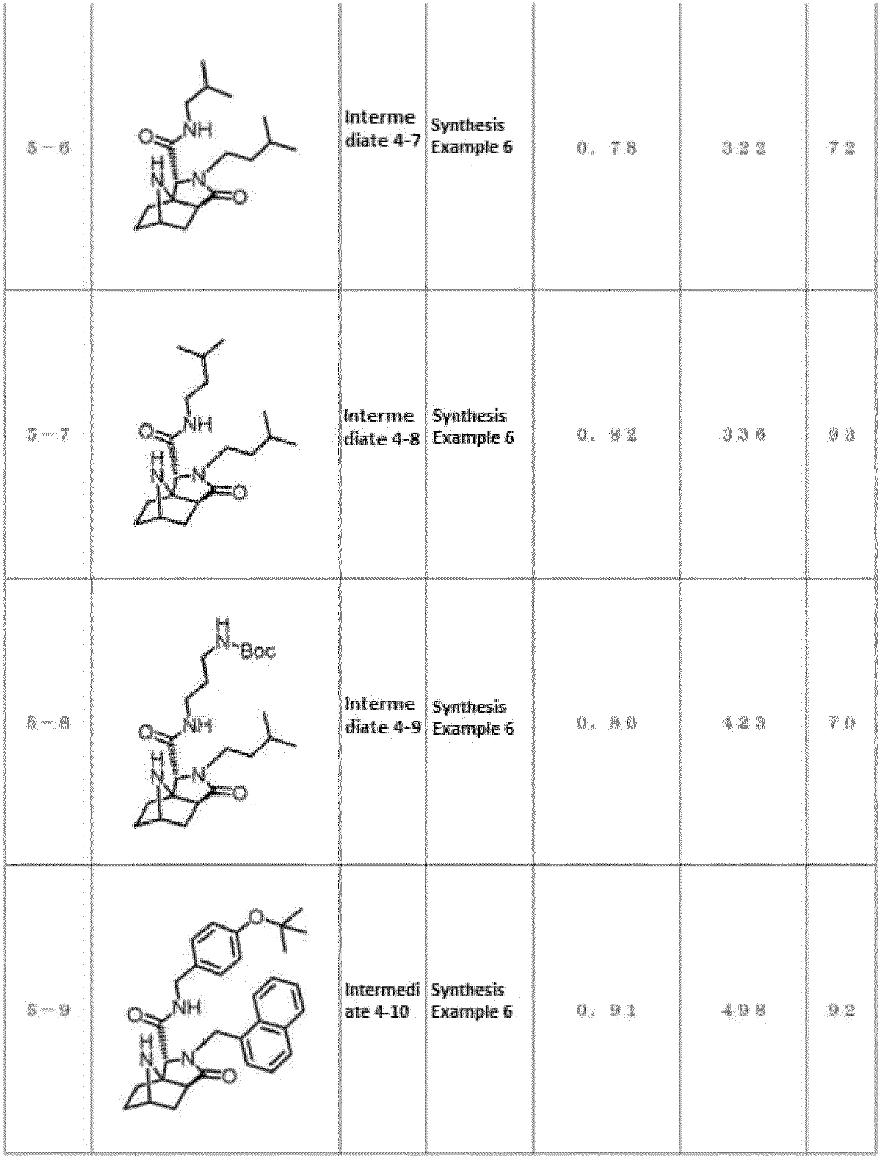

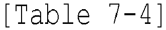

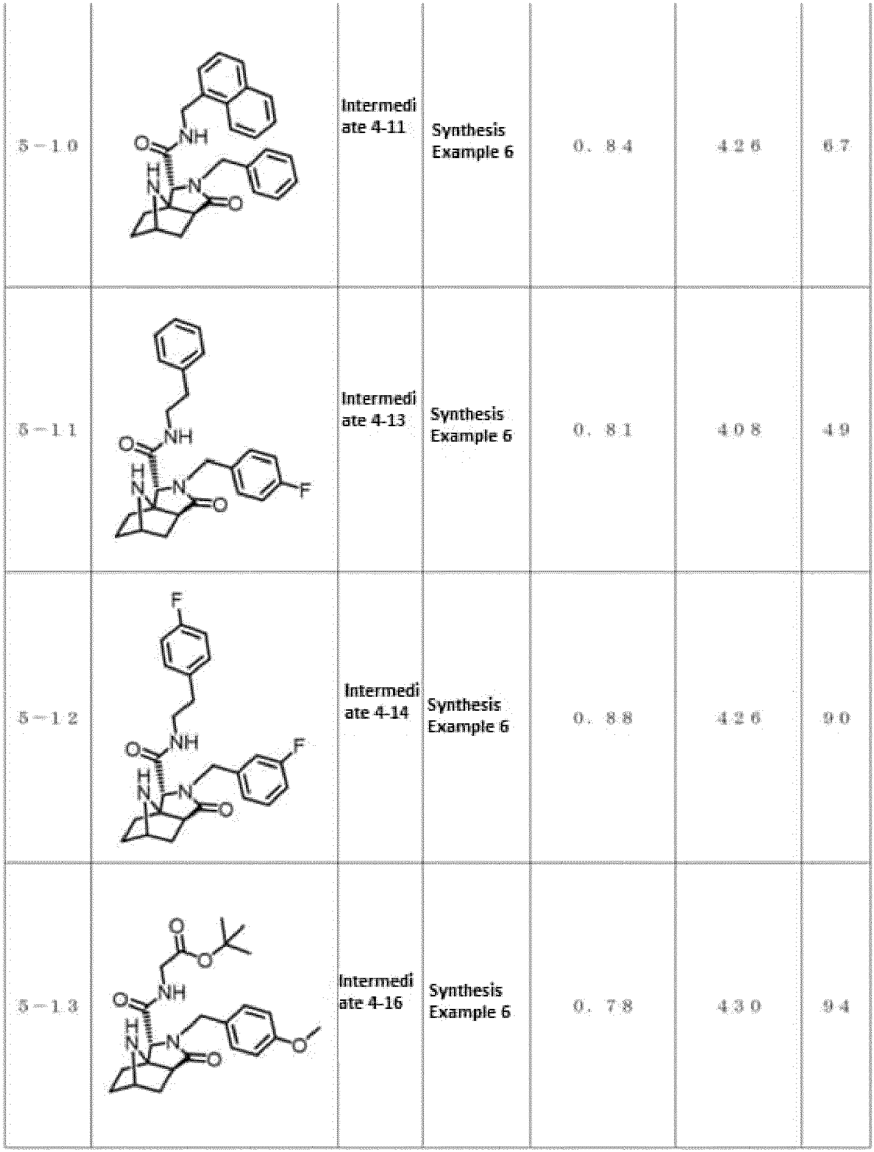

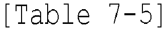

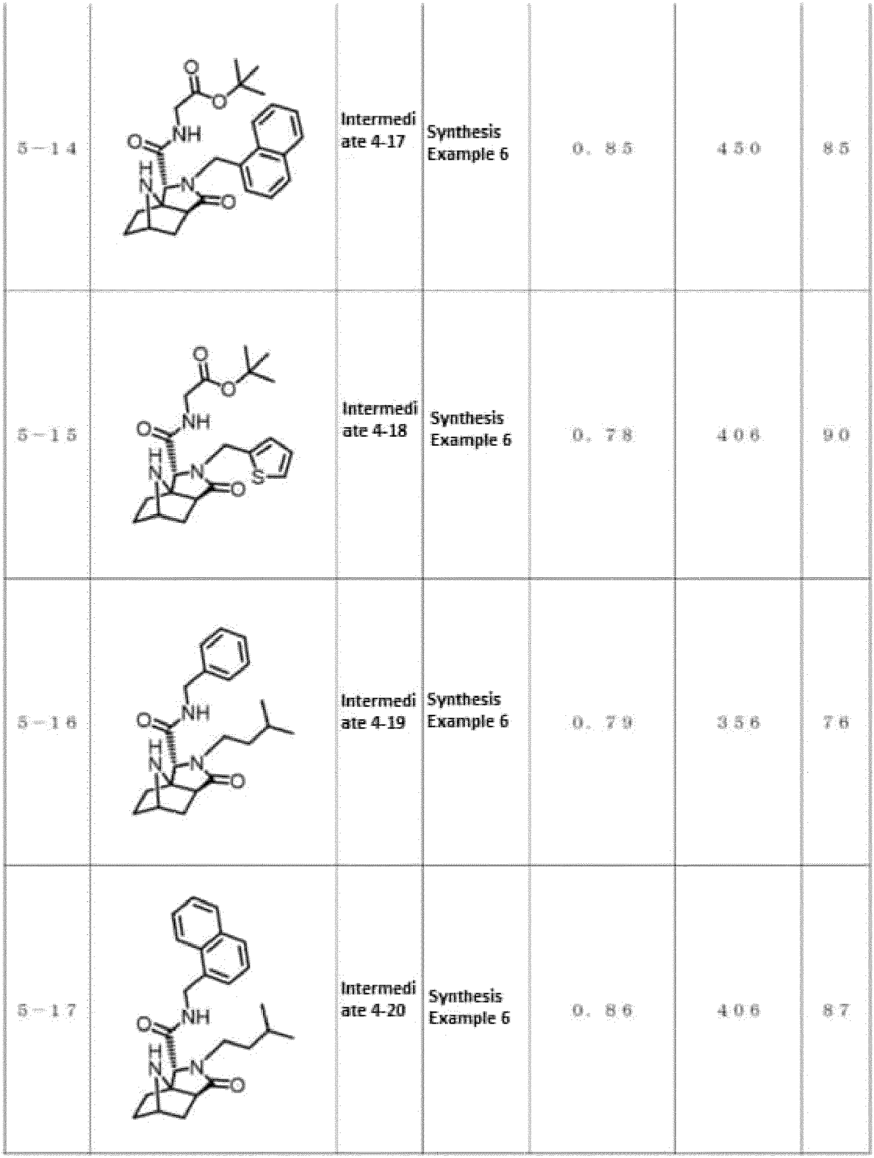

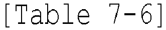

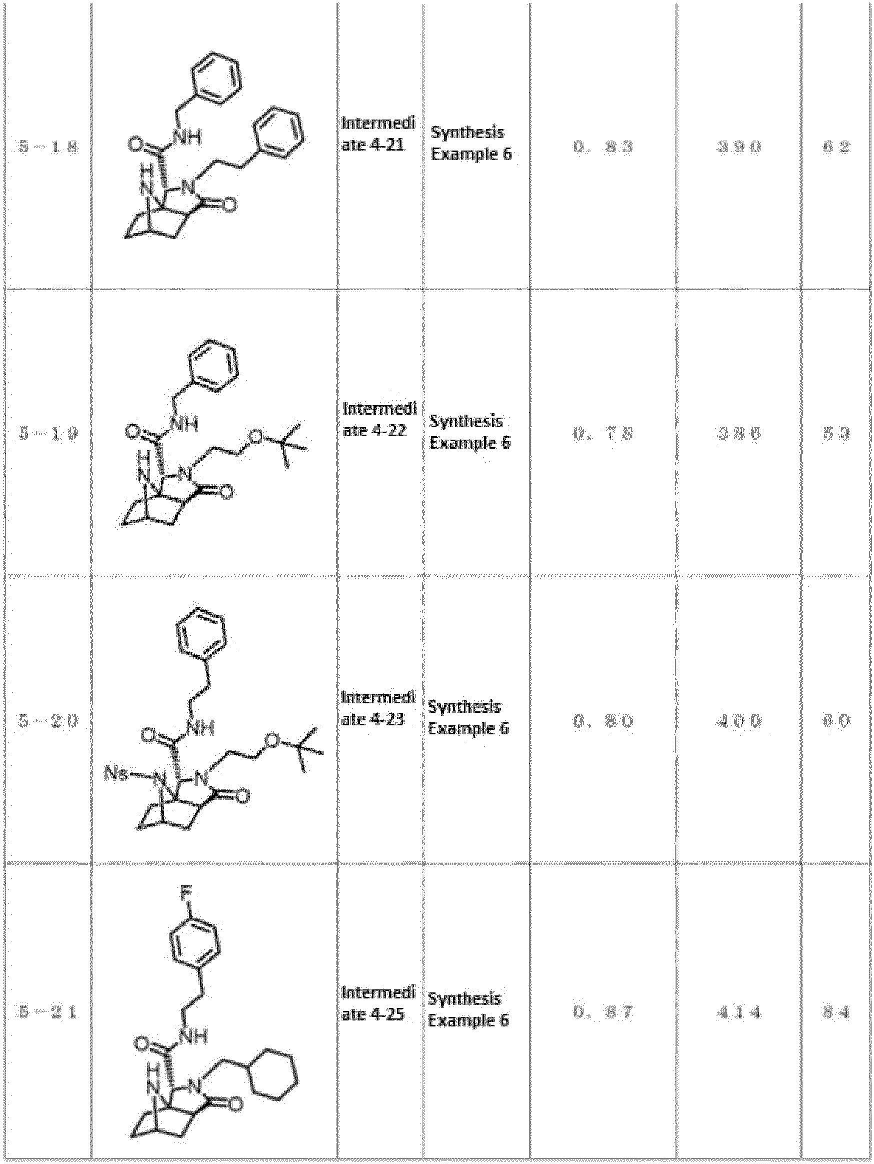

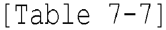

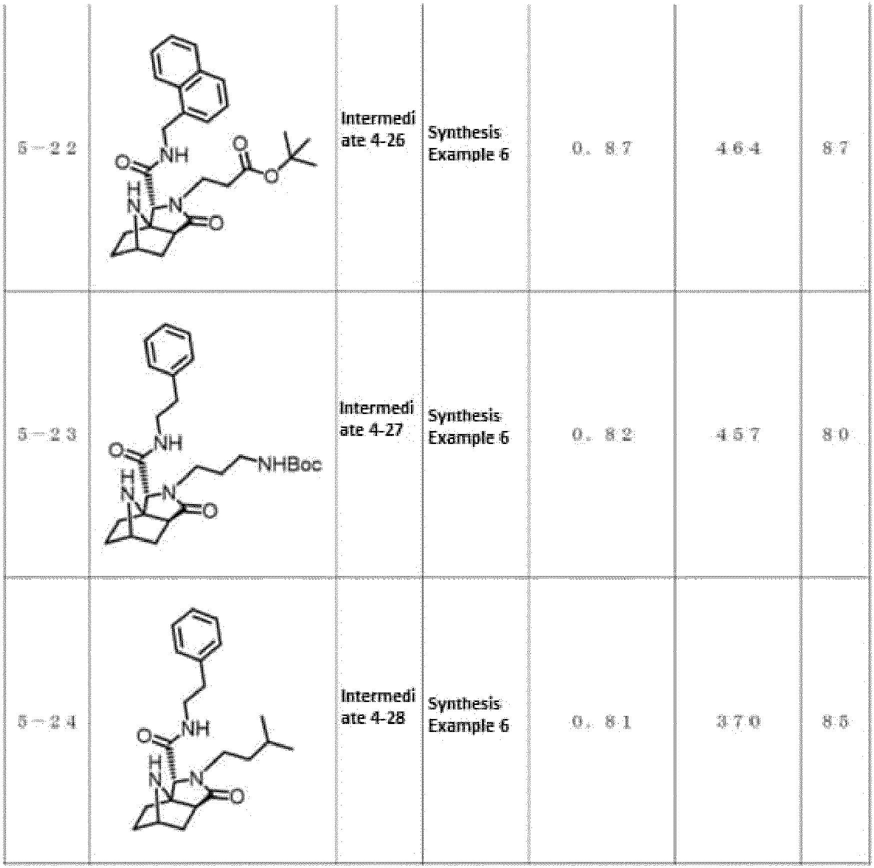

### [Synthesis Example 7: Coupling reaction-1]

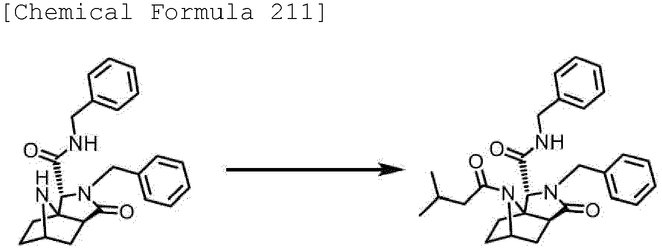

### Synthesis of (3R*, 3aR*, 6S*, 7aS*) -N, 2-dibenzyl-8- (3-methylbutanoyl)-1-oxooctahydro-3a,6-epiiminoisoindole-3-carboxamide (compound number 26)

Intermediate 5-1 (13.5 mg) was dissolved in a mixture of chloroform (0.5 mL) and a saturated aqueous sodium hydrogen carbonate solution (0.5 mL). Isovaleryl chloride (7.0 µL) was added, and the mixture was stirred for 17 hours at room temperature. The organic layer was separated by using a Phase Separator, and then the solvent was evaporated under reduced pressure. The resulting residue was purified by silica gel column chromatography (CHROMATOREX Q-PACK SI30 SIZE 10, eluent: hexane:ethyl acetate 4:1-0:1) to obtain the title compound (14.1 mg, yield 85%, RT = 1.07 minutes, [M+H]+ = 460) as a colorless oily substance.
¹H-NMR (300 MHz, CDCl₃) : δ 7.38-7.16 (m, 10H), 6.23 (t, J = 5.7 Hz, 1H), 5.41 (s, 1H), 4.95 (d, J = 15.1 Hz, 1H), 4.39 (d, J = 6.0 Hz, 2H), 4.26 (t, J = 4.5 Hz, 1H), 4.03 (d, J = 15.1 Hz, 1H), 2.94 (q, J = 5.2 Hz, 1H), 2.22-2.13 (m, 2H), 2.07-1.80 (m, 5H), 1.69-1.58 (m, 2H), 0.92 (d, J = 6.4 Hz, 3H), 0.89 (d, J = 6.4 Hz, 3H).

### [Synthesis Example 8: Coupling reaction-2]

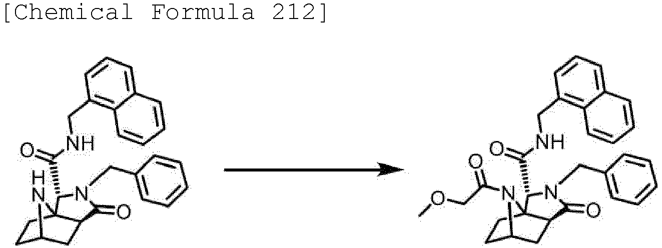

### Synthesis of (3R*,3aR*,6S*,7aS*)-2-benzyl-8-(2-methoxyacetyl)-N-(naphthalen-1-ylmethyl)-1-oxooctahydro-3a,6-epiiminoisoindole-3-carboxamide (compound number 232)

Intermediate 5-10 (10.0 mg) was dissolved in chloroform (1.0 mL), and methoxyacetic acid (2.2 µL), HATU (13 mg), and triethylamine (13 µL) were sequentially added. The mixture was stirred for 1.5 hours at room temperature. The reaction was quenched by adding a saturated aqueous sodium hydrogen carbonate solution to the reaction solution, and the organic layer was separated by using a Phase Separator. The residue obtained by evaporating the solvent under reduced pressure was purified by silica gel column chromatography (CHROMATOREX Q-PACK NH30 SIZE 10, eluent: hexane:ethyl acetate 4:1-0:1) to obtain the title compound (10.4 mg, yield 87%, RT = 1.05 minutes, [M+H]+ = 498) as a colorless oily substance.
¹H-NMR (300 MHz, CDCl₃) : δ 7.98-7.84 (m, 3H), 7.63-7.52 (m, 2H), 7.47-7.35 (m, 2H), 7.21-6.93 (m, 5H), 6.09 (d, J = 5.3 Hz, 1H), 5.40 (s, 1H), 4.89-4.72 (m, 3H), 4.42 (t, J = 4.3 Hz, 1H), 4.14 (d, J = 15.1 Hz, 1H), 3.91 (d, J = 14.4 Hz, 1H), 3.82 (d, J = 14.4 Hz, 1H), 3.31 (s, 3H), 2.96 (dd, J = 9.8, 5.3 Hz, 1H), 2.17-2.14 (m, 1H), 1.90-1.83 (m, 3H), 1.70-1.58 (m, 2H).

### [Synthesis Example 9: Reductive amination reaction-1]

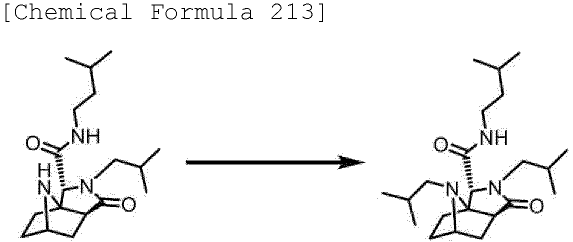

### Synthesis of (3R*,3aR*,6S*,7aS*)-2,8-diisobutyl-N-isopentyl-1-oxooctahydro-3a,6-epiiminoisoindole-3-carboxamide (compound number 256)

Intermediate 5-5 (10.0 mg) was dissolved in chloroform (1.0 mL), and isobutylaldehyde (4.2 µL) and sodium triacetoxyborohydride (20 mg) were sequentially added. The mixture was stirred for 15 hours at room temperature. The reaction was quenched by adding a saturated aqueous sodium hydrogen carbonate solution to the reaction solution, and the organic layer was separated by using a Phase Separator. The residue obtained by evaporating the solvent under reduced pressure was purified by silica gel column chromatography (CHROMATOREX Q-PACK SI30 SIZE 10, eluent: hexane:ethyl acetate 4:1-0:1) to obtain the title compound (9.3 mg, yield 79%, RT = 0.84 minutes, [M+H]+ = 378) as a colorless oily substance.
¹H-NMR (300 MHz, CDCl₃): δ 5.95 (t, J = 5.3 Hz, 1H), 4.00 (s, 1H), 3.50 (dd, J = 13. 6, 9. 6 Hz, 1H), 3.42 (t, J = 5.3 Hz, 1H), 3.38-3.28 (m, 2H), 2.65 (dd, J = 13.6, 5.3 Hz, 1H), 2.45 (dd, J = 9.6, 4.5 Hz, 1H), 2.21-2.05 (m, 2H), 1.99-1.84 (m, 4H), 1.67-1.56 (m, 3H), 1.49-1.28 (m, 4H), 0.95-0.88 (m, 18H)

### [Synthesis Example 10: Reductive amination reaction-2]

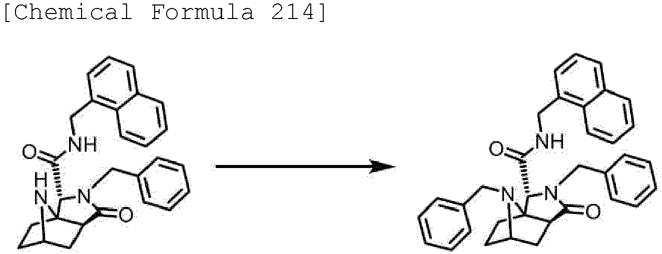

### Synthesis of (3R*,3aR*,6S*,7aS*)-2,8-dibenzyl-N- (naphthalen-1-ylmethyl)-1-oxooctahydro-3a,6-epiiminoisoindole-3-carboxamide (compound number 307)

Intermediate 5-10 (5.0 mg) was dissolved in chloroform (1.0 mL), and benzaldehyde (3.6 µL), acetic acid (1.0 µL), and sodium triacetoxyborohydride (7.5 mg) were sequentially added. The mixture was stirred for 17 hours at room temperature. The reaction was quenched by adding a saturated aqueous sodium hydrogen carbonate solution to the reaction solution, and the organic layer was separated by using a Phase Separator. The residue obtained by evaporating the solvent under reduced pressure was purified by silica gel column chromatography (CHROMATOREX Q-PACK SI30 SIZE 10, eluent: hexane:ethyl acetate 4:1-0:1) to obtain the title compound (5.2 mg, yield 84%, RT = 0.94 minutes, [M+H]+ = 516) as a white solid.
¹H-NMR (300 MHz, CDCl₃) : δ 7.99-7.87 (m, 2H), 7.63-7.55 (m, 2H), 7.50-7.40 (m, 2H), 7.33-7.27 (m, 5H), 7.25-7.22 (m, 2H), 7.14-7.09 (m, 4H), 6.11 (t, J = 5.5 Hz, 1H), 4.95 (dd, J = 14.4, 5.7 Hz, 1H), 4.86 (d, J = 15.1 Hz, 1H), 4.79 (dd, J = 14.4, 5.7 Hz, 1H), 3.93 (s, 1H), 3.82 (d, J = 15.1 Hz, 1H), 3.50 (d, J = 14.0 Hz, 1H), 3.30 (t, J = 4.5 Hz, 1H), 3.22 (d, J = 14.0 Hz, 1H), 2.58-2.48 (m, 1H), 2.07-1.97 (m, 3H), 1.64-1.47 (m, 3H).

### [Synthesis Example 11: Urea synthesis]

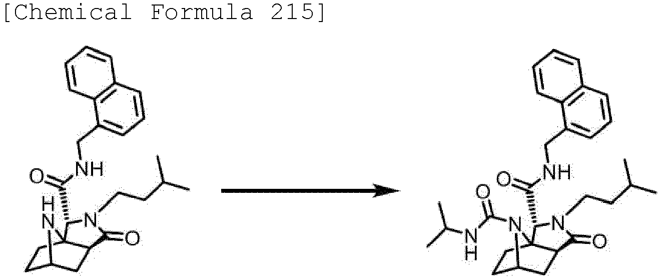

### Synthesis of (3R*, 3aR*, 6S*, 7aS*) -2-isopentyl-N8-isopropyl-N3-(naphthalen-1-ylmethyl)-1-oxooctahydro-3a,6-epiiminoisoindole-3,8-dicarboxamide (compound number 388)

Intermediate 5-17 (10.0 mg) was dissolved in chloroform (1.0 mL), and isopropylisocyanate (3.7 uL) was added. The mixture was stirred for 1 hour at room temperature. After concentrating the reaction solution under reduced pressure, the resulting residue was purified by silica gel column chromatography (CHROMATOREX Q-PACK SI30 SIZE 10, eluent: hexane:ethyl acetate 4:1-0:1) to obtain the title compound (11.2 mg, yield 91%, RT = 1.13 minutes, [M+H]+ = 491) as a white solid.
¹H-NMR (300 MHz, CDCl₃) : δ 8.08-8.04 (m, 1H), 7.92-7.89 (m, 1H), 7.87-7.84 (m, 1H), 7.56-7.43 (m, 4H), 6.48 (t, J = 5.5 Hz, 1H), 5.26 (s, 1H), 5.11 (dd, J = 14.5, 5.5 Hz, 1H), 4.86 (dd, J = 14.5, 5.5 Hz, 1H), 4.72 (d, J = 7.9 Hz, 1H), 3.90 (t, J = 5.1 Hz, 1H), 3.84-3.77 (m, 1H), 3.65-3.55 (m, 1H), 3.04-2.97 (m, 1H), 2.78 (dd, J = 10.2, 5.1 Hz, 1H), 2.09-1.98 (m, 2H), 1.86 (s, 1H), 1.70 (dd, J = 12.1, 10.2 Hz, 1H), 1.61-1.47 (m, 2H), 1.36-1.28 (m, 3H), 1.13 (d, J = 1.9 Hz, 3H), 1.11 (d, J = 1.9 Hz, 3H), 0.85 (d, J = 1.1 Hz, 3H), 0.83 (d, J = 1.1 Hz, 3H).

### [Synthesis Example 12: Deprotection reaction of Boc group]

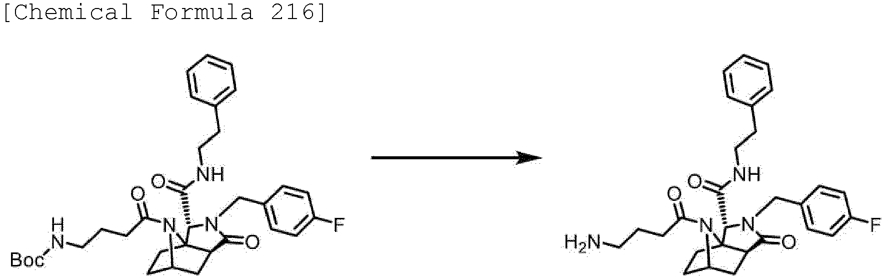

### Synthesis of (3R*,3aR*,6S*,7aS*)-8-(4-aminobutanoyl)-2-(4-fluorobenzyl)-1-oxo-N-phenethyloctahydro-3a,6-epiiminoisoindole-3-carboxamide (compound number 241)

Compound number 238 (38.3 mg) was dissolved in chloroform (0.3 mL), and trifluoroacetic acid (0.3 mL) was added. The mixture was stirred for 3 hours at room temperature. After concentrating the reaction solution under reduced pressure, chloroform and saturated sodium hydrogen carbonate were added to the resulting residue, and the organic layer was separated with a Phase Separator. After evaporating the solvent under reduced pressure, the resulting residue was purified by silica gel column chromatography (CHROMATOREX Q-PACK NH30 SIZE 10, eluent: ethyl acetate:methanol 1:0-4:1) to obtain the title compound (24.1 mg, yield 75%, RT = 0.80 minutes, [M+H]+ = 493) as a colorless oily substance.
¹H-NMR (300 MHz, CDCl₃) : δ 7.35-7.23 (m, 4H), 7.20-7.09 (m, 3H), 6.99-6.93 (m, 2H), 5.97 (s, 1H), 5.33 (s, 1H), 4.79 (d, J = 15.1 Hz, 1H), 4.29-4.28 (m, 1H), 3.96 (d, J = 15.1 Hz, 1H), 3.57-3.46 (m, 2H), 2.90-2.76 (m, 3H), 2.67 (td, J = 6.8, 1.5 Hz, 2H), 2.39-2.07 (m, 3H), 1.86-1.74 (m, 3H), 1.70-1.59 (m, 3H).

### [Synthesis Example 13: reductive amination reaction (dimethylation)]

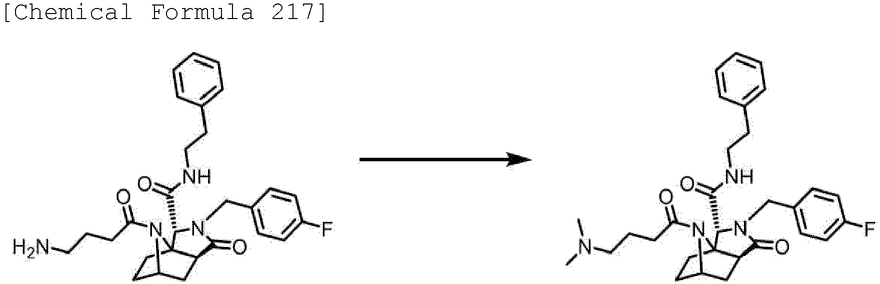

### Synthesis of (3R*,3aR*,6S*,7aS*)-8-[4-(dimethylamino)butanoyl]-2-(4-fluorobenzyl)-1-oxo-N-phenethyloctahydro-3a,6-epiiminoisoindole-3-carboxamide (compound number 243)

Compound number 242 (15.7 mg) was dissolved in chloroform (1.0 mL), and an aqueous 37% formaldehyde solution (15 µL) and sodium triacetoxyborohydride (41 mg) were sequentially added. The mixture was stirred for 30 minutes at room temperature. The reaction was quenched by adding a saturated aqueous sodium hydrogen carbonate solution to the reaction solution, and the organic layer was separated by using a Phase Separator. The residue obtained by evaporating the solvent under reduced pressure was purified by silica gel column chromatography (CHROMATOREX Q-PACK NH30 SIZE 10, eluent: ethyl acetate:methanol 1:0-4:1) to obtain the title compound (14.5 mg, yield 87%, RT = 0.81 minutes, [M+H]+ = 521) as a colorless oily substance.
¹H-NMR (300 MHz, CDCl₃) : δ 7.35-7.22 (m, 3H), 7.20-7.17 (m, 2H), 7.14-7.09 (m, 2H), 6.99-6.93 (m, 2H), 5.99-5.96 (m, 1H), 5.34 (s, 1H), 4.79 (d, J = 15.1 Hz, 1H), 4.29 (t, J = 4.5 Hz, 1H), 3.96 (d, J = 15.1 Hz, 1H), 3.59-3.43 (m, 2H), 2.90-2.75 (m, 3H), 2.39-2.09 (m, 5H), 2.22 (s, 6H), 1.86-1.78 (m, 3H), 1.73-1.48 (m, 3H).

### [Synthesis Example 14: Guanidination reaction and deprotection reaction of Boc group]

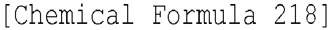

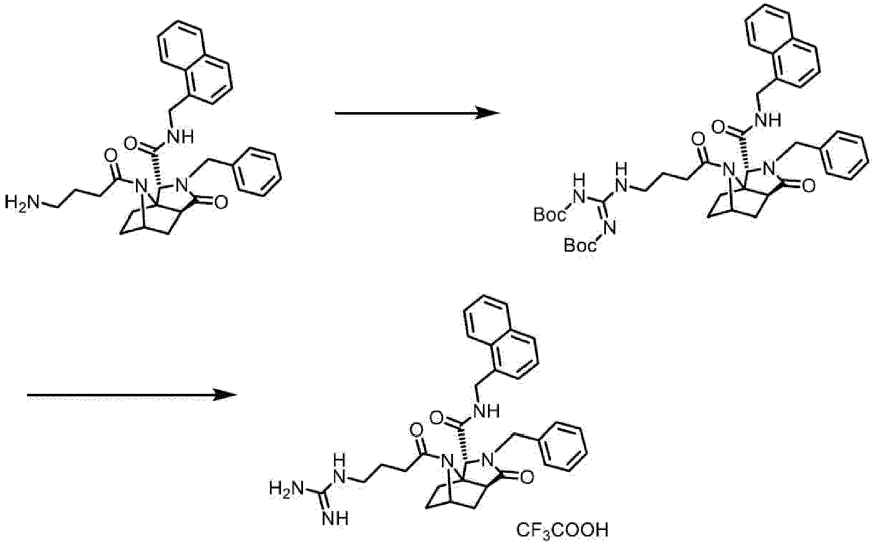

### Synthesis of (3R*,3aR*,6S*,7aS*)-2-benzyl-8-(4-guanidinobutanoyl)-N-(naphthalen-1-ylmethyl)-1-oxooctahydro-3a,6-epiiminoisoindole-3-carboxamide trifluoroacetate (compound number 244)

Compound number 237 (9.9 mg) was dissolved in tetrahydrofuran (1.0 mL), and a Goodman reagent (11.4 mg) and triethylamine (8.1 µL) were sequentially added. The mixture was stirred for 3 hours at room temperature. The reaction solution was concentrated under reduced pressure, and a saturated aqueous sodium hydrogen carbonate solution and chloroform were added. The organic layer was separated by using a Phase Separator. The residue obtained by evaporating the solvent under reduced pressure was purified by silica gel column chromatography (CHROMATOREX Q-PACK SI30 SIZE 10, eluent: hexane:ethyl acetate 4:1-0:1).

The compound obtained above was dissolved in chloroform (0.5 mL), and trifluoroacetic acid (0.5 mL) was added. The mixture was stirred for 5 hours at room temperature. The reaction solution was concentrated under reduced pressure to obtain the title compound (10.3 mg, yield 79% (2 steps), RT = 0.86 minutes, [M+H]+ = 553) as a colorless oily substance.

### [Synthesis Example 15: tert-butyl ester deprotection]

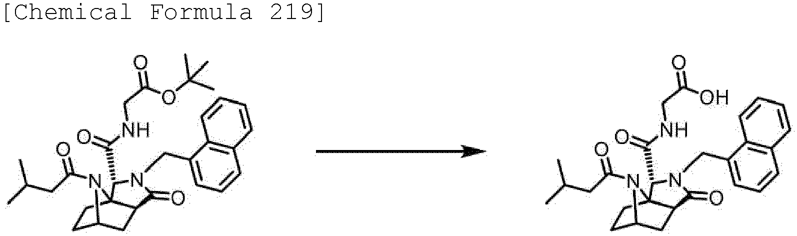

### Synthesis of [(3R*, 3aR*, 6S*, 7aS*) -8- (3-methylbutanoyl) -2-(naphthalen-1-ylmethyl)-1-oxooctahydro-3a,6-epiiminoisoindole-3-carbonyl]glycine (compound number 47)

Compound number 44 (39.7 mg) was dissolved in chloroform (0.5 mL), and trifluoroacetic acid (0.5 mL) was added. The mixture was stirred for 15 hours at room temperature. After concentrating the reaction solution under reduced pressure, the resulting residue was purified by silica gel column chromatography (CHROMATOREX Q-PACK SI30 SIZE 10, eluent: hexane:ethyl acetate 1:1-0:1) to obtain the title compound (23.2 mg, yield 65%, RT = 0.97 minutes, [M+H]+ = 478) as a white solid.

### [Synthesis Example 16: Carboxamide synthesis]

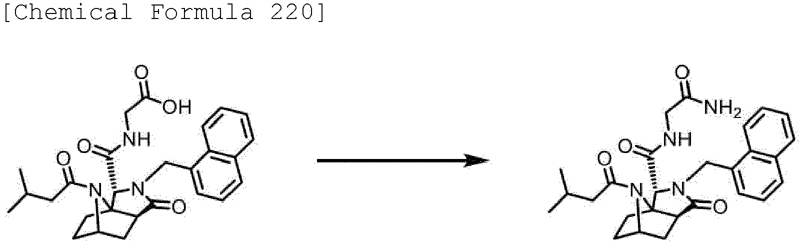

### Synthesis of (3R*, 3aR*, 6S*, 7aS*) -N- (2-amino-2-oxoethyl) -8-(3-methylbutanoyl)-2-(naphthalen-1-ylmethyl)-1-oxooctahydro-3a,6-epiiminoisoindole-3-carboxamide (compound number 50)

Compound number 47 (19.0 mg) was dissolved in methanol (1.0 mL), and 7 mol/L ammonium-methanol solution (17.1 µL) and DMT-MM (33.0 mg) were sequentially added. The mixture was stirred for 2.5 days at room temperature. A saturated aqueous sodium hydrogen carbonate solution and chloroform were added to the reaction solution, and the organic layer was separated by using a Phase Separator. The residue obtained by evaporating the solvent under reduced pressure was purified by silica gel column chromatography (CHROMATOREX Q-PACK SI30 SIZE 10, eluent: ethyl acetate:methanol 1:0-4:1) to obtain the title compound (6.1 mg, yield 32%, RT = 0.93 minutes, [M+H]+ = 477) as a colorless solid.
¹H-NMR (300 MHz, DMSO-D₆): δ 8.38-8.35 (m, 1H), 8.05-8.02 (m, 1H), 7.93-7.90 (m, 1H), 7.85-7.83 (m, 1H), 7.53-7.49 (m, 2H), 7.39-7.31 (m, 3H), 7.01 (s, 1H), 5.56 (s, 1H), 5.20 (d, J = 15.9 Hz, 1H), 4.36-4.30 (m, 2H), 3.64 (t, J = 6.2 Hz, 2H), 2.86-2.82 (m, 1H), 2.11-2.03 (m, 1H), 1.96-1.68 (m, 6H), 1.56-1.53 (m, 2H), 0.74-0.67 (m, 6H).

### [Synthesis Example 17: tert-butyl ether deprotection reaction-1]

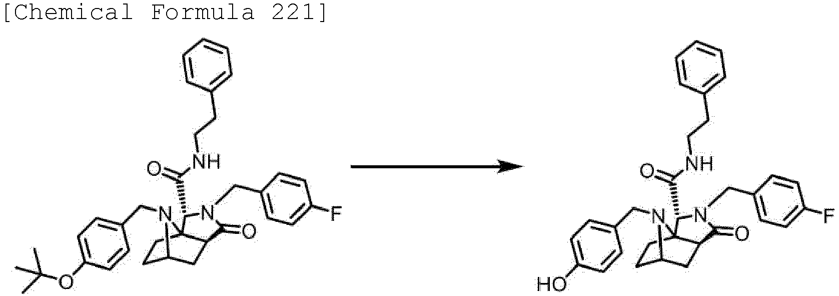

### Synthesis of (3R*, 3aR*, 6S*, 7aS*) -2- (4-fluorobenzyl) -8- (4-hydroxybenzyl)-1-oxo-N-phenethyloctahydro-3a,6-epiiminoisoindole-3-carboxamide (compound number 327)

Compound number 324 (13.1 mg) was dissolved in chloroform (0.25 mL), and trifluoroacetic acid (0.25 mL) was added. The mixture was stirred for 6 hours at room temperature. The reaction solution was concentrated under reduced pressure, and a saturated aqueous sodium hydrogen carbonate solution and chloroform were added. The organic layer was separated by using a Phase Separator. The residue obtained by evaporating the solvent under reduced pressure was purified by silica gel column chromatography (CHROMATOREX Q-PACK SI30 SIZE 10, eluent:hexane:ethyl acetate 1:1-0:1) to obtain the title compound (10.5 mg, yield 89%, RT = 0.86 minutes, [M+H]+ = 514) as a colorless oily substance.
¹H-NMR (300 MHz, CDCl₃) : δ 7.40-7.26 (m, 3H), 7.23-7.21 (m, 2H), 7.18-7.13 (m, 2H), 7.02-6.99 (m, 2H), 6.86-6.80 (m, 2H), 6.77-6.74 (m, 2H), 5.92 (t, J = 5.5 Hz, 1H), 5.19 (s, 1H), 4.88 (d, J = 15.5 Hz, 1H), 3.79 (s, 1H), 3.69-3.50 (m, 3H), 3.35 (d, J = 13.6 Hz, 1H), 3.28 (t, J = 4.5 Hz, 1H), 3.11 (d, J = 13.6 Hz, 1H), 2.88-2.84 (m, 2H), 2.47-2.42 (m, 1H), 1.99-1.91 (m, 3H), 1.45-1.39 (m, 1H), 1.31-1.21 (m, 1H).

### [Synthesis Example 18:tert-butyl ether deprotection reaction-1]

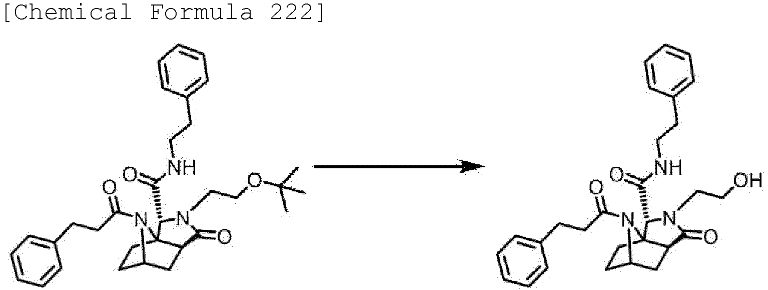

### Synthesis of (3R*, 3aR*, 6S*, 7aS*) -2- (2-hydroxyethyl) -1-oxo-N-phenethyl-8-(3-phenylpropanoyl)octahydro-3a,6-epiiminoisoindole-3-carboxamide (compound number 198)

Compound number 197 (11.4 mg) was dissolved in chloroform (0.25 mL), and trifluoroacetic acid (0.25 mL) was added. The mixture was stirred for 19 hours at room temperature. After concentrating the reaction solution under reduced pressure, a mixture of methanol-water (1:1) and potassium carbonate were sequentially added. The mixture was further stirred for 30 minutes at room temperature. Chloroform was added to the reaction solution, and the organic layer was separated by using a Phase Separator. The residue obtained by evaporating the solvent under reduced pressure was purified by silica gel column chromatography (CHROMATOREX Q-PACK SI30 SIZE 10, eluent: ethyl acetate:methanol 1:0-4:1) to obtain the title compound (2.8 mg, yield 28%, RT = 0.96 minutes, [M+H]+ = 476) as a colorless oily substance.
¹H-NMR (300 MHz, CDCl₃): δ 7.37-7.17 (m, 10H), 6.31-6.29 (m, 1H), 5.70 (s, 1H), 4.17 (t, J = 4.5 Hz, 1H), 3.71-3.54 (m, 5H), 3.48-3.40 (m, 1H), 3.32-3.27 (m, 1H), 2.94-2.86 (m, 4H), 2.78 (dd, J = 9.6, 5.1 Hz, 1H), 2.65-2.50 (m, 2H), 1.94-1.90 (m, 1H), 1.77-1.70 (m, 1H), 1.64-1.57 (m, 1H), 1.51-1.44 (m, 3H) .

### [Synthesis Example 19: Deprotection reaction of TBS group]

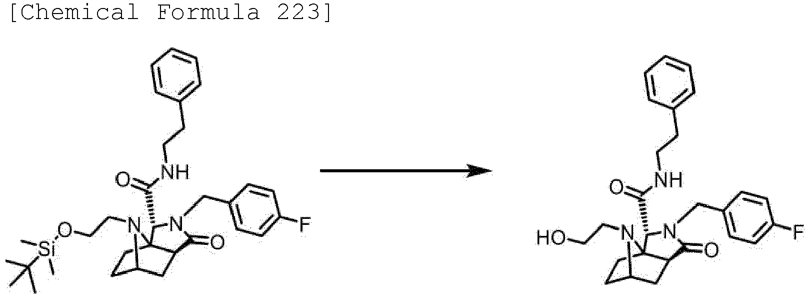

### Synthesis of (3R*, 3aR*, 6S*, 7aS*) -2- (4-fluorobenzyl) -8- (2-hydroxyethyl)-1-oxo-N-phenethyloctahydro-3a,6-epiiminoisoindole-3-carboxamide (compound number 340)

Compound number 339 (17.6 mg) was dissolved in chloroform (0.25 mL), and trifluoroacetic acid (0.25 mL) was added. The mixture was stirred for 20 hours at room temperature. The reaction solution was concentrated under reduced pressure, and then trifluoroacetic acid (0.3 mL) was added again. The mixture was further stirred for 4 hours at room temperature. The reaction solution was concentrated under reduced pressure, and then a saturated aqueous sodium hydrogen carbonate solution and chloroform were added. The organic layer was separated by using a Phase Separator. The residue obtained by evaporating the solvent under reduced pressure was purified by silica gel column chromatography (CHROMATOREX Q-PACK SI30 SIZE 10, eluent: ethyl acetate:methanol 1:0-4:1) to obtain the title compound (8.7 mg, yield 62%, RT = 0.81 minutes, [M+H]+ = 452) as a white solid.
¹H-NMR (300 MHz, CDCl₃) : δ 7.39-7.25 (m, 3H), 7.21-7.14 (m, 4H), 7.06-6.99 (m, 2H), 5.87 (t, J = 5.5 Hz, 1H), 4.79 (d, J = 14.7 Hz, 1H), 3.76 (d, J = 14.7 Hz, 1H), 3.73 (s, 1H), 3.68-3.57 (m, 1H), 3.51-3.38 (m, 3H), 3.34-3.27 (m, 1H), 2.82 (t, J = 7.0 Hz, 2H), 2.44-2.33 (m, 2H), 2.27-2.20 (m, 1H), 1.99-1.87 (m, 2H), 1.75-1.61 (m, 3H), 1.47-1.39 (m, 1H), 1.31-1.16 (m, 2H).

### [Synthesis Example 20: Catalytic hydrogenation]

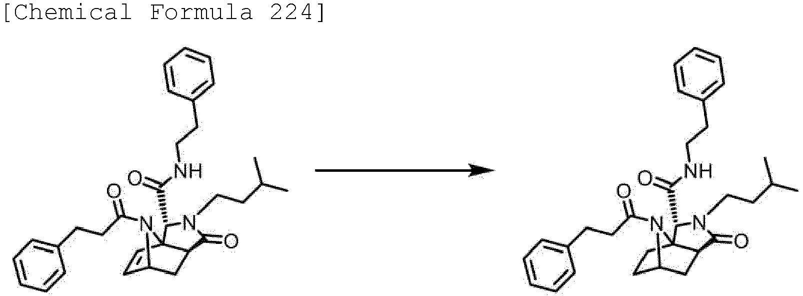

### Synthesis of (3R*, 3aR*, 6S*, 7aS*) -2-isopentyl-1-oxo-N-phenethyl-8-(3-phenylpropanoyl)octahydro-3a,6-epiiminoisoindole-3-carboxamide (compound number 192)

Compound number 193 (38.4 mg) was dissolved in methanol (1.5 mL), and palladium on carbon (Pd 10%) (4.1 mg) was added. The mixture was then stirred for 2 hours at room temperature under a hydrogen atmosphere. After filtering the reaction solution with Celite, the solvent was evaporated under reduced pressure. The resulting residue was purified by silica gel column chromatography (CHROMATOREX Q-PACK SI30 SIZE 10, eluent: hexane:ethyl acetate 4:1-0:1) to obtain the title compound (30.3 mg, yield 78%, RT = 1.15 minutes, [M+H]+ = 502) as a colorless oily substance.
¹H-NMR (300 MHz, CDCl₃) δ 7.36-7.18 (m, 10H), 6.13 (t, J = 6.0 Hz, 1H), 5.63 (s, 1H), 4.15 (t, J = 4.2 Hz, 1H), 3.69-3.51 (m, 3H), 2.95-2.87 (m, 5H), 2.75 (dd, J = 9.6, 5.5 Hz, 1H), 2.62-2.48 (m, 2H), 2.01 (s, 1H), 1.78-1.68 (m, 2H), 1.56-1.46 (m, 4H), 1.37-1.29 (m, 2H), 0.91 (d, J = 6.8 Hz, 6H) .

The compounds in the following tables were synthesized in accordance with the methods of Synthesis Example 7 to Synthesis Example 20 by using each corresponding raw material. The backbone is selected from either formula I or formula II, and R₁, R₂, and R₃ are each selected from a corresponding table.

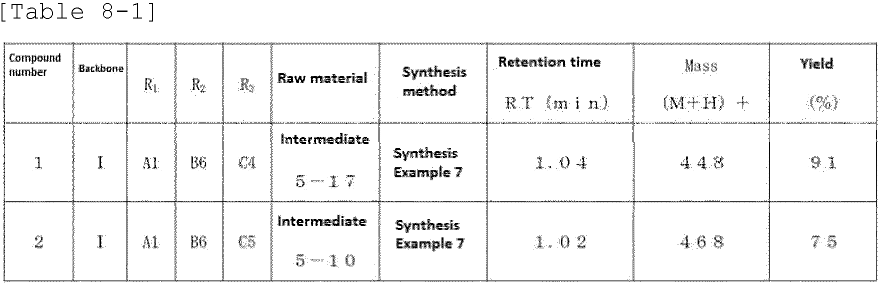

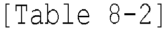

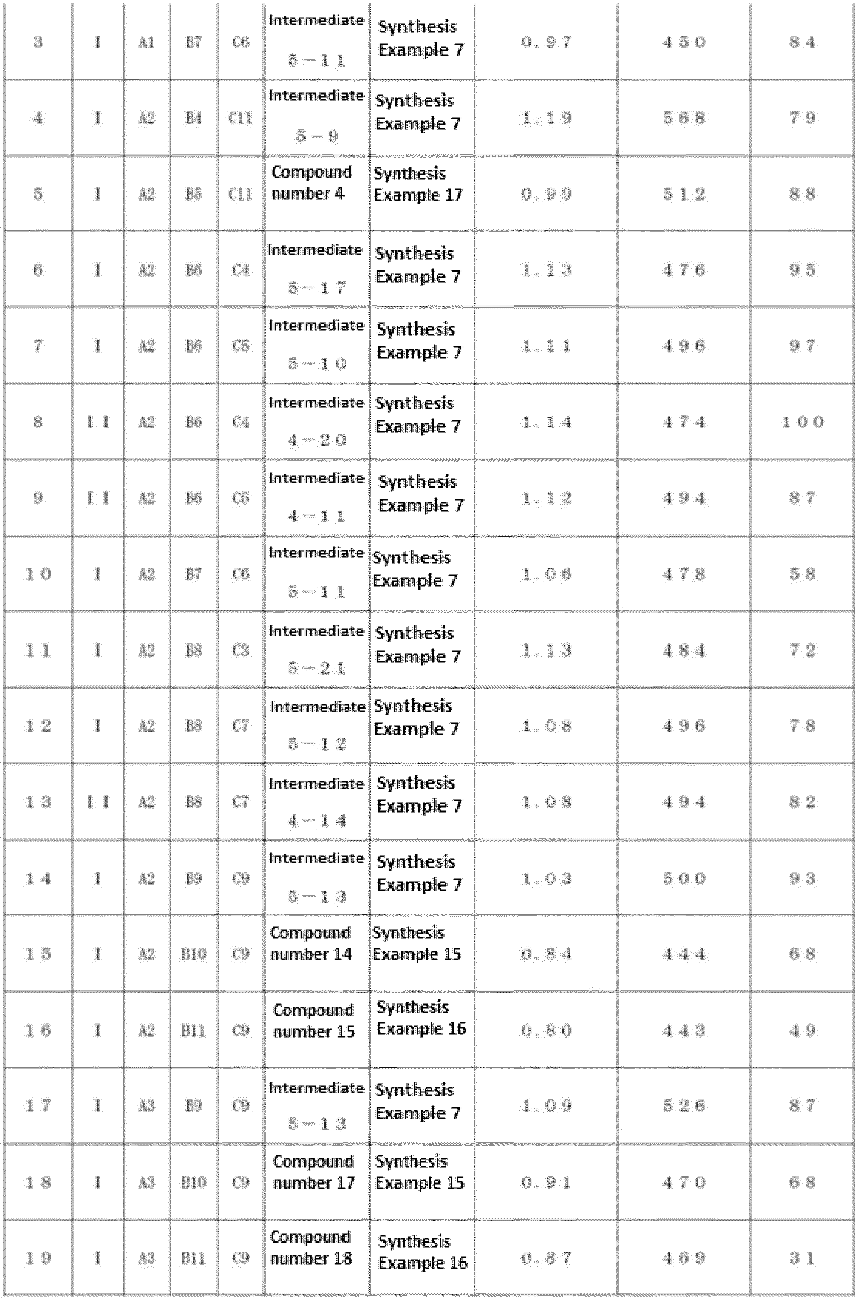

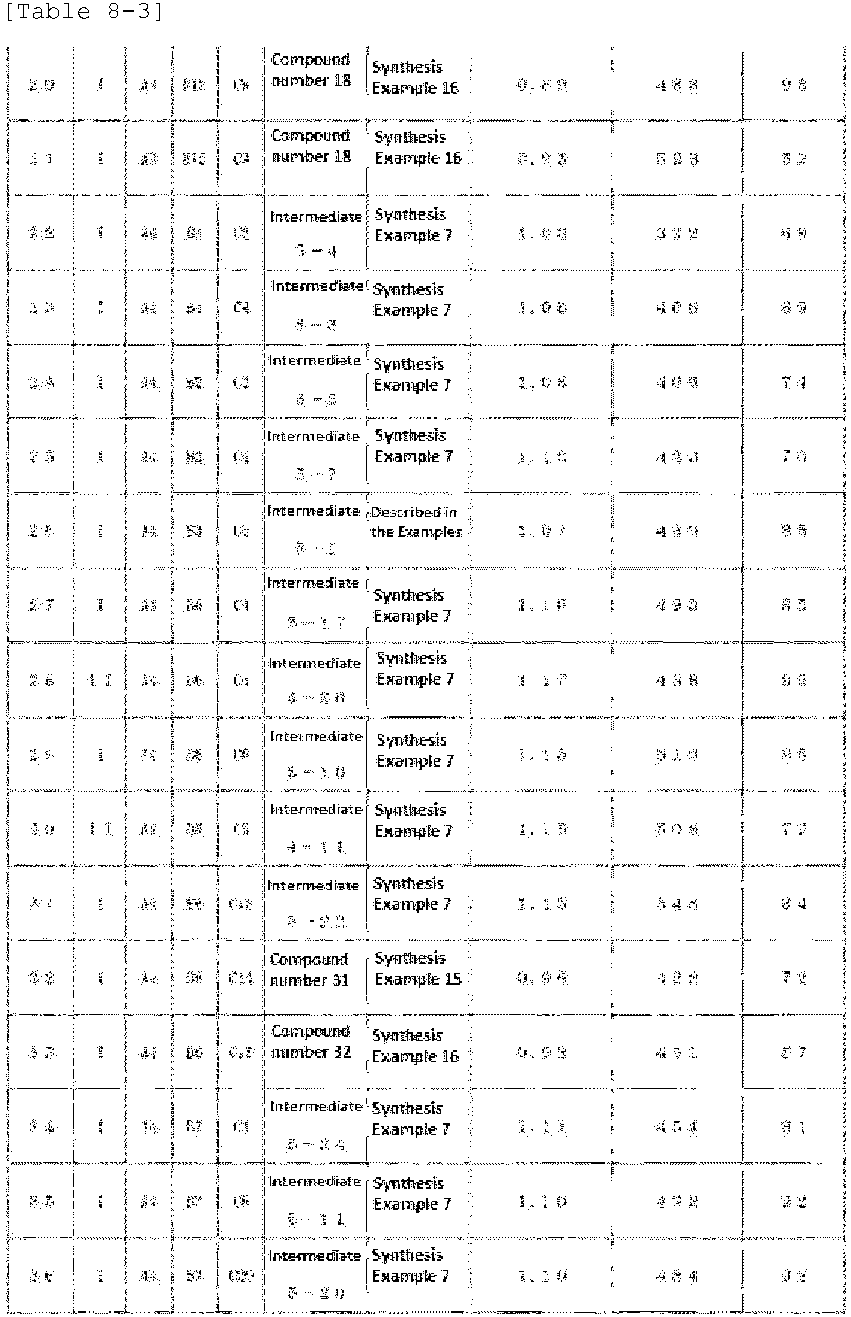

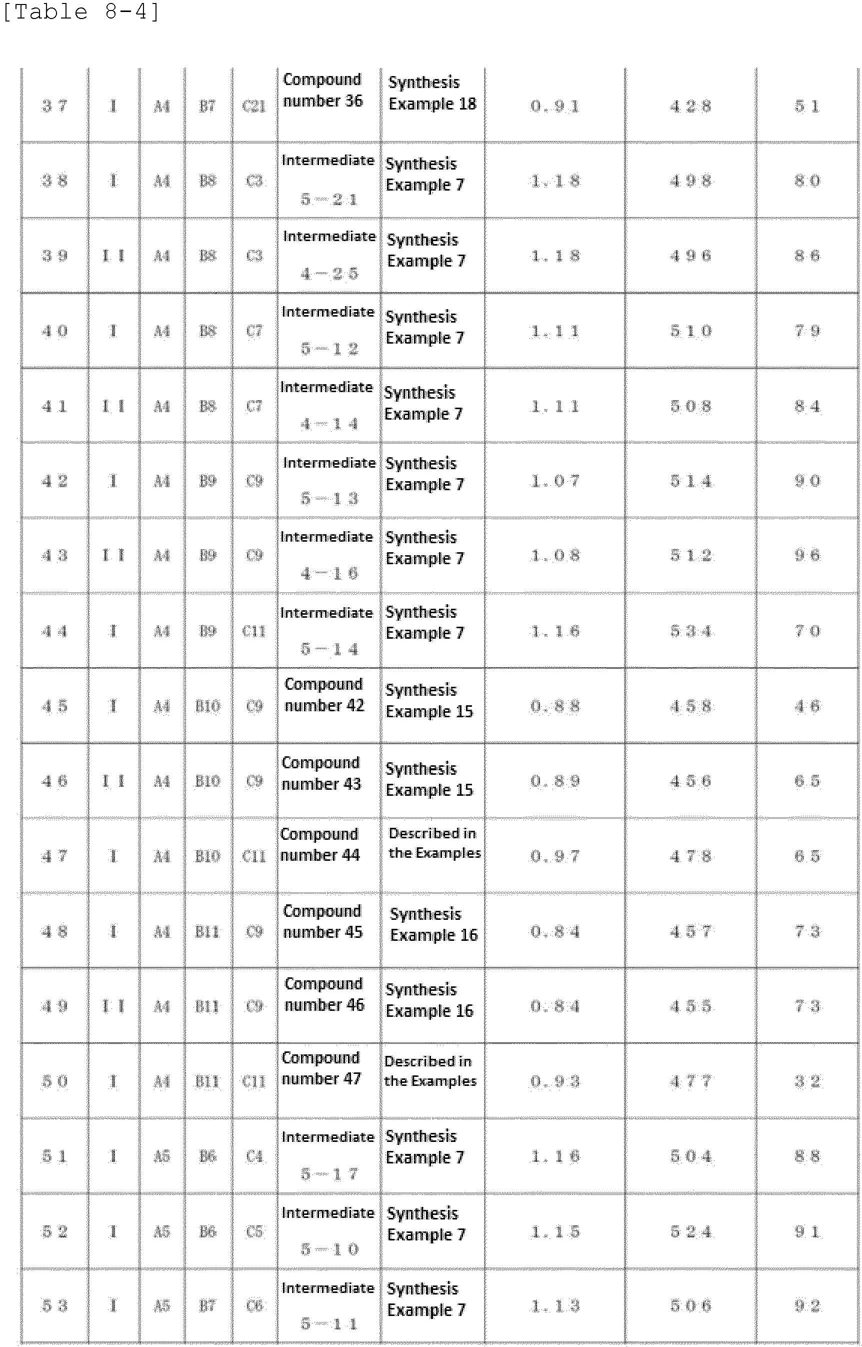

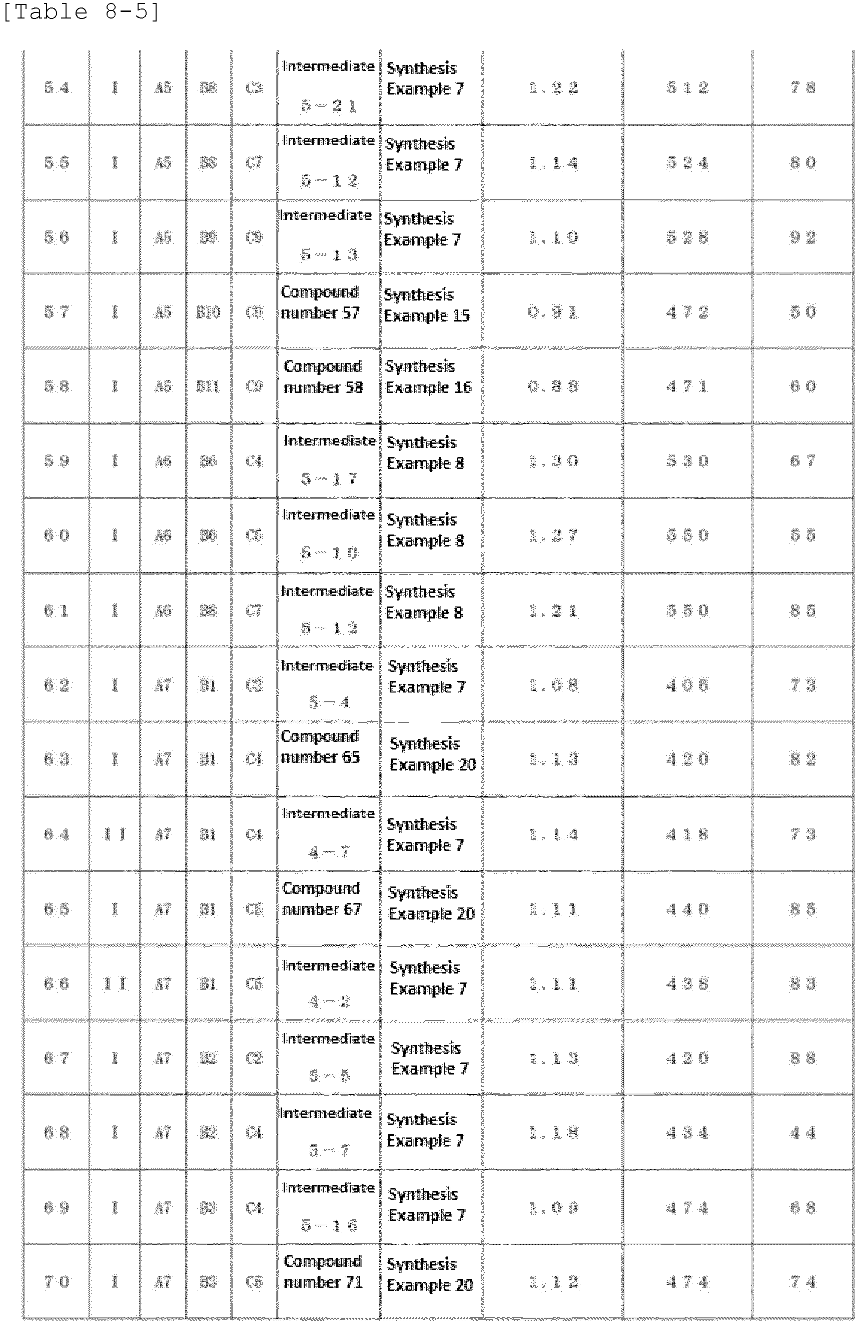

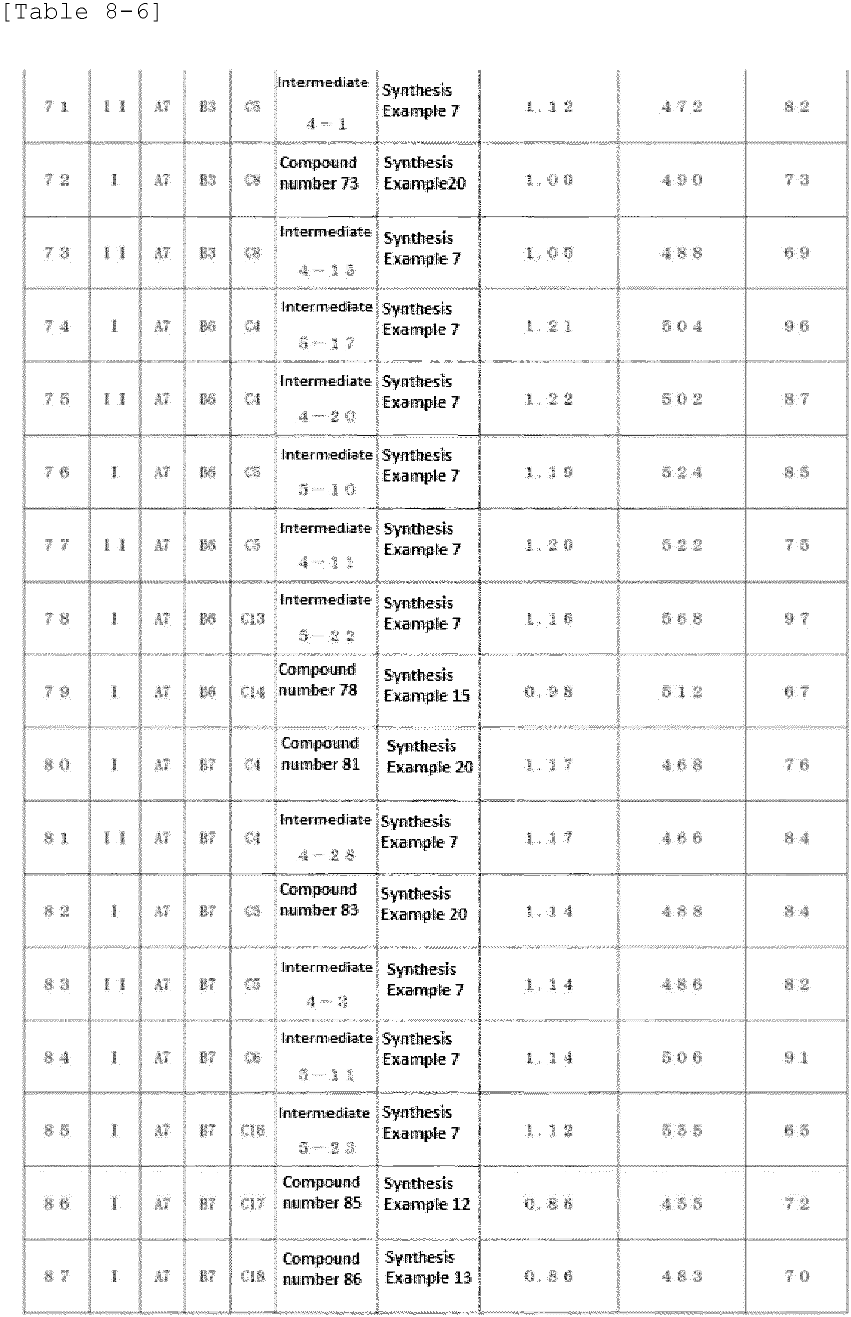

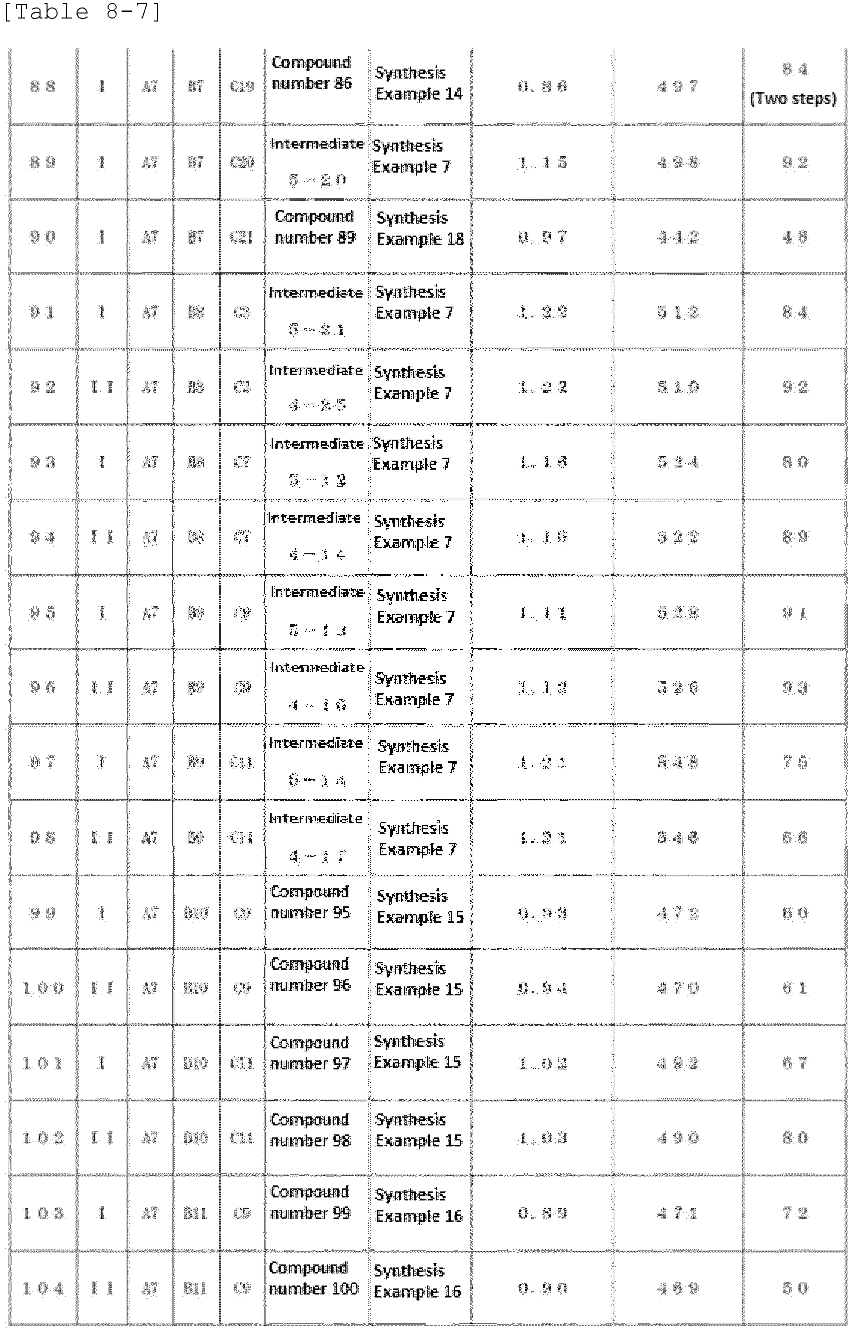

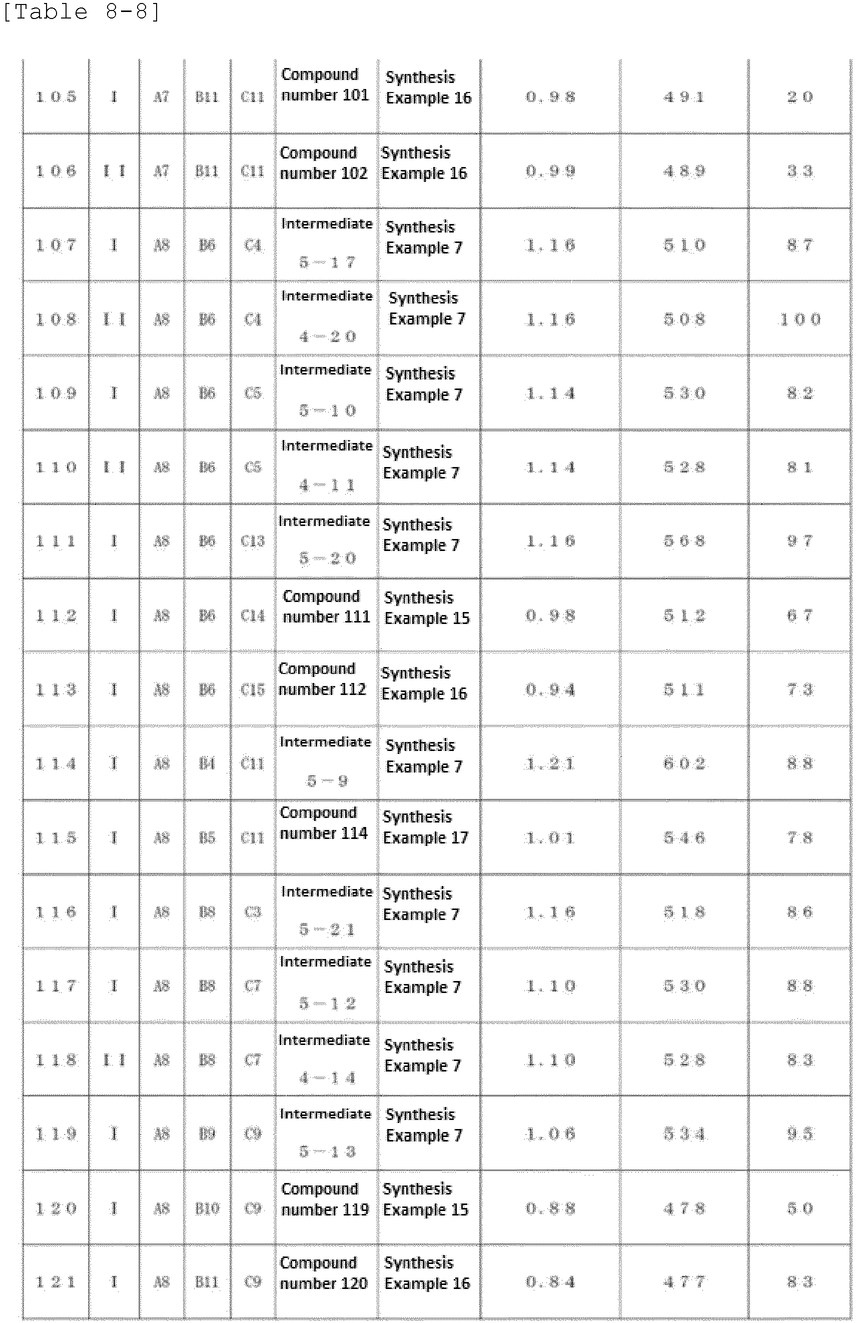

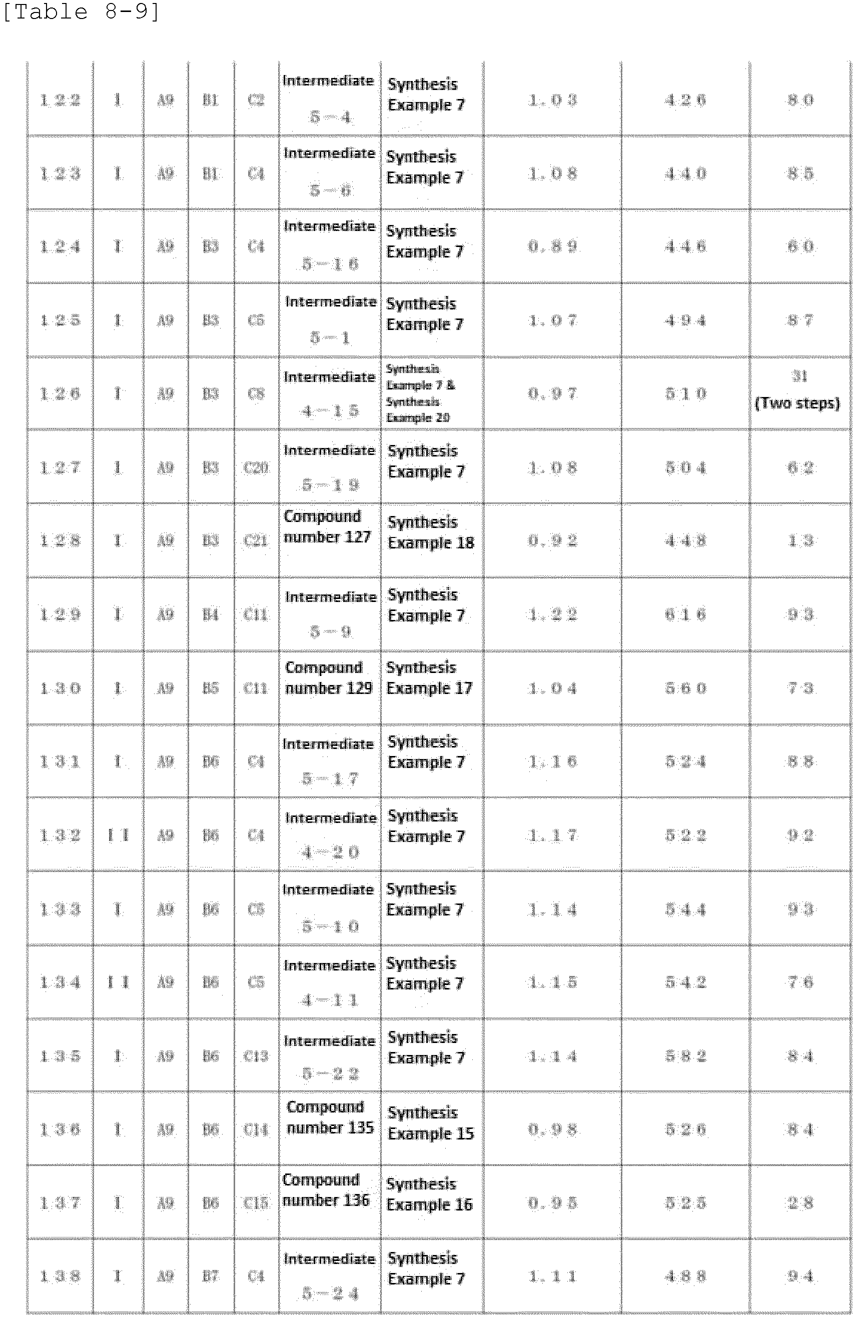

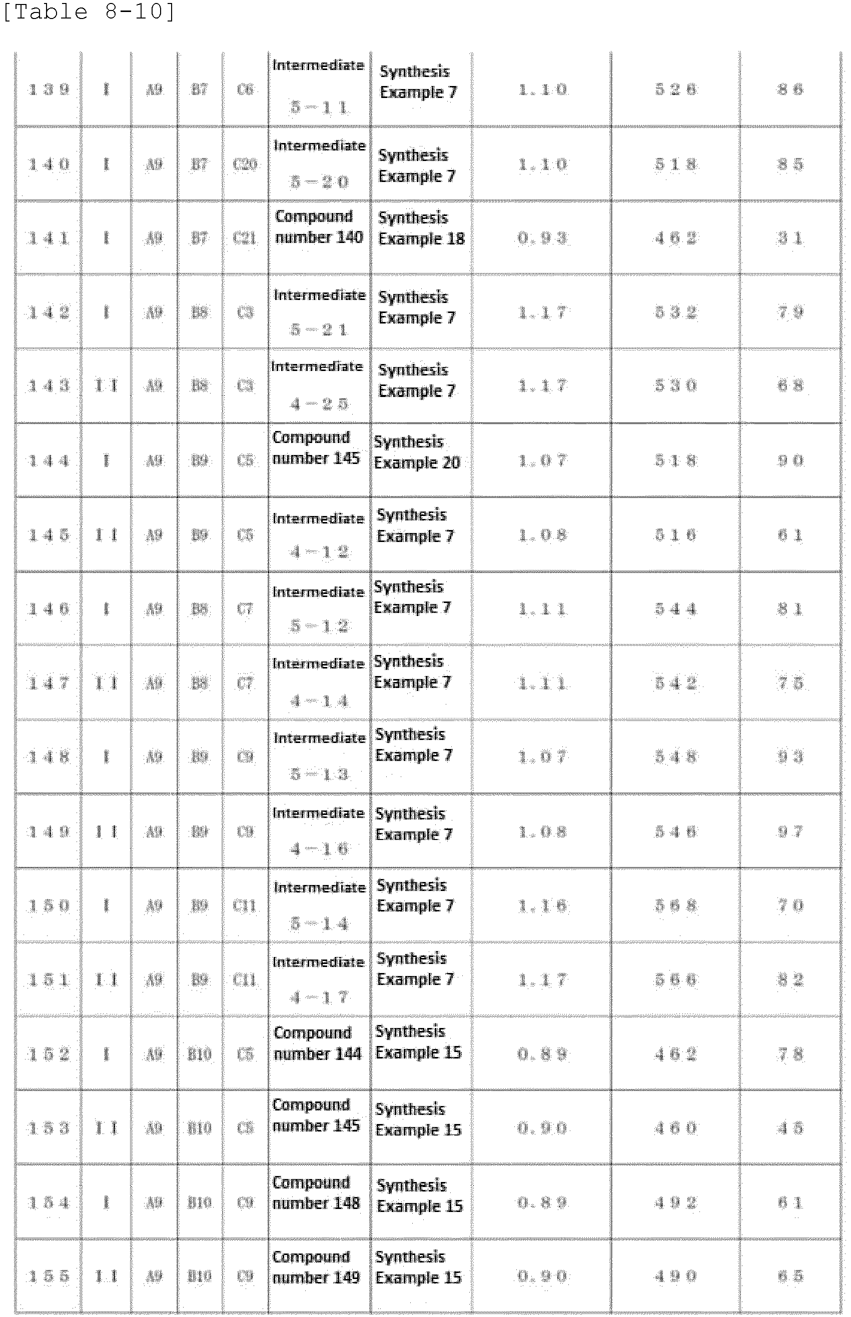

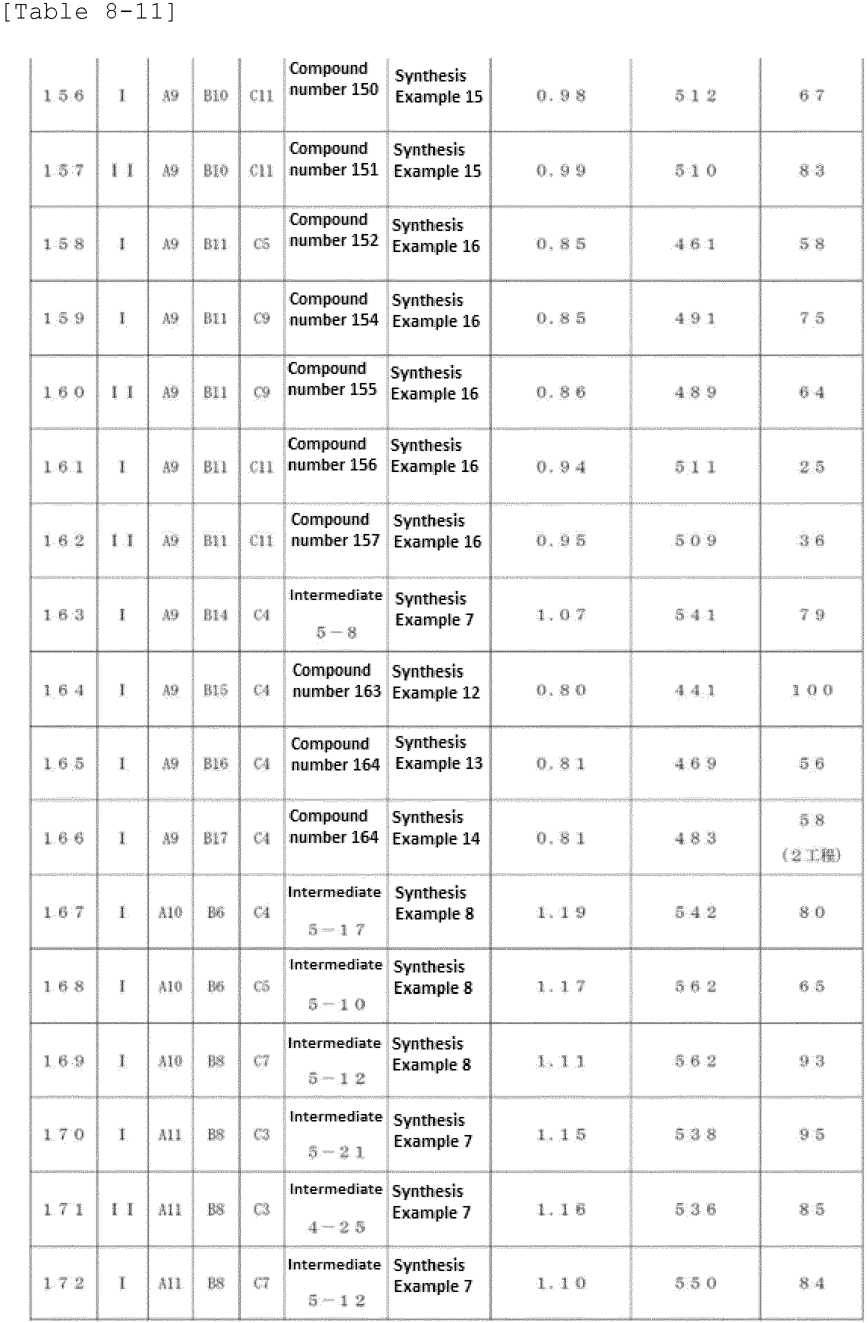

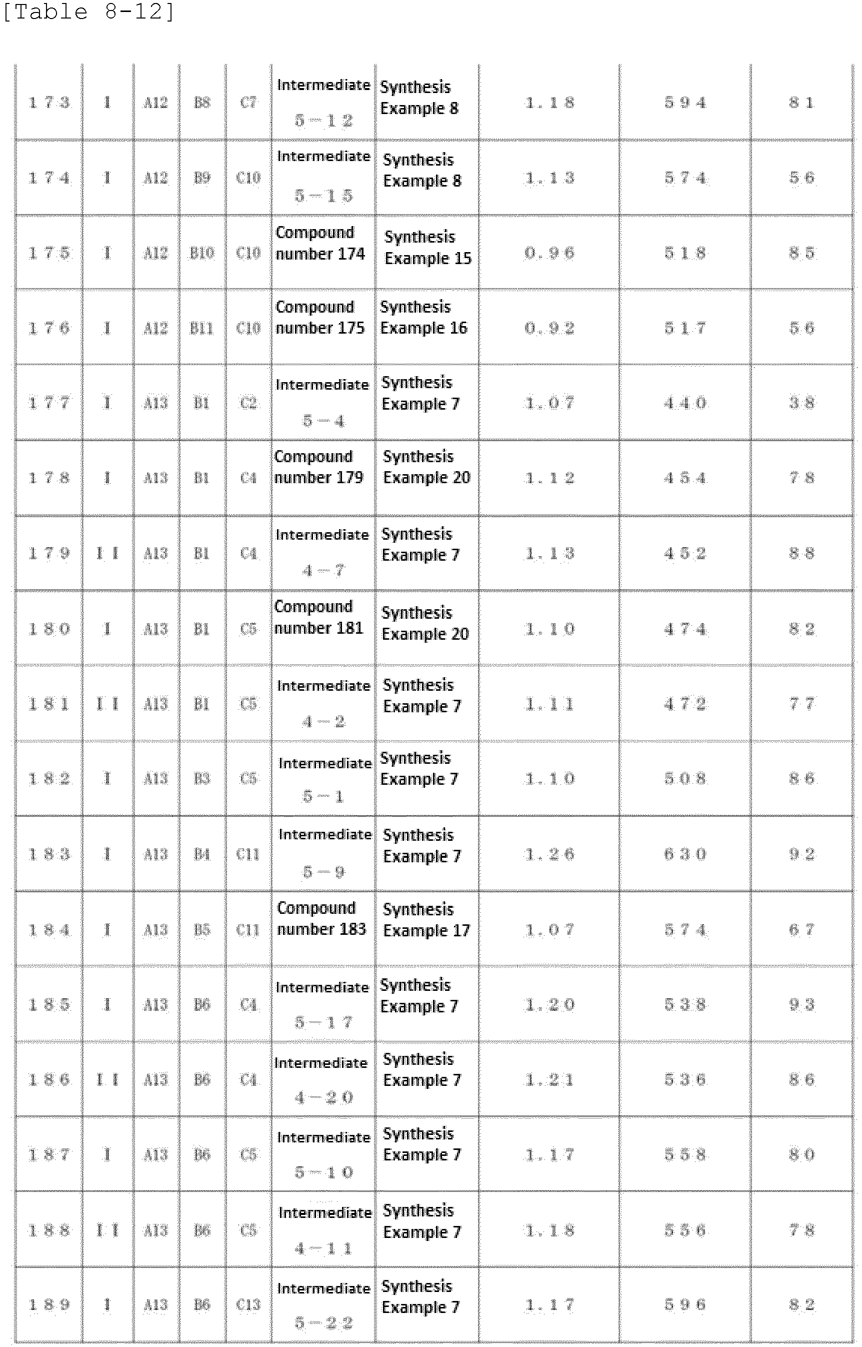

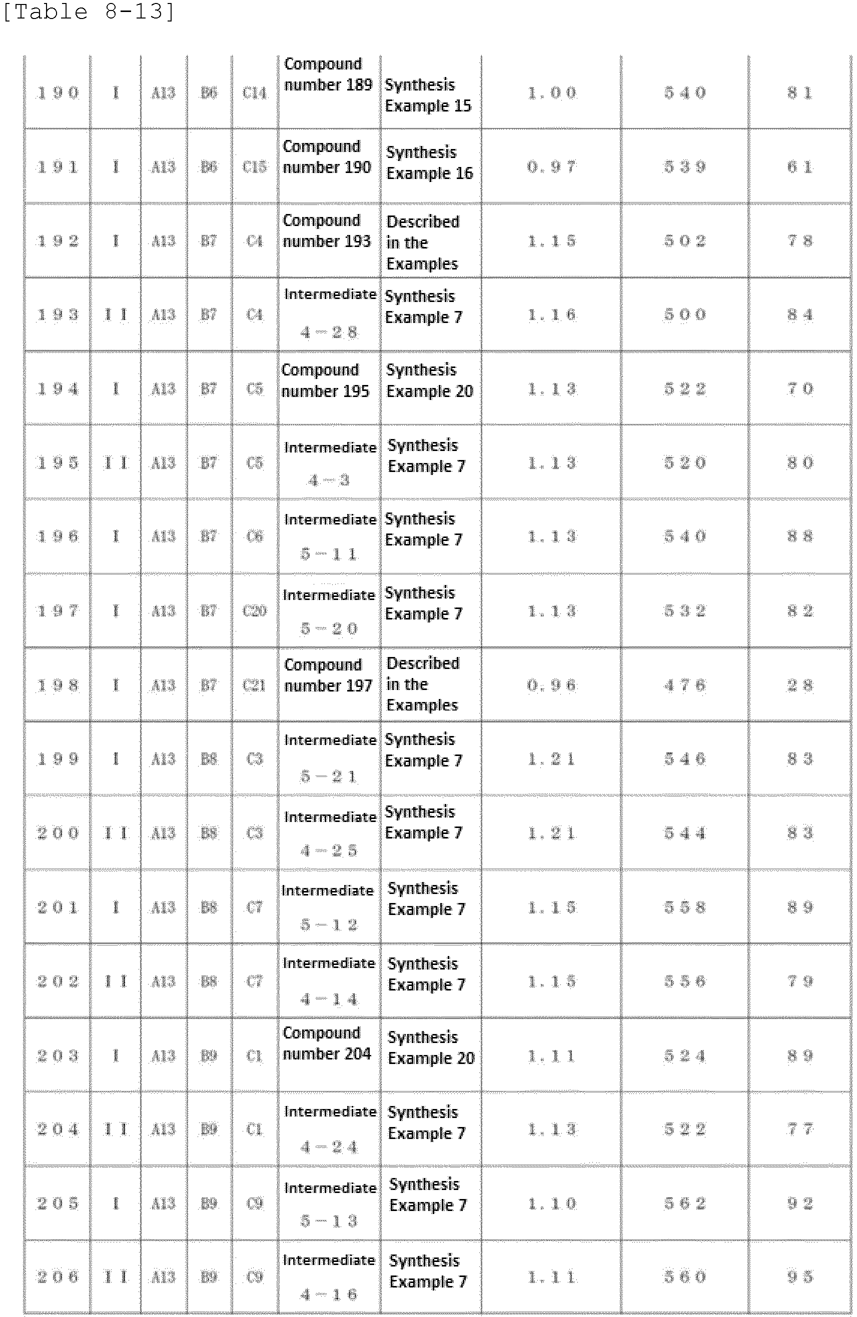

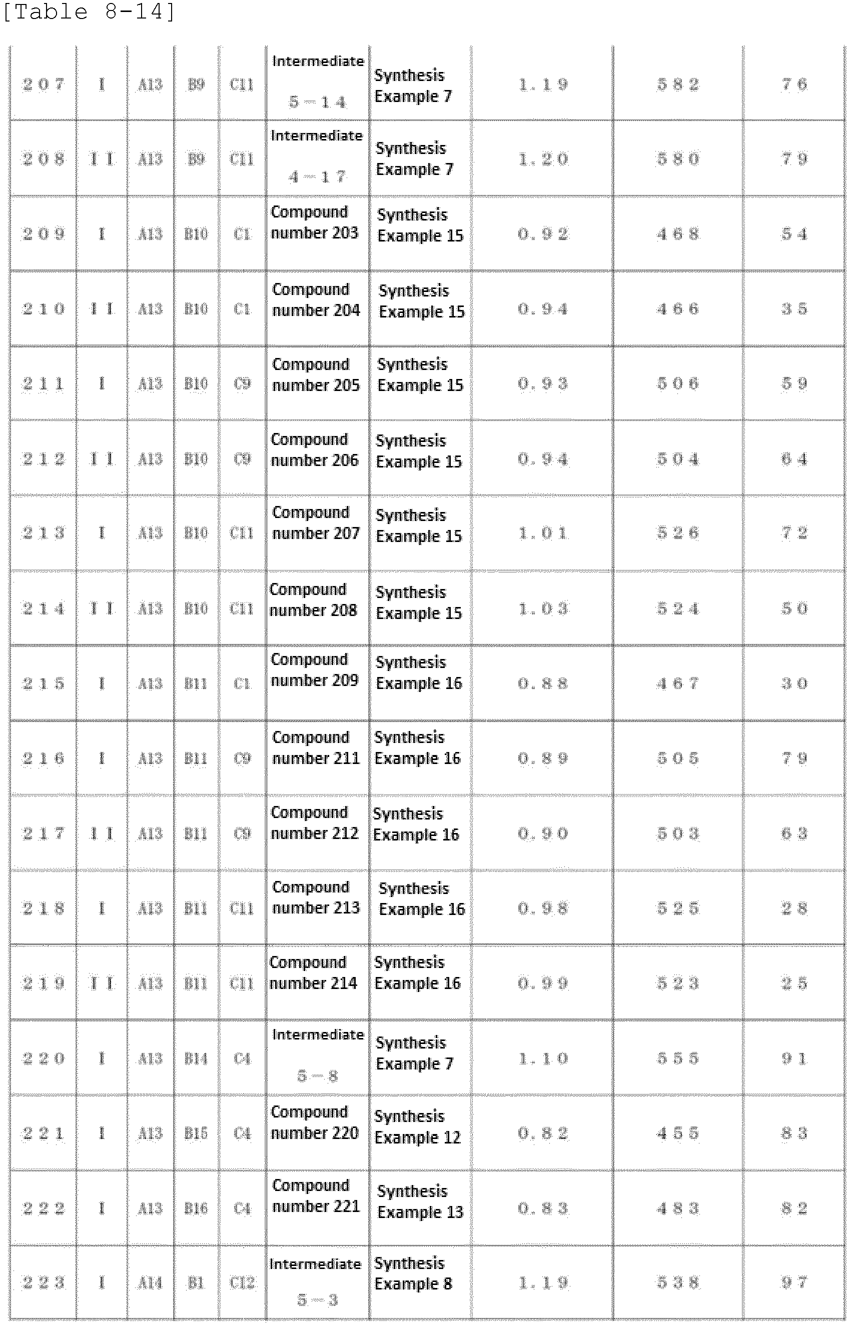

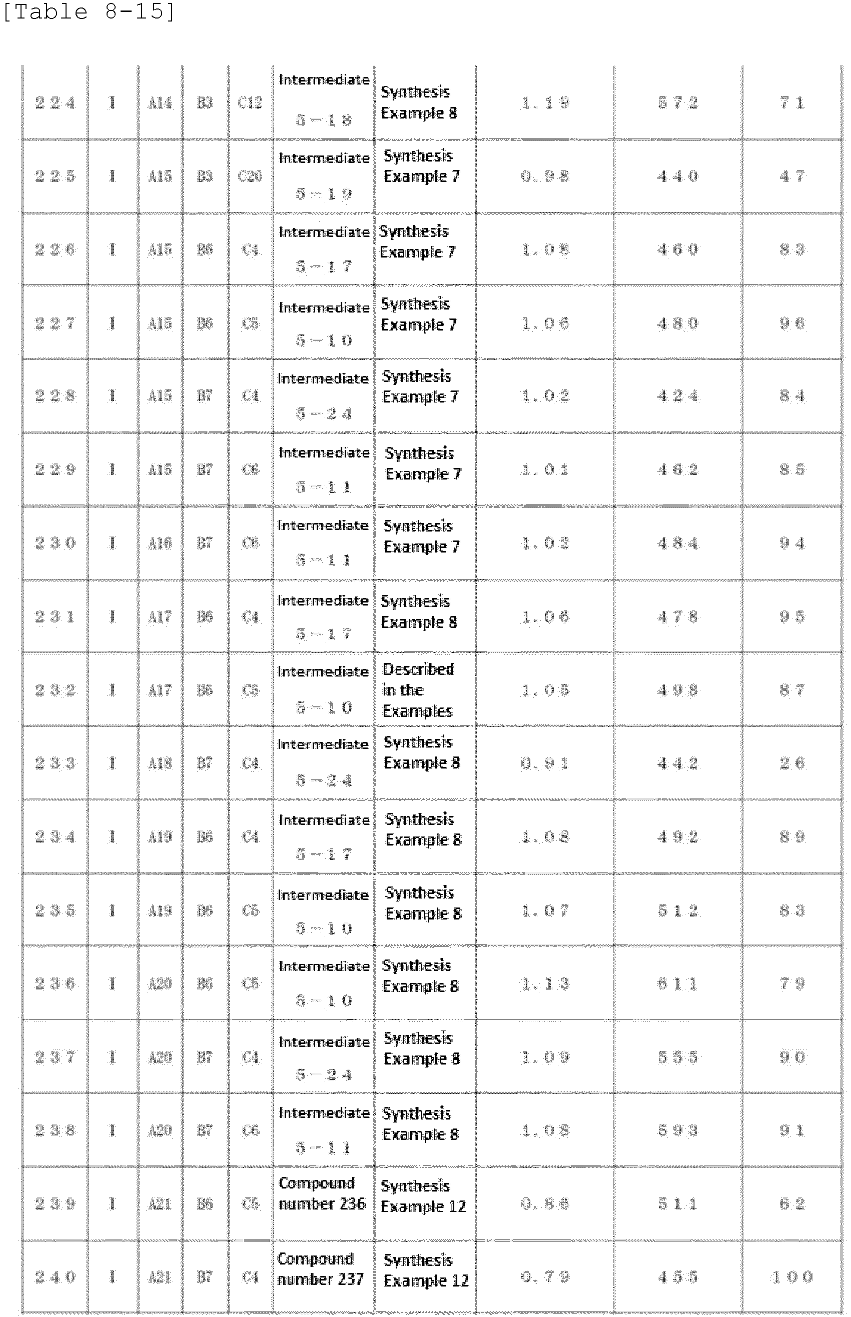

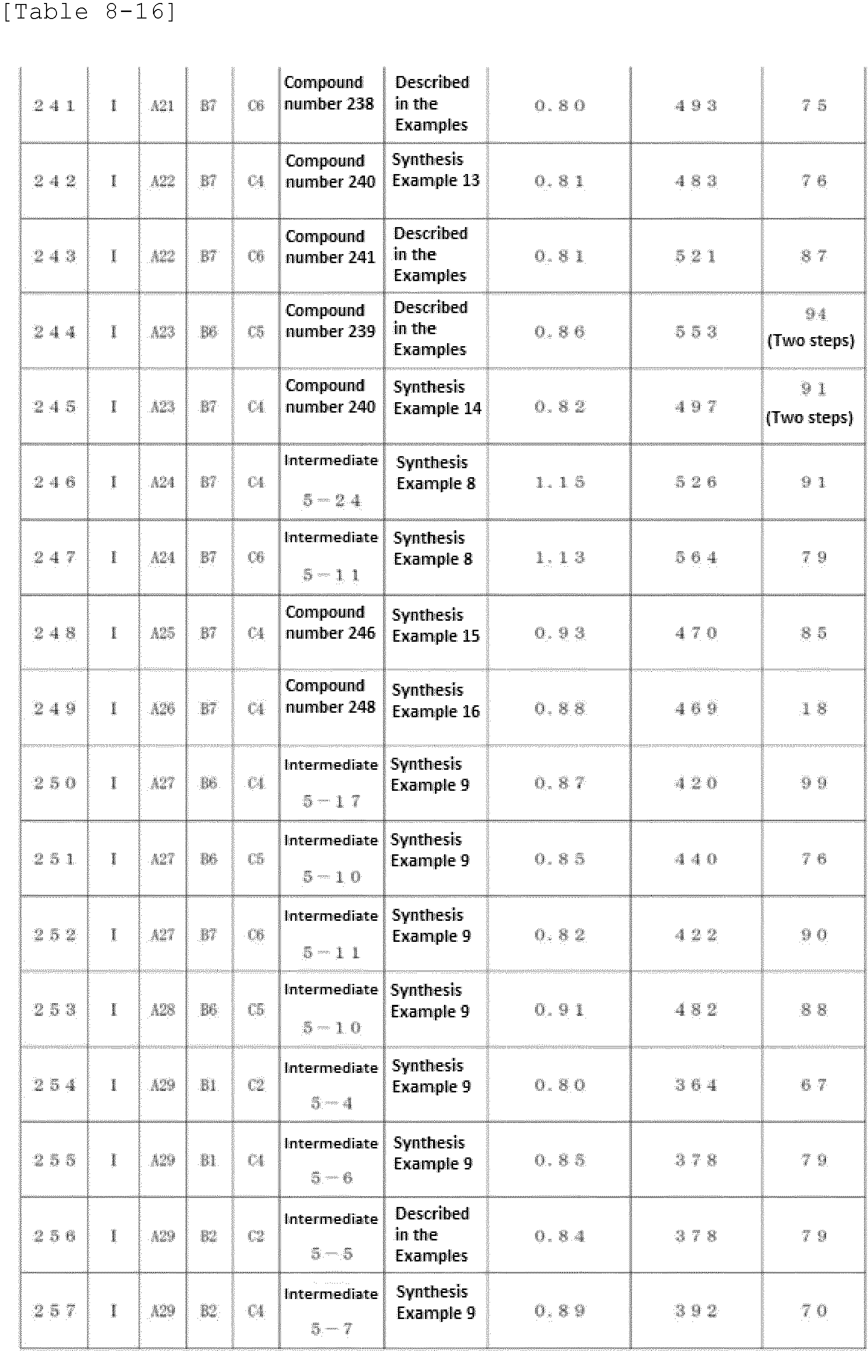

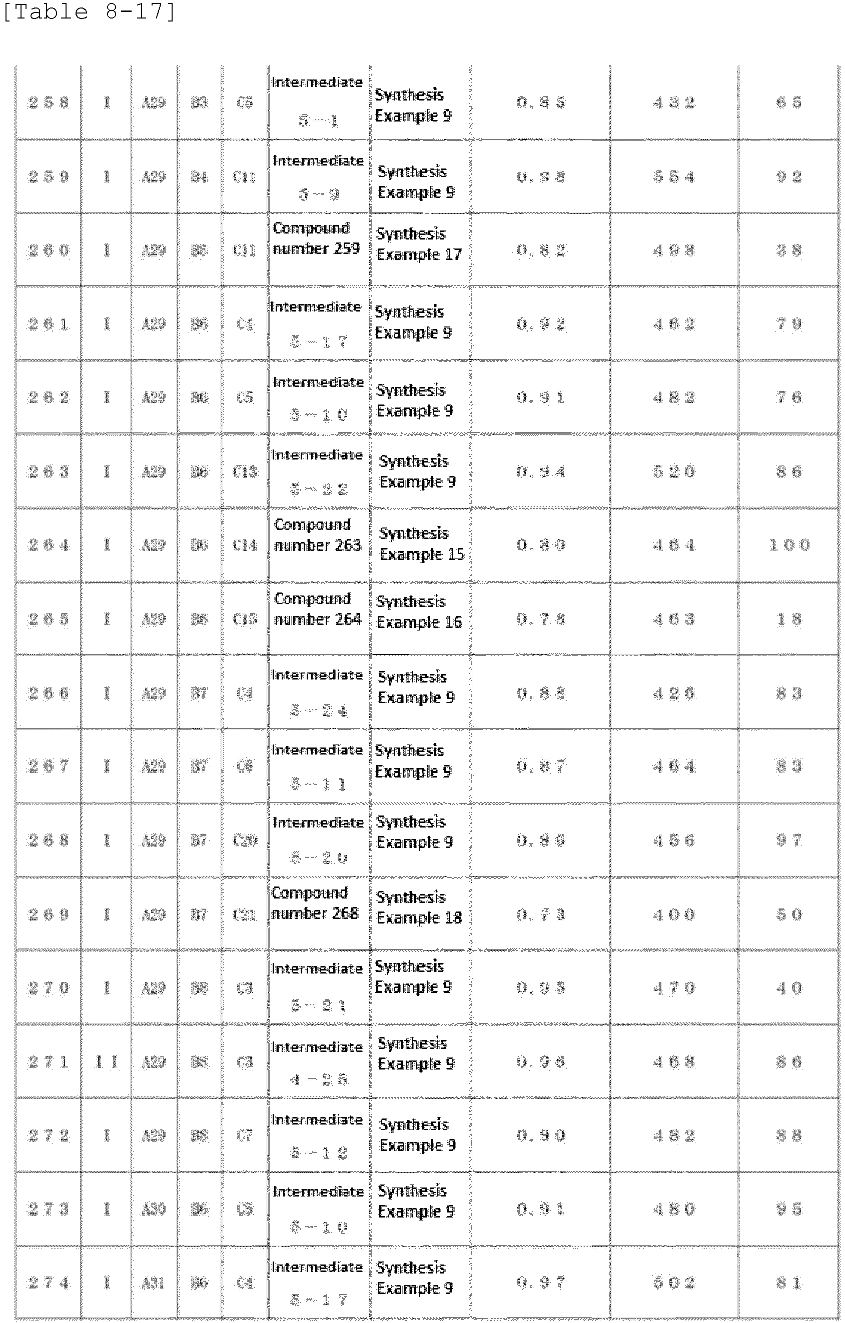

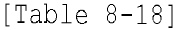

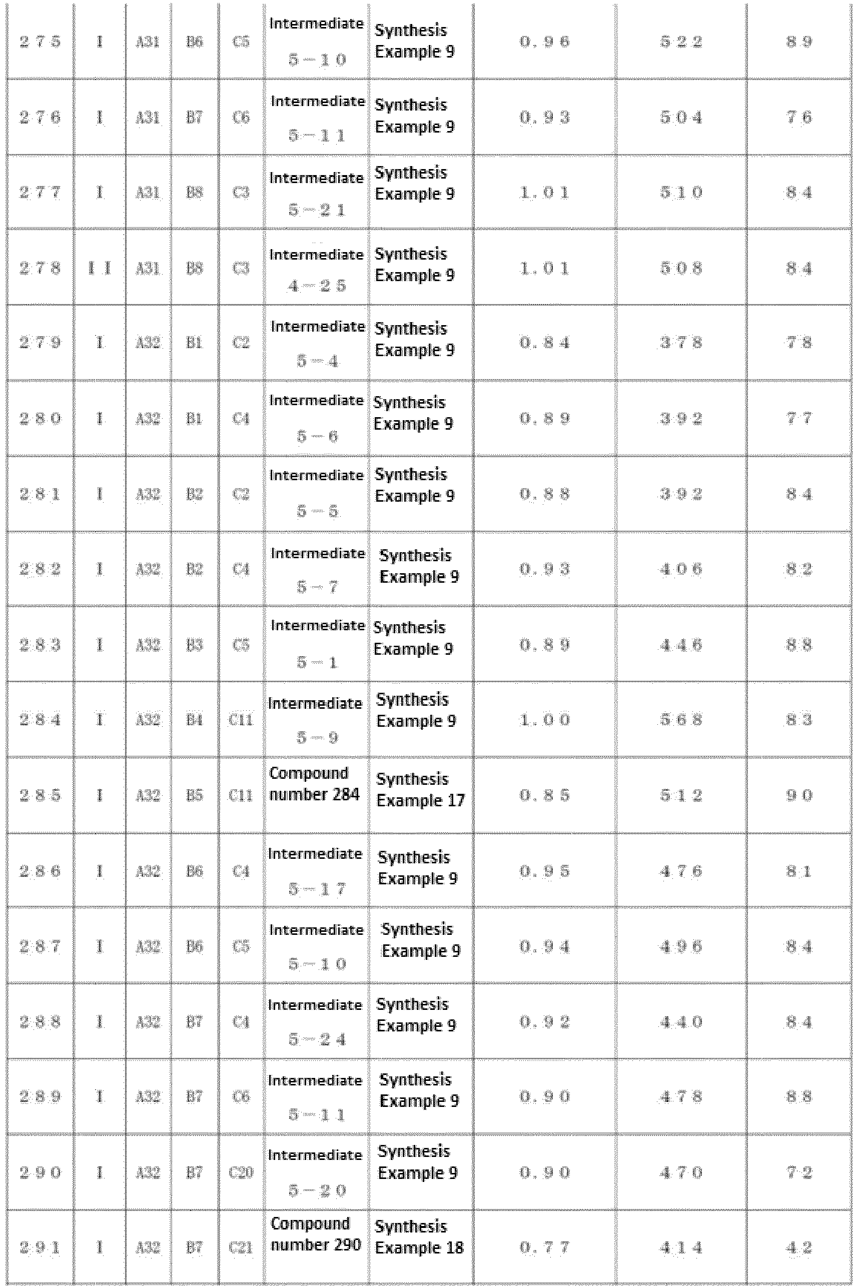

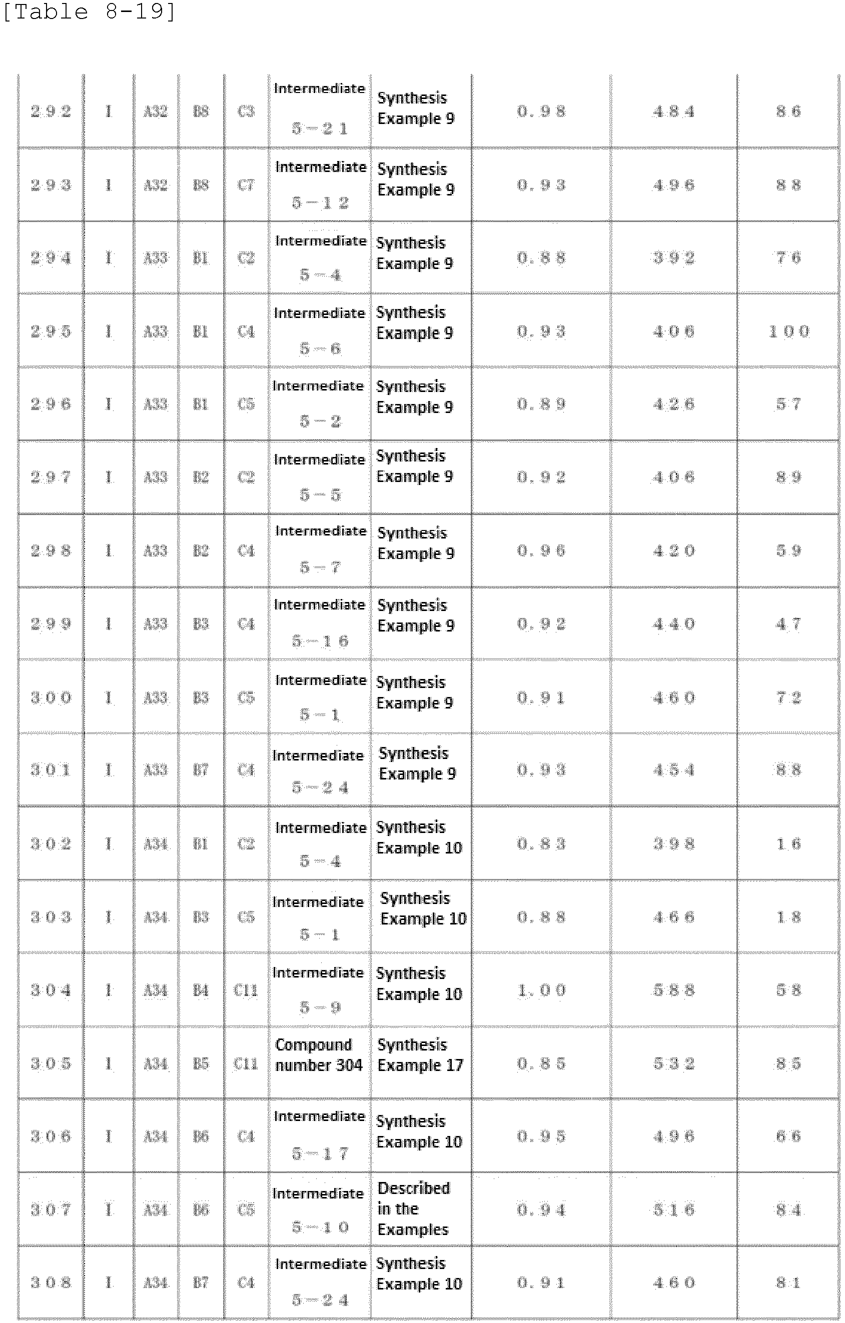

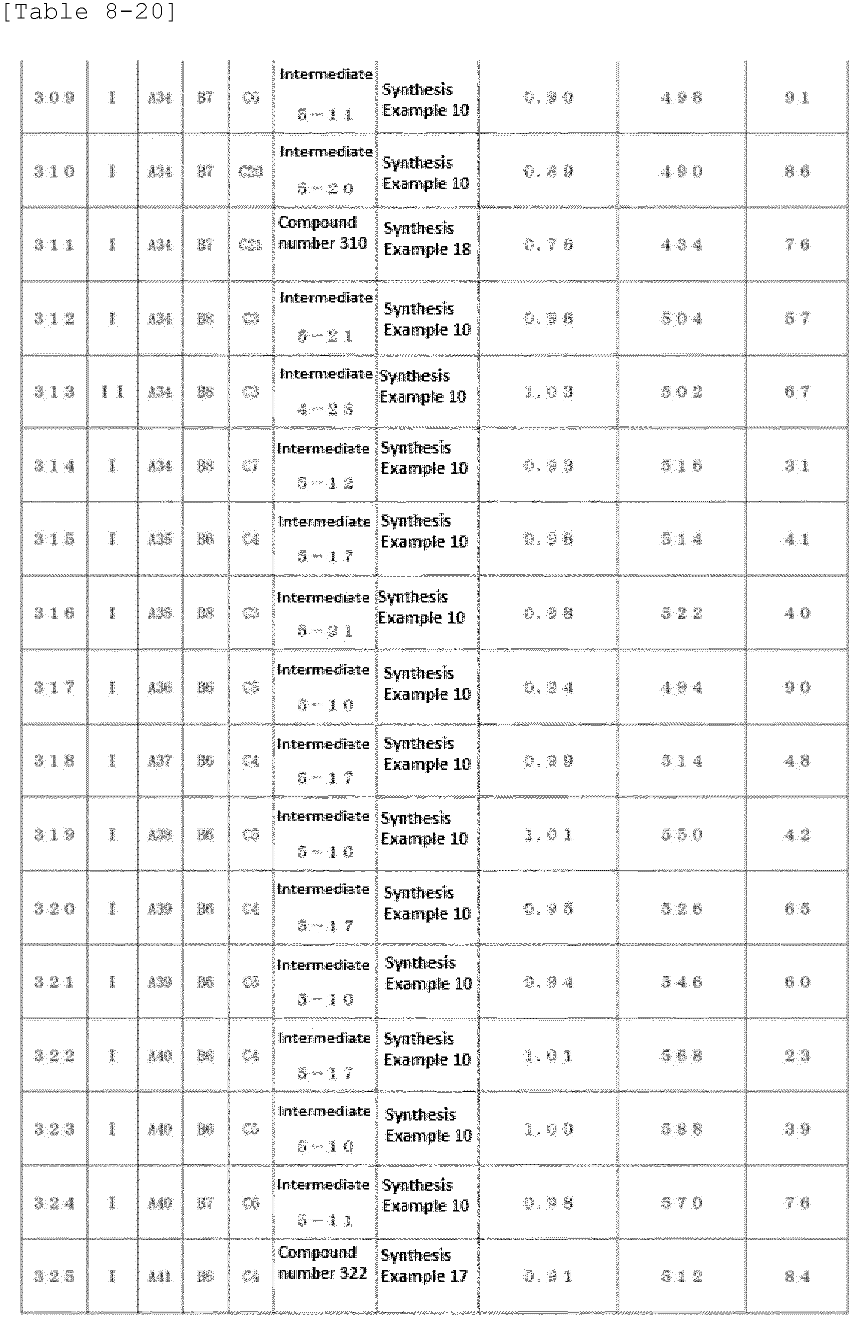

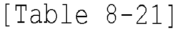

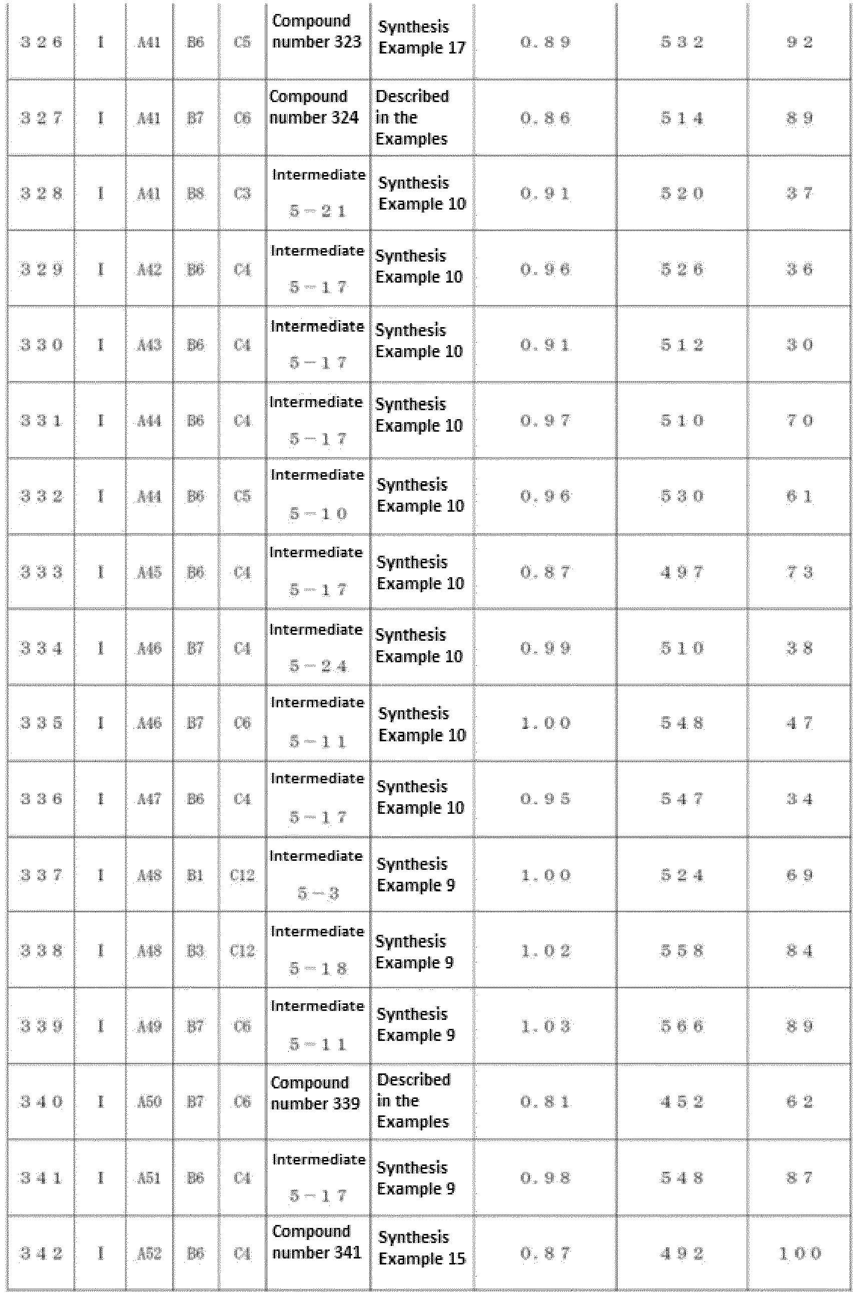

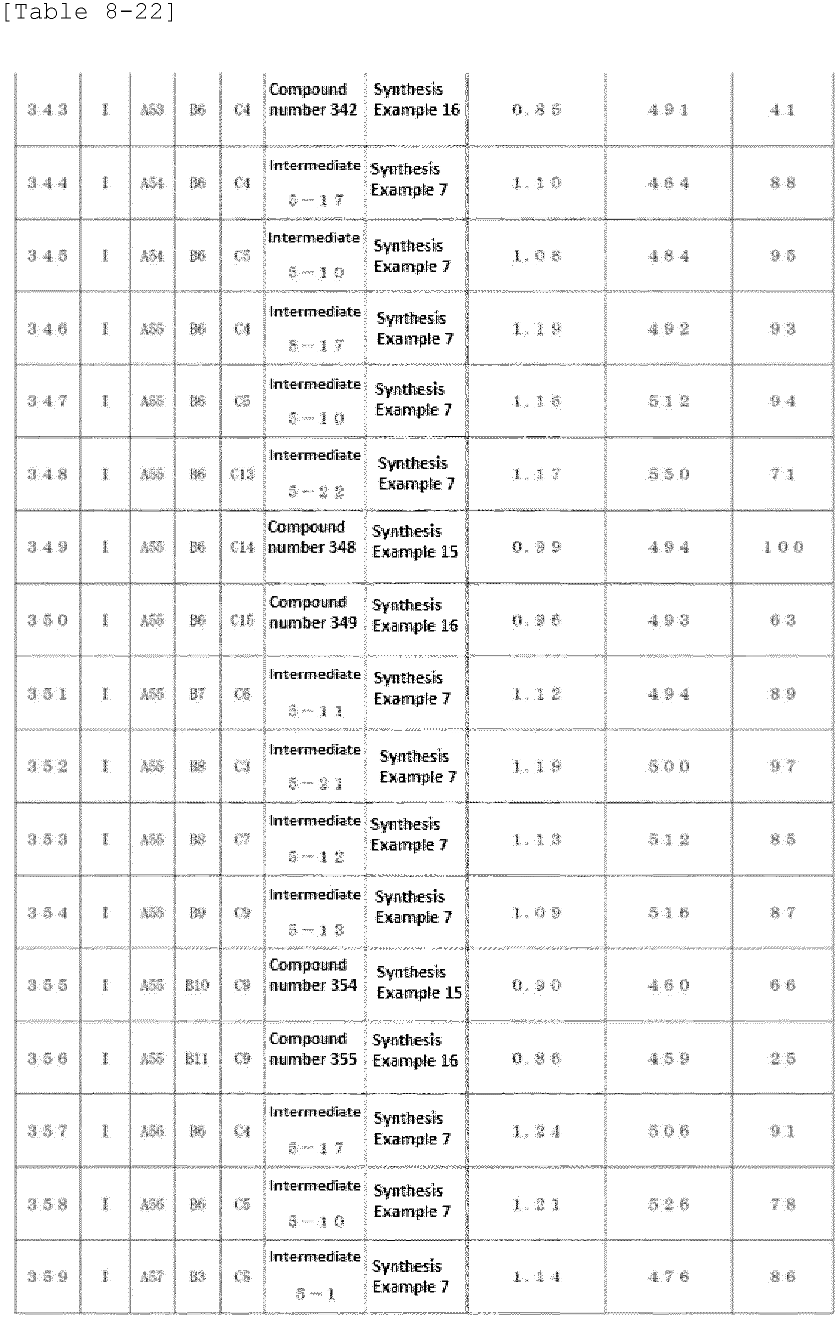

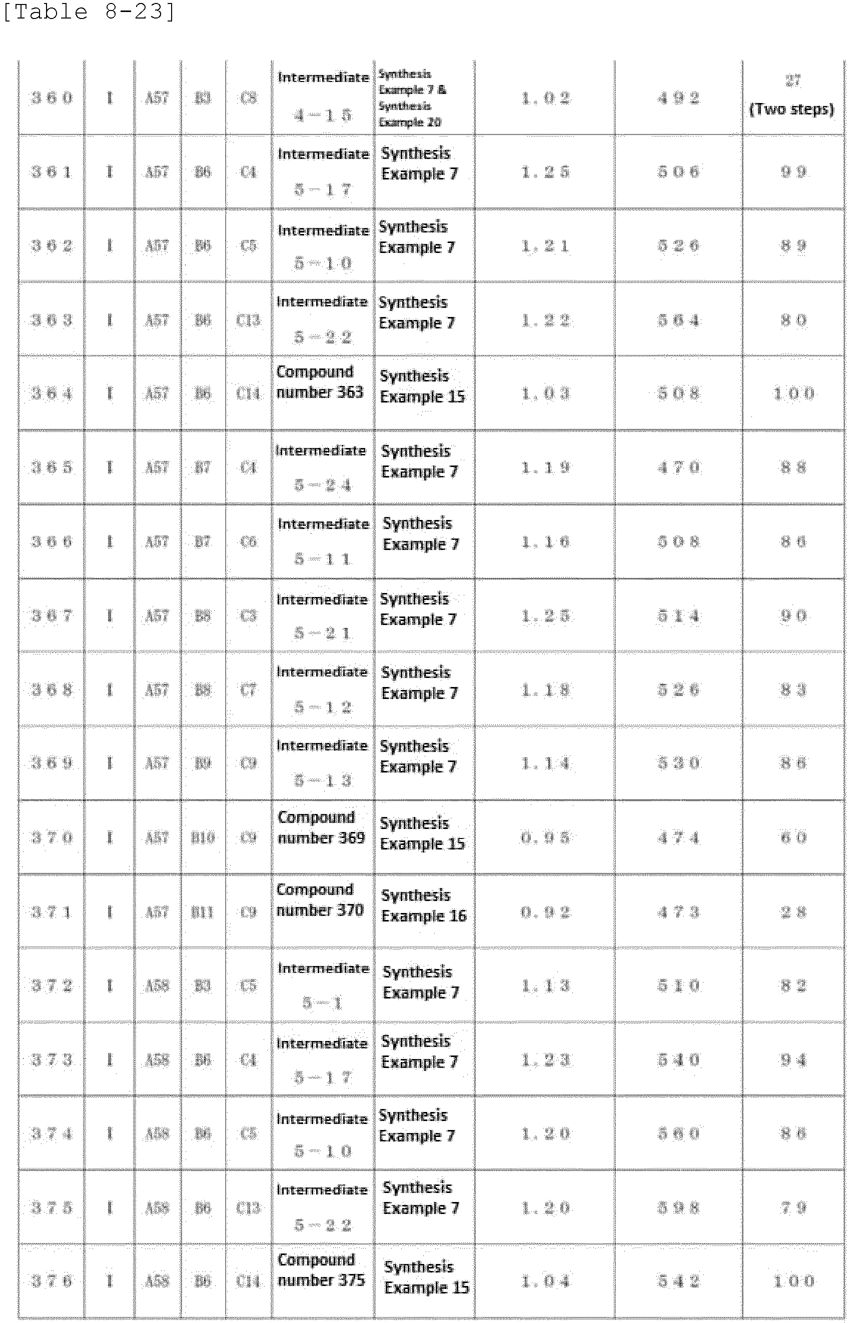

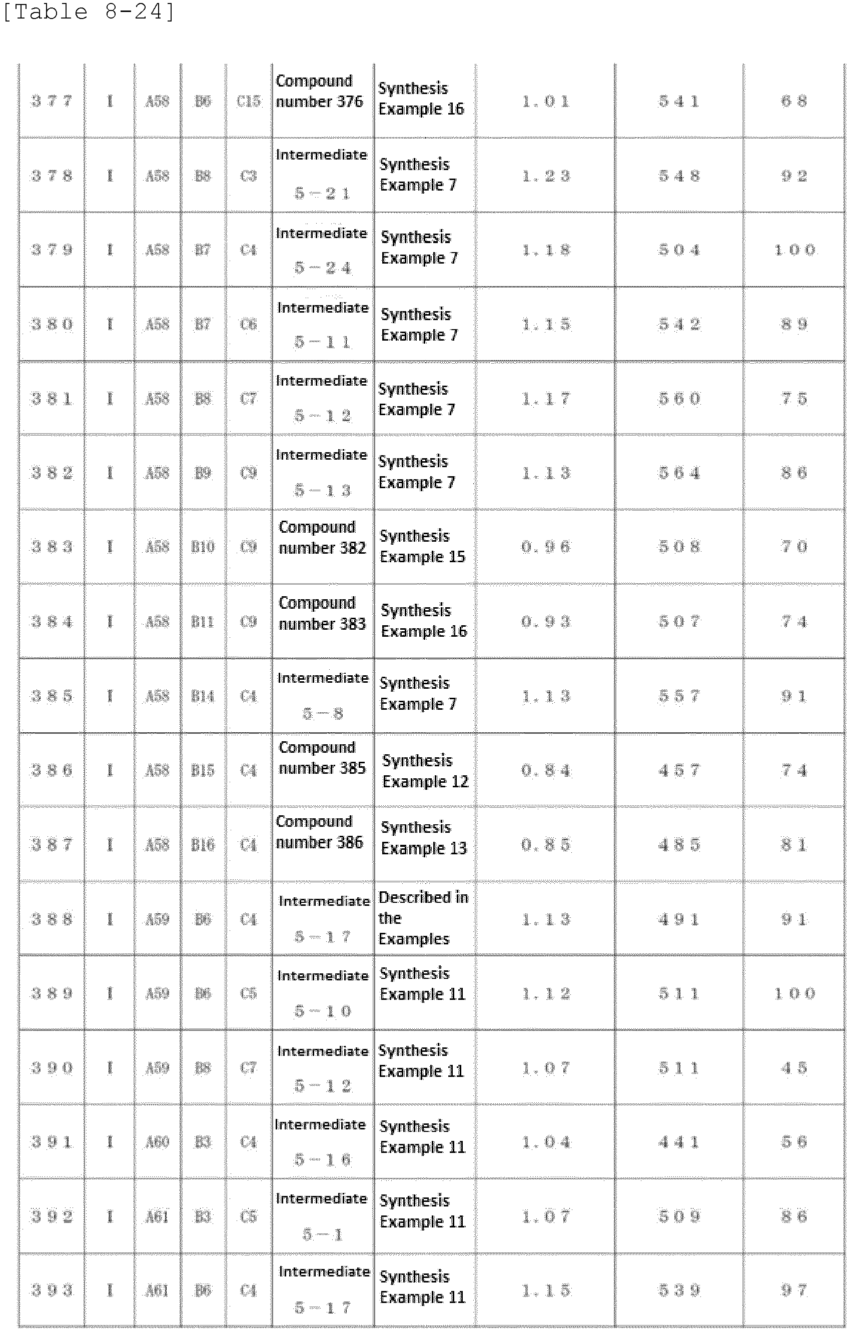

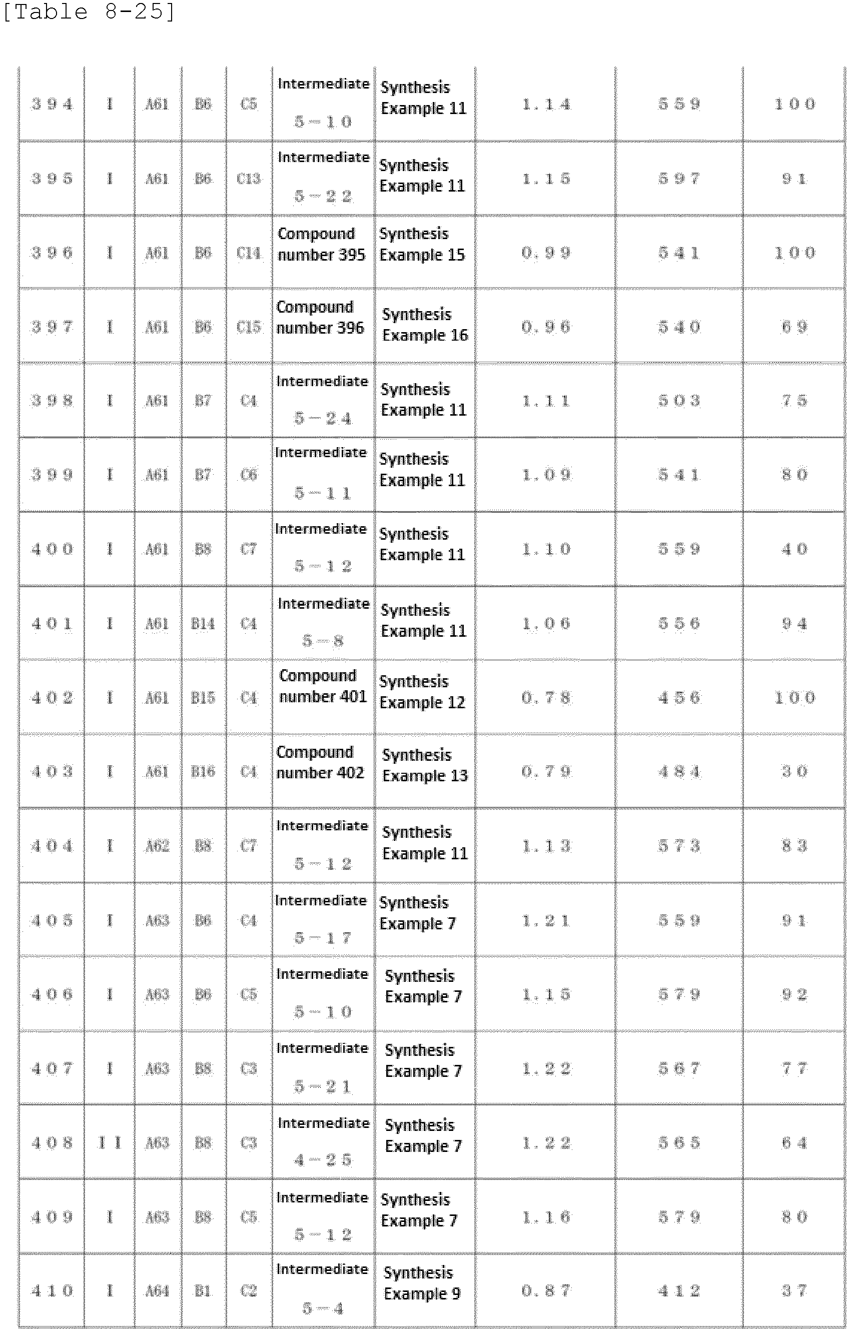

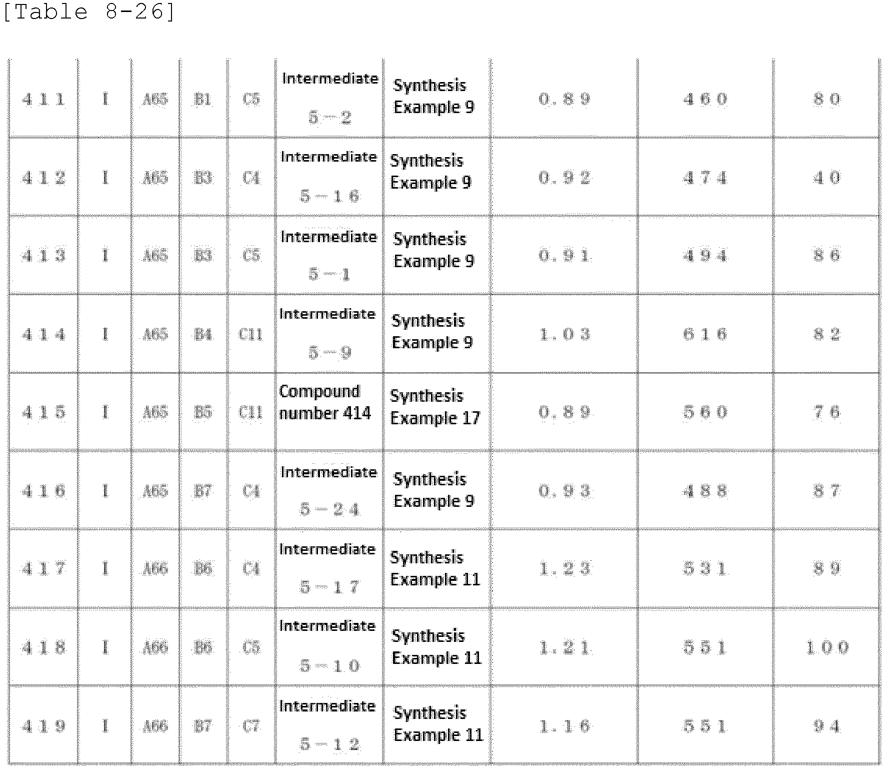

In this regard, R₁, R₂, and R₃ in the tables described above are selected from the following tables.
R₁ is selected from the following table.

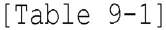

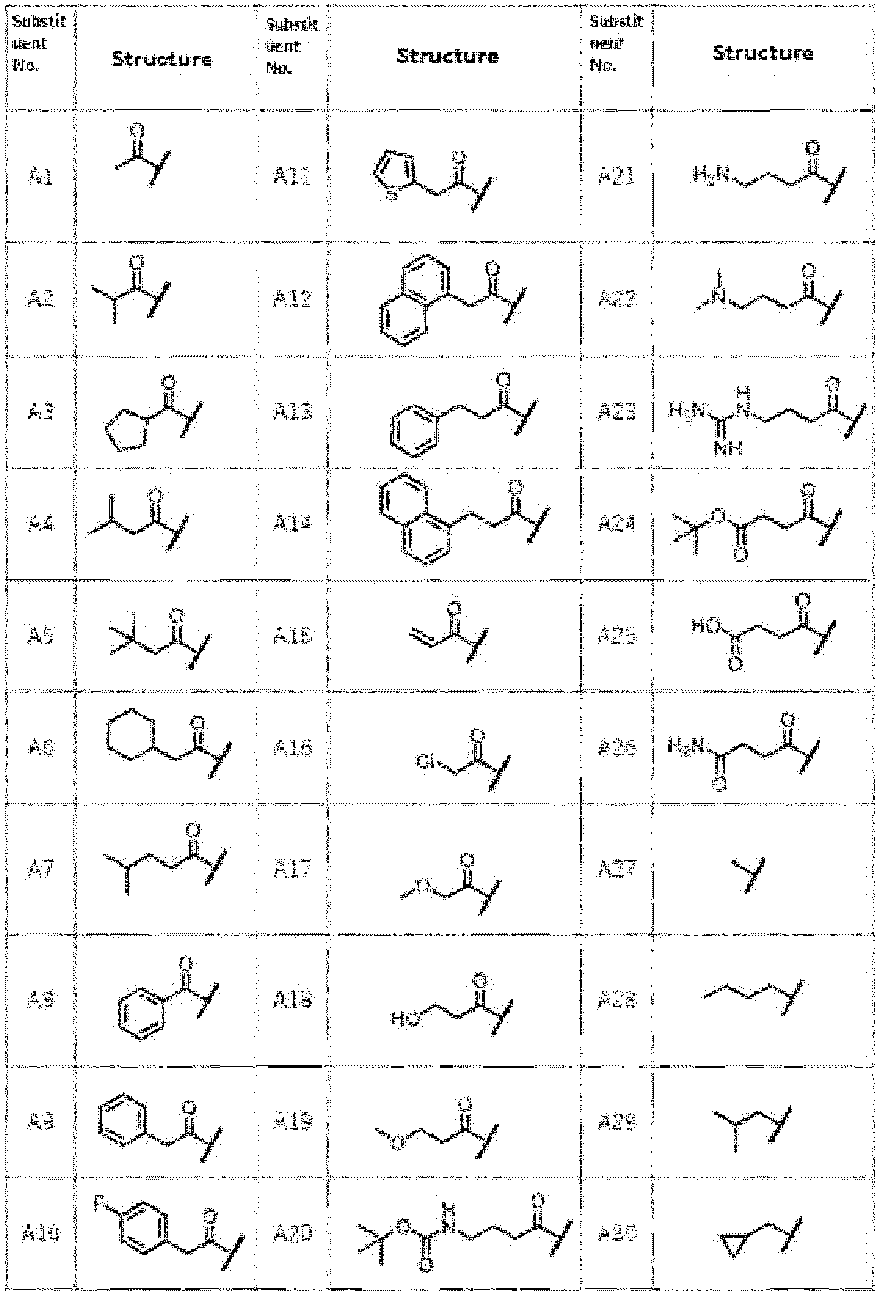

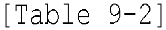

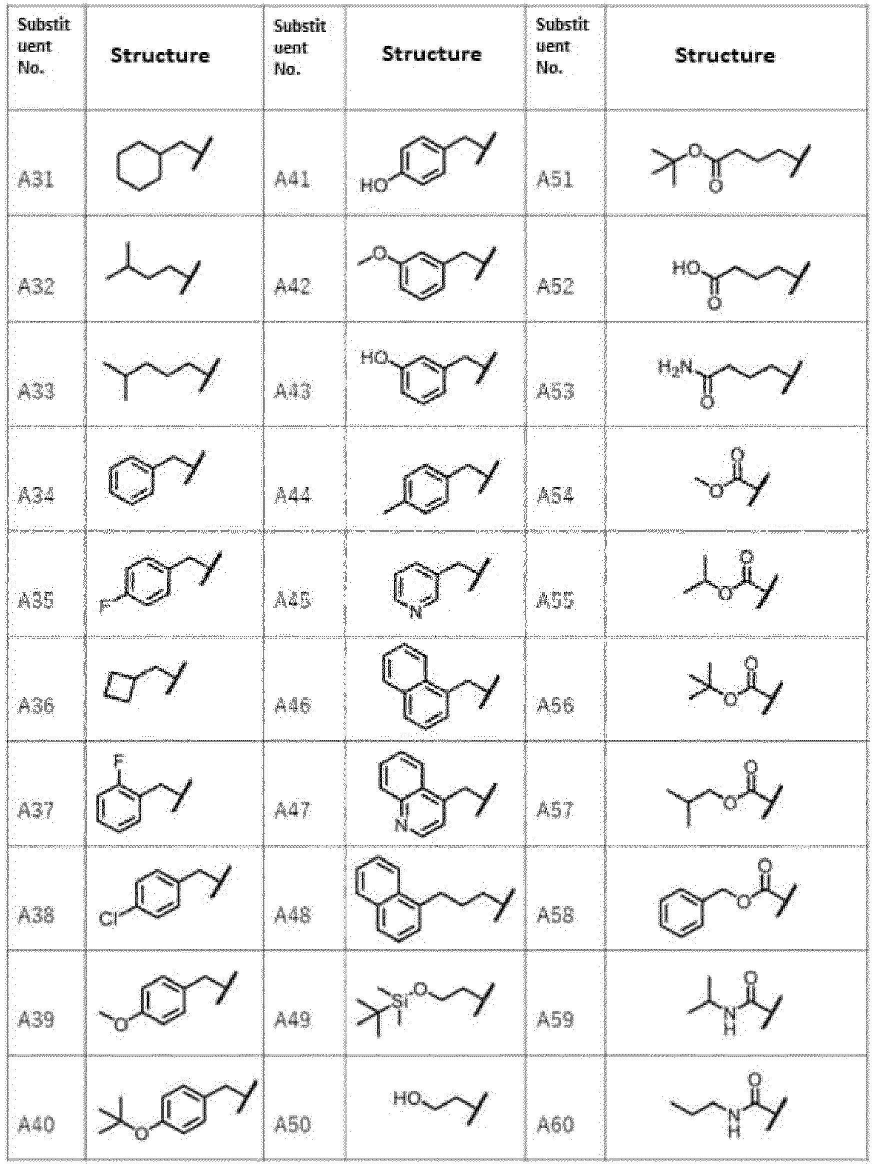

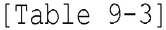

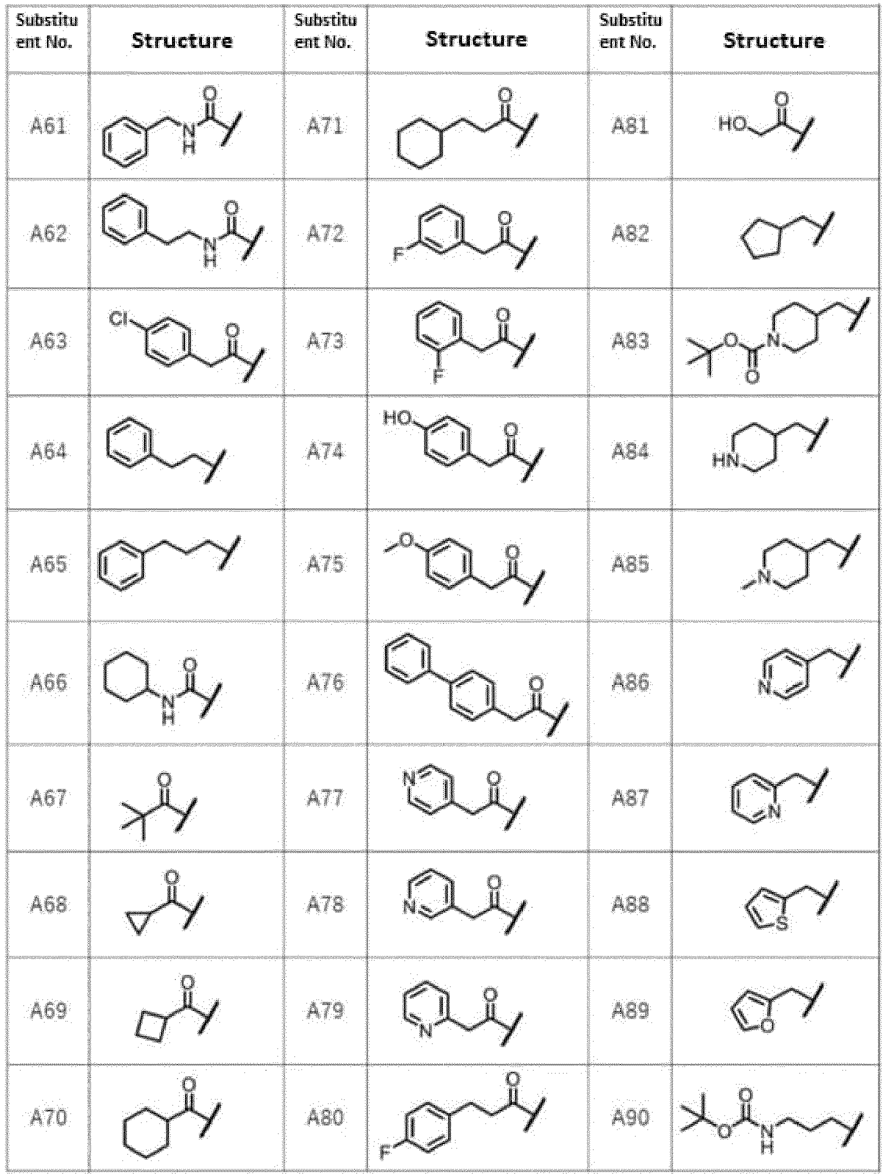

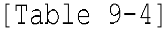

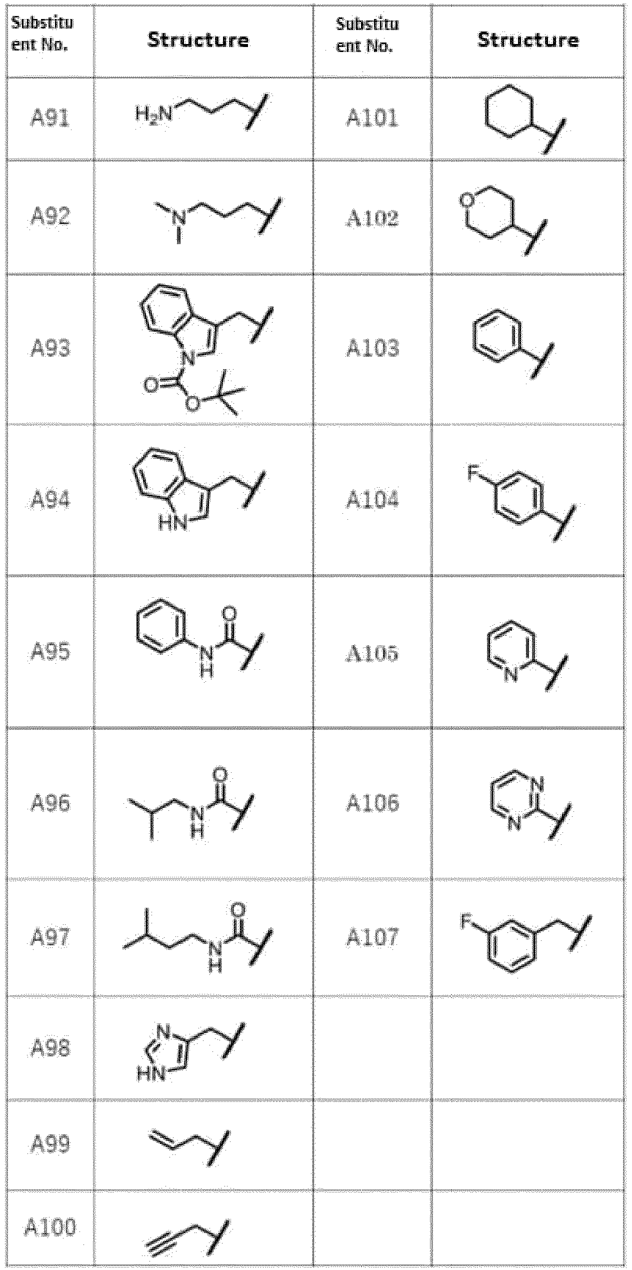

R₂ is a group selected from the following tables.

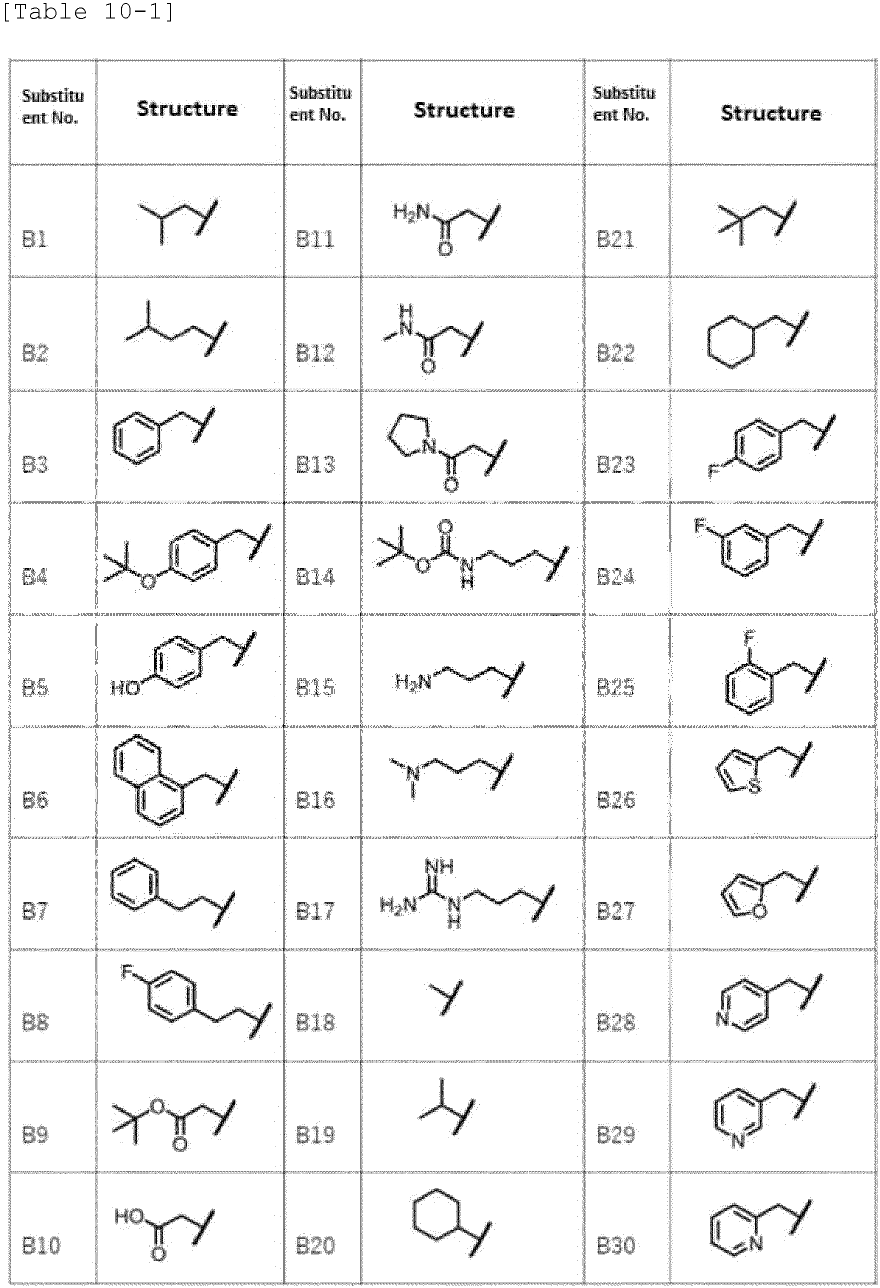

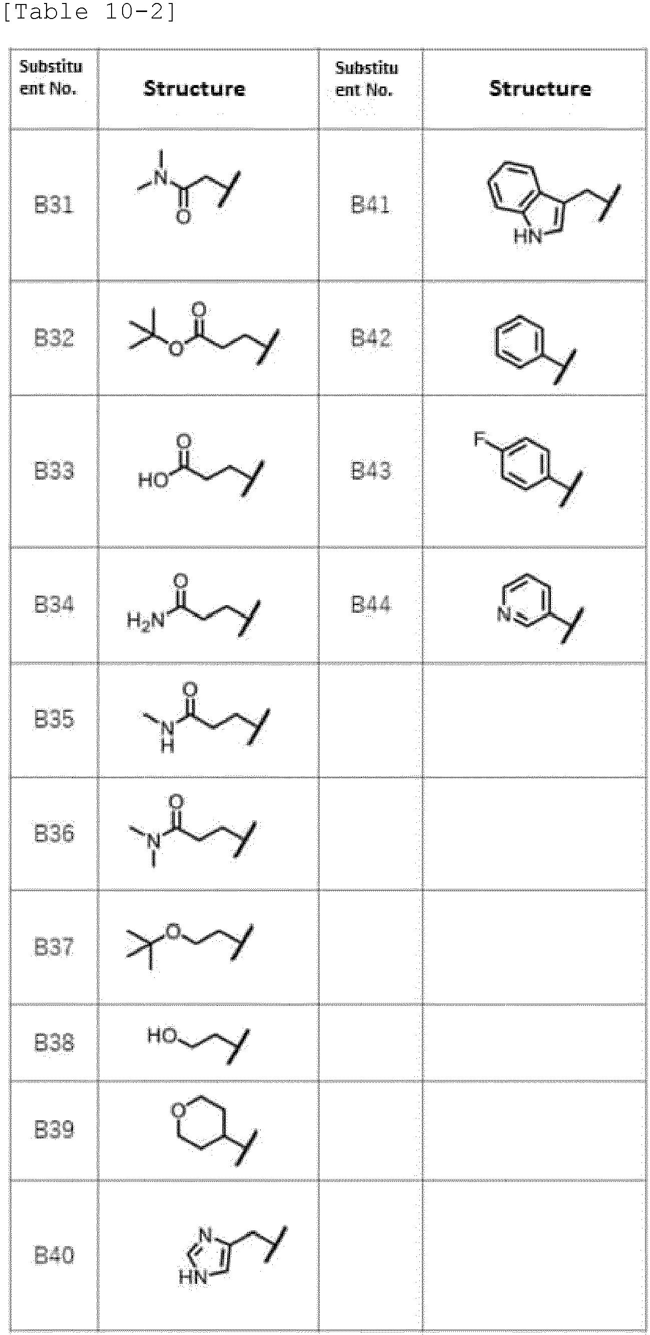

R₃ is a group selected from the following tables.

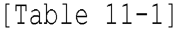

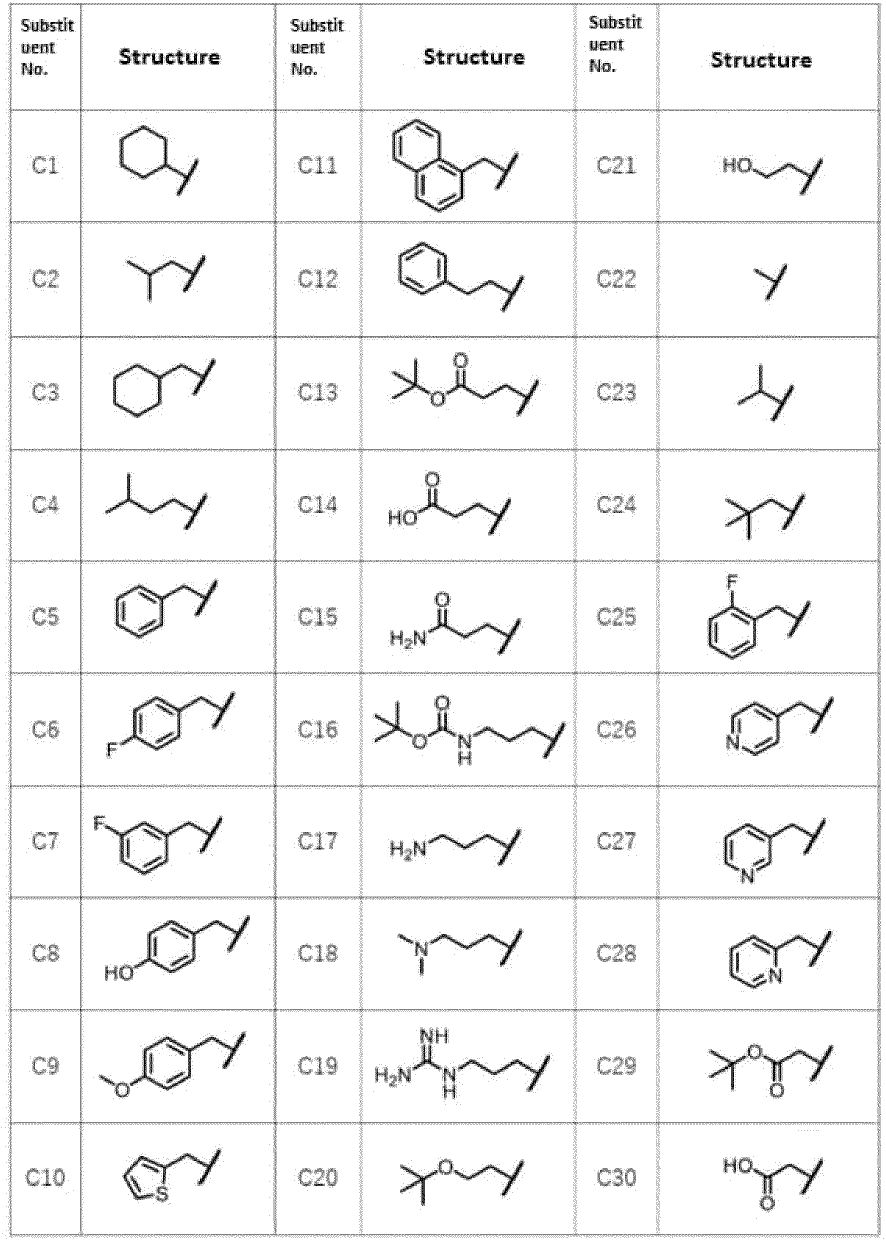

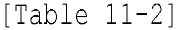

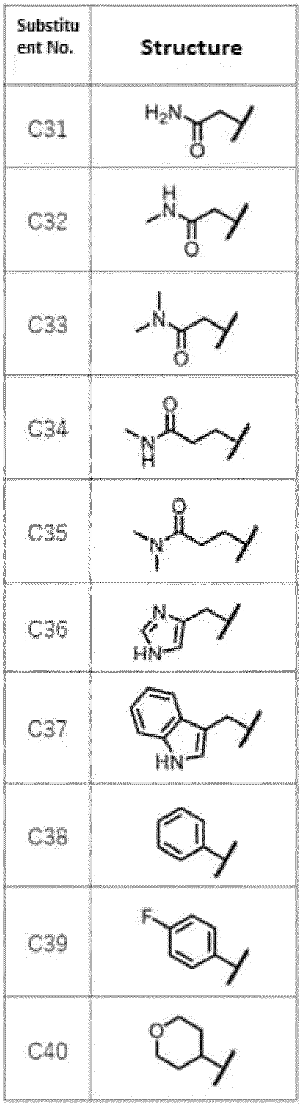

### (Example 9: Manufacture of peptidomimetic molecule)

### Synthesis of (3S*,3aS*,6R*,7R*,7aS*)-N-benzyl-7a-ethyl-7-methyl-1-(naphthalen-1-ylmethyl)-1,2,3,6,7,7a-hexahydro-3aH-3,6-methanopyrrolo[3,2-b]pyridine-3a-carboxamide

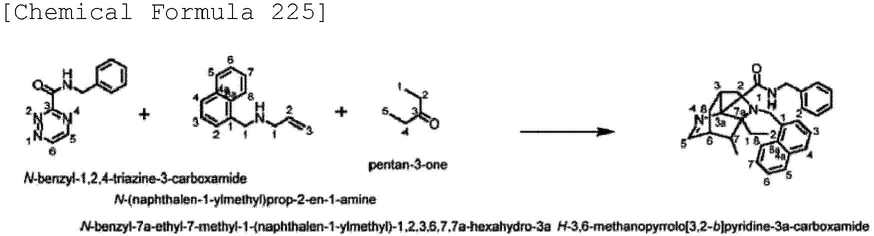

N-benzyl-1,2,4-triazine-3-carboxamide + N-(naphthalen-1-ylmethyl)prop-2-en-1-amine + pentan-3-one → N-benzyl-7a-ethyl-7-methyl-1-(naphthalen-1-ylmethyl)-1,2,3,6,7,7a-hexahydro-3aH-3,6-methanopyrrolo[3,2-b]pyridine-3a-carboxamide

4A molecular sieve (50 mg), toluene (100 µL) solution of pentan-3-one (9.6 mg, 0.11 mmol), and N-benzyl-1,2,4-triazine (6.5 mg, 0.03 mmol), and toluene (50 µL) were added to a toluene (150 µL) solution of N-(naphthalen-1-ylmethyl)prop-2-en-1-amine (18 mg, 0.09 mmol), and the mixture was heated for 2 days at 100°C. When the reaction mixture was analyzed by LCMS, a peak was found through the mass of a compound of interest ([M+1]+ = 452) at a retention time of 0.99 minutes. The reaction mixture can be concentrated and purified by using HPLC or (and) PTLC to obtain the title compound.

### Synthesis of (3S*,3aS*,6R*,7R*,7aS*)-N-benzyl-7a-ethyl-7-methyl-1-(naphthalen-1-ylmethyl)-5-oxooctahydro-3aH-3,6-methanopyrrolo[3,2-b]pyridine-3a-carboxamide

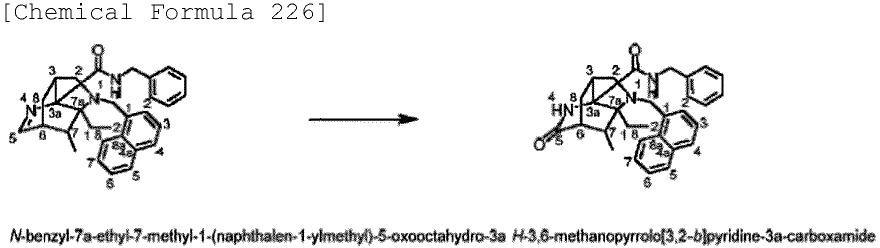

### N-benzyl-7a-ethyl-7-methyl-1-(naphthalen-1-ylmethyl)-5-oxooctahydro-3aH-3,6-methanopyrrolo[3,2-b]pyridine-3a-carboxamide

Previously synthesized (3S*,3aS*,6R*,7R*,7aS*)-N-benzyl-7a-ethyl-7-methyl-1-(naphthalen-1-ylmethyl)-1,2,3,6,7,7a-hexahydro-3aH-3,6-methanopyrrolo[3,2-b]pyridine-3a-carboxamide (4.5 mg,10 µmol) is dissolved in tetrahydrofuran (200 µL), and 2,3-dimethyl-2-butene (5.9 µL), trifluoroacetic acid (1.5 µL), aqueous 5M sodium dihydrogenphosphate solution (7.0 µL), and aqueous 5M sodium chlorite solution (3.0 µL) were added. The mixture is stirred for 2 hours at room temperature. The reaction mixture is added with ethyl acetate, washed with sodium bicarbonate water and saline, dried with anhydrous sodium sulfate, then filtered. Ethyl acetate is evaporated under reduced pressure from the filtrate, and the resulting compound is purified by using HPLC or PTLC, whereby the title compound can be obtained.

### (Example 10: Studying the application of peptidomimetic molecule)

In order to study the biological application of the compounds synthesized in the Examples described above, activity against rabies viruses was studied.

### Assay method for anti-rabies virus activity

Each tested compound prepared as a 10 mM stock in DMSO was first diluted to 100 µM or 40 µM with a 10% fetal bovine serum-supplemented Eagle's medium (hereinafter, medium), and further diluted with the medium so that the concentration would reach the final concentration of interest. 50 µl of the diluent was added dropwise to each well of a 96-well plate. Furthermore, 50 µl of a medium comprising 4 × 10² infection units of recombinant rabies virus 1088 strain (1088/GLuc) expressing Gaussia Luciferase (GLuc) and 4 × 10⁴ Neuro-2a cells was added to each well and shaken for 30 seconds in a multiple tandem microplate mixer NS-4P (AS ONE Corporation) and then cultured for 3 days at 37°C in the presence of 5% CO₂. After incubation, 25 µL of coelenterazine, which is a substrate of the luciferase, was added dropwise to each well of the plate, and the plate was immediately loaded into a luminescent plate reader LuMate (Awareness Technology) and shaken for 10 seconds. The relative light unit (RLU) was then measured.

The activity of Example 3 was as follows.

The following tables show the anti-rabies activity of the compound of formula IF or formula IB (wherein X is - N(R_{2A})-, R_{2A} is H, R₂ is R_{2B}, and R₄ is H) . The compounds are classified as A if IC₅₀ is 10 µM or less, and B if 10 µM or greater and 30 µM or less. Na indicates not applicable. In the following tables, compound number is represented by "IF-" and a number if the assayed compound is a compound of formula IF, and represented by "IB-" and a number if the assayed compound is a compound of formula IB.

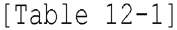

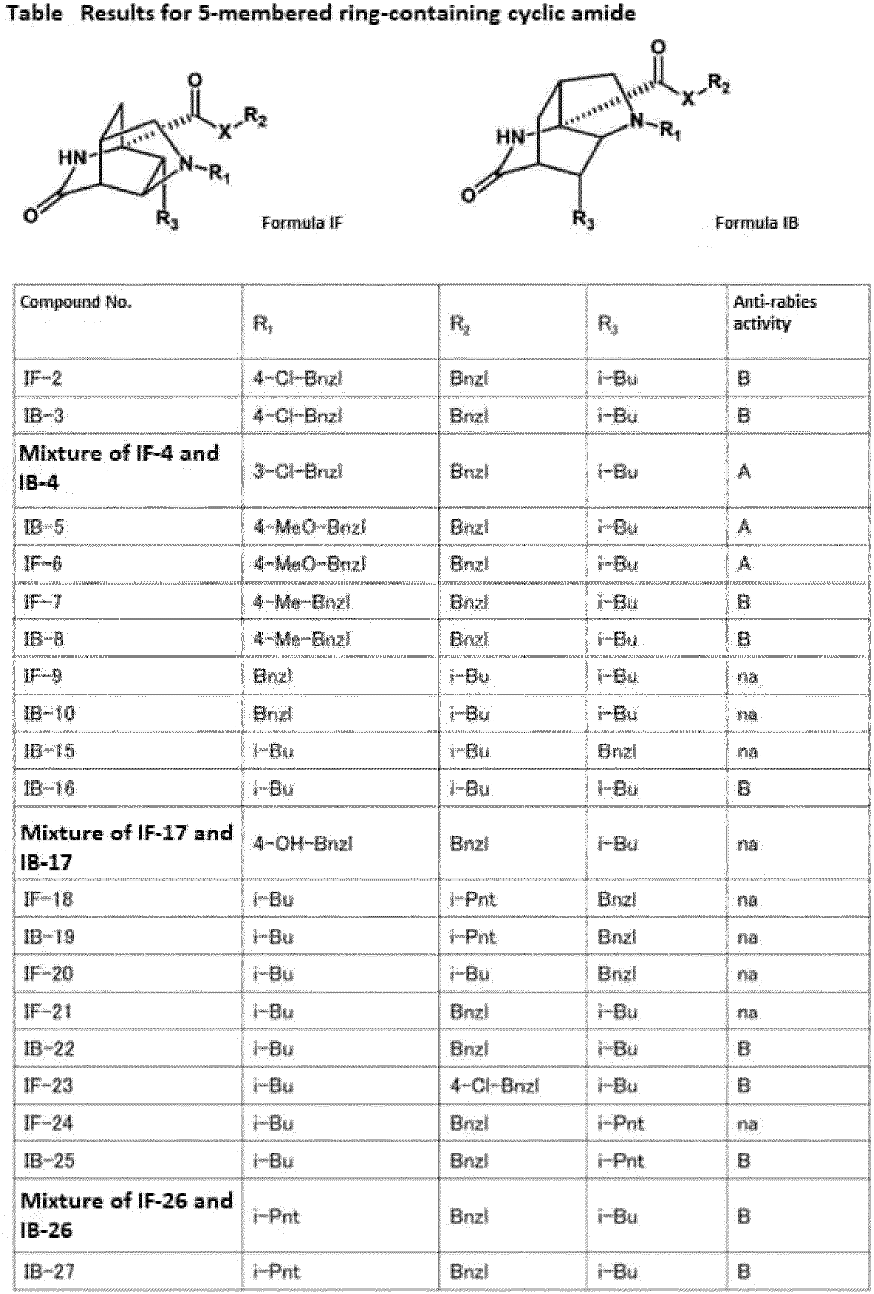

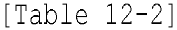

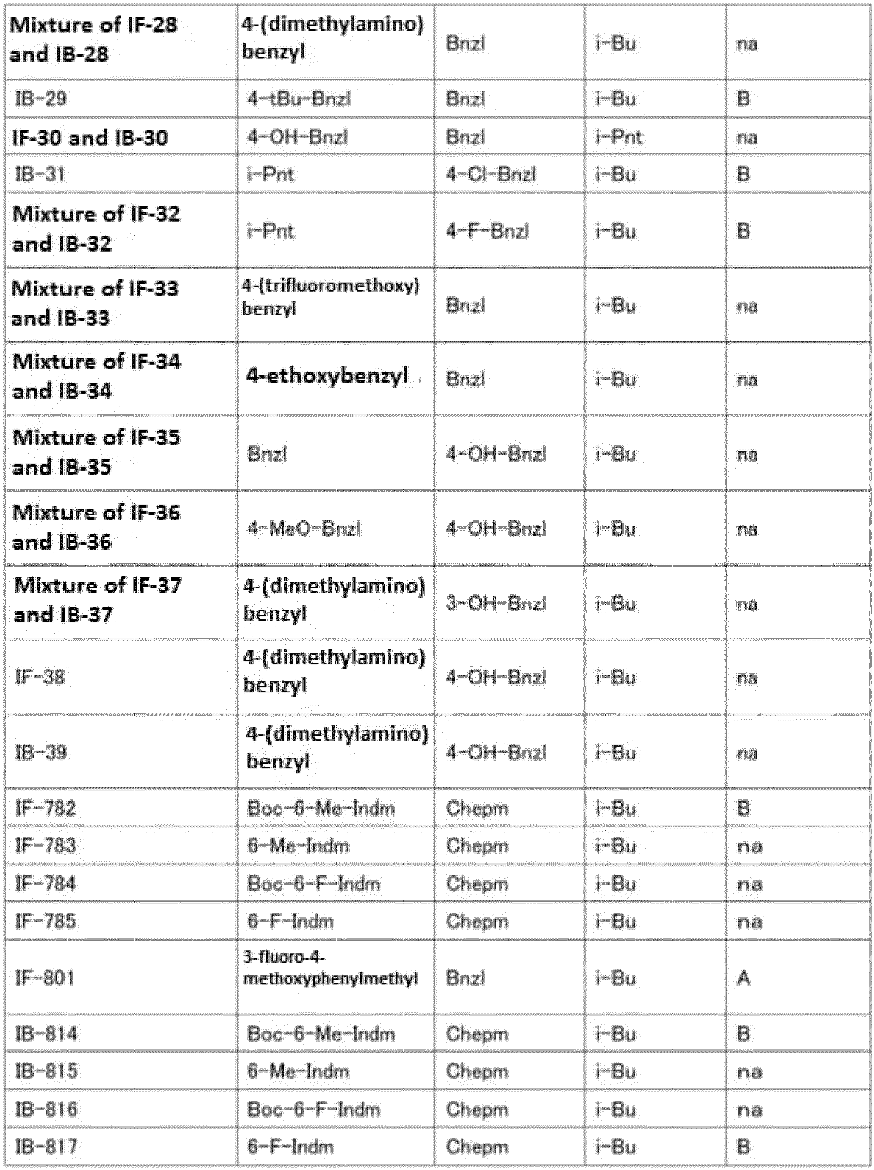

The activity of the compound obtained in Example 5 was as follows. In the following table, the compound is classified as A if IC₅₀ is 10 µM or less, and B if 10 µM or greater and 30 µM or less.

**[Table 13]**

| **Compound No.** | R₁ | R₂ | R3 | **Anti-rabies activity** |
|---|---|---|---|---|
| 12aab | Bnzl | Bnzl | i-Bu | A |

### (Example 11: Fitting an amino acid-like structure)

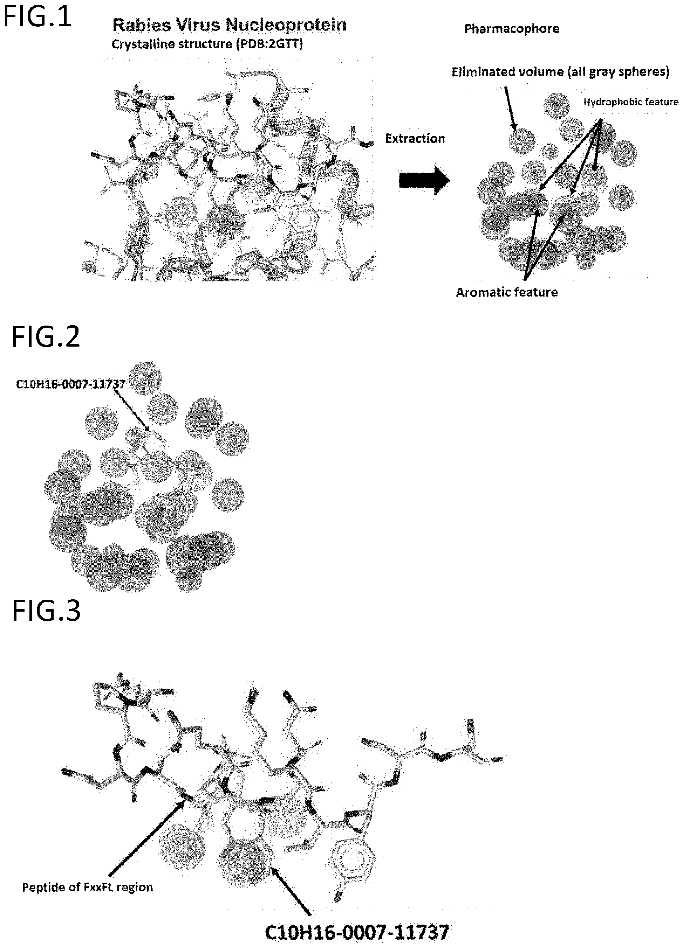

This Example mimicked a peptide of the FxxFL region located at the C-terminus of Rabies Virus Nucleoprotein. First, a pharmacophore of the FxxFL region was extracted by using LigandScout (see Figure 2).

To this pharmacophore, 12,447 peptidomimetic molecule frames shown in Example 2 were fitted with LigandScout. As a result of fitting, 2,649 frames satisfied the pharmacophore, and pharmacophore scores were calculated. The pharmacophore scores of the remaining 9798 frames that do not satisfy the pharmacophore were assumed to be 0.

In this Example, C10H16-0007-11737 (F form, R1 = benzyl, R2 = benzyl, R3 = isobutyl) was selected from the top 2500 pharmacophore scores. The pharmacophore score of C10H16-0007-11737 was 0.6 (no. 2068) (see Figure 3).

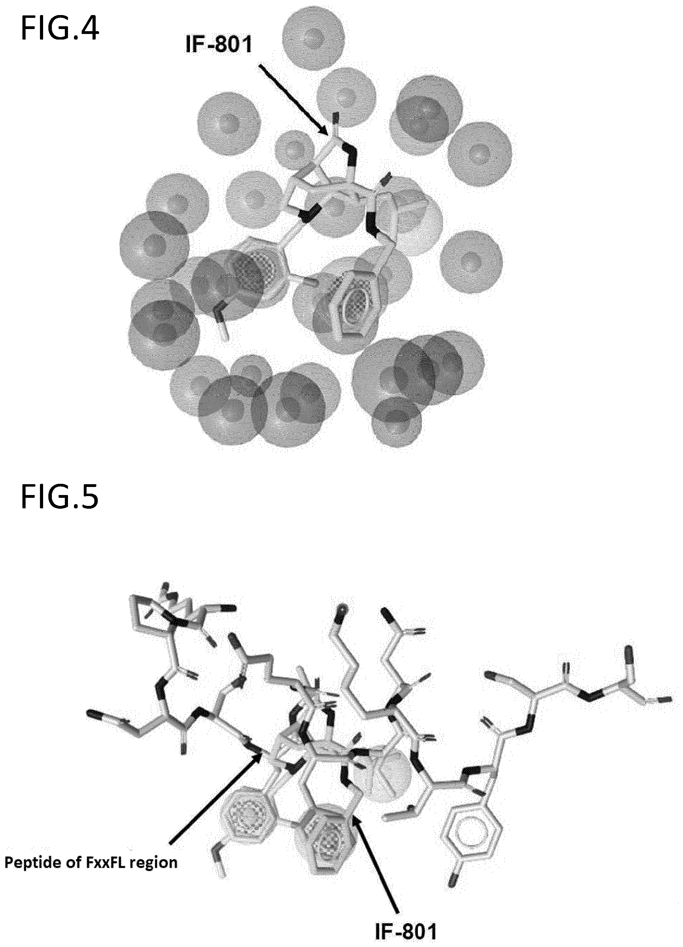

C10H16-0007-11737 superposed on the FxxFL region is shown in Figure 4.

### (Example 12)

This Example synthesized IF-801 based on the molecular frame C10H16-0007-11737 by using the same procedure as the Examples described above and fitted IF-801 to the pharmacophore. The same fitting as C10H16-0007-11737 was able to be achieved (Figure 5).

IF-801 superposed on the FxxFL region is shown in Figure 6.

### (Example 13: Comprehensive synthesis of other backbones)

Backbone synthesis can only be studied uniquely for the backbone by methods such as reverse synthesis analysis developed by Elias James Corey, etc. As shown in the fundamental textbooks described above, synthesis methods can construct a backbone by using reductive amination using substituted amine and substituted aldehyde, alkylamination using substituted amine and substituted halide, introduction of an allyl group and/or an alkyl group through a Diels-Alder reaction, amidation using substituted carboxylic acid and substituted amino group, substituted amidation using substituted ester and a substituted amino group, amidation using substituted active ester and a substituted amino group, an Ugi reaction using substituted imine, substituted carboxylic acid, and substituted isonitrile, a Horner-Emmons reaction, synthesis of unsaturated ester through aldol condensation, condensation reaction using the Woodward-Hoffmann rules, [2,3]-Wittig rearrangement, Pinnick oxidation, Click reaction using substituted alkyne and substituted azide, etc.

### [Note]

As disclosed above, the present disclosure is exemplified by the use of its preferred embodiments. However, it is understood that the scope of the present disclosure should be interpreted based solely on the Claims. The present application claims priority to Japanese Patent Application No. 2020 - 204334 in Japan. The entirety thereof is incorporated herein by reference. It is understood that any patent, any patent application, and any reference cited herein should be incorporated herein by reference in the same manner as the contents are specifically described herein.

### [Industrial Applicability]

The present disclosure provides design and production of a useful carbon backbone, design and production of a peptidomimetic molecule, and application and use based thereon. A technology that can be utilized in industries (pharmaceutical, etc.) based on such technologies is provided.

## Claims

1. A method of designing a compound backbone with a desired structure, comprising the steps of causing a computer to:
(1) generate a molecular formula of a compound comprising carbon;
(2) randomly generate molecules using an adjacency matrix to extract a compound falling under the molecular formula among the randomly generated molecules; and
(3) select a compound satisfying a predefined condition.

2. The method of claim 1, wherein the predefined structure is a cage-like carbon backbone.

3. The method of claim 1 or 2, wherein the predefined condition is to satisfy at least one of:
condition 1: all carbon atoms and bonds are within a 5- or 6-membered ring;
condition 2: there is no spiro ring;
condition 3: there are two or more atoms that are present in common in at least three ring structures within a molecule (ring structure under condition 1 is a 5- or 6-membered ring); and
condition 4: energy is 150 kcal/mol or less in an MMFF (Merck Molecular Force Field) when all are calculated as a single bond upon generation of a 3D structure.

4. The method of claim 3, wherein all of condition 1 to condition 4 are satisfied.

5. The method of any one of claims 1 to 4, wherein the molecular formula is one selected from the group consisting of C₁₀H₁₆, C₁₁H₁₆, C₁₁H₁₈, C₁₂H₁₈, C₁₂H₂₀, C₁₃H₂₀, and C₁₃H₂₂.

6. A method of generating a compound having a compound backbone with a desired structure, comprising the steps of:
(1) causing a computer to generate a molecular formula of a compound comprising carbon;
(2) causing a computer to randomly generate molecules using an adjacency matrix to extract a compound falling under the molecular formula among the randomly generated molecules;
(3) causing a computer to select a compound satisfying a predefined condition; and
(4) producing a compound having a backbone structure selected in (3).

7. The method of claim 6, wherein the compound comprises at least one type of bond selected from a single bond, a double bond, and a triple bond.

8. A method of designing a peptidomimetic molecule framework, comprising the steps of causing a computer to:
(A) provide structural data for a carbon moiety of a predefined carbon backbone;
(B) generate an attachment point (AP), which is a position where a side chain structure is given from the structural data for the carbon backbone;
(C) randomly select APs at M locations;
(D) optionally, generate a carbon backbone with N APs by repeating steps (A) to (C) N times, wherein N is a positive integer;
(E) optionally, comprehensively add the same or different amino acid-like structure to each AP at M locations to generate an amino acid-like structure-containing carbon structure;
(F) fit the amino acid-like structure-containing carbon structure to a pharmacophore obtained from a three-dimensional structure of a predefined peptide, and select a carbon structure that has fit as a peptidomimetic molecule framework.

9. The method of claim 8, wherein the carbon backbone is a cage-like carbon backbone.

10. The method of claim 8 or 9, wherein N is 1000 or greater in step (D).

11. The method of any one of claims 8 to 10, wherein M is an integer from 2 to 6.

12. The method of any one of claims 8 to 11, wherein the amino acid comprises at least one selected from the group consisting of leucine, phenylalanine, isoleucine, and tryptophan in step (E).

13. The method of any one of claims 8 to 12, wherein step (F) comprises causing the computer to select top K carbon backbones with a best fitting score of the pharmacophore.

14. The method of any one of claims 8 to 13, wherein the carbon backbone is a backbone generated by the method of any one of claims 1 to 5.

15. A method of generating a compound having a peptidomimetic molecule framework, comprising the steps of:
(A) providing to a computer structural data for a carbon moiety of a predefined carbon backbone;
(B) causing a computer to generate an attachment point (AP), which is a position where a side chain structure is given from the structural data for the carbon backbone;
(C) causing a computer to randomly select APs at M locations;
(D) optionally, causing a computer to generate a carbon backbone with N APs by repeating step (C) N times, wherein N is a positive integer;
(E) optionally, causing a computer to comprehensively add the same or different amino acid-like structure to each AP at M locations to generate a carbon backbone with an amino acid side chain structure;
(F) causing a computer to fit the carbon structure with the amino acid side chain structure to a pharmacophore obtained from a three-dimensional structure of a predefined peptide, and select a carbon structure that has fit as a peptidomimetic molecule framework; and
(G) producing a compound with the peptidomimetic molecule framework selected in (F).

16. The method of claim 15, wherein M is 3 or greater.

17. The method of claim 15 or 16, wherein the method comprises step (D), and N is 1000 or greater.

18. The method of any one of claims 15 to 17, wherein the compound comprises at least one type selected from a single bond, a double bond, and a triple bond.

19. A cyclic compound having a backbone satisfying, when calculated in terms of hydrocarbon:
condition 1: all carbon atoms and bonds are within a 5- or 6-membered ring;
condition 2: there is no spiro ring;
condition 3: there are two or more atoms that are present in common in at least three ring structures within a molecule (ring structure under condition 1 is a 5- or 6-membered ring); and
condition 4: energy is 150 kcal/mol or less in an MMFF upon generation of a 3D structure;
wherein the cyclic compound optionally has a heteroatom in place of a carbon atom within a backbone and optionally has one or more substituents.

20. A peptidomimetic compound having a backbone satisfying, when calculated in terms of hydrocarbon:
condition 1: all carbon atoms and bonds are within a 5- or 6-membered ring;
condition 2: there is no spiro ring;
condition 3: there are two or more atoms that are present in common in at least three ring structures within a molecule (ring structure under condition 1 is a 5- or 6-membered ring); and
condition 4: energy is 150 kcal/mol or less in an MMFF upon generation of a 3D structure;
and M substituents (wherein M is any integer from 1 to (number of atoms in a backbone moiety that can have a substituent)) corresponding to M amino acid-like structures (amino acid side chain or an equivalent thereof) from the backbone.

21. Following backbones:
